# EUROPEAN PATENT APPLICATION

(11) **EP 2 594 583 A1**
(43) Date of publication of application: **22.05.2013**
(21) Application number: 12189417.4
(22) Date of filing: 08.08.2008
(51) Int. Cl.: C07K 14/79, A61K 47/48, A61K 38/40, C12N 5/10

(54) **Transferrin variants and conugates**

(30) Priority: 08.08.2007 EP 07114012; 02.04.2008 EP 08153938
(62) Divisional of application: 08787067.1
(71) Applicant: Novozymes Biopharma DK A/S, 2880 Bagsvaerd (DK)
(72) Inventor: Sleep, Darrell, Nottingham, Nottinghamshire NG2 5DS (GB); Friis, Esben Peter, 2730 Herlev (DK); Hay, Joanna, Nottingham, Nottinghamshire NG2 6QW (GB); Finnis, Christopher John Arthur, Nottingham, Nottinghamshire NG7 1JB (GB)
(74) Representative: Williams, Rachel Clare

(57) **Abstract**

Based on the three-dimensional structure of transferrin, the inventors have designed variant polypeptides (muteins) which have one or more Cysteine residues with a free thiol group (hereinafter referred to as "thiotransferrin"). The variant polypeptide may be conjugated through the sulphur atom of the Cysteine residue to a bioactive compound.

## Description

### Reference to sequence listing

This application contains a Sequence Listing in computer readable form. The computer readable form is incorporated herein by reference.

### FIELD OF THE INVENTION

The present invention relates to conjugates of a polypeptide and at least one bioactive compound, to polypeptides for making the conjugates and to polynucleotides encoding them.

### BACKGROUND OF THE INVENTION

It is known to use conjugates of transferrin with bioactive compounds for receptor-mediated endocytosis to improve the transcellular delivery. Thus, N.J. Kavimandam et al., Bioconjugate Chem., 2006, 17, 1376-1384 is titled "Synthesis and Characterization of Insulin-Transferrin Conjugates". D. Shah et al., Journal of Pharmaceutical Sciences, Vol. 85, No. 12, December 1996, is titled "Transcellular Delivery of an Insulin-Transferrin Conjugate in Enterocyte-like Caco-2 Cells" and Fritzer et al., (1996) Biochem. Pharm., 51, 489-493 is titled "Cytodoxic Effects of a Doxorubicin-Transferrin Conjugate in Multidrug-Resistant KB Cells. US 20030221201 and 20040023334 describe fusion proteins comprising a transferrin protein fused to a therapeutic protein.

Human serum transferrin (HST) is known to be a single-chain polypeptide of 679 amino acid residues which contain 38 Cysteine residues linked in 19 disulfide bridges with the capacity to bind two ferric ions. The three-dimensional structure of human transferrin was published in J. Wally et al., Journal of Biological Chemistry, 281 (34), 24934-24944 (2006). According to the human transferrin crystal structure from Wally *et al.,* HST comprises an N-lobe consisting of amino acids 1-331, a C-lobe consisting of amino acids 339-679, and an interlobe linker consisting of amino acids 332-338.

When a transferrin protein loaded with iron encounters a transferrin receptor (TfR) on the surface of a cell, it binds to it and is consequently transported into the cell in a vesicle. The cell will acidify the vesicle, causing transferrin to release its iron ions. The receptor is then transported through the endocytic cycle back to the cell surface, ready for another round of iron uptake.

Cheng, Y., Cell (Cambridge, Mass.) v116, pp. 565-576 (2004) describes a model for the structure of the Human Transferrin Receptor-Transferrin Complex. The structure is found as 1 SUV in the Protein Data Bank.

J. Wally et al., Journal of Biological Chemistry, 281 (34), 24934-24944 (2006) describes a three-dimensional structure of iron-free human transferrin. The structure is found as 2HAV in the Protein Data Bank.

L. A. Lambert et al., Comparative Biochemistry and Physiology, Part B 142 (2005) 129-141 is titled "Evolution of the transferrin family: Conservation of residues associated with iron and anion binding".

The transferrins form a group of proteins with high sequence homology, including human serum transferrin (HST), lactoferrin, melanotransferrin and ovotransferrin. Native HST is known to contain two lobes (N- and C-Iobes) with 19 disulfide bridges, an O-glycosylation site at S32, and two N-glycosylation sites at N413 and N611.

Woodworth, R. C., et al. (1991), Biochemistry 30, 10824-9, discloses mutants of the N-terminal half-molecule of human serum transferrin including the mutant D63C. Muralidhara, B. K. and Hirose, M. (2000), Protein Sci 9, 1567-1575, discloses selective reduction of the isolated C-Iobe of ovotransferrin. J. Williams et al., Biochem. J. (1985) 228, 661-665 discloses selective reduction of a disulphide bridge in ovotransferrin or the C-terminal half-molecule. US 5986067 (Funk et al.) discloses a recombinant HST mutant which does not allow glycosylation.

### SUMMARY OF THE INVENTION

Based on a three-dimensional structure of a transferrin, the inventors have designed variant polypeptides (muteins) which have one or more Cysteine residues with a free thiol group (hereinafter referred to as "thiotransferrin"). The variant polypeptide may be conjugated through the sulphur atom of the Cysteine residue of the polypeptide to a bioactive compound.

Accordingly, the invention provides a polypeptide which has iron binding capacity and binds to a receptor.

It has an amino acid sequence which is at least 40 % or at least 60 % identical to residues 1-679 or 339-679 of SEO ID NO: 1, residues 1-691 of SEO ID NO:11 or residues 1-738 of SEQ ID NO:15 and comprises one or more Cysteine residues with a free thiol group.

The term thiotransferrin is used herein to describe a transferrin variant which comprises one or more unpaired cysteines. Similar the terms thiolactoferrin and thiomelanotransferrin are used to describe variants of lactoferrin and melanotransferin respectively, which comprises one or more unpaired Cysteine residues with free thio groups.

It may be created by insertion of a cysteine residue (the amino acid chain length is increased), substitution of two or more adjacent residues with a cysteine (the amino acid chain length is decreased) or substitution of an amino acid residue with a cysteine (the amino acid chain length is unchanged), deletion of a cysteine residue or combinations of the above.

In another aspect the invention relates to conjugates comprising at least one bioactive compound and a polypeptide of the invention.

### BRIEF DESCRIPTION OF DRAWINGS

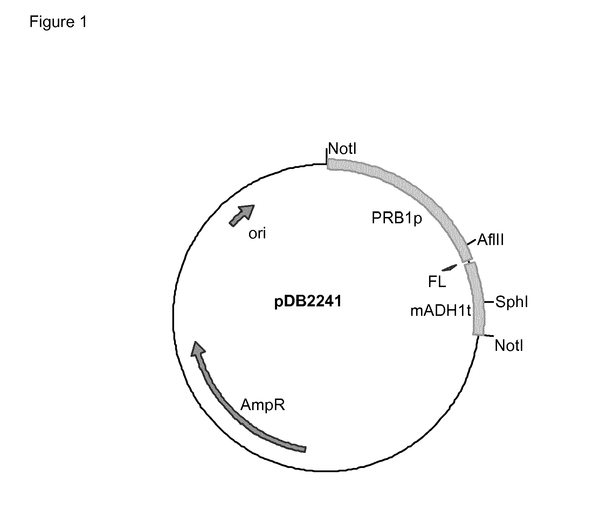

Figure 1, shows the structure of plamid pDB2241
Figure 2 shows the structure of plasmid pDB3237
Figure 3 show the structure of plasmid pDB3191

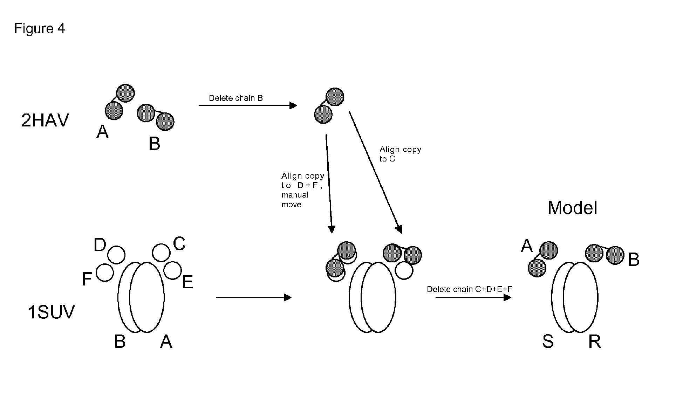

Figure 4 shows the building of a three-dimensional transferrin model.
Figure 5 shows data for residues in the two Tf chains in a 3D model. Details are given in the examples.
Figures 6-9 show alignments of various transferrin family proteins with HST (SEQ ID NO: 1), identified as NP_001054.
Figure 10 shows the map of 3.259kb *Not*I expression cassette from pDB3191 labeled with the positions of selected residues for modification and restriction endonuclease site to facilitate cloning
Figure 11 shows the structure of plasmid pDB3806.
Figure 12 shows the structure of plasmid pDB3809
Figure13 shows the structure of Lactoferrin subcloning plasmids pDB3815 and pDB3816
Figure 14 shows the structure of thiolactoferrin subcloning plasmids pDB3817
Figure 15 shows the structure of Lactoferrin expression plasmids pDB3818 and pDB3819
Figure 16 shows the structure of Thiolactoferrin expression plasmid pDB3820

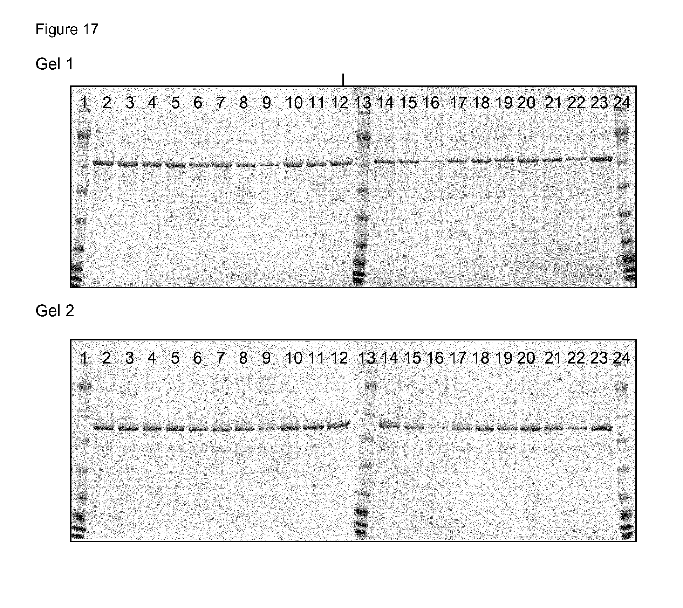

Figure 17 shows SDS-PAGE analysis of recombinant transferrin (S415A, T613A) compared to secretion recombinant 'thiotransferrin' from S. cerevisiae strain Strain 1 containing recombinant transferrin (S415A, T613A) expression plasmid pDB3237, or the appropriate recombinant thiotransferrin expression plasmid 10mL BMMD shake flasks were inoculated with 100µL cryopreserved yeast stock and incubated for 5-days at 30°C. Gel 1 corresponds to 20µL supernatant analysed on non-reducing SDS-PAGE (4-12% NuPAGE®, MOPS buffer, Invitrogen) with GelCode® Blue reagent (Pierce). Gel 2 corresponds to 20µL supernatant analysed on reducing SDS-PAGE (4-12% NuPAGE®, MOPS buffer, Invitrogen) with GelCode@ Blue reagent (Pierce).
   In Gels1 and 2 of figure 17, the lanes correspond to the following samples: 1, =10µL SeeBlue Plus Markers (Invitrogen); 2 =20µL Strain 1 [pDB3237], 3 =20µL Strain 1 [pDB3766] 4 =20µL Strain 1 [pDB3767], 5 =20µL Strain 1 [pDB3778], 6 =20µL Strain 1 [pDB3769], 7 =20µL Strain 1 [pDB3789], 8 =20µL Strain 1 [pDB3770], 9 =20µL Strain 1 [pDB3771], 10 =20µL Strain 1 [pDB3779], 11 =20µL Strain 1 [pDB3757], 12 =20µL Strain 1 [pDB3761], 13 =10µL SeeBlue Plus Markers (Invitrogen), 14 =20µL Strain 1 [pDB3773], 15 =20µL Strain 1 [pDB3763], 16 =20µL Strain 1 [pDB3760],17 =20µL Strain 1 [pDB3745], 18 =20µL Strain 1 [pDB3775], 19 =20µL Strain 1 [pDB3758], 20 =20µL Strain 1 [pDB3777], 21 =20µL Strain 1 [pDB3765], 22 =20µL Strain 1 [pDB3759], 23 =20µL Strain 1 [pDB3237], 24 =10µL SeeBlue Plus Markers (Invitrogen).

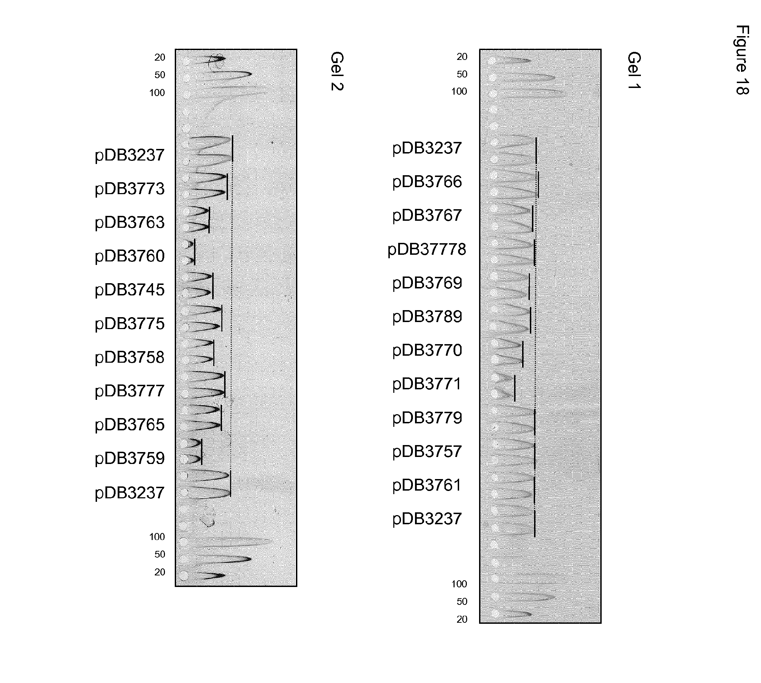

Figure 18 shows Rocket immunoelectrophoresis analysis of thiotransferrin variant secretion from a proprietary *S*. *cerevisiae* strains containing thiotransferrin variant expression plasmids compared to transferrin (S415A, T613A) secretion from *S. cerevisiae* Strain 1 containing transferrin (S415A, T613A) expression plasmid pDB3237. 10mL BMMD shake flasks were inoculated with 100µL cryopreserved yeast stock and incubated for 5-days at 30°C. 4µL culture supernatant was loaded in duplicate per well of a rocket immunoelectrophoresis gel (30µL goat anti-Tf / 50mL agarose). Recombinant human transferrin (S415A, T613A) (Deltaferrin^{™}) standards concentrations were loaded at 20-100µg/mL. Precipin was stained with Coomassie blue. Gel 1 corresponds to expression levels for N-Iobe and interlobe transferrin variant compared with expression from Strain 1 [pDB3237] expressing recombinant human transferrin (S415A, T613A). Gel 2 corresponds to expression levels for C-Iobe transferrin variants compared with expression from Strain 1 [pDB3237] expressing recombinant human transferrin (S415A, T613A).

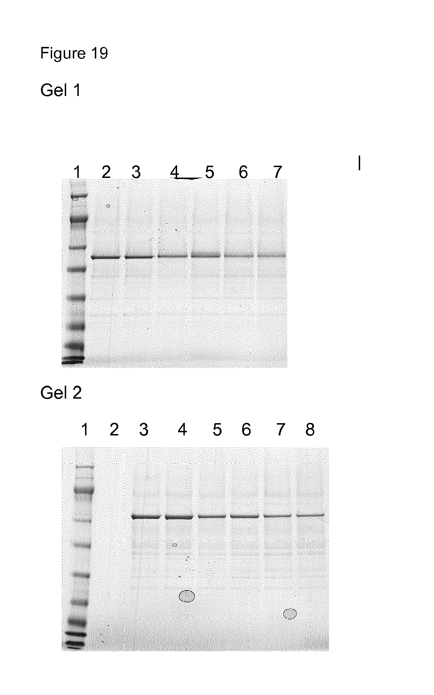

Figure 19 shows SDS-PAGE analysis of recombinant thiotransferrin (S28C, S415C, T613A) from S. cerevisiae Strain 1 [pDB3809] compared to secretion of recombinant thiotransferrin (S28C, S415A, T613A), recombinant thiotransferrin (S415C, T613A) and transferrin (S415A, T613A) recombinant 'thiotransferrin from S. cerevisiae Strain 1 [pDB3767], Strain 1 [pDB3773], Strain 1 [pDB3237]. 10mL BMMD shake flasks were inoculated with 100µL cryopreserved yeast stock and incubated for 5-days at 30°C. Gel 1 corresponds to 20µL supernatant analysed on non-reducing SDS-PAGE (4-12% NuPAGE^{®}, MOPS buffer, Invitrogen) with GelCode^{®} Blue reagent (Pierce). In Gel1 of figure 19, the lanes correspond to the following samples: 1, =10µL SeeBlue Plus Markers (Invitrogen); 2 =20µL Strain 1 [pDB3237] , 3 =20µL Strain 1 [pDB3237] 4 =20µL Strain 1 [pDB3767] 5 =20µL Strain 1 [pDB3773], 6 =20µL Strain 1 [pDB3809], 7 =20µL Strain 1 [pDB3809], In Gel 2 of figure 19, the lanes correspond to the following samples: 1, =10µL SeeBlue Plus Markers (Invitrogen); 2 =no sample, 3 =20µL Strain 1 [pDB3237] , 4 =20µL Strain 1 [pDB3237] 5 =20µL Strain 1 [pDB3767] 6 =20µL Strain 1 [pDB3773], 7 =20µL Strain 1 [pDB3809], 8 =20µL Strain 1 [pDB3809]

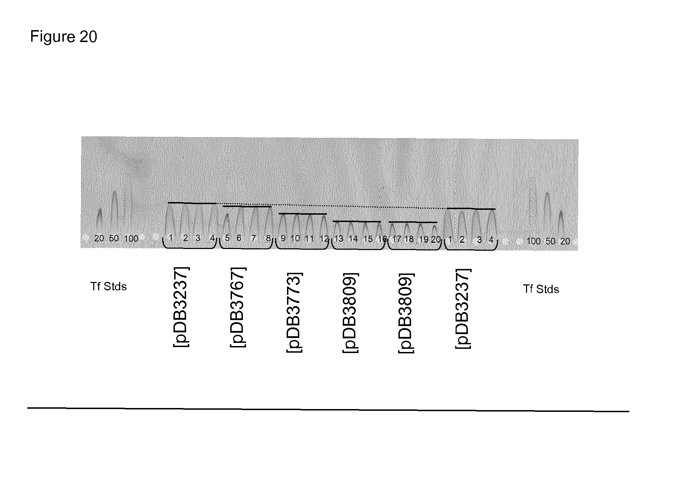

Figure 20 shows Rocket immunoelectrophoresis analysis of recombinant thiotransferrin (S28C, S415C, T613A) from proprietary *S*. *cerevisiae* Strain 1 [pDB3809] compared to secretion of recombinant thiotransferrin (S28C, S415A, T613A), recombinant thiotransferrin (S415C, T613A) and transferrin (S415A, T613A) recombinant 'thiotransferrin from proprietary *S. cerevisiae* Strain 1 [pDB3767], Strain 1 [pDB3773], Strain 1 [pDB3237]. 10mL BMMD shake flasks were inoculated with 100µL cryopreserved yeast stock and incubated for 5-days at 30°C. 4µL culture supernatant was loaded in duplicate per well of a rocket immunoelectrophoresis gel (30µL goat anti-Tf / 50mL agarose). Recombinant human transferrin (S415A, T613A) (Deltaferrin^{™}) standards concentrations were loaded at 20-100µg.mL⁻¹. Precipin was stained with Coomassie blue.

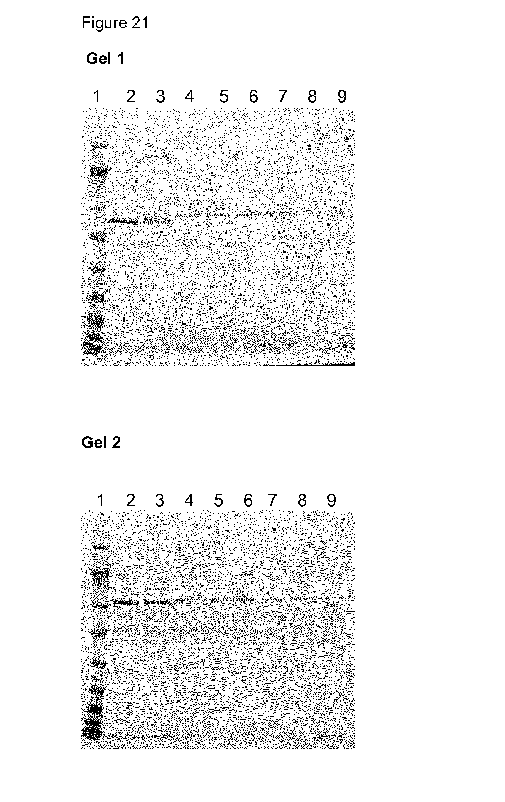

Figure 21 shows SDS-PAGE analysis of recombinant lactoferrin (T139A, T480A, S625A) from S. cerevisiae Strain 1 [pDB3818] recombinant lactoferrin (T139A, T480A, S625A) from S. cerevisiae Strain 1 [pDB3819] and recombinant thiolactoferrin (T139A, S421 C, T480A, S625A) from S. cerevisiae Strain 1 [pDB3820] compared to recombinant transferrin (S415A, T613A) from S. cerevisiae Strain 1 [pDB3237] and recombinant thiotransferrin (S415C) from S. cerevisiae Strain 1 [pDB3773]. 10mL BMMD shake flasks were inoculated with 100µL cryopreserved yeast stock and incubated for 5-days at 30°C. Gel 1 corresponds to 20µL supernatant analysed on non-reducing SDS-PAGE (4-12% NuPAGE^{®}, MOPS buffer, Invitrogen) with GelCode^{®} Blue reagent (Pierce). In both gels of Figure 21, the lanes correspond to the following samples: 1, =10µL SeeBlue Plus Markers (Invitrogen), 2 = 20µL Strain 1 [pDB3237] , 3 =20µL Strain 1 [pDB3773] 4= 20µL Strain 1 [pDB3818] 5 =20µL Strain 1 [pDB3818] 6 =20µL Strain 1 [pDB3819] , 7 =20µL Strain 1 [pDB3819], 8 =20µL Strain 1 [pDB3820] and 9 =20µL Strain 1 [pDB3820].

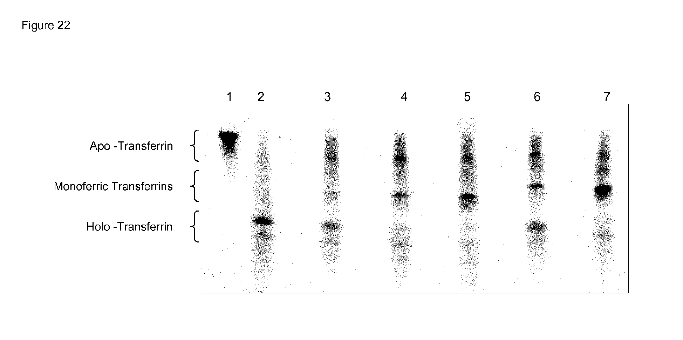

Figure 22 shows analytical TBE-urea gel analysis of purified recombinant transferrin (S415A, T613A) compared to purified recombinant thiotransferrin (S28C, S415A, T613A), purified thiotransferrin (S32C, S415A, T613A), purified thiotransferrin (A215C, S415A, T613A), purified thiotransferrin (S415C, T613A), and purified thiotransferrin (S4125A, N553C, T613A). Samples were prepared according to the protocol described in the following example. 5 µg samples were separated on 6% TBE Urea PAGE (Invitrogen) and stained with Coomassie G250 (Pierce).
   Lanes 1-2 shows Strain 1 [pDB3237] samples; Lane 3 shows Strain 1 [pDB3767], Lane 4 shows Strain 1 [pDB3779], Lane 5 shows Strain 1 [pDB3758], Lane 6 shows Strain 1 [pDB3773 and Lane 7 shows Strain 1 [pDB3778]
   Lanes 1 shows an iron-free preparation of recombinant transferrin (S415A, T613A) mutant; Lanes 2 shows iron-loaded preparation of recombinant transferrin (S415A, T613A) mutant, Lane 3 shows iron-loaded preparation of recombinant thiotransferrin (S28C, S415A, T613A) mutant, Lane 3 shows iron-loaded preparation of recombinant thiotransferrin (S415A, N553C, T613A) mutant, Lane 3 shows iron-loaded preparation of recombinant thiotransferrin (S415C, T613A) mutant, Lane 3 shows iron-loaded preparation of recombinant thiotransferrin (S32C, S415A, T613A) mutant.

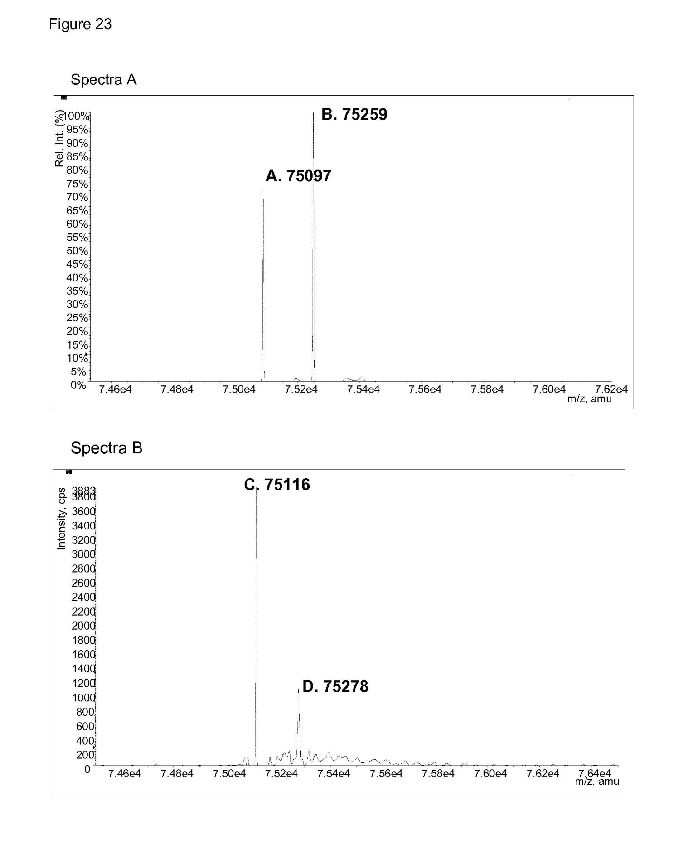

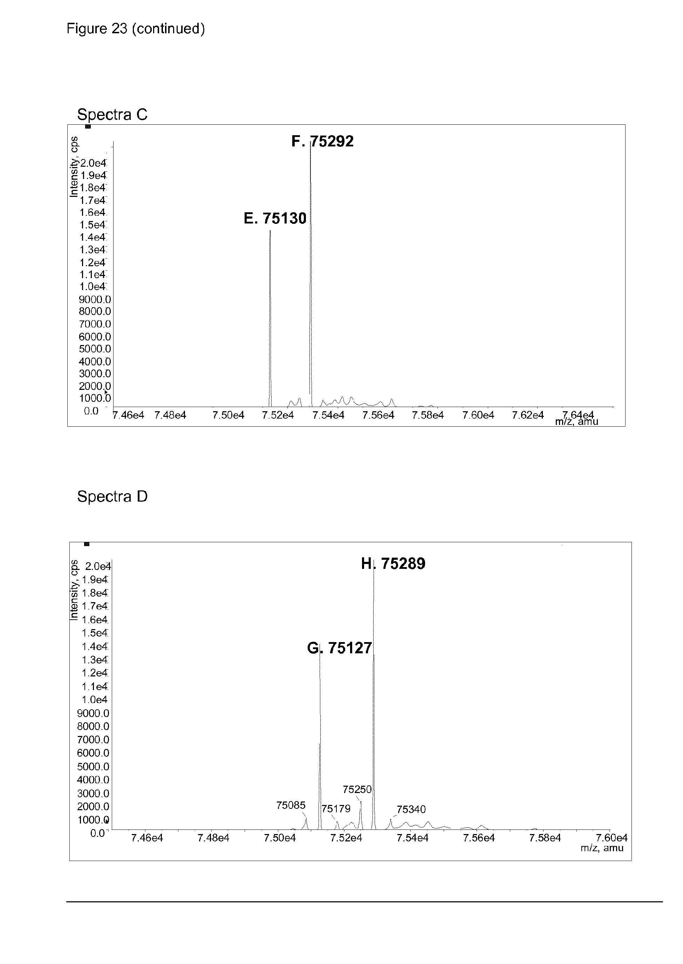

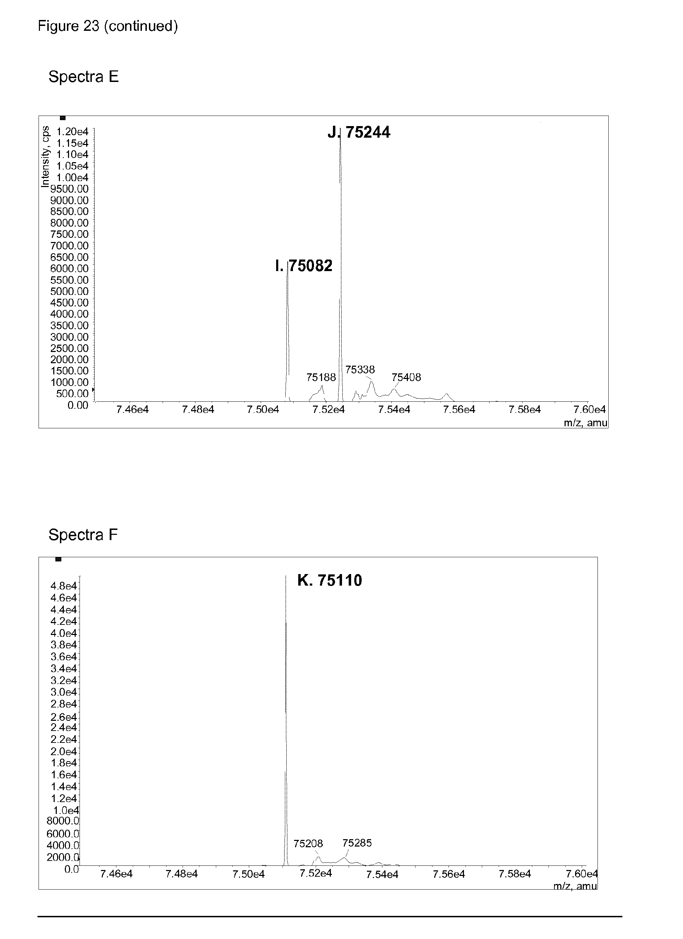

Figure 23 shows deconvolved mass spectra from analysis of thiotransferrin (S28C, S415A, T613A), thiotransferrin (A215C, S415A, T613A), thiotransferrin (S415C, T613A), thiotransferrin (S415A, N553C, T613A) and thiotransferrin (S32C, S415A, T613A) variants compared to transferrin (S415A, T613A) using ESI-TOF mass spectrometry. Spectrum A shows the mass spectrum of transferrin (S415A, T613A) purified from high cell density fermentation of YBX7 [pDB3237]. Peak identification A) native molecule (theoretical mass 75098Da), B) native molecule +1 hexose (theoretical mass 75259Da). Spectrum B shows the mass spectrum of thiotransferrin (S28C, S415A, T613A) variant from high cell density fermentation of Strain 1 [pDB3767] purified by the first chromatographic step. Peak identification C) native molecule (theoretical mass 75114Da), D) native molecule +1 hexose (theoretical mass 75274Da). Spectrum C shows the mass spectrum of thiotransferrin (A215C, S415A, T613A) variant from high cell density fermentation of Strain 1 [pDB3779] purified by the first chromatographic step. Peak identification E) native molecule (theoretical mass 75130Da), F) native molecule +1 hexose (theoretical mass 75292Da). Spectrum D shows the mass spectrum of thiotransferrin (S415C, T613A) variant from high cell density fermentation of Strain 1 [pDB3773] purified by the first chromatographic step. Peak identification G) native molecule (theoretical mass 75130Da), H) native molecule +1 hexose (theoretical mass 75292Da). Spectrum E shows the mass spectrum of thiotransferrin (S415A, N553C, T613A) variant from high cell density fermentation of Strain 1 [pDB3758] purified by the first chromatographic step. Peak identification I) native molecule (theoretical mass 75087Da), J) native molecule +1 hexose (theoretical mass 75249Da). Spectrum F shows the mass spectrum of thiotransferrin (S32C, S415A, T613A) variant from high cell density fermentation of Strain 1 [pDB3778] purified by the first chromatographic step. Peak identification K) native molecule (theoretical mass 75114Da).

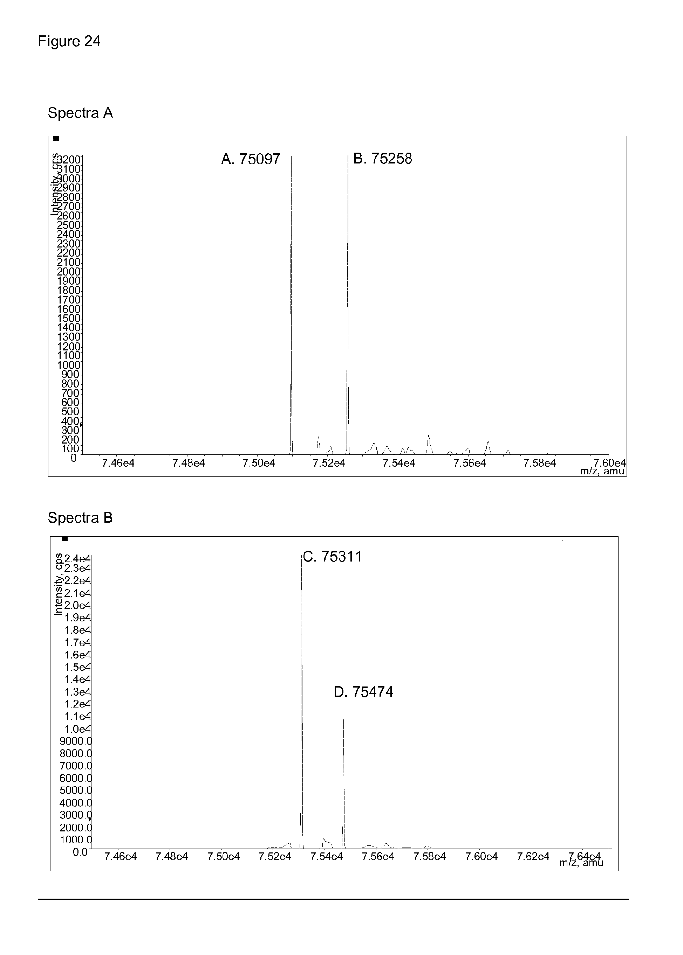

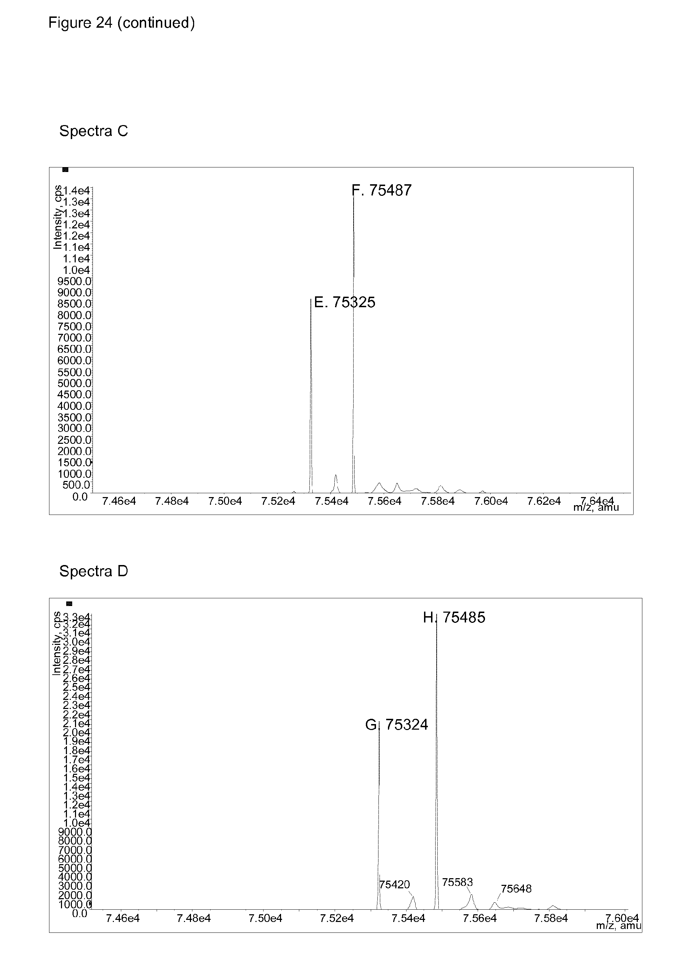

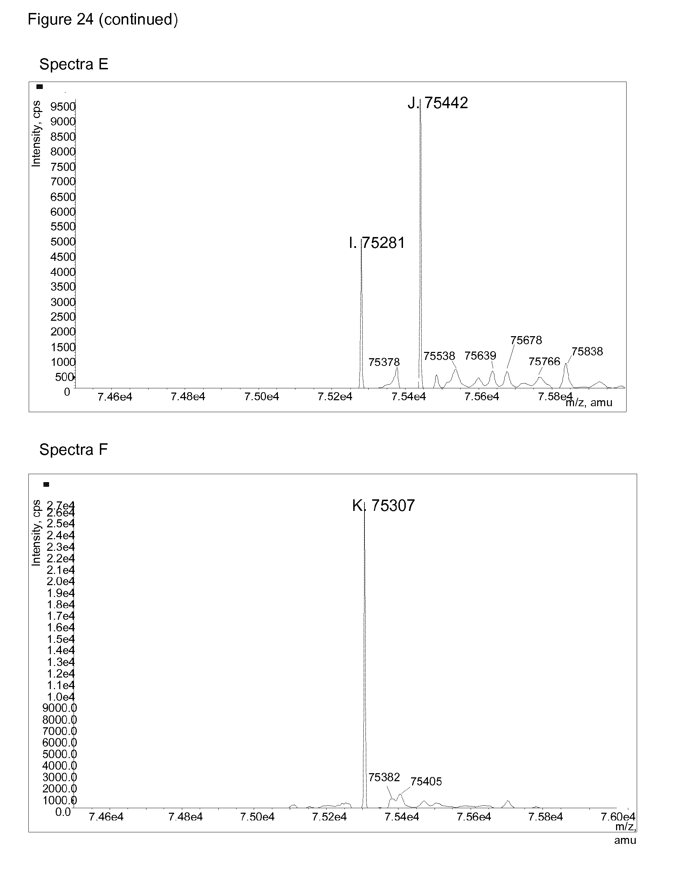

Figure 24 shows deconvolved mass spectra from analysis of thiotransferrin (S28C, S415A, T613A), thiotransferrin (A215C, S415A, T613A), thiotransferrin (S415C, T613A), thiotransferrin (N553C, S415A, T613A) and thiotransferrin (S32C, S415A, T613A) variants treated with DTNB compared to transferrin (S415A, T613A) treated with DTNB using ESI-TOF mass spectrometry. Spectrum A shows the mass spectrum of transferrin (S415A, T613A) purified from high cell density fermentation of YBX7 [pDB3237]. Peak identification A) native molecule (theoretical mass 75098Da), B) native molecule +1 hexose (theoretical mass 75259Da). Spectrum B shows the mass spectrum of thiotransferrin (S28C, S415A, T613A) variant from high cell density fermentation of Strain 1 [pDB3767] purified by the first chromatographic step and treated with DTNB. Peak identification C) native molecule +NTB (theoretical mass 75311Da), D) native molecule +1 hexose +NTB (theoretical mass 75473Da). Spectrum C shows the mass spectrum of thiotransferrin (A215C, S415A, T613A) variant from high cell density fermentation of Strain 1 [pDB3779] purified by the first chromatographic step and treated with DTNB. Peak identification E) native molecule +NTB (theoretical mass 75327Da), F) native molecule +1 hexose +NTB (theoretical mass 75489Da). Spectrum D shows the mass spectrum of thiotransferrin (S415C, T613A) variant from high cell density fermentation of Strain 1 [pDB3773] purified by the first chromatographic step and treated with DTNB. Peak identification G) native molecule +NTB (theoretical mass 75327Da), H) native molecule +1 hexose +NTB (theoretical mass 75489Da). Spectrum E shows the mass spectrum of thiotransferrin (N553C, S415A, T613A) variant from high cell density fermentation of Strain 1 [pDB3758] purified by the first chromatographic step and treated with DTNB. Peak identification I) native molecule +NTB (theoretical mass 75284Da), J) native molecule +1 hexose +NTB (theoretical mass 75446Da). Spectrum F shows the mass spectrum of thiotransferrin (S32C, S415A, T613A) variant from high cell density fermentation of Strain 1 [pDB3778] purified by the first chromatographic step and treated with DTNB. Peak identification K) native molecule +NTB (theoretical mass 75311Da).

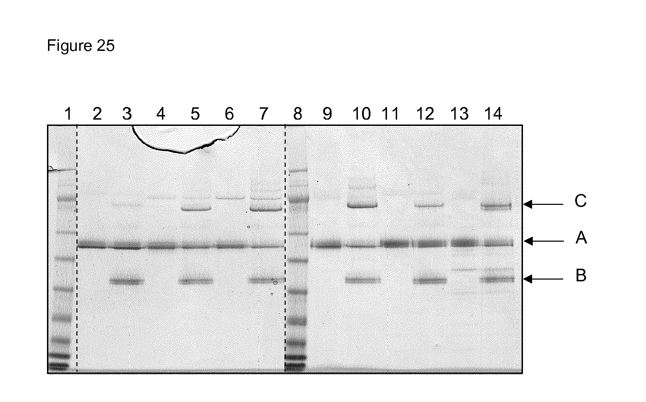

Figure 25. shows SDS-PAGE analysis of thiotransferrin variants compared to thiotransferrin variants conjugated to horse radish peroxidase. Proteins purified by the first chromatographic step were treated with a 4 fold molar excess of EZ-Link Maleimide Activated Horseradish Peroxidase (Pierce). Gel 1 corresponds to 20µL sample analysed on non-reducing SDS-PAGE (4-12% NuPAGE®, MOPS buffer, Invitrogen) with GeICode^{®} Blue reagent (Pierce). Gel 2 corresponds to 20µL supernatant analysed on reducing SDS-PAGE (4-12% NuPAGE®, MOPS buffer, Invitrogen) with GelCode® Blue reagent (Pierce). Lane 1, =10µL SeeBlue Plus Markers (Invitrogen); 2 =Transferrin (S415A, T613A), 3 =Transferrin (S415A, T613A) + EZ-Link Maleimide Activated Horseradish Peroxidase 4 =Thiotransferrin (S28C, S415A, T613A), 5 =Thiotransferrin (S28C, S415A, T613A) + EZ-Link Maleimide Activated Horseradish Peroxidase, 6 =Thiotransferrin (S32C, S415A, T613A), 7= Thiotransferrin (S32C, S415A, T613A) + EZ-Link Maleimide Activated Horseradish Peroxidase, 8= 10µL SeeBlue Plus Markers (Invitrogen), 9= Thiotransferrin (A215C, S415A, T613A), 10= Thiotransferrin (A215C, S415A, T613A) + EZ-Link Maleimide Activated Horseradish Peroxidase, 11= Thiotransferrin (S415C, T613A), 12= Thiotransferrin (S415C, T613A) + EZ-Link Maleimide Activated Horseradish Peroxidase, 13= Thiotransferrin (N553C, S415A, T613A), 14= Thiotransferrin (N553C, S415A, T613A) + EZ-Link Maleimide Activated Horseradish Peroxidase.

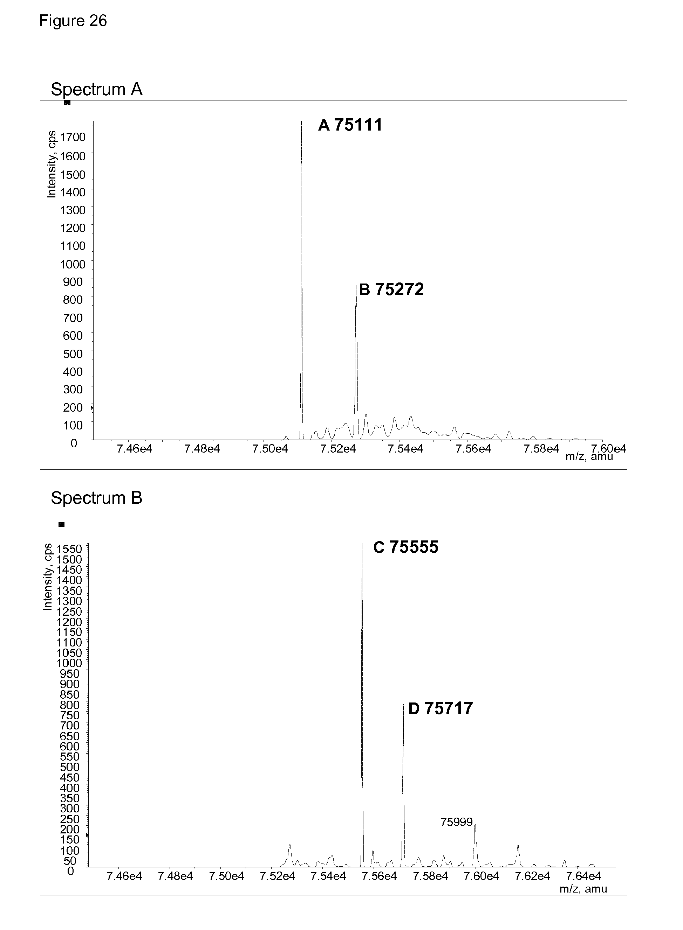

Figure 26 shows deconvolved mass spectra from analysis of thiotransferrin (S28C, S415A, T613A), compared to fluorescein conjugated thiotransferrin (S28C, S415A, T613A) using ESITOF mass spectrometry. Spectrum A shows the mass spectrum of thiotransferrin (S28C, S415A, T613A) variant. Peak identification A) native molecule (theoretical mass 75114Da), B) native molecule +1 hexose (theoretical mass 75272Da). Spectrum B shows the mass spectrum of fluorescein conjugated thiotransferrin (S28C, S415A, T613A) variant. Peak identification C) native molecule + fluorescein (theoretical mass 75541 Da), D) native molecule + fluorescein +1 hexose (theoretical mass 75701 Da).

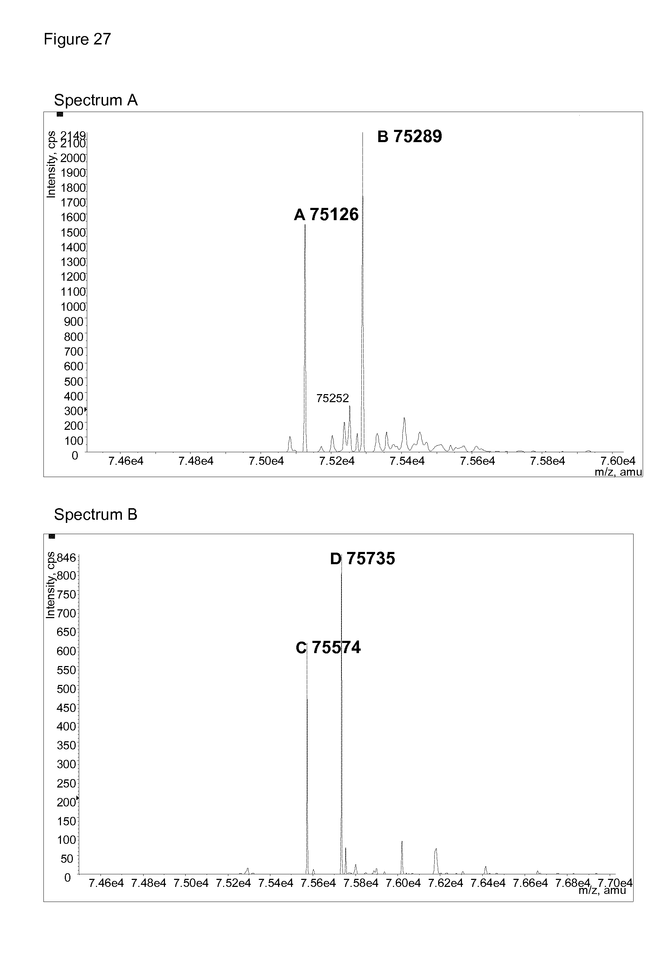

Figure 27 shows deconvolved mass spectra from analysis of thiotransferrin (S415C, T613A), compared to fluorescein conjugated thiotransferrin (S415C, T613A) using ESI-TOF mass spectrometry. Spectrum A shows the mass spectrum of thiotransferrin (S415C, T613A) variant. Peak identification A) native molecule (theoretical mass 75130Da), B) native molecule +1 hexose (theoretical mass 75292Da). Spectrum B shows the mass spectrum of fluorescein conjugated thiotransferrin (S415C, T613A) variant. Peak identification C) native molecule + fluorescein (theoretical mass 75553Da), D) native molecule + fluorescein +1 hexose (theoretical mass 75716Da).

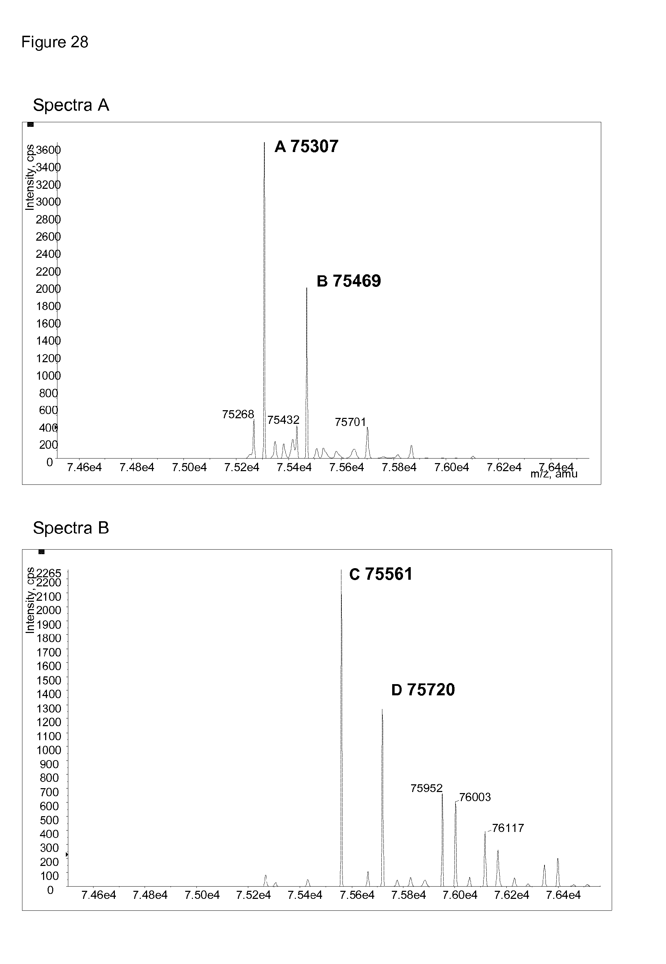

Figure 28 shows deconvolved mass spectra from analysis of thiotransferrin (S28C, S415A, T613A) treated with DTNB, compared to fluorescein conjugated thiotransferrin (S28C, S415A, T613A) treated with DTNB using ESI-TOF mass spectrometry. Spectrum A shows the mass spectrum of thiotransferrin (S28C, S415A, T613A) variant treated with DTNB. Peak identification A) native molecule +NTB (theoretical mass 75311Da), B) native molecule +1 hexose +NTB (theoretical mass 75473Da). Spectrum B shows the mass spectrum of fluorescein conjugated thiotransferrin (S28C, S415A, T613A) variant treated with DTNB. Peak identification C) native molecule + fluorescein (theoretical mass 75541 Da), D) native molecule + fluorescein +1 hexose (theoretical mass 75701 Da).

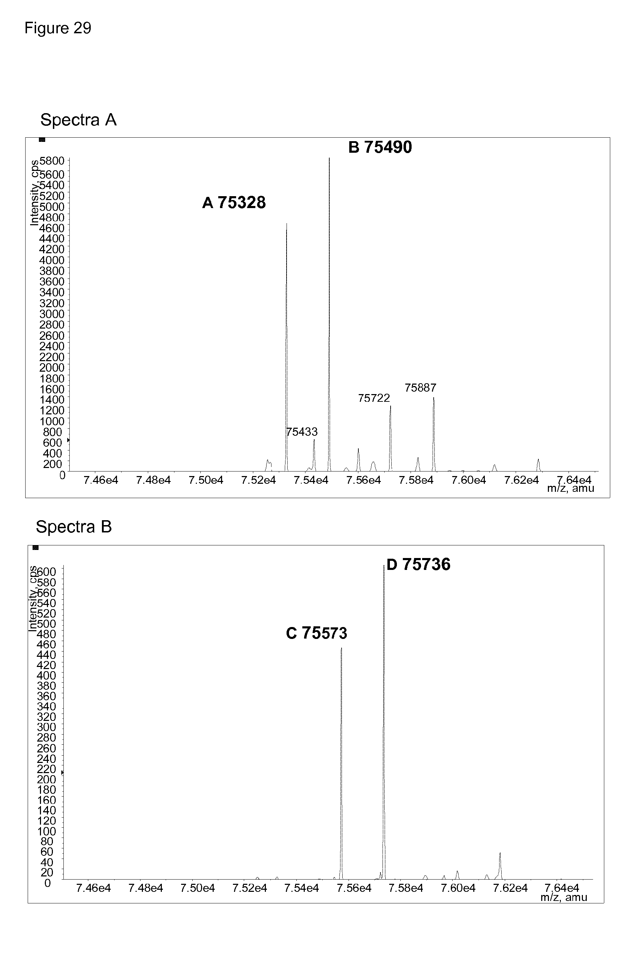

Figure 29 shows deconvolved mass spectra from analysis of thiotransferrin (S415C, T613A) treated with DTNB, compared to fluorescein conjugated thiotransferrin (S415C, T613A) treated with DTNB using ESI-TOF mass spectrometry. Spectrum A shows the mass spectrum of thiotransferrin (S415C, T613A) variant treated with DTNB. Peak identification A) native molecule +NTB (theoretical mass 75327Da), B) native molecule +1 hexose +NTB (theoretical mass 75489Da). Spectrum B shows the mass spectrum of fluorescein conjugated thiotransferrin (S28C, S415A, T613A) variant treated with DTNB. Peak identification C) native molecule + fluorescein (theoretical mass 75553Da), D) native molecule + fluorescein +1 hexose (theoretical mass 75716Da).

### DETAILED DESCRIPTION OF THE INVENTION

### Transferrin

The transferrin used in the invention may be any protein with iron binding capacity which belongs to the transferrin family as described, e.g., by Lambert et al., Comparative Biochemistry and Physiology, Part B, 142 (2005), 129-141, and by Testa, Proteins of iron metabolism, CRC Press, 2002; Harris & Aisen, Iron carriers and iron proteins, Vol. 5, Physical Bioinorganic Chemistry, VCH, 1991.

Examples of transferrin family proteins are serum transferrin, ovotransferrin, melanotransferrin and lactoferrin and their derivatives and variants, such as mutant transferrins (Mason et al., (1993) Biochemistry, 32, 5472; Mason et al., (1998), Biochem. J., 330, 35), truncated transferrins, transferrin lobes (Mason et al., (1996) Protein Expr. Purif., 8, 119; Mason et al., (1991) Protein Expr. Purif., 2, 214), mutant lactoferrins, truncated lactoferrins, lactoferrin lobes, mutant ovotransferrin, truncated ovotransferrin, melanotransferrin lobes, truncated melanotransferrin or fusions of any of the above to other peptides, polypeptides or proteins (Shin et al., (1995) Proc. Natl. Acad. Sci. USA, 92, 2820; Ali et al., (1999) J. Biol. Chem., 274, 24066; Mason et al., (2002) Biochemistry, 41, 9448). Serum transferrins are preferred, particularly a human serum transferrin (HST) having the amino acid sequence of SEQ ID NO: 1 with 679 amino acids, also called the C1 variant (Accession number NP_001054). Lactoferrins are preferred, particularly a human lactoferrin having the amino acid sequence of SEQ ID NO: 11 with 691 amino acids. Melanotransferrins are preferred, particularly human melanotransferrin having the amino acid sequence residue 20-738 of SEQ ID NO: 15.

The transferrin generally has an amino acid sequence which has at least 25% identity to SEQ ID NO: 1, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9%. More particularly, it may have 100% identity.

The lactoferrin generally has an amino acid sequence which has at least 25% identity to SEQ ID NO: 11, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1 %, 99.2%, 99.3%, 99.4%, 99.5%, 99.6%, 99.7%, 99.8%, or 99.9%. More particularly, it may have 100% identity.

The melanotransferrin generally has an amino acid sequence which has at least 25% identity to SEQ ID NO: 15, particularly at least 30%, 35%, 40%, 45%, 50%, 55%, 60%, 65%, 70%, 75%, 80%, 85%, 90%, 91%, 92%, 93%, 94%, 95%, 96%, 97%, 98%, 99%, 99.1 %, 99.2%, 99.3%, 99.4%, 99.5%, 99.6%, 99.7%, 99.8%, or 99.9%. More particularly, it may have 100% identity.

The amino acid sequence of the transferrin family protein may have a length of at least 167 amino acids, particularly at least 300 amino acids, 630 amino acids, 691 amino acids, 694 amino acids or 695 amino acids. The length is typically at most 1274 amino acids, particularly at most 819 amino acids, at most 722 amino acids, at most 717 amino acids or at most 706 amino acids. The length may particularly be 679 amino acids. It may be a transferrin from vertebrae with 691-717 amino acids or a mammalian transferrin with a length of 695-706 amino acids.

The transferrin family protein, particularly one having a length of 694-706 amino acids, generally contains two domains (called the N- and C-Iobes), each having around 331-341 residues and each binding one atom of Fe (III) and one carbonate anion, connected by an interlobe linker of about 7 residues. Typically, each iron is coordinated to four conserved amino acid residues: one Asp, two Tyr and one His, and the anion is bound to an Arg and a Thr.

The transferrin family protein used in the invention has at least 40% or at least 60% identity to full-length HST (residues 1-679 of SEQ ID NO: 1) or to the C-Iobe of HST (residues 339-679 of SEQ ID NO:1). The primary receptor-recognition site of HST for the TfR is in the C-Iobe, and proteolytically isolated HST C-Iobe is able to deliver ferric iron to cells.

The human serum transferrin may be the variant designated TfC₁, TfC₂ or TfC₃.

A number of proteins are known to exist within the transferrin family and a non-exclusive list is shown below. The list indicates the full length of the sequences, including the mature protein and the leader sequence.

| **Protein** | **Species** | **Common Name** | **Accession Number** | **Identity to ID NO: 1** | **Length** |
|---|---|---|---|---|---|
| Transferrin | Homo sapiens | Human | NP_001054 | 100 % | 698 aa |
| Transferrin | Canis lupus familiaris | Dog | XP_864515 | 73.2 | 706 aa |
| Transferrin | Mus musculus | House mouse | NP_598738 | 73.7 | 697 aa |
| Transferrin | Rattus norvegicus | Norway rat | NP_001013128 | 73.6 | 698 aa |
| Transferrin | Sus scrofa | Pig | CAA30943 | 71.6 | 696 aa |
| Transferrin | Oryctolagus cuniculus | Rabbit | AAB94136 | 79.0 | 695 aa |
| Transferrin | Equus caballus | Horse | NP_001075415 | 73.7 | 706 aa |
| Transferrin | Bos taurus | Cattle | NP_803450 | 70.1 | 704 aa |
| Transferrin | Gallus gallus | Chicken | NP_990635 | 53.1 | 705 aa |
| Transferrin | Marmota monax | Woodchuck | AAP37129 | | 694 aa |
| Transferrin | Xenopus laevis | African clawed frog | NP_001079812 | | 717 aa |
| Transferrin | Xenopus tropicalis | African clawed frog | NP_001027487 | 49.6 | 703 aa |
| Transferrin | Chamaeleo calyptratus | Veiled Chameleons | CAK18229 | | 710 aa |
| Transferrin | Lacerta agilis | Sand Lizard | CAK18228 | | 714 aa |
| Transferrin | Eublepharis macularius | Leopard gecko | CAK18227 | | 703 aa |
| Transferrin | Pogona vitticeps | Central bearded dragon | CAK18226 | | 702 aa |
| Transferrin | Anolis sagrei | Brown anole | CAK18225 | | 710 aa |
| Transferrin | Lamprophis fuliginosus | African House Snake | CAK18223 | | 711 aa |
| Transferrin | Natrix natrix | Grass Snake | CAK18221 | 50.0 | 710 aa |
| Transferrin | Oncorhynchus mykiss | Rainbow trout | BAA84103 | | 691 aa |
| Transferrin | Oryzias latipes | Japanese medaka | BAA10901 | | 690 aa |
| Transferrin | Oreochromis niloticus | Nile tilapia | ABB70391 | | 694 aa |
| Transferrin | Oncorhynchus tshawytscha | Chinook salmon | AAF03084 | | 672 aa |
| Transferrin | Gadus morhua | Atlantic cod | AAB08440 | | 642 aa |
| Transferrin | Salmo trutta | Brown trout | BAA84102 | | 691 aa |
| Transferrin | Salvelinus namaycush | Lake trout | BAA84101 | | 691 aa |
| Transferrin | Salvelinus fontinalis | Brook trout | BAA84100 | | 691 aa |
| Transferrin | Salvelinus pluvius | Japanese fish | BAA84099 | | 691 aa |
| Transferrin | Oncorhynchus masou | Cherry salmon | BAA84098 | | 691 aa |
| Transferrin | Oncorhynchus rhodurus | Amago | BAA84097 | | 691 aa |
| Transferrin | Oncorhynchus nerka | Sockeye salmon | BAA84096 | 50.2 | 691 aa |
| Transferrin | Paralichthys olivaceus | Bastard halibut | BAA28944 | | 685 aa |
| Transferrin | Oncorhynchus kisutch | Coho salmon | BAA13759 | | 687 aa |
| Transferrin | Oreochromis aureus | Tilapia (cichlid) | CAC59954 | | 167 aa |
| Transferrin | Danio rerio | Zebrafish | DAA01798 | | 675 aa |
| Transferrin | Pagrus major | Red seabream | AAP94279 | | 691 aa |
| Melanotransferrin | Homo sapiens | Human | NP_005920 | 45.7 | 738 aa |
| Meanotransferrin | Canis lupus familiaris | Dog | XP_545158 | 42.3 | 1193 aa |
| Melanotransferrin | Mus musculus | House mouse | NP_038928 | 44.4 | 738 aa |
| Melanotransferrin | Rattus norvegicus | Norway rat | XP_237839 | 44.7 | 738 aa |
| Melanotransferrin | Gallus gallus | Chicken | NP_990538 | 43.6 | 738 aa |
| Lactotransferrin | H.sapiens | Human | NP_002334 | 61.8 | 710 aa |
| Lactotransferrin | Pan troglodytes | Chimpanzee | XP_516417 | 62.0 | 711 aa |
| Lactotransferrin | Canis lupus familiaris | Dog | XP_864480 | 61.9 | 724 aa |
| Lactotransferrin | Mus musculus | House mouse | NP_032548 | 57.6 | 707 aa |
| Lactotransferrin | Rattus norvegicus | Norway rat | XP_236657 | 53.8 | 729 aa |
| Lactoferrin | Sus scrofa | Pig | AAA31059 | 61.1 | 703 aa |
| Lactoferrin | Camelus dromedarius | Arabian camel | CAB53387 | 62.0 | 708 aa |
| Lactoferrin | Equus caballus | Horse | CAA09407 | 63.7 | 695 aa |
| Lactoferrin | Bos taurus | Cattle | AAA30610 | 62.0 | 708 aa |
| Lactoferrin | Bubalus bubalis | Water buffalo | CAA06441 | 62.1 | 708 aa |
| Lactoferrin | Capra hircus | Goat | ABD49106 | 62.2 | 708 aa |
| Lactoferrin | Bos grunniens | Domestic yak | ABD49105 | 62.0 | 708 aa |
| Lactoferrin | Ovis aries | Sheep | AAV92908 | 62.4 | 708 aa |
| Ovotransferrin | Anas platyrhynchos | Mallard duck | P56410 | 54.4 | 686 aa |
| Ovotransferrin | Gallus gallus | Chicken | CAA26040 | 53.1 | 705 aa |

The transferrin may optionally be fused to another protein, particularly a bioactive protein such as those described below. The fusion may be at the N- or C-terminal or comprise insertions. The skilled person will also appreciate that the open reading frame may encode a protein comprising any sequence, be it a natural protein (including a zymogen), or a variant, or a fragment (which may, for example, be a domain) of a natural protein; or a totally synthetic protein; or a single or multiple fusion of different proteins (natural or synthetic). Examples of transferrin fusions are given in US patent applications US2003/026778, US2003/0221201 and US2003/0226155, Shin, et al., 1995, Proc Natl Acad Sci U S A, 92, 2820, Ali, et al., 1999, J Biol Chem, 274, 24066, Mason, et al., 2002, Biochemistry, 41, 9448, the contents of which are incorporated herein by reference.

### 3D model

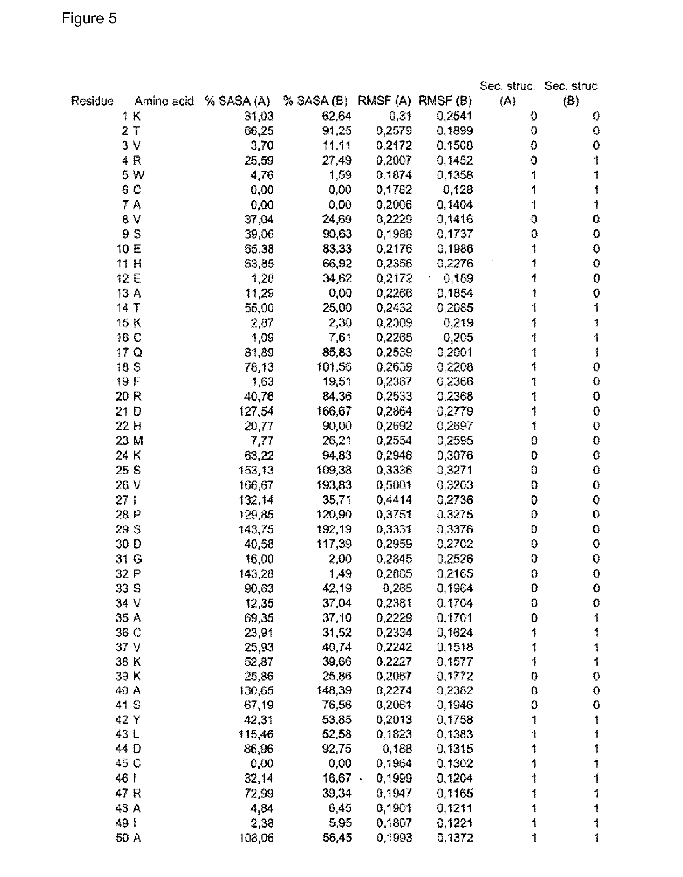

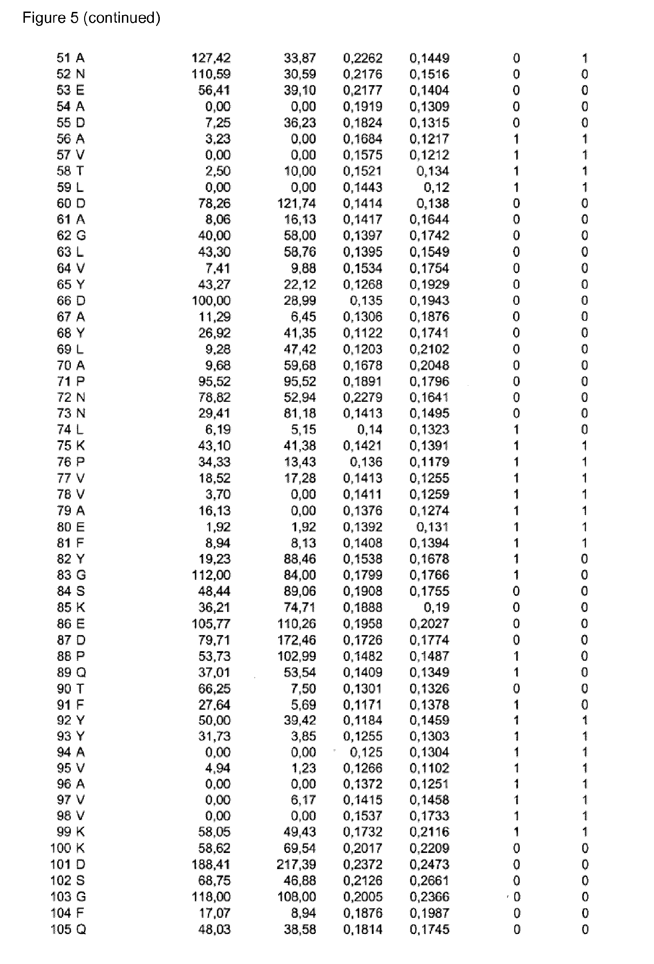

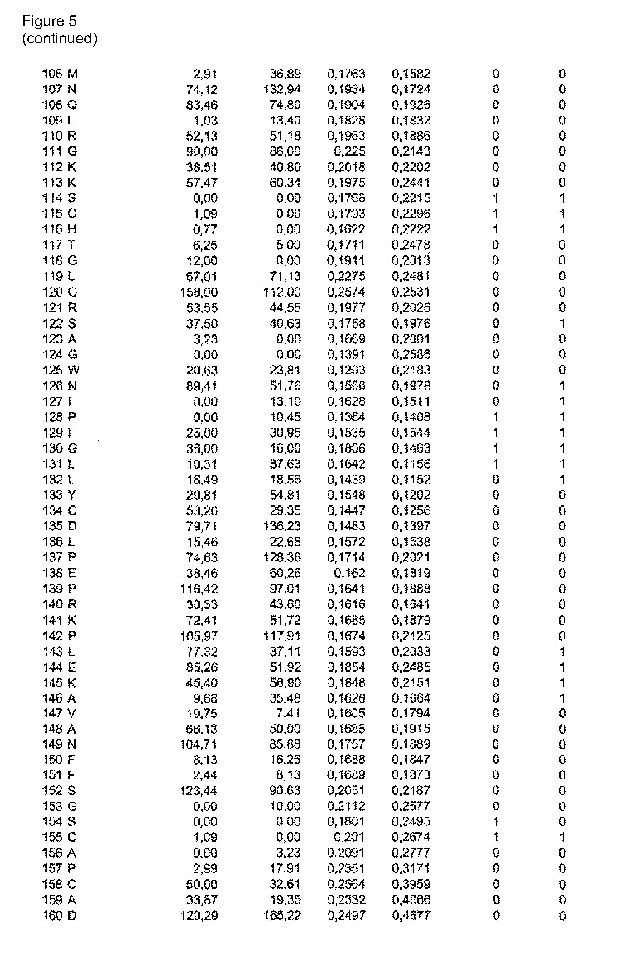

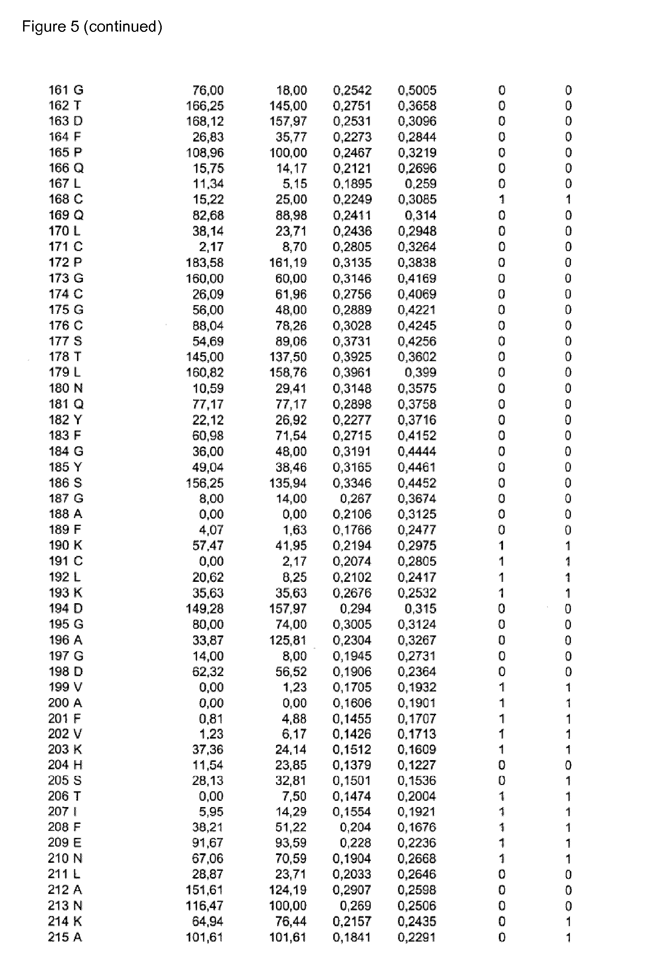

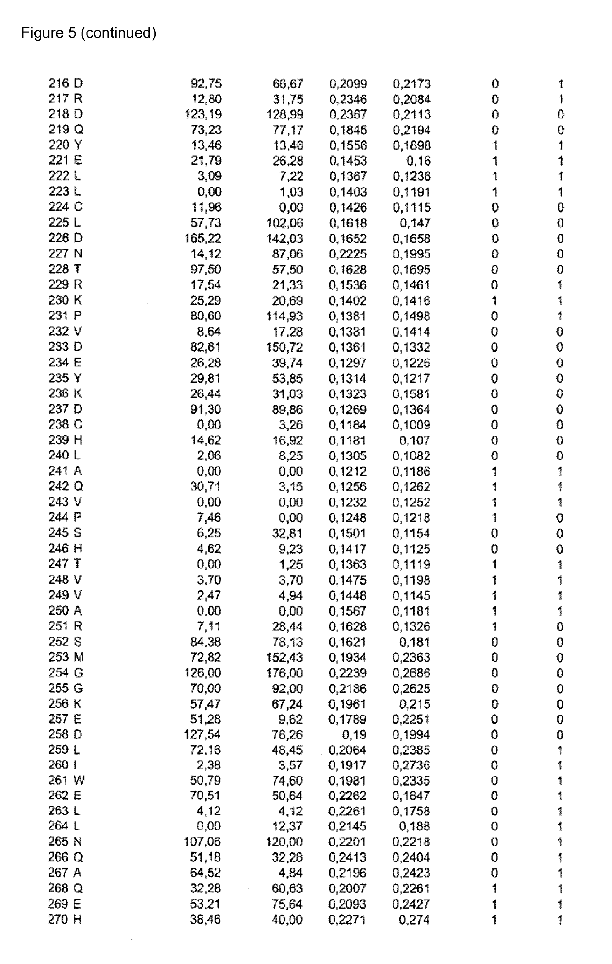

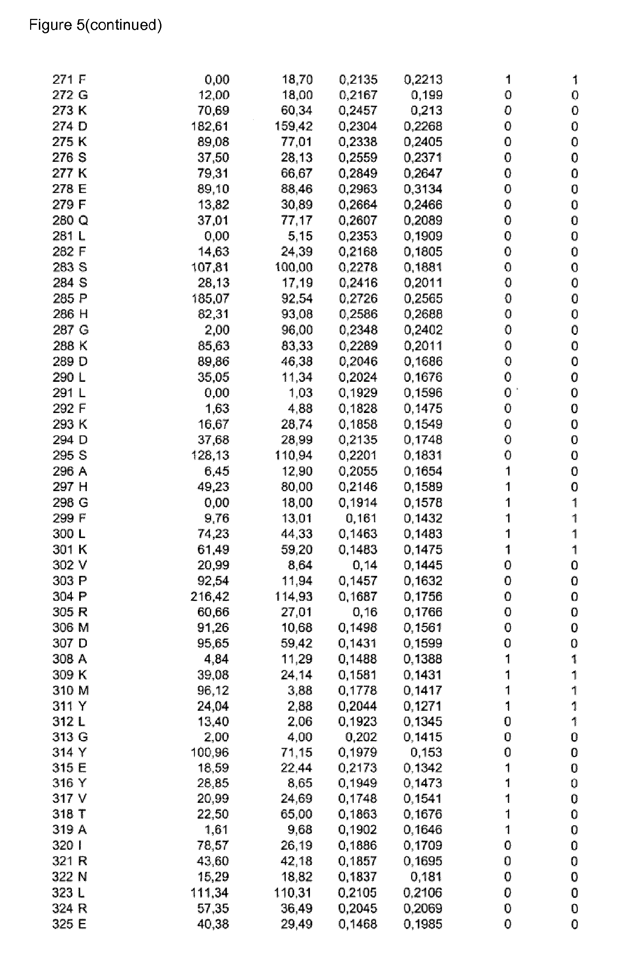

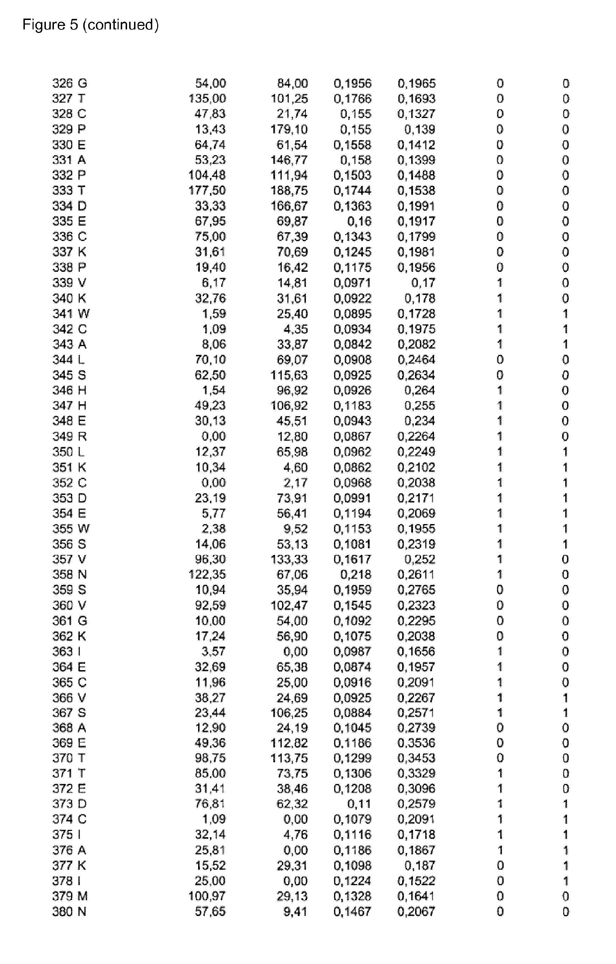

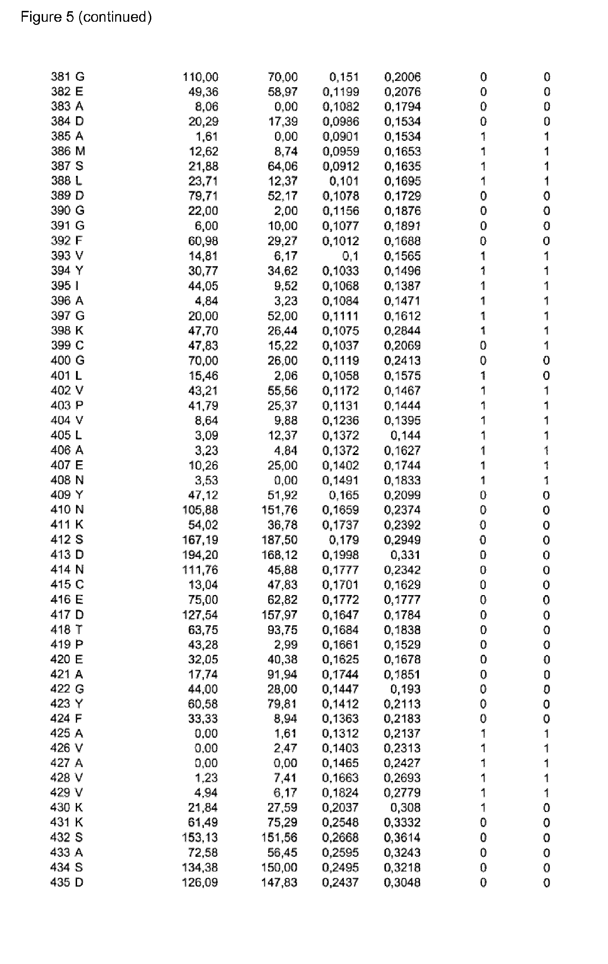

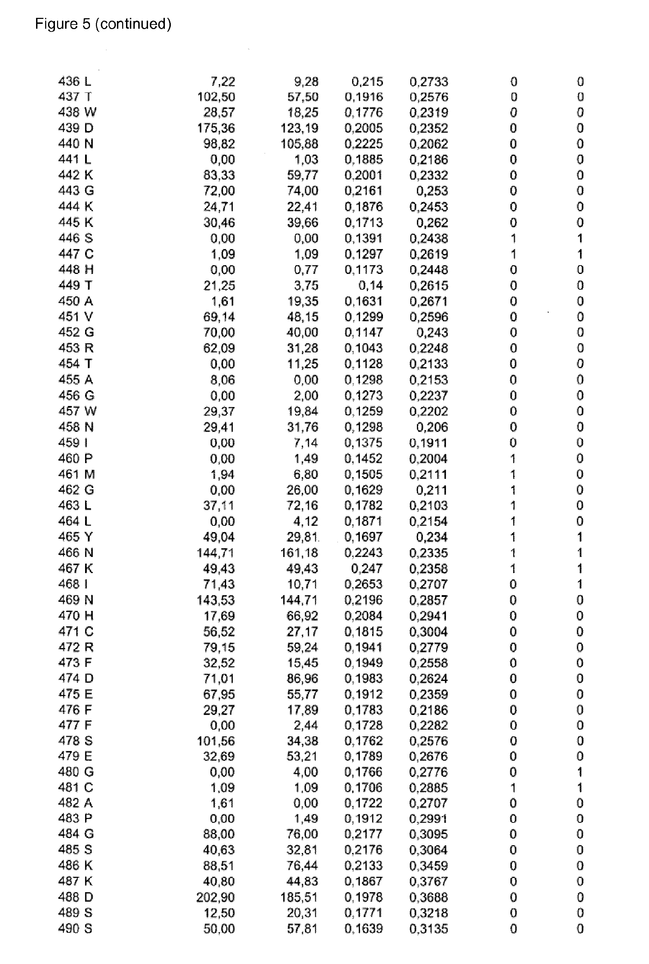

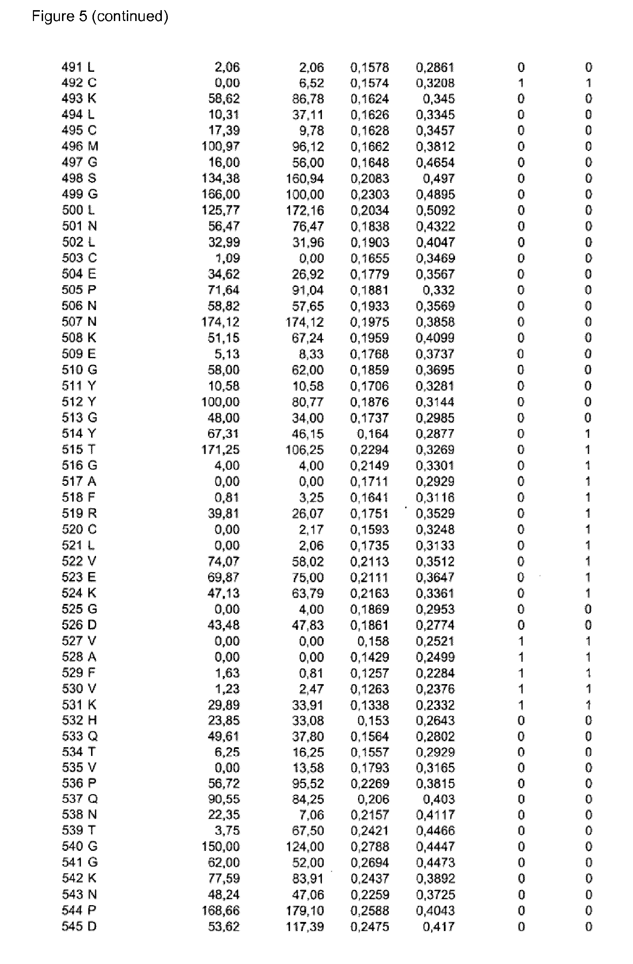

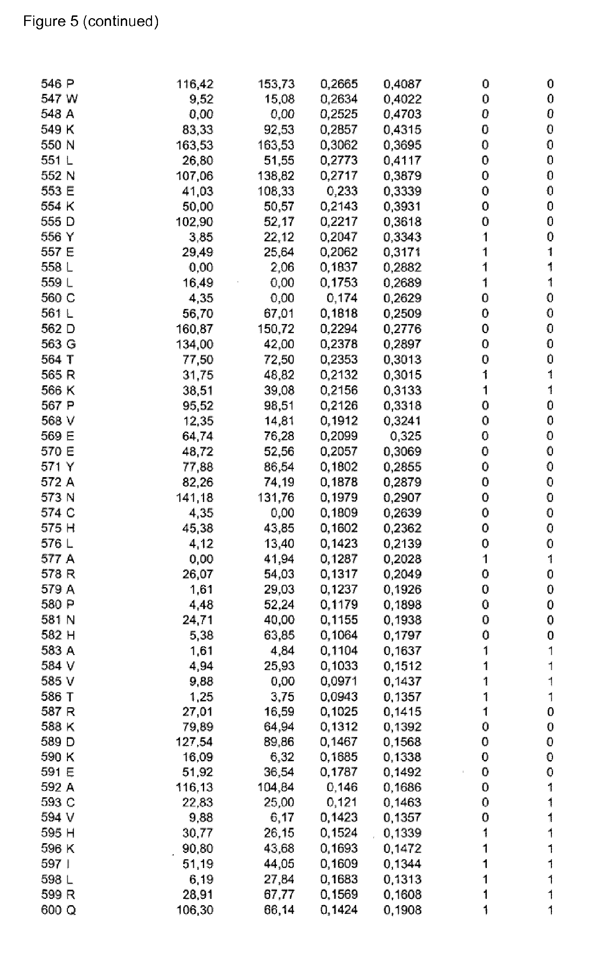

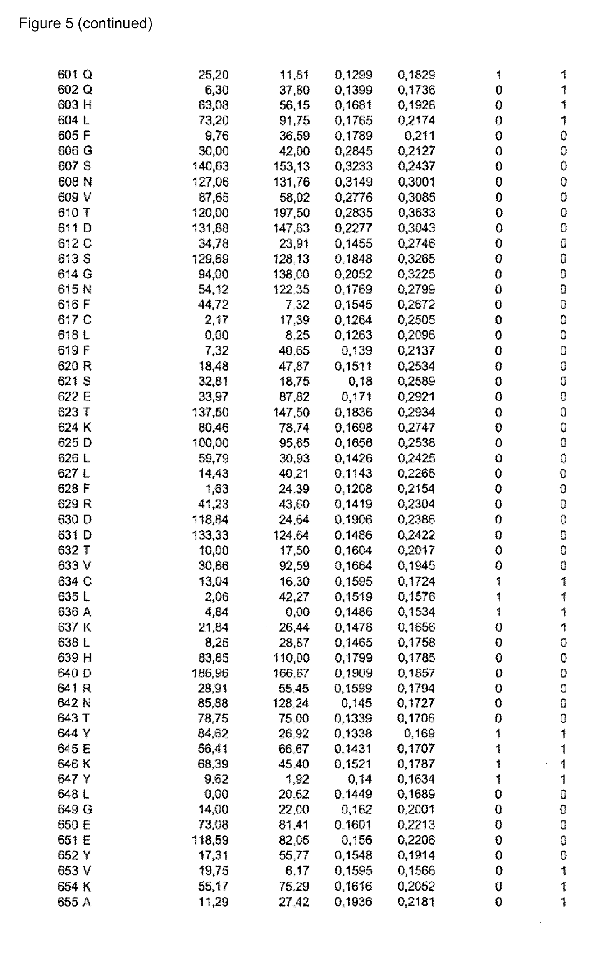

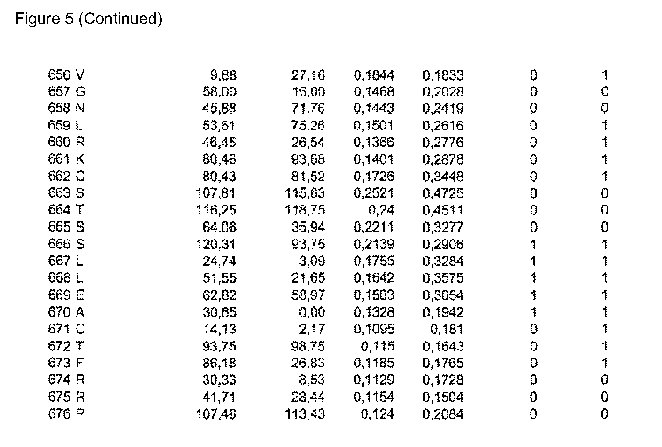

The 3D model used in the invention comprises at least one molecule of transferrin and at least one receptor. Figure 5 gives the coordinates for a model with two molecules of HST and two molecules of TfR. The building of this model is described in Example 4.

Other models including a transferrin family protein and a receptor can be built similarly on the basis of published 3D structures such as 1 CB6, 1 B0L, 1 LFG, 1 DTZ, 1AIV, 1 DOT, 1OVT 1H76, 1JNF, 2HAV and 1SUV (Protein Data Bank).

### Selection of amino acid residues and regions - Step 1

Based on the location of the C-alpha atom of each residue in the 3D model, residues are selected which meet the following criteria.
- Mobility corresponding to RMSF >0.14, >0.16, >0.18, >0.20, >0.22, >0.24, >0.26, >0.28 or >0.30.
- Accessibility >30%, >40%, >50%, >60%, >70%, >80%, >90%, >100%, >110%, >120%, >130% or >140%.

The following lists some particular combinations of mobility and Accessibility and the amino acid residues selected by these criteria based on the properties of amino acid residues shown in Figure 5. The numbering of residues disregards the first three residues in SEQ ID NO: 1 which are unresolved in the 3D model and thus starts with K4 of SEQ ID NO: 1 as position 1.

### Accessibility > 100 %, RMSF > 0.14

D 21 + S 25 + V 26 + P 28 + S 29 + A 40 + E 86 + D 101 + G 103 + G 120 + P 142 + D 160 + T 162 + D 163 + P 172 + T 178 + L 179 + S 186 + D 194 + A 212 + A 215 + D 218 + D 226 + G 254 + N 265 + D 274 + S 295 + 304 + L 323 + T 327 + P 332 + T 333 + N 410 + S 412 + D 413 + D 417 + S 432 + S 434 + D 435 + D 439 + N 466 + N 469 + D 488 + S 498 + L 500 + N 507 + T 515 + G 540 + P 544 + P 546 + N 550 + N 552 + D 562 + N 573 + A 592 + S 607+N608+T610+D611 +S613+T623+D631 +D640+S663+T664+

### Accessibility > 110 %, RMSF > 0.14

D 21 + V 26 + P 28 + S 29 + A 40 + D 101 + G 120 + D 160 + T 162 + D 163 + P 172 + T 178 + L 179 + S 186 + D 194 + A 212 + D 218 + D 226 + G 254 + D 274 + S 295 + P 304 + L 323 + T 333 + S 412 + D 413 + D 417 + S 432 + S 434 + D 435 + D 439 + N 466 + N 469 + D 488 + S 498 + L 500 + N 507 + G 540 + P 544 + P 546 + N 550 + D 562 + N 573 + S 607+N608+T610+D611 +S613+T623+D631 +D640+T664+

### Accessibility > 120 %, RMSF > 0.14

D21 + V 26 + P 28 + S 29 + A 40 + D 101 + D 160 + T 162 + D 163 + P 172 + T 178 + L 179 + S 186 + D 194 + A 212 + D 218 + D 226 + G 254 + D 274 + T 333 + S 412 + D 413 + D 417 + S 432 + S 434 + D 435 + D 439 + N 466 + N 469 + D 488 + S 498 + L 500 + N 507 + G 540 + P 544 + N 550 + D 562 + N 573 + S 607 + N 608 + D 611 +S613+T623+D631 +D640+

### Accessibility > 130 %, RMSF > 0.14

V 26 + S 29 + A40 + D 101 + T 162 + D 163 + P 172 + T 178 + L 179 + S 186 + D 194 + D 226 + D 274 + T 333 + S 412 + D 413 + S 432 + S 434 + N 466 + N 469 + D 488 + S 498 + N 507 + P 544 + N 550 + D 562 + N 573 + S 607 + D 611 +T623+D640+

### Accessibility > 140 %, RMSF > 0.14

V 26 + S 29 + D 101 +T162+D163+P172+L179+D194+D226+D274+T 333 + S 412 + D 413 + S 432 + N 466 + N 469 + D 488 + N 507 + P 544 + N 550 + D 562 + S 607 + D 640 +

### Accessibility > 90 %, RMSF > 0.16

D21 + S 25 + V 26 + P 28 + S 29 + A 40 + P 71 + E 86 + D 101 + G 103 + G 120 + P 139 + P 142 + S 152 + D 160 + T 162 + D 163 + P 165 + P 172 + T 178 + L 179 + S 186 + D 194 + E 209 + A 212 + N 213 + A 215 + D 218 + D 226 + G 254 + N 265 + D 274 + S 283 + P 285 + S 295 + P 304 + L 323 + T 327 + V 357 + N 410 + S 412 + D 413 + D 417 + S 432 + S 434 + D 435 + D 439 + N 440 + N 466 + N 469 + D 488 + M 496 + S 498 + G 499 + L 500 + N 507 + T 515 + G 540 + P 544 + P 546 + N 550 + N 552 + D 562 + P 567 + N 573 + S 607 +N608+T610+D611 +S613+G614+T623+D625+D640+S663+T664+S666 +

### Accessibility > 100 %, RMSF > 0.16

D21 + S 25 + V 26 + P 28 + S 29 + A 40 + E 86 + D 101 + G 103 + G 120 + P 142 + D 160 + T 162 + D 163 + P 172 + T 178 + L 179 + S 186 + D 194 + A 212 + A 215 + D 218 + D 226 + G 254 + N 265 + D 274 + S 295 + P 304 + L 323 + T 327 + N 410 + S 412 + D 413 + D 417 + S 432 + S 434 + D 435 + D 439 + N 466 + N 469 + D 488 + S 498 + L 500 + N 507 + T 515 + G 540 + P 544 + P 546 + N 550 + N 552 + D 562 + N 573 + S 607 + N 608 + T 610 + D611 +S613+T623+D640+S663+T664+

### Accessibility > 110 %, RMSF > 0.16

D 21 + V 26 + P 28 + S 29 + A 40 + D 101 + G 120 + D 160 + T 162 + D 163 + P 172 + T 178 + L 179 + S 186 + D 194 + A 212 + D 218 + D 226 + G 254 + D 274 + S 295 + P 304 + L 323 + S 412 + D 413 + D 417 + S 432 + S 434 + D 435 + D 439 + N 466 + N 469 + D 488 + S 498 + L 500 + N 507 + G 540 + P 544 + P 546 + N 550 + D 562 + N 573 + S 607 + N 608+T610+D611 +S613+T623+D640+T664+

### Accessibility > 120 %, RMSF > 0.16

D21 + V 26 + P 28 + S 29 + A 40 + D 101 + D 160 + T 162 + D 163 + P 172 + T 178 + L 179 + S 186 + D 194 + A 212 + D 218 + D 226 + G 254 + D 274 + S 412 + D 413 + D 417 + S 432 + S 434 + D 435 + D 439 + N 466 + N 469 + D 488 + S 498 + L 500 + N 507 + G 540 + P 544 + N 550 + D 562 + N 573 + S 607 + N 608 + D 611 + S 613 + T 623 + D 640 +

### Accessibility > 130 %, RMSF > 0.16

V 26 + S 29 + A 40 + D 101 + T 162 + D 163 + P 172 + T 178 + L 179 + S 186 + D 194 + D 226 + D 274 + S 412 + D 413 + S 432 + S 434 + N 466 + N 469 + D 488 + S 498 + N 507+P544+N550+D562+N573+S607+D611 +T623+D640+

### Accessibility > 140 %, RMSF > 0.16

V 26 + S 29 + D 101 + T 162 + D 163 + P 172 + L 179 + D 194 + D 226 + D 274 + S 412 + D 413 + S 432 + N 466 + N 469 + D 488 + N 507 + P 544 + N 550 + D 562 + S 607 + D 640 +

### Accessibility > 90 %, RMSF > 0.18

D21 + S 25 + V 26 + P 28 + S 29 + A 40 + E 86 + D 101 +G 103+G 120+S 152+ D 160 + T 162 + D 163 + P 165 + P 172 + T 178 + L 179 + S 186 + D 194 + E 209 + A 212 + N 213 + A 215 + D 218 + G 254 + N 265 + D 274 + S 283 + P 285 + S 295 + L 323 + D 413 + S 432 + S 434 + D 435 + D 439 + N 440 + N 466 + N 469 + D 488 + S 498 + G 499 + L 500 + N 507 + T 515 + G 540 + P 544 + P 546 + N 550 + N 552 + D 562 + P 567 + N 573 + S 607 + N 608 + T 610 + D 611 + S 613 + G 614 + T 623 + D 640 + S 663 + T 664 + S 666 +

### Accessibility > 100 %, RMSF > 0.18

D 21 + S 25 + V 26 + P 28 + S 29 + A40 + E 86 + D 101 + G 103 + G 120 + D 160 + T 162 + D 163 + P 172 + T 178 + L 179 + S 186 + D 194 + A 212 + A 215 + D 218 + G 254 + N 265 + D 274 + S 295 + L 323 + D 413 + S 432 + S 434 + D 435 + D 439 + N 466 + N 469 + D 488 + S 498 + L 500 + N 507 + T 515 + G 540 + P 544 + P 546 + N 550 + N 552 + D 562 + N573+S607+N608+T610+D611 +S613+T623+D640+S663+T664+

### Accessibility > 110 %, RMSF > 0.18

D 21 + V 26 + P 28 + S 29 + A 40 + D 101 + G 120 + D 160 + T 162 + D 163 + P 172 + T 178 + L 179 + S 186 + D 194 + A 212 + D 218 + G 254 + D 274 + S 295 + L 323 + D 413 + S 432 + S 434 + D 435 + D 439 + N 466 + N 469 + D 488 + S 498 + L 500 + N 507 + G 540+P544+P546+N550+D562+N573+S607+N608+T610+D611 +S613+T 623 + D 640 + T 664 +

### Accessibility > 120 %, RMSF > 0.18

D21 + V 26 + P 28 + S 29 + A 40 + D 101 + D 160 + T 162 + D 163 + P 172 + T 178 + L 179 + S 186 + D 194 + A 212 + D 218 + G 254 + D 274 + D 413 + S 432 + S 434 + D 435 + D 439 + N 466 + N 469 + D 488 + S 498 + L 500 + N 507 + G 540 + P 544 + N 550 + D 562 +N573+S607+N608+D611 +S613+T623+D640+

### Accessibility > 130 %, RMSF > 0.18

V 26 + S 29 + A 40 + D 101 +T162+D163+P172+T178+L179+S186+D 194 + D 274 + D 413 + S 432 + S 434 + N 466 + N 469 + D 488 + S 498 + N 507 + P 544 + N 550+D562+N573+S607+D611 +T623+D640+

### Accessibility > 140 %, RMSF > 0.18

V 26 + S 29 + D 101 + T 162 + D 163 + P 172 + L 179 + D 194 + D 274 + D 413 + S 432 + N 466 + N 469 + D 488 + N 507 + P 544 + N 550 + D 562 + S 607 + D 640 +

### Accessibility > 70 %, RMSF > 0.2

Q 17 + S 18 + D 21 + S 25 + V 26 + P 28 + S 29 + A 40 + D 101 + G 103 + G 111 + G 120 + S 152 + D 160 + T 162 + D 163 + P 165 + Q 169 + P 172 + C 176 + T 178 + L 179 + Q 181 + S 186 + D 194 + G 195 + E 209 + A 212 + N 213 + D 218 + G 254 + N 265 + D 274 + K 275 + E 278 + P 285 + H 286 + K 288 + L 323 + S 432 + S 434 + D 435 + D 439 + N 440 + G 443 + N 466 + N 469 + G 484 + K 486 + S 498 + G 499 + L 500 + T 515 + Q 537 + G 540 + K 542 + P 544 + P 546 + K 549 + N 550 + N 552 + D 562 + T 564 + P 567 + S 607 + N 608+T610+D611 +G614+S663+T664+S666+

### Accessibility > 80 %, RMSF > 0.2

Q17+D21 + S 25 + V 26 + P 28 + S 29 + A 40 + D 101 + G 103 + G 111 +G120 + S 152 + D 160 + T 162 + D 163 + P 165 + Q 169 + P 172 + T 178 + L 179 + S 186 + D 194 + E 209 + A 212 + N 213 + D 218 + G 254 + N 265 + D 274 + E 278 + P 285 + H 286 + K 288 + L 323 + S 432 + S 434 + D 435 + D 439 + N 440 + N 466 + N 469 + S 498 + G 499 + L 500 + T 515 + Q 537 + G 540 + P 544 + P 546 + K 549 + N 550 + N 552 + D 562 + P 567 + S 607 +N608+T610+D611 +G614+S663+T664+S666+

### Accessibility > 90 %, RMSF > 0.2

D21 + S 25 + V 26 + P 28 + S 29 + A 40 + D 101 + G 103 + G 120 + S 152 + D 160 + T 162 + D 163 + P 165 + P 172 + T 178 + L 179 + S 186 + D 194 + E 209 + A 212 + N 213 + D 218 + G 254 + N 265 + D 274 + P 285 + L 323 + S 432 + S 434 + D 435 + D 439 + N 440 + N 466 + N 469 + S 498 + G 499 + L 500 + T 515 + G 540 + P 544 + P 546 + N 550 + N 552 +D562+P567+S607+N608+T610+D611 +G614+S663+T664+S666+

### Accessibility > 100 %, RMSF > 0.2

D21 + S 25 + V 26 + P 28 + S 29 + A 40 + D 101 +G103+G120+D160+T162 + D 163 + P 172 + T 178 + L 179 + S 186 + D 194 + A 212 + D 218 + G 254 + N 265 + D 274 + L 323 + S 432 + S 434 + D 435 + D 439 + N 466 + N 469 + S 498 + L 500 + T 515 + G 540 + P 544 + P 546 + N 550 + N 552 + D 562 + S 607 + N 608 + T 610 + D 611 + S 663 + T 664 +

### Accessibility > 110 %, RMSF > 0.2

D 21 + V 26 + P 28 + S 29 + A 40 + D 101 + G 120 + D 160 + T 162 + D 163 + P 172 + T 178 + L 179 + S 186 + D 194 + A 212 + D 218 + G 254 + D 274 + L 323 + S 432 + S 434 + D 435 + D 439 + N 466 + N 469 + S 498 + L 500 + G 540 + P 544 + P 546 + N 550 + D 562+S607+N608+T610+D611 +T664+

### Accessibility > 120 %, RMSF > 0.2

D21 + V 26 + P 28 + S 29 + A 40 + D 101 + D 160 + T 162 + D 163 + P 172 + T 178 + L 179 + S 186 + D 194 + A 212 + D 218 + G 254 + D 274 + S 432 + S 434 + D 435 + D 439 + N 466 + N 469 + S 498 + L 500 + G 540 + P 544 + N 550 + D 562 + S 607 + N 608 + D 611 +

### Accessibility > 130 %, RMSF > 0.2

V 26 + S 29 + A 40 + D 101 + T 162 + D 163 + P 172 + T 178 + L 179 + S 186 + D 194 + D 274 + S 432 + S 434 + N 466 + N 469 + S 498 + P 544 + N 550 + D 562 + S 607 + D 611 +

### Accessibility > 140 %, RMSF > 0.2

V 26 + S 29 + D 101 + T 162 + D 163 + P 172 + L 179 + D 194 + D 274 + S 432 + N 466 + N 469 + P 544 + N 550 + D 562 + S 607 +

### Accessibility > 50 % ,RMSF > 0.22

H 11 + S 18 + D 21 + K 24 + S 25 + V 26 + P 28 + S 29 + A 40 + D 101 + L 119 + G 120 + D 160 + T 162 + D 163 + P 165 + Q 169 + P 172 + G 173 + C 176 + S 177 + T 178 + L 179+Q 181 + F 183 + S 186 + D 194 + G 195 + E 209 + A 212 + N 213 + G 254 + N 265 + D 274 + K 275 + K 277 + E 278 + P 285 + H 286 + K 431 + S 432 + A 433 + S 434 + D 435 + N 466 + G 499 + T 515 + P 536 + G 540 + G 541 + K 542 + P 544 + D 545 + P 546 + K 549 + N 550 + N 552 + D 555 + D 562 + T 564 + S 607 + N 608 + V 609 + T 610 + D 611 + S 663 + T 664 +

### Accessibility > 60 %, RMSF > 0.22

H 11 + S 18 + D 21 + K 24 + S 25 + V 26 + P 28 + S 29 + A 40 + D 101 + L 119 + G 120 + D 160 + T 162 + D 163 + P 165 + Q 169 + P 172 + C 176 + T 178 + L 179 + Q 181 + F 183 + S 186 + D 194 + G 195 + E 209 + A 212 + N 213 + G 254 + N 265 + D 274 + K 275 + K 277 + E 278 + P 285 + H 286 + K 431 + S 432 + S 434 + D 435 + N 466 + G 499 + T 515 + G 540 + K 542 + P 544 + P 546 + K 549 + N 550 + N 552 + D 562 + T 564 + S 607 + N 608 + T 610+D611 +S663+T664+

### Accessibility > 70 %, RMSF > 0.22

S 18 + D 21 +S25+V26+P28+S29+A40+D 101 + G 120 + D 160 + T 162 + D 163 + P 165 + Q 169 + P 172 + C 176 + T 178 + L 179 + Q 181 + S 186 + D 194 + G 195 + E 209 + A 212 + N 213 + G 254 + N 265 + D 274 + K 275 + E 278 + P 285 + H 286 + S 432 + S 434 + D 435 + N 466 + G 499 + T 515 + G 540 + K 542 + P 544 + P 546 + K 549 + N 550 + N 552 + D 562 + T 564 + S 607 + N 608 + T 610 + D 611 +S663+T664+

### Accessibility > 80 %, RMSF > 0.22

D 21 + S 25 + V 26 + P 28 + S 29 + A 40 + D 101 + G 120 + D 160 + T 162 + D 163 + P 165 + Q 169 + P 172 + T 178 + L 179 + S 186 + D 194 + E 209 + A 212 + N 213 + G 254 + N 265 + D 274 + E 278 + P 285 + H 286 + S 432 + S 434 + D 435 + N 466 + G 499 + T 515 + G 540 + P 544 + P 546 + K 549 + N 550 + N 552 + D 562 + S 607 + N 608 + T 610 + D 611 + S 663 + T 664 +

### Accessibility > 90 %, RMSF > 0.22

D 21 + S 25 + V 26 + P 28 + S 29 + A 40 + D 101 + G 120 + D 160 + T 162 + D 163 + P 165 + P 172 + T 178 + L 179 + S 186 + D 194 + E 209 + A 212 + N 213 + G 254 + N 265 + D 274 + P 285 + S 432 + S 434 + D 435 + N 466 + G 499 + T 515 + G 540 + P 544 + P 546 + N 550 + N 552 + D 562 + S 607 + N 608 + T 610 + D 611 +S663+T664+

### Accessibility > 100 %, RMSF > 0.22

D 21 + S 25 + V 26 + P 28 + S 29 + A 40 + D 101 + G 120 + D 160 + T 162 + D 163 + P 172 + T 178 + L 179 + S 186 + D 194 + A 212 + G 254 + N 265 + D 274 + S 432 + S 434 + D 435 + N 466 + T 515 + G 540 + P 544 + P 546 + N 550 + N 552 + D 562 + S 607 + N 608 +T610+D611 +S663+T664+

### Accessibility > 110 %, RMSF > 0.22

D21 + V 26 + P 28 + S 29 + A 40 + D 101 +G 120 + D 160 + T 162 + D 163 + P 172 + T 178 + L 179 + S 186 + D 194 + A 212 + G 254 + D 274 + S 432 + S 434 + D 435 + N 466 + G 540 + P 544 + P 546 + N 550 + D 562 + S 607 + N 608 + T 610 + D 611 +T664+

### Accessibility > 120 %, RMSF > 0.22

D21 + V 26 + P 28 + S 29 + A 40 + D 101 + D 160 + T 162 + D 163 + P 172 + T 178 + L 179 + S 186 + D 194 + A 212 + G 254 + D 274 + S 432 + S 434 + D 435 + N 466 + G 540 +P544+N550+D562+S607+N608+D611 +

### Accessibility > 130 %, RMSF > 0.22

V 26 + S 29 + A 40 + D 101 + T 162 + D 163 + P 172 + T 178 + L 179 + S 186 + D 194 + D 274 + S 432 + S 434 + N 466 + P 544 + N 550 + D 562 + S 607 + D 611 +

### Accessibility > 140 %, RMSF > 0.22

V 26 + S 29 + D 101 + T 162 + D 163 + P 172 + L 179 + D 194 + D 274 + S 432 + N 466 + P 544 + N 550 + D 562 + S 607 +

### Accessibility > 40 %, RMSF > 0.24

D 21 + K 24 + S 25 + V 26 + P 28 + S 29 + D 30 + G 120 + D 160 + T 162 + D 163 + P 165 + Q 169 + P 172 + G 173 + G 175 + C 176 + S 177 + T 178 + L 179 + Q 181 + F 183 + S 186 + D 194 + G 195 + A 212 + N 213 + K 277 + E 278 + P 285 + H 286 + K 431 + S 432 + A 433 + S 434 + D 435 + G 540 + G 541 + K 542 + P 544 + D 545 + P 546 + K 549 + N 550 + N 552 + S 607 + N 608 + V 609 + T 610 + S 663 +

### Accessibility > 50 %, RMSF > 0.24

D 21 + K 24 + S 25 + V 26 + P 28 + S 29 + G 120 + D 160 + T 162 + D 163 + P 165 + Q 169 + P 172 + G 173 + C 176 + S 177 + T 178 + L 179 + Q 181 + F 183 + S 186 + D 194 + G 195 + A 212 + N 213 + K 277 + E 278 + P 285 + H 286 + K 431 + S 432 + A 433 + S 434 +D435+G540+G541 + K 542 + P 544 + D 545 + P 546 + K 549 + N 550 + N 552 + S 607 + N 608 + V 609 + T 610 + S 663 +

### Accessibility > 60 %, RMSF > 0.24

D 21 + K 24 + S 25 + V 26 + P 28 + S 29 + G 120 + D 160 + T 162 + D 163 + P 165 + Q 169 + P 172 + C 176 + T 178 + L 179 + Q 181 + F 183 + S 186 + D 194 + G 195 + A 212 + N 213 + K 277 + E 278 + P 285 + H 286 + K 431 + S 432 + S 434 + D 435 + G 540 + K 542 + P 544 + P 546 + K 549 + N 550 + N 552 + S 607 + N 608 + T 610 + S 663 +

### Accessibility > 70 %, RMSF > 0.24

D 21 + S 25 + V 26 + P 28 + S 29 + G 120 + D 160 + T 162 + D 163 + P 165 + Q 169 + P 172 + C 176 + T 178 + L 179 + Q 181 + S 186 + D 194 + G 195 + A 212 + N 213 + E 278 + P 285 + H 286 + S 432 + S 434 + D 435 + G 540 + K 542 + P 544 + P 546 + K 549 + N 550 + N 552 + S 607 + N 608 + T 610 + S 663 +

### Accessibility > 80 %, RMSF > 0.24

D 21 + S 25 + V 26 + P 28 + S 29 + G 120 + D 160 + T 162 + D 163 + P 165 + Q 169 + P 172 + T 178 + L 179 + S 186 + D 194 + A 212 + N 213 + E 278 + P 285 + H 286 + S 432 + S 434 + D 435 + G 540 + P 544 + P 546 + K 549 + N 550 + N 552 + S 607 + N 608 + T 610+S663+

### Accessibility > 90 %, RMSF > 0.24

D21 + S 25 + V 26 + P 28 + S 29 + G 120 + D 160 + T 162 + D 163 + P 165 + P 172 + T 178 + L 179 + S 186 + D 194 + A 212 + N 213 + P 285 + S 432 + S 434 + D 435 + G 540 + P 544 + P 546 + N 550 + N 552 + S 607 + N 608 + T 610 + S 663 +

### Accessibility > 100 %, RMSF > 0.24

D21 + S 25 + V 26 + P 28 + S 29 + G 120 + D 160 + T 162 + D 163 + P 172 + T 178 + L 179 + S 186 + D 194 + A 212 + S 432 + S 434 + D 435 + G 540 + P 544 + P 546 + N 550 + N 552 + S 607 + N 608 + T 610 + S 663 +

### Accessibility > 110 %, RMSF > 0.24

D 21 + V 26 + P 28 + S 29 + G 120 + D 160 + T 162 + D 163 + P 172 + T 178 + L 179 + S 186 + D 194 + A 212 + S 432 + S 434 + D 435 + G 540 + P 544 + P 546 + N 550 + S 607+N608+T610+

### Accessibility > 120 %, RMSF > 0.24

D 21 + V 26 + P 28 + S 29 + D 160 + T 162 + D 163 + P 172 + T 178 + L 179 + S 186 + D 194 + A 212 + S 432 + S 434 + D 435 + G 540 + P 544 + N 550 + S 607 + N 608 +

### Accessibility > 130 %, RMSF > 0.24

V 26 + S 29 + T 162 + D 163 + P 172 + T 178 + L 179 + S 186 + D 194 + S 432 + S 434 + P 544 + N 550 + S 607 +

### Accessibility > 140 %, RMSF > 0.24

V 26 + S 29 + T 162 + D 163 + P 172 + L 179 + D 194 + S 432 + P 544 + N 550 + S 607 +

### Accessibility > 40 %, RMSF > 0.26

D21 + K 24 + S 25 + V 26 + P 28 + S 29 + D 30 + T 162 + P 172 + G 173 + G 175 + C 176 + S 177 + T 178 + L 179 + Q 181 + F 183 + S 186 + D 194 + G 195 + K 277 + E 278 + S 432 + G 540 + G 541 + P 546 + K 549 + N 550 + N 552 + N 608 + V 609 + T 610 +

### Accessibility > 50 %, RMSF > 0.26

D 21 + K 24 + S 25 + V 26 + P 28 + S 29 + T 162 + P 172 + G 173 + C 176 + S 177 + T 178 + L 179 + Q 181 + F 183 + S 186 + D 194 + G 195 + K 277 + E 278 + S 432 + G 540 + G 541 + P 546 + K 549 + N 550 + N 552 + N 608 + V 609 + T 610 +

### Accessibility > 60 %, RMSF > 0.26

D 21 + K 24 + S 25 + V 26 + P 28 + S 29 + T 162 + P 172 + C 176 + T 178 + L 179 + Q 181 + F 183 + S 186 + D 194 + G 195 + K 277 + E 278 + S 432 + G 540 + P 546 + K 549 + N 550 + N 552 + N 608 + T 610 +

### Accessibility > 70 %, RMSF > 0.26

D 21 + S 25 + V 26 + P 28 + S 29 + T 162 + P 172 + C 176 + T 178 + L 179 + Q 181 + S 186 + D 194 + G 195 + E 278 + S 432 + G 540 + P 546 + K 549 + N 550 + N 552 + N 608 + T 610 +

### Accessibility > 80 %, RMSF > 0.26

D21 + S 25 + V 26 + P 28 + S 29 + T 162 + P 172 + T 178 + L 179 + S 186 + D 194 + E 278 + S 432 + G 540 + P 546 + K 549 + N 550 + N 552 + N 608 + T 610 +

### Accessibility > 90 %, RMSF > 0.26

D21 + S 25 + V 26 + P 28 + S 29 + T 162 + P 172 + T 178 + L 179 + S 186 + D 194 + S 432 + G 540 + P 546 + N 550 + N 552 + N 608 + T 610 +

### Accessibility > 100 %, RMSF > 0.26

D 21 + S 25 + V 26 + P 28 + S 29 + T 162 + P 172 + T 178 + L 179 + S 186 + D 194 + S 432 + G 540 + P 546 + N 550 + N 552 + N 608 + T 610 +

### Accessibility > 110 %, RMSF > 0.26

D 21 + V 26 + P 28 + S 29 + T 162 + P 172 + T 178 + L 179 + S 186 + D 194 + S 432 + G 540 + P 546 + N 550 + N 608 + T 610 +

### Accessibility > 120 %, RMSF > 0.26

D 21 + V 26 + P 28 + S 29 + T 162 + P 172 + T 178 + L 179 + S 186 + D 194 + S 432 + G 540 + N 550 + N 608 +

### Accessibility > 130 %, RMSF > 0.26

V 26 + S 29 + T 162 + P 172 + T 178 + L 179 + S 186 + D 194 + S 432 + N 550 +

### Accessibility > 140 %, RMSF > 0.26

V 26 + S 29 + T 162 + P 172 + L 179 + D 194 + S 432 + N 550 +

### Accessibility > 40 %, RMSF > 0.28

K 24 + S 25 + V 26 + P 28 + S 29 + P 172 + G 173 + G 175 + C 176 + S 177 + T 178 + L 179 + Q 181 + S 186 + D 194 + G 195 + E 278 + K 549 + N 550 + N 608 + T 610 +

### Accessibility > 50 %, RMSF > 0.28

K 24 + S 25 + V 26 + P 28 + S 29 + P 172 + G 173 + C 176 + S 177 + T 178 + L 179 + Q 181 + S 186 + D 194 + G 195 + E 278 + K 549 + N 550 + N 608 + T 610 +

### Accessibility > 60 %, RMSF > 0.28

K 24 + S 25 + V 26 + P 28 + S 29 + P 172 + C 176 + T 178 + L 179 + Q 181 + S 186 + D 194 + G 195 + E 278 + K 549 + N 550 + N 608 + T 610 +

### Accessibility > 70 %, RMSF > 0.28

S 25 + V 26 + P 28 + S 29 + P 172 + C 176 + T 178 + L 179 + Q 181 + S 186 + D 194 + G 195 + E 278 + K 549 + N 550 + N 608 + T 610 +

### Accessibility > 80 %, RMSF > 0.28

S 25 + V 26 + P 28 + S 29 + P 172 + T 178 + L 179 + S 186 + D 194 + E 278 + K 549 + N 550 + N 608 + T 610 +

### Accessibility > 90 %, RMSF > 0.28

S 25 + V 26 + P 28 + S 29 + P 172 + T 178 + L 179 + S 186 + D 194 + N 550 + N 608+T610+

### Accessibility > 100 %, RMSF > 0.28

S 25 + V 26 + P 28 + S 29 + P 172 + T 178 + L 179 + S 186 + D 194 + N 550 + N 608+T610+

### Accessibility > 110 %, RMSF > 0.28

V 26 + P 28 + S 29 + P 172 + T 178 + L 179 + S 186 + D 194 + N 550 + N 608 + T 610 +

### Accessibility > 120 %, RMSF > 0.28

V 26 + P 28 + S 29 + P 172 + T 178 + L 179 + S 186 + D 194 + N 550 + N 608 +

### Accessibility > 130 %, RMSF > 0.28

V 26 + S 29 + P 172+T178+L 179+S 186 + D 194 + N 550 +

### Accessibility > 140 %, RMSF > 0.28

V 26 + S 29 + P 172 + L 179 + D 194 + N 550 +

### Accessibility > 40 %, RMSF > 0.3

S 25 + V 26 + P 28 + S 29 + P 172 + G 173 + C 176 + S 177 + T 178 + L 179 + S 186 + G 195 + N 550 + N 608 +

### Accessibility > 50 %, RMSF > 0.3

S 25 + V 26 + P 28 + S 29 + P 172 + G 173 + C 176 + S 177 + T 178 + L 179 + S 186 + G 195 + N 550 + N 608 +

### Accessibility > 60 %, RMSF > 0.3

S 25 + V 26 + P 28 + S 29 + P 172 + C 176 + T 178 + L 179 + S 186 + G 195 + N 550 + N 608 +

### Accessibility > 70 %, RMSF > 0.3

S 25 + V 26 + P 28 + S 29 + P 172 + C 176 + T 178 + L 179 + S 186 + G 195 + N 550 + N 608 +

### Accessibility > 80 %, RMSF > 0.3

S 25 + V 26 + P 28 + S 29 + P 172 + T 178 + L 179 + S 186 + N 550 + N 608 +

### Accessibility > 90 %, RMSF > 0.3

S 25 + V 26 + P 28 + S 29 + P 172 + T 178 + L 179 + S 186 + N 550 + N 608 +

### Accessibility > 100 %, RMSF > 0.3

S 25 + V 26 + P 28 + S 29 + P 172 + T 178 + L 179 + S 186 + N 550 + N 608 +

### Accessibility > 110 %, RMSF > 0.3

V 26 + P 28 + S 29 + P 172 + T 178 + L 179 + S 186 + N 550 + N 608 +

### Accessibility > 120 %, RMSF > 0.3

V 26 + P 28 + S 29 + P 172 + T 178 + L 179 + S 186 + N 550 + N 608 +

### Accessibility > 130 %, RMSF > 0.3

V 26 + S 29 + P 172 + T 178 + L 179 + S 186 + N 550 +

### Accessibility > 140 %, RMSF > 0.3

V 26 + S 29 + P 172 + L 179 + N 550 +

### Selection of amino acid residues and regions - Step 2

Next, the three unresolved N terminal amino acids (Wally et al., (2006) Journal of Biological Chemistry, 281 (34), 24934-24944), V 1, P 2 and D 3 are added to the list of selected amino acids and the assigned position numbers of the selected amino acid shown above are corrected for the addition of the unresolved four N terminal amino acids.

Further, residues and regions may be selected on the basis of their relation to secondary structures in the 3D model (alpha-helices and beta-strands). An analysis on the basis of Table 2 of J. Wally et al., Journal of Biological Chemistry, 281 (34), 24934-24944 (2006) or figure 5 of this application, indicates that the selected amino acids are located as follows in relation to secondary structures:

**Table 1**

| N-lobe | | Interlobe + linker C-lobe | |
|---|---|---|---|
| Amino acids | Structural location | Amino acids | Structural location |
| V 1, P 2, D 3, K 4 | Before βa | P 335, T 336 | Within interlobe linker |
| T 5 | Beginning of βa | | |
| H 14, Q 20, S 21, D 24 | Within α1 | N 413, S 415, D 416 | Loop between α3 - α3a |
| K 27, S 28, V 29, P 31, S 32, D 33 | Loop between α1 - βb | D 420 | End of α3a |
| A43 | Loop between βb - α2 | | |
| E 89 | Loop between βd - βe | | |
| D104,G106 | Loop between βe - α4 | K 434, S 435, A 436, S 437, D 438 | Loop between βe - α4 |
| G 114 | Loop between α4 - βf | D 442, N 443 | Within α4 |
| L 122, G 123 | Loop between βf - α5 | G 446 | Loop between α4 - βf |
| P 145 | Loop between α5a - α6 | N 469 | Within α5a |
| S 155 | Loop between α6 - βg | N 472 | Loop between α5a - α6 |
| D 163, T 165, D 166 | Loop between βg - α6a | G 487, K 489, D 491, S 501 | Loop between βg - α6a |
| P 168 | Beginning of α6a | G 502 | Beginning of α6a |
| P 175 | Beginning of α6b | L 503 | Within α6a |
| G 176 | Within α6b | | |
| G 178, C 179, S 180, T 181, L 182, Q 184 | Loop between α6a - α7 | N 510 | Loop between α6a - α7 |
| F 187 | Beginning α7 | T 518 | Within α7 |
| S 189 | Within α7 | P 539, Q540 | Within α7a |
| D 197, G 198 | Loop between α7 - βh | G 543, G 544, K 545, P 547, D 548 | Loop between α7a - α8 |
| E 212 | Within α8 | P 549 | Beginning of α8 |
| A 215, N 216 | Loop between α8 - α8a | K 552, N 553, N 555 | Loop between α8 - α8a1 |
| A 218 | Within α8a | D 558 | Loop between α8a1 - βi |
| D 221 | Loop between α8a - βi | D 565, T 567 | Loop between βi - βia |
| D 229 | Loop between βi - α8b | P 570 | End of βia |
| G 257 | Loop between βja - α9 | N 576 | End of α8b |
| N 268 | Within α9 | A 595 | Within α9 |
| D 277, K 278, K 280, E 281, S 287, P 288, H 289, K 291, S 298 | Loop between α9 - βk | S610,N611,V 612, T 613, D 614, S 616, G 617, T 626, D 634 | Loop between α9 - βk |
| P 307 | Loop between βk - α10 | D 643 | Loop between βk - α10 |
| L 326 | Within α11 | S 666, T 667 | Loop between α11 - α12 |
| T 330 | Loop between α11 and linker | S 669 | Beginning of α12 |

Based on the HST 3D structure described in the examples, the Accessibility and RMSF analysis performed (Figure 5) and the mapping analysis performed above, the preferred regions for introduction of the one or more Cys residues with a free thiol groups may be taken as all the loops, sheets and helices identified in the table above, i.e. V1-T5, E13-H25, M26-S36, K42-S44, Y85-T93, K103-Q108, L112-K115, T120-W128, L139-P145, F154-G156, A159-F167, P168-L170; P175-C177, G178-F186, G187-K196, D197-D201, I210-N213, L214-N216, K217-R220, D221-Q222, L226-P234, S255-K259, E260-H273, F274-H300, V305-D310, Y317-E328, G329- P341, C402-N417, C418-D420, K434-T440, W441-N443, L444-G446, Y468-K470, I471-R475, A485-S501, G502-N504, L505-Y515, G516-V526, T537-Q540, N541-D548, P549-A551, K552-N555, D558-Y559, C563-T567, R568-P570, E572-N576, E594-F608, G609-V636, L641-T646, R663-S668, S669-R677. Particularly preferred ranges include all the loops identified in table 1 and so excludes the sheets and helices], i.e. V1-K4, M26-P35, K42-S44, Y85-T93, K103-Q108, L112-K115, T120-W128, L139-P145, F154-S155, A159-F167, G178-F186, D197-D201, L214-N216, D221-Q222, L226-P234, S255-K259, F274-H300, V305-D310, G329- P341, C402-N417, K434-T440, L444-G446, I471-R475, A485-S501, L505-Y515, N541-D548, K552-N555, D558, C563-T567, G609-V636, L641-T646, R663-S668.

For a transferrin family protein with two lobes, the lobes may be aligned as described in J. Wally et al., Journal of Biological Chemistry, 281 (34), 24934-24944 (2006), and residues may be selected which meet the criteria in both lobes. Most preferred ranges cover loops which were identified in both the N-lobe and C-Iobe of table 1, i.e. V1-K4, K103-Q108, L112-K115, L139-P145, A159-F167, G178-F186, F274-H300, V305-D310, G329-P341, K434-T440, L444-G446, I471-R475, A485-S501, L505-Y515, G609-V636, L641-T646.

Most especially preferred positions for introduction of the one or more Cys residues with a free thiol groups is defined as the amino acids identified in table 1, i.e. V1, P2, D3, K4 T5, H14, Q20, S21, D24, K27, S28, V29, P31, S32, D33, A43, E89, D104, G106, G114, L122, G123, P145, S155, D163, T165, D166, P168, P175, G176, G178, C179, S180, T181, L182, Q184, F187, S189, D197, G198, E212, A215, N216, A218, D221, D229, G257, N268, D277, K278, K280, E281, S287, P288, H289, K291, S298, P307, L326, T330, P335, T336, N413, S415, D416, D420, K434, S435, A436, S437, D438, D442, N443, G446, N469, N472, G487, K489, D491, S501, G502, L503, N510, T518, P539, Q540, G543, G544, K545, P547, D548, P549, K552, N553, N555, D558, D565, T567, P570, N576, A595, S610, N611, V612, T613, D614, S616, G617, T626, D634, D643, S666, T667, S669.

In the following the invention is described infurther details in particular with reference to transferrin, however, the skilled person will appreciate that the teaching applies likewise to other members of the transferrin family, such as lactoferrin and melanotransferrin.

### Alteration of transferrin amino acid sequence

A free thiol group may be introduced into the transferrin molecule by altering the amino acid sequence by substitution or insertion at a position selected as described above. Thus, the selected residue may be substituted with Cys (the amino acid length is unchanged), or Cys may be inserted at the N- or C-terminal side of the selected residue (the amino chain length is increased), or one or more adjacent residues including the selected residue may be substituted with Cys (the amino acid chain length is reduced). Multiple alterations may be made to the polypeptide.

Alternatively, one of the Cys residues present in the transferrin molecule (38 in the case of HST) may be selected, and the selected cysteine may be deleted or may be substituted with a different amino acid, particularly Ser, Thr, Val or Ala. Cys residues in the regions selected above correspond to C19, C158, C161, C177, C179, C194, C227, C331, C339, C402, C418, C474, C495, C448, C506, C523, C563, C596, C615, C620, C665. The selected Cys residue may in particular correspond to C227, C241, C474, C577, C563 or C665.

### Production of thiotransferrin

The thiotransferrin or fusions of thiotransferrin and another protein or proteins can be prepared by methods know to the art (Sanker, (2004), Genetic Eng. News, 24, 22-28, Schmidt, (2004), Appl. Microbiol. Biotechnol., 65, 363-372) including but not limited to expression in mammalian cell culture (Mason et al., (2004), Protein Expr. Purif., 36, 318-326; Mason et al., (2002), Biochemistry, 41, 9448-9454) from cells lines such as CHO (and its variants), NS0, BHK, HEK293, Vero or PERC6 cells by transformation or transient expression; insect cell culture (Lim et al., (2004) Biotechnol. Prog., 20, 1192-1197); plant cell culture from such plants as Lemna; transgenic animals (Dyck et al., (2003) Trends in Biotechnology, 21, 394-399); transgenic plants (Ma et al., (2003) Nature Reviews Genetics, 4, 794-805); Gram +ve and Gram -ve bacteria such as *Bacillus* and *Escherichia coli* (Steinlein, and Ikeda, (1993), Enzyme Microb. Technol., 15, 193-199); filamentous fungi including but not restricted to *Aspergillus* spp (EP 238023, US 5,364,770, US 5,578,463, EP184438, EP284603, WO 2000/056900, WO9614413), *Trichoderma* spp and *Fusarium* spp (Navalainen et al., (2005), Trends in Biotechnology, 23, 468-473).

Polypeptides which are variants of full-length HST may be expressed recombinantly from baby hamster kidney (BHK) cells (Mason et al., (2004), Protein Expr. Purif., 36, 318-326, Mason et al., (2002), Biochemistry, 41, 9448-9454), *D. melanogaster* S2 cells (Lim et al., (2004), Biotechnol Prog., 20, 1192-1197) and as a non-N-linked glycosylated mutant from BHK cells (Mason et al., (2001), Protein Expr. Purif., 23, 142-150, Mason et al., (1993), Biochemistry, 32, 5472-5479) and *S. cerevisiae* (Sargent et al., (2006), Biometals 19, 513-519). The Polypeptides which are variants of C-Iobe of HST (NTf/2C) may be expressed from BHK cells (Mason et al., (1997), Biochem. J., 326 (Pt 1), 77-85). In one embodiment the host cell is a yeast cell, such as a member of the *Saccharomyces, Kluyveromyces,* or *Pichia* genus, such as *Saccharomyces cerevisiae, Kluyveromyces lactis, Pichia pastoris* (Mason et al., (1996), Protein Expr. Purif., 8, 119-125, Steinlein et al., 1995 Protein Expr. Purif., 6, 619-624), *Pichia methanolica* (Mayson et al., (2003) Biotechnol. Bioeng., 81, 291-298) and *Pichia membranaefaciens,* or *Zygosaccharomyces rouxii* (formerly classified as *Zygosaccharomyces bisporus), Zygosaccharomyces bailii, Zygosaccharomyces fermentati, Hansenula polymorpha* (also known as *Pichia angusta*) or *Kluyveromyces drosophilarum* are preferred.

The host cell may be any type of cell. The host cell may or may not be an animal (such as mammalian, avian, insect, etc.), plant, fungal or bacterial cell. Bacterial and fungal, such as yeast, host cells may or may not be preferred.

Typical prokaryotic vector plasmids are: pUC18, pUC19, pBR322 and pBR329 available from Biorad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Methods well known to those skilled in the art can be used to construct expression vectors containing the coding sequence and, for example appropriate transcriptional or translational controls. One such method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic double stranded oligonucleotide linkers and adaptors. DNA fragments with blunt ends are generated by bacteriophage T4 DNA polymerase or *E.coli* DNA polymerase I which remove protruding 3' termini and fill in recessed 3' ends. Synthetic linkers and pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end. Alternatively a DNA fragment or DNA fragments can be ligated together by the action of DNA ligase in the presence or absence of one or more synthetic double stranded oligonucleotides optionally containing cohesive ends.

Synthetic linkers containing a variety of restriction endonuclease sites are commercially available from a number of sources including Sigma-Genosys Ltd, London Road, Pampisford, Cambridge, United Kingdom.

The thiotransferrin or fusions of thiotransferrin and another protein or proteins will be expressed from a nucleotide sequence, which may or may not contain one or more introns. Additionally the nucleotide sequence may or may not be codon optimised for the host by methods known to the art.

The thiotransferrin or fusions of thiotransferrin and another protein or proteins can be expressed as variants with reduced N-linked glycosylation. Accordingly, in case of human serum transferring (HST), N413 can be changed to any amino acid, preferably, Q, D, E or A; S415 can be changed to any amino acid except S or T, preferably, A; T613 can be changed to any amino acid except S or T, preferably, A; N611 can be changed to any amino acid; or combinations of the above. Where the transferrin is not HST, reduction in N-glycosylation can be achieved by similar modification to the protein primary. For clarity, the thiotransferrin or fusions of thiotransferrin and another protein or proteins can be both a transferrin variant of the invention and have reduced N-linked glycosylation.

It may be particularly advantageous to use a yeast deficient in one or more protein mannosyl transferases involved in O-glycosylation of proteins, for instance by disruption of the gene coding sequence. Recombinantly expressed proteins can be subject to undesirable post-translational modifications by the producing host cell. The mannosylated transferrin would be able to bind to the lectin Concanavalin A. The amount of mannosylated transferrin produced by the yeast can be reduced by using a yeast strain deficient in one or more of the *PMT* genes (WO 94/04687). The most convenient way of achieving this is to create a yeast which has a defect in its genome such that a reduced level of one of the Pmt proteins is produced. For example, there may or may not be a deletion, insertion or transposition in the coding sequence or the regulatory regions (or in another gene regulating the expression of one of the *PMT* genes) such that little or no Pmt protein is produced. Alternatively, the yeast could be transformed to produce an anti-Pmt agent, such as an anti-Pmt antibody. Alternatively, the yeast could be cultured in the presence of a compound that inhibits the activity of one of the PMT genes (Duffy et al, "Inhibition of protein mannosyltransferase 1 (PMT1) activity in the pathogenic yeast Candida albicans", International Conference on Molecular Mechanisms of Fungal Cell Wall Biogenesis, 26-31 August 2001, Monte Verita, Switzerland, Poster Abstract P38; the poster abstract may be viewed at http://www.micro.biol.ethz.ch/cellwall/). If a yeast other than *S. cerevisiae* is used, disruption of one or more of the genes equivalent to the *PMT* genes of *S. cerevisiae* is also beneficial, e.g. in *Pichia pastoris* or *Kluyveromyces lactis.* The sequence of *PMT1* (or any other *PMT* gene) isolated from *S. cerevisiae* may be used for the identification or disruption of genes encoding similar enzymatic activities in other fungal species. The cloning of the *PMT1* homologue of *Kluyveromyces lactis* is described in WO 94/04687.

The yeast may or may not also have a deletion of the *HSP150* and/or *YAP3* genes as taught respectively in WO 95/33833 and WO 95/23857.

The HST variant may be produced by recombinant expression and secretion. Where the expression system (i.e. the host cell) is yeast, such as *Saccharomyces cerevisiae,* suitable promoters for *S. cerevisiae* include those associated with the *PGK1* gene, *GAL1* or *GAL10* genes, *TEF1, TEF2, PYK1, PMA1, CYC1, PHO5, TRP1, ADH1, ADH2,* the genes for glyceraldehyde-3-phosphate dehydrogenase, hexokinase, pyruvate decarboxylase, phosphofructokinase, triose phosphate isomerase, phosphoglucose isomerase, glucokinase, α-mating factor pheromone, α-mating factor pheromone, the *PRB1* promoter, the *PRA1* promoter, the *GPD1* promoter, and hybrid promoters involving hybrids of parts of 5' regulatory regions with parts of 5' regulatory regions of other promoters or with upstream activation sites (e.g. the promoter of EP-A-258 067).

Suitable transcription termination signals are well known in the art. Where the host cell is eukaryotic, the transcription termination signal is preferably derived from the 3' flanking sequence of a eukaryotic gene, which contains proper signals for transcription termination and polyadenylation. Suitable 3' flanking sequences may, for example, be those of the gene naturally linked to the expression control sequence used, i.e. may correspond to the promoter. Alternatively, they may be different. In that case, and where the host is a yeast, preferably *S. cerevisiae,* then the termination signal of the *S*. *cerevisiae ADH1, ADH2, CYC1,* or *PGK1* genes are preferred.

It may be beneficial for the promoter and open reading frame of the gene encoding the recombinant protein comprising the sequence of a transferrin mutant to be flanked by transcription termination sequences so that the transcription termination sequences are located both upstream and downstream of the promoter and open reading frame, in order to prevent transcriptional read-through into any neighbouring genes, such as 2µm genes, and vice versa.

In one embodiment, the favoured regulatory sequences in yeast, such as *Saccharomyces cerevisiae,* include: a yeast promoter (e.g. the *Saccharomyces cerevisiae PRB1* promoter), as taught in EP 431 880; and a transcription terminator, preferably the terminator from *Saccharomyces ADH1,* as taught in EP 60 057.

It may be beneficial for the non-coding region to incorporate more than one DNA sequence encoding a translational stop codon, such as UAA, UAG or UGA, in order to minimise translational read-through and thus avoid the production of elongated, non-natural fusion proteins. The translation stop codon UAA is preferred.

In one preferred embodiment, the recombinant protein comprising the sequence of a transferrin mutant is secreted. In that case, a sequence encoding a secretion leader sequence may be included in the open reading frame. Thus, a polynucleotide according to the present invention may comprise a sequence that encodes a recombinant protein comprising the sequence of a transferrin mutant operably linked to a polynucleotide sequence that encodes a secretion leader sequence. Leader sequences are usually, although not necessarily, located at the N-terminus of the primary translation product of an ORF and are generally, although not necessarily, cleaved off the protein during the secretion process, to yield the "mature" protein. Thus, in one embodiment, the term "operably linked" in the context of leader sequences includes the meaning that the sequence that encodes a recombinant protein comprising the sequence of a transferrin mutant is linked, at its 5' end, and in-frame, to the 3' end of a polynucleotide sequence that encodes a secretion leader sequence. Alternatively, the polynucleotide sequence that encodes a secretion leader sequence may be located, in-frame, within the coding sequence of the recombinant protein comprising the sequence of a transferrin mutant, or at the 3' end of the coding sequence of the recombinant protein comprising the sequence of a transferrin mutant.

Numerous natural or artificial polypeptide leader sequences (also called secretion pre regions and pre/pro regions) have been used or developed for secreting proteins from host cells. Leader sequences direct a nascent protein towards the machinery of the cell that exports proteins from the cell into the surrounding medium or, in some cases, into the periplasmic space.

For production of proteins in eukaryotic species such as the yeasts *Saccharomyces cerevisiae, Zygosaccharomyces* species, *Kluyveromyces lactis* and *Pichia pastoris,* a secretion leader sequence may be used. This may comprise a signal (pre) sequence or a prepro leader sequence. Signal sequences are known to be heterogeneous in their amino acid sequence (Nothwehr and Gordon 1990, Bioessays 12, 479-484, or Gierasch 1989, Biochemistry 28, p923-930). In essence, signal sequences are generally N-terminally located, have a basic n-region, a hydrophobic h-region and a polar c-region. As long as this structure is retained the signal sequence will work, irrespective of the amino acid composition. How well they work, i.e. how much mature protein is secreted, depends upon the amino acid sequence. Accordingly, the term "signal peptide" is understood to mean a presequence which is predominantly hydrophobic in nature and present as an N-terminal sequence of the precursor form of an extracellular protein expressed in yeast. The function of the signal peptide is to allow the expressed protein to be secreted to enter the endoplasmic reticulum. The signal peptide is normally cleaved off in the course of this process. The signal peptide may be heterologous or homologous to the yeast organism producing the protein. Known leader sequences include those from the *S. cerevisiae* acid phosphatase protein (Pho5p) (see EP 366 400), the invertase protein (Suc2p) (see Smith et al. (1985) Science, 229, 1219-1224) and heat-shock protein-150 (Hsp150p) (see WO 95/33833). Additionally, leader sequences from the *S*. *cerevisiae* mating factor alpha-1 protein (MFα-1) and from the human lysozyme and human serum albumin (HSA) protein have been used, the latter having been used especially, although not exclusively, for secreting human albumin. WO 90/01063 discloses a fusion of the MFα-1 and HSA leader sequences. In addition, the natural transferrin leader sequence may or may not be used to direct secretion of the recombinant protein comprising the sequence of a transferrin mutant.

The skilled person will appreciate that any suitable plasmid may be used, such as a centromeric plasmid. The examples provide suitable plasmids (centromeric YCplac33-based vectors) for use to transform yeast host cells of the present invention. Alternatively, any other suitable plasmid may be used, such as a yeast-compatible 2µm-based plasmid.

Plasmids obtained from one yeast type can be maintained in other yeast types (Irie et al, 1991, Gene, 108(1), 139-144; Irie et al, 1991, Mol. Gen. Genet., 225(2), 257-265). For example, pSR1 from *Zygosaccharomyces rouxii* can be maintained in *Saccharomyces cerevisiae.* In one embodiment the plasmid may or may not be a 2µm-family plasmid and the host cell will be compatible with the 2µm-family plasmid used (see below for a full description of the following plasmids). For example, where the plasmid is based on pSR1, pSB3 or pSB4 then a suitable yeast cell is *Zygosaccharomyces rouxii;* where the plasmid is based on pSB1 or pSB2 then a suitable yeast cell is *Zygosaccharomyces bailli;* where the plasmid is based on pSM1 then a suitable yeast cell is *Zygosaccharomyces fermentati;* where the plasmid is based on pKD1 then a suitable yeast cell is *Kluyveromyces drosophilarum;* where the plasmid is based on pPM1 then a suitable yeast cell is *Pichia membranaefaciens;* where the plasmid is based on the 2µm plasmid then a suitable yeast cell is *Saccharomyces cerevisiae* or *Saccharomyces carlsbergensis.* Thus, the plasmid may be based on the 2µm plasmid and the yeast cell may be *Saccharomyces cerevisiae.* A 2µm-family plasmid can be said to be "based on" a naturally occurring plasmid if it comprises one, two or preferably three of the genes *FLP, REP1* and *REP2* having sequences derived from that naturally occurring plasmid.

Useful yeast episomal plasmid vectors are pRS403-406 and pRS413-416 and are generally available from Stratagene Cloning Systems (La Jolla, CA 92037, USA), YEp24 (Botstein, D., et al. (1979) Gene 8, 17-24), and YEplac122, YEplac195 and YEplac181 (Gietz, R.D. and Sugino. A. (1988) Gene 74, 527-534). Other yeast plasmids are described in WO 90/01063 and EP 424 117, as well as the "disintegration vectors of EP-A-286 424 and W02005061719. Plasmids pRS403, pRS404, pRS405 and pRS406 are Yeast Integrating plasmids (Ylps) and incorporate the yeast selectable markers *HIS3, TRP1, LEU2* and *URA3,* as are YIplac204, YIplac211 and YIplac128 (Gietz, R.D. and Sugino. A. (1988) Gene 74, 527-534). Plasmids pRS413-416 are Yeast Centromere plasmids (YCps) as are YCplac22, YCplac33 and YCplac111 (Gietz, R.D. and Sugino. A. (1988) Gene 74, 527-534).

Where one or more of the helper protein(s) and/or protein product of choice are encoded by a plasmid-borne polynucleotide sequence, the host cell type may be selected for compatibility with the plasmid type being used. Such plasmids are disclosed in WO2005061719. Perferred helper proteins include *PDI1, AHA1, ATP11, CCT2, CCT3, CCT4, CCT5*, *CCT6, CCT7, CCT8, CNS1, CPR3, CPR6, DER1, DER3, DOA4, ERO1, EUG1, ERV2, EPS1, FKB2, FMO1, HCH1, HRD3, HSP10, HSP12, HSP104, HSP26, HSP30, HSP42, HSP60, HSP78, HSP82, KAR2, JEM1, MDJ1, MDJ2, MPD1, MPD2, PD11, PFD1, ABC1, APJ1, ATP11, ATP12, BTT1, CDC37, CPR7, HSC82, KAR2, LHS1, MGE1, MRS11, NOB1, ECM10, SCJ1, SSA1, SSA2, SSA3, SSA4,* SSB1, SSB2, *SSC1, SSE2, SIL1, SLS1, ORM1, ORM2, PER1, PTC2, PSE1, UBC7, UB14* and *HAC1* or a truncated intronless *HAC1* (Valkonen et al. 2003, Applied Environ. Micro., 69, 2065). Such helper proteins are disclosed in WO 2005/061718, WO 2006/067511 and WO 2006/136831.

Plasmids as defined above may be introduced into a host through standard techniques. With regard to transformation of prokaryotic host cells, see, for example, Cohen et al (1972) Proc. Natl. Acad. Sci. USA 69, 2110 and Sambrook et al (2001) Molecular Cloning, A Laboratory Manual, 3rd Ed. Cold Spring Harbor Laboratory, Cold Spring Harbor, NY. Transformation of yeast cells is described in Sherman et al (1986) Methods In Yeast Genetics, A Laboratory Manual, Cold Spring Harbor, NY. The method of Beggs (1978) Nature 275, 104-109 is also useful. Methods for the transformation of *S. cerevisiae* are taught generally in EP 251 744, EP 258 067 and WO 90/01063, all of which are incorporated herein by reference. With regard to vertebrate cells, reagents useful in transfecting such cells, for example calcium phosphate and DEAE-dextran or liposome formulations, are available from Stratagene Cloning Systems, or Life Technologies Inc., Gaithersburg, MD 20877, USA.

Electroporation is also useful for transforming cells and is well known in the art for transforming fungal (including yeast) cell, plant cells, bacterial cells and animal (including vertebrate) cells. Methods for transformation of yeast by electroporation are disclosed in Becker & Guarente (1990) Methods Enzymol. 194, 182.

Generally, the plasmid will transform not all of the hosts and it will therefore be necessary to select for transformed host cells. Thus, a plasmid may comprise a selectable marker, including but not limited to bacterial selectable marker and/or a yeast selectable marker. A typical bacterial selectable marker is the β-lactamase gene although many others are known in the art. Typical yeast selectable marker include *LEU2, TRP1, HIS3, HIS4, URA3, URA5, SFA1, ADE2, MET15, LYS5, LYS2, ILV2, FBA1, PSE1, PDI1* and *PGK1.* Those skilled in the art will appreciate that any gene whose chromosomal deletion or inactivation results in an unviable host, so called essential genes, can be used as a selective marker if a functional gene is provided on the plasmid, as demonstrated for *PGK1* in a *pgk1* yeast strain (Piper and Curran, 1990, Curr. Genet. 17, 119). Suitable essential genes can be found within the Stanford Genome Database (SGD), (http:://db.yeastgenome.org). Any essential gene product (e.g. PDI1, PSE1, PGK1 or FBA1) which, when deleted or inactivated, does not result in an auxotrophic (biosynthetic) requirement, can be used as a selectable marker on a plasmid in a host cell that, in the absence of the plasmid, is unable to produce that gene product, to achieve increased plasmid stability without the disadvantage of requiring the cell to be cultured under specific selective conditions. By "auxotrophic (biosynthetic) requirement" we include a deficiency which can be complemented by additions or modifications to the growth medium. Therefore, preferred "essential marker genes" in the context of the present application are those that, when deleted or inactivated in a host cell, result in a deficiency which cannot be complemented by additions or modifications to the growth medium. Additionally, a plasmid may comprise more than one selectable marker.

Transformed host cells may be cultured for a sufficient time and under appropriate conditions known to those skilled in the art, and in view of the teachings disclosed herein, to permit the expression of the helper protein(s) and the protein product of choice.

The culture medium may be non-selective or place a selective pressure on the maintenance of a plasmid.

Methods for culturing prokaryotic host cells, such as *E.coli*, and eukaryotic host cells, such as mammalian cells are well known in the art. Methods for culturing yeast are generally taught in EP 330 451 and EP 361 991.

The thus produced protein product of choice may be present intracellularly or, if secreted, in the culture medium and/or periplasmic space of the host cell.

Accordingly, the present invention also provides a method for producing a protein product of choice, the method comprising: a) providing a host cell of the invention comprising a polynucleotide encoding protein product of choice as defined above; and b) growing the host cell (for example, culturing the host cell in a culture medium); thereby to produce a cell culture or recombinant organism comprising an increased level of the protein product of choice compared to the level of production of the protein product of choice achieved by growing (for example, culturing), under the same conditions, the same host cell that has not been genetically modified to cause over-expression of one or more helper proteins.

The step of growing the host cell may or may not involve allowing a host cell derived from a multicellular organism to be regrown into a multicellular recombinant organism (such as a plant or animal) and, optionally, producing one or more generations of progeny therefrom.

The method may or may not further comprise the step of purifying the thus expressed protein product of choice from the cultured host cell, recombinant organism or culture medium.

The step of "purifying the thus expressed protein product of choice from the cultured host cell, recombinant organism or culture medium" optionally comprises cell immobilisation, cell separation and/or cell breakage, but always comprises at least one other purification step different from the step or steps of cell immobilisation, separation and/or breakage.

Thiotransferrin of the invention may be purified from the culture medium by any technique that has been found to be useful for purifying such proteins. Similarly, cell separation techniques, such as centrifugation, filtration (e.g. cross-flow filtration, expanded bed chromatography and the like) are well known in the art. Likewise, methods of cell breakage, including beadmilling, sonication, enzymatic exposure and the like are well known in the art.

The "at least one other purification step" may be any other step suitable for protein purification known in the art. For example purification techniques for the recovery of recombinantly expressed albumin have been disclosed in: WO 92/04367, removal of matrix-derived dye; EP 464 590, removal of yeast-derived colorants; EP 319 067, alkaline precipitation and subsequent application of the albumin to a lipophilic phase; and WO 96/37515, US 5 728 553 and WO 00/44772, which describe complete purification processes; all of which are incorporated herein by reference. Suitable methods include ammonium sulphate or ethanol precipitation, acid or solvent extraction, anion or cation exchange chromatography, phosphocellulose chromatography, hydrophobic interaction chromatography, affinity chromatography, hydroxyapatite chromatography, lectin chromatography, concentration, dilution, pH adjustment, diafiltration, ultrafiltration, high performance liquid chromatography ("HPLC"), reverse phase HPLC, conductivity adjustment and the like.

In one embodiment, any one or more of the above mentioned techniques may or may not be used to further purifying the thus isolated protein to a commercially or industrially acceptable level of purity. By commercially or industrially acceptable level of purity, we include the provision of the protein at a concentration of at least 10⁻⁴g.L⁻¹, 10⁻³ g.L⁻¹, 0.01 g.L⁻¹, 0.02 g.L⁻¹, 0.03 g.L⁻¹, 0.04 g.L⁻¹, 0.05 g.L⁻¹, 0.06 g.L⁻¹,0.07 g.L⁻¹, 0.08 g.L⁻¹, 0.09 g.L⁻¹, 0.1 g.L⁻¹, 0.2 g.L⁻¹, 0.3 g.L⁻¹, 0.4 g.L⁻¹, 0.5 g.L⁻¹, 0.6 g.L⁻¹, 0.7 g.L⁻¹, 0.8 g.L⁻¹, 0.9 g.L⁻¹, 1 g.L⁻¹, 2 g.L⁻¹, 3 g.L-¹, 4 g.L⁻¹, 5 g.L⁻¹, 6 g.L⁻¹, 7 g.L⁻¹, 8 g.L⁻¹, 9 g.L⁻¹, 10 g.L⁻¹, 15 g.L⁻¹, 20 g.L⁻¹, 25 g.L⁻¹, 30 g.L⁻¹, 40 g.L⁻¹,50 g.L⁻¹, 60 g.L⁻¹, 70 g.L⁻¹, 70 g.L⁻¹, 90 g.L⁻¹, 100 g.L⁻¹, 150 g.L⁻¹, 200 g.L⁻¹, 250 g.L⁻¹, 300 g.L⁻¹, 350 g.L⁻¹, 400 g.L⁻¹, 500 g.L⁻¹, 600 g.L⁻¹, 700 g.L⁻¹, 800 g.L⁻¹, 900 g.L⁻¹, 1000 g.L⁻¹, or more.

A commercially or industrially acceptable level of purity may be obtained by a relatively crude purification method by which the protein product of choice is put into a form suitable for its intended purpose. A protein preparation that has been purified to a commercially or industrially acceptable level of purity may, in addition to the protein product of choice, also comprise, for example, cell culture components such as host cells or debris derived therefrom. Alternatively, high molecular weight components (such as host cells or debris derived therefrom) may or may not be removed (such as by filtration or centrifugation) to obtain a composition comprising the protein product of choice and, optionally, a functionally acceptable level of low molecular weight contaminants derived from the cell culture process.

The protein may or may not be purified to achieve a pharmaceutically acceptable level of purity. A protein has a pharmaceutically acceptable level of purity if it is essentially pyrogen free and can be used for it's intended purpose and hence be administered in a pharmaceutically efficacious amount without causing medical effects not associated with the activity of the protein.

A method of the present invention may or may not further comprise the step of formulating the purified protein product of choice with a carrier or diluent and optionally presenting the thus formulated protein in a unit dosage form.

Although it is possible for a therapeutically useful protein obtained by a process of the invention to be administered alone, it is preferable to present it as a pharmaceutical formulation, together with one or more acceptable carriers or diluents. The carrier(s) or diluent(s) must be "acceptable" in the sense of being compatible with the desired protein. Typically, the carriers or diluents will be water or saline which will be sterile and pyrogen free.

Alternatively, a method of the present invention may or may not further comprise the step of lyophilising the thus purified protein product of choice.

### Formulation of thiotransferrin or conjugate

The thiotransferrin may be formulated by strategies given in "Protein Formulation and Delivery", E. J. McNally (Ed.), published by Marcel Dekker Inc. New York 2000 and "Rational Design of Satble Protein Formulations - Theory and Practice"; J. F. Carpenter and M. C. Manning (Ed.) Pharmaceutical Biotechnology Vol 13. Kluwer Academic/Plenum Publishers, New York 2002, Yazdi and Murphy, (1994) Cancer Research 54, 6387-6394, Widera et al., (2003) Pharmaceutical Research 20, 1231-1238; Lee et al., (2005) Arch. Pharm. Res. 28, 722-729. Examples of formulation methods are as follows:

Method #1: Following purification the free thiol containing transferrin mutein of the invention or the conjugate can be stored at 4°C, -20°C or -80°C in 0.01 M - 0.1 M phosphate buffered saline (pH 7.0 - 8.0) containing 0.01 M - 0.2 M NaCl.

Method #2: Following purification the free thiol containing transferrin mutein of the invention or the conjugate can be stored at 4°C, -20°C or -80°C in 0.01 M - 0.1 M phosphate buffered saline (pH 7.0 - 8.0) containing 0.01 M - 0.2 M NaCl and containing 10-20mg/L Polysorbate 80.

Method #3: Following purification the free thiol containing transferrin mutein of the invention or the conjugate can be stored at 4°C, -20°C or -80°C in 0.01 M - 0.2 M NaCl (pH 7.0 - 8.0).

Method #4: Following purification the free thiol containing transferrin mutein of the invention or the conjugate can be stored at 4°C, -20°C or -80°C in 0.01 M - 0.2 M NaCl (pH 7.0 - 8.0) containing 10-20mg/L Polysorbate 80.

### Freeze-dried formulations

Method #5: Following purification the free thiol containing transferrin mutein of the invention or the conjugate can be dialysed against water, freeze dried and stored at 4°C, - 20°C or -80°C.

Method #6: Following purification the free thiol containing transferrin mutein of the invention or the conjugate can be dialysed against 0.01 M - 0.2 M NaCl (pH 7.0 - 8.0), freeze dried and stored at 4°C, -20°C or -80°C.

### Nanoparticles

Method #7: Following purification the free thiol containing transferrin mutein of the invention or the conjugate can be formulated into nanoparticles prepared according to known procedures for preparing nanoparticles, such as procedures disclosed in WO 2004/071536 A1 and WO 2008/007146 A1, both included by reference.

### Bioactive compound

The bioactive compound may be a therapeutic or diagnostic compound. The therapeutic compound may be a chemotherapy drug for use in cancer chemotherapy. It may be cytostatic or cytotoxic; it may be a tumor-inhibiting agent.

The bioactive compound may already contain a free thiol group, e.g. a polypeptide containing a Cysteine residue with a free thiol group. Alternatively, the bioactive compound may be modified so as to contain a free thiol group. Thus, the amino acid sequence of a polypeptide may be altered so as to include a Cysteine residue with a free thiol group, or the bioactive compound may be chemically derivatized to include a free thiol group.

The bioactive compound may be a polypeptide (protein), particularly a recombinant protein pharmaceutical. It may be a chemotherapy or radiotherapy drug used to treat cancers and other related diseases.

The free thiol containing transferrin mutein of the invention (thiotransferrin) can be conjugated via the free thiol group, or groups if the transferrin mutein of the invention contains more than one free thiol, to at least one bioactive compound by methods know to the art. The bioactive compound includes but is not limited to, peptides, polypeptides or proteins (either natural, recombinant, or synthetic) (Debinski, (2002) Cancer Investigation 20, 801-809, O'Keefe and Draper et al., (1985) JBC 260, 932-937, Xia et al., (2000) J. Pharmacology Experimental Therapeutics 295, 594-600, Kavimandan et al., (2006) Bioconjugate Chem. 17, 1376-1384, Humphries, et al., (1994) J. Tissue Culture Methods 16, 239-242, Wenning et al., (1998) Biotech. Bioeng. 57, 484-496, Yazdi and Murphy, (1994) Cancer Research 54, 6387-6394, Weaver and Laske (2003) J. Neuro-Oncology 65, 3-13, Widera et al., (2003) Pharmaceutical Research 20, 1231-1238, Daniels, T.R. et al. (2006) Clinical Immunology 121, 159-176 and the references included therein); therapeutic and diagnostic drugs or compounds (Mishra et al., (2006) J. Drug Targeting 14, 45-53, Lim and Shen, (2004) Pharmaceutical Research 21, 1985-1992, Fritzer et al., (1996) Biochemical Pharmacology 51, 489-493, Lubgan and Jozwiak (2002) Cell. Mol. Biol. Lett. 7, 98, Daniels, T.R. et al. (2006) Clinical Immunology 121, 159-176 and the references included therein); high molecular weight complexes including but not limited to liposomes, viruses and nanoparticles (Mishra et al., (2006) J. Drug Targeting 14, 45-53, Daniels, T.R. et al. (2006) Clinical Immunology 121, 159-176 and the references included therein); nucleic acids and radionuclides, including DNA, RNA (including siR-NA) and their analogs (Lee et al., (2005) Arch. Pharm. Res. 28, 722-729, Huang et al., (2007) FASEB J. 21, 1117- 1125, Daniels, T.R. et al. (2006) Clinical Immunology 121, 159-176 and the references included therein) and devices (Humphries, et al., (1994) J. Tissue Culture Methods 16, 239-242 and the references included therein). Additionally the entity can itself be modified by methods known to the art.

### Therapeutic compounds

4-1 BB ligand, 5-helix, , A human C-C chemokine, A human L105 chemokine, A human L105 chemokine designated huL105_3., A monokine induced by gamma-interferon (MIG), A partial CXCR4B protein, A platelet basic protein (PBP), α1-antitrypsin, □□ACRP-30 Homologue; Complement Component C1 q C, Adenoid-expressed chemokine (ADEC), aFGF; FGF-1, AGF, AGF Protein, albumin, an etoposide, angiostatin, Anthrax vaccine, Antibodies specific for collapsin, antistasin, Anti-TGF beta family antibodies, antithrombin III, APM-1; ACRP-30; Famoxin, apo-lipoprotein species, , Arylsulfatase B, b57 Protein, BCMA, Betathromboglobulin protein (beta-TG), bFGF; FGF2, Blood coagulation factors, BMP Processing Enzyme Furin, BMP-10, BMP-12, BMP-15, BMP-17, BMP-18, BMP-2B, BMP-4, BMP-5, BMP-6, BMP-9, Bone Morphogenic Protein-2, calcitonin, Calpain-10a, Calpain-10b, Calpain-10c, Cancer Vaccine, Carboxypeptidase, C-C chemokine, MCP2, CCR5 variant, CCR7, CCR7, CD11a Mab, CD137; 4-1BB Receptor Protein, CD20 Mab, CD27, CD27L, CD30, CD30 ligand, CD33 immunotoxin, CD40, CD40L, CD52 Mab, Cerebus Protein, Chemokine Eotaxin., Chemokine hIL-8, Chemokine hMCP1, Chemokine hMCP1a, Chemokine hMCP1b, Chemokine hMCP2, Chemokine hMCP3, Chemokine hSDF1b, Chemokine MCP-4, chemokine TECK and TECK variant, Chemokine-like protein IL-8M1 Full-Length and Mature, Chemokine-like protein IL-8M10 Full-Length and Mature, Chemokine-like protein IL-8M3, Chemokine-like protein IL-8M8 Full-Length and Mature, Chemokine-like protein IL-8M9 Full-Length and Mature, Chemokine-like protein PF4-414 Full-Length and Mature, Chemokine-like protein PF4-426 Full-Length and Mature, Chemokine-like protein PF4-M2 Full-Length and Mature, Cholera vaccine, Chondromodulin-like protein, c-kit ligand; SCF; Mast cell growth factor; MGF; Fibrosarcoma-derived stem cell factor, CNTF and fragment thereof (such as CNTFAx15'(Axokine™)), , coagulation factors in both pre and active forms, collagens, Complement C5 Mab, Connective tissue activating protein-III, CTAA16.88 Mab, CTAP-III, CTLA4-Ig, CTLA-8, CXC3, CXC3, CXCR3; CXC chemokine receptor 3, cyanovirin-N, , Darbepoetin, designated exodus, designated huL105_7., DIL-40 , Dnase, EDAR, EGF Receptor Mab, ENA-78, Endostatin, Eotaxin, Epithelial neutrophil activating protein-78, EPO receptor; EPOR, erythropoietin (EPO) and EPO mimics, Eutropin , Exodus protein, Factor IX, , Factor VII, , Factor VIII, , Factor X and Factor XIII, FAS Ligand Inhibitory Protein (DcR3), FasL, FasL, FasL, FGF, FGF-12; Fibroblast growth factor homologous factor-1, FGF-15, FGF-16, FGF-18, FGF-3; INT-2, FGF-4; gelonin, HST-1; HBGF-4, FGF-5, FGF-6; Heparin binding secreted transforming factor-2, FGF-8, FGF-9; Glia activating factor, fibrinogen, , flt-1, flt-3 ligand, Follicle stimulating hormone Alpha subunit, Follicle stimulating hormone Beta subunit, Follitropin, Fractalkine, fragment. myofibrillar protein Troponin I, FSH, Galactosidase, Galectin-4, G-CSF, GDF-1, Gene therapy, Glioma-derived growth factor, glucagon, , glucagon-like peptides, Glucocerebrosidase, glucose oxidase, Glucosidase, Glycodelin-A; Progesterone-associated endometrial protein, GM-CSF, gonadotropin, Granulocyte chemotactic protein-2 (GCP-2), Granulocyte-macrophage colony stimulating factor, growth hormone, , Growth related oncogene-alpha (GRO-alpha), Growth related oncogene-beta (GRO-beta), Growth related oncogene-gamma (GRO-gamma), hAPO-4; TROY, hCG, Hepatitus B surface Antigen, Hepatitus B Vaccine, HER2 Receptor Mab, hirudin, HIV gp120, HIV gp41, HIV Inhibitor Peptide, HIV Inhibitor Peptide, HIV Inhibitor Peptide, HIV protease inhibiting peptides, HIV-1 protease inhibitors, HPV vaccine, Human 6CKine protein, Human Act-2 protein, Human adipogenesis inhibitory factor, human B cell stimulating factor-2 receptor, Human beta-chemokine H1305 (MCP-2), Human C-C chemokine DGWCC, Human CC chemokine ELC protein, Human CC type chemokine interleukin C, Human CCC3 protein, Human CCF18 chemokine, Human CC-type chemokine protein designated SLC (secondary lymphoid chemokine), Human chemokine beta-8 short forms, Human chemokine C10, Human chemokine CC-2, Human chemokine CC-3, Human chemokine CCR-2, Human chemokine Ckbeta-7, Human chemokine ENA-78, Human chemokine eotaxin, Human chemokine GRO alpha, Human chemokine GROalpha, Human chemokine GRObeta, Human chemokine HCC-1, Human chemokine HCC-1, Human chemokine I-309, Human chemokine IP-10, Human chemokine L105_3, Human chemokine L105_7, Human chemokine MIG, Human chemokine MIG-beta protein, Human chemokine MIP-1alpha, Human chemokine MIP1beta, Human chemokine MIP-3alpha, Human chemokine MIP-3beta, Human chemokine PF4, Human chemokine protein 331D5, Human chemokine protein 61164, Human chemokine receptor CXCR3, Human chemokine SDF1alpha, Human chemokine SDF1beta, Human chemokine ZSIG-35, Human Chr19Kine protein, Human CKbeta-9, Human CKbeta-9, Human CX3C 111 amino acid chemokine, Human DNAX interleukin-40, Human DVic-1 C-C chemokine, Human EDIRF I protein sequence, Human EDIRF II protein sequence, Human eosinocyte CC type chemokine eotaxin, Human eosinophil-expressed chemokine (EEC), Human fast twitch skeletal muscle troponin C, Human fast twitch skeletal muscle troponin I, Human fast twitch skeletal muscle Troponin subunit C, Human fast twitch skeletal muscle Troponin subunit I Protein, Human fast twitch skeletal muscle Troponin subunit T, Human fast twitch skeletal muscle troponin T, Human foetal spleen expressed chemokine, FSEC, Human GM-CSF receptor, Human gro-alpha chemokine, Human gro-beta chemokine, Human gro-gamma chemokine, Human IL-16 protein, Human IL-1RD10 protein sequence, Human IL-1RD9, Human IL-5 receptor alpha chain, Human IL-6 receptor, Human IL-8 receptor protein hIL8RA, Human IL-8 receptor protein hIL8RB, Human IL-9 receptor protein, Human IL-9 receptor protein variant #3, Human IL-9 receptor protein variant fragment, Human IL-9 receptor protein variant fragment#3, Human interleukin 1 delta, Human Interleukin 10, Human Interleukin 10, Human interleukin 18, Human interleukin 18 derivatives, Human interleukin-1 beta precursor, Human interleukin-1 beta precursor., Human interleukin-1 receptor accessory protein, Human interleukin-1 receptor antagonist beta, Human interleukin-1 type-3 receptor, Human Interleukin-10 (precursor), Human Interleukin-10 (precursor), Human interleukin-11 receptor, Human interleukin-12 40 kD subunit, Human interleukin-12 beta-1 receptor, Human interleukin-12 beta-2 receptor, Human Interleukin-12 p35 protein, Human Interleukin-12 p40 protein, Human interleukin-12 receptor, Human interleukin-13 alpha receptor, Human interleukin-13 beta receptor, Human interleukin-15, Human interleukin-15 receptor from clone P1, Human interleukin-17 receptor, Human interleukin-18 protein (IL-18), Human interleukin-3, human interleukin-3 receptor, Human interleukin-3 variant, Human interleukin-4 receptor, Human interleukin-5, Human interleukin-6, Human interleukin-7, Human interleukin-7., Human interleukin-8 (IL-8), Human intracellular IL-1 receptor antagonist, Human IP-10 and HIV-1 gp120 hypervariable region fusion protein, Human IP-10 and human Muc-1 core epitope (VNT) fusion protein, human liver and activation regulated chemokine (LARC), Human Lkn-1 Full-Length and Mature protein, Human mammary associated chemokine (MACK) protein Full-Length and Mature, Human mature chemokine Ckbeta-7, Human mature gro-alpha, Human mature gro-gamma polypeptide used to treat sepsis, Human MCP-3 and human Muc-1 core epitope (VNT) fusion protein, Human MI10 protein, Human MI1A protein, Human monocyte chemoattractant factor hMCP-1, Human monocyte chemoattractant factor hMCP-3, Human monocyte chemotactic proprotein (MCPP) sequence, Human neurotactin chemokine like domain, Human non-ELR CXC chemokine H174, Human non-ELR CXC chemokine IP10, Human non-ELR CXC chemokine Mig, Human PAI-1 mutants, Human protein with IL-16 activity, Human protein with IL-16 activity, Human secondary lymphoid chemokine (SLC), Human SISD protein, Human STCP-1, Human stromal cell-derived chemokine, SDF-1, Human T cell mixed lymphocyte reaction expressed chemokine (TMEC), Human thymus and activation regulated cytokine (TARC), Human thymus expressed, Human TNF-alpha, Human TNF-alpha, Human TNF-beta (LT-alpha), Human type CC chemokine eotaxin 3 protein sequence, Human type II interleukin-1 receptor, Human wild-type interleukin-4 (hIL-4) protein, Human ZCHEMO-8 protein, Humanized Anti-VEGF Antibodies, and fragments thereof, Humanized Anti-VEGF Antibodies, and fragments thereof, Hyaluronidase, ICE 10 kD subunit., ICE 20 kD subunit., ICE 22 kD subunit., Iduronate-2-sulfatase, Iduronidase, IL-1 alpha, IL-1 beta, IL-1 inhibitor (IL-1i)., IL-1 mature, IL-10 receptor, I L-11, IL-11, IL-12 p40 subunit., IL-13, IL-14, IL-15, IL-15 receptor, IL-17, IL-17 receptor, II-17 receptor, II-17 receptor, IL-19, IL-1i fragments, IL1-receptor antagonist, , IL-21 (TIF), IL-3 containing fusion protein., IL-3 mutant proteins, IL-3 variants, IL-3 variants, IL-4, IL-4 mutein, IL-4 mutein Y124G, IL-4 mutein Y124X, IL-4 muteins, II-5 receptor, IL-6, II-6 receptor, IL-7 receptor clone, IL-8 receptor, IL-9 mature protein variant (Met117 version), immunoglobulins or immunoglobulin-based molecules or fragment of either (e.g. a Small Modular ImmunoPharmaceutical™ ("SMIP") or dAb, Fab' fragments, F(ab')2, scAb, scFv or scFv fragment), including but not limited to plasminogen, Influenza Vaccine, Inhibin alpha, Inhibin beta, insulin, insulin-like growth factor, Integrin Mab, inter-alpha trypsin inhibitor, inter-alpha trypsin inhibitor, Interferon gamma-inducible protein (IP-10), interferons (such as interferon alpha species and sub-species, interferon beta species and sub-species, interferon gamma species and sub-species), interferons (such as interferon alpha species and sub-species, interferon beta species and sub-species, interferon gamma species and sub-species), Interleukin 6, Interleukin 8 (IL-8) receptor, Interleukin 8 receptor B, Interleukin-1alpha, Interleukin-2 receptor associated protein p43, interleukin-3, interleukin-4 muteins, Interleukin-8 (IL-8) protein., interleukin-9, Interleukin-9 (IL-9) mature protein (Thr117 version), interleukins (such as IL10, IL11 and IL2), interleukins (such as IL10, IL11 and IL2), Japanese encephalitis vaccine, Kalikrein Inhibitor, Keratinocyte growth factor, Kunitz domain protein (such as aprotinin, amyloid precursor protein and those described in WO 03/066824, with or without albumin fusions), Kunitz domain protein (such as aprotinin, amyloid precursor protein and those described in WO 03/066824, with or without albumin fusions), LACI, lactoferrin, Latent TGF-beta binding protein II, leptin, Liver expressed chemokine-1 (LVEC-1), Liver expressed chemokine-2 (LVEC-2), LT-alpha, LT-beta, Luteinization Hormone, Lyme Vaccine, Lymphotactin, Macrophage derived chemokine analogue MDC (n+1), Macrophage derived chemokine analogue MDC-eyfy, Macrophage derived chemokine analogue MDC-yl, Macrophage derived chemokine, MDC, Macrophage-derived chemokine (MDC), Maspin; Protease Inhibitor 5, MCP-1 receptor, MCP-1 a, MCP-1 b, MCP-3, MCP-4 receptor, M-CSF, , Melanoma inhibiting protein, Membrane-bound proteins, Met117 human interleukin 9, MIP-3 alpha, MIP-3 beta, MIP-Gamma, MIRAP, Modified Rantes, monoclonal antibody, MP52, Mutant Interleukin 6 S176R, myofibrillar contractile protein Troponin I, Natriuretic Peptide, Nerve Growth Factor-beta, Nerve Growth Factor-beta2, Neuropilin-1, Neuropilin-2, Neurotactin, Neurotrophin-3, Neurotrophin-4, Neurotrophin-4a, Neurotrophin-4b, Neurotrophin-4c, Neurotrophin-4d, Neutrophil activating peptide-2 (NAP-2), NOGO-66 Receptor, NOGO-A, NOGO-B, NOGO-C, Novel beta-chemokine designated PTEC, N-terminal modified chemokine GroHEK/hSDF-1 alpha, N-terminal modified chemokine GroHEK/hSDF-1 beta., N-terminal modified chemokine met-hSDF-1 alpha, N-terminal modified chemokine met-hSDF-1 beta, OPGL, Osteogenic Protein-1; OP-1; BMP-7, Osteogenic Protein-2, OX40; ACT-4, OX40L, Oxytocin (Neurophysin I), parathyroid hormone, , Patched, Patched-2, PDGF-D, Pertussis toxoid, Pituitary expressed chemokine (PGEC), Placental Growth Factor, Placental Growth Factor-2, Plasminogen Activator Inhibitor-1; PAI-1, Plasminogen Activator Inhibitor-2; PAI-2, Plasminogen Activator Inhibitor-2; PAI-2, Platelet derived growth factor, Platelet derived growth factor Bv-sis, Platelet derived growth factor precursor A, Platelet derived growth factor precursor B, Platelet Mab, platelet-derived endothelial cell growth factor (PD-ECGF), Platelet-Derived Growth Factor A chain, Platelet-Derived Growth Factor B chain, polypeptide used to treat sepsis, Preproapolipoprotein "milano" variant, Preproapolipoprotein "paris" variant, pre-thrombin, Primate CC chemokine "ILINCK", Primate CXC chemokine "IBICK", proinsulin, Prolactin, Prolactin2, prosaptide, Protease inhibitor peptides, Protein C, , Protein S, pro-thrombin, prourokinase, RANTES, RANTES 8-68, RANTES 9-68, RANTES peptide, RANTES receptor, Recombinant interleukin-16, Resistin, restrictocin, Retroviral protease inhibitors, ricin, Rotavirus Vaccine, RSV Mab, saporin, sarcin, Secreted and Transmembrane polypeptides, Secreted and Transmembrane polypeptides, serum cholinesterase, serum protein (such as a blood clotting factor), Soluble BMP Receptor Kinase Protein-3, Soluble VEGF Receptor, Stem Cell Inhibitory Factor, Straphylococcus Vaccine, Stromal Derived Factor-1 alpha, Stromal Derived Factor-1 beta, Substance P (tachykinin), T1249 peptide, T20 peptide, T4 Endonuclease, TACI, Tarc, TGF-beta 1, TGF-beta 2, Thr117 human interleukin 9, thrombin, , thrombopoietin, Thrombopoietin derivative1, Thrombopoietin derivative2, Thrombopoietin derivative3, Thrombopoietin derivative4, Thrombopoietin derivative5, Thrombopoietin derivative6, Thrombopoietin derivative7, Thymus expressed chemokine (TECK), Thyroid stimulating Hormone, tick anticoagulant peptide,, Tim-1 protein, TNF-alpha precursor, TNF-R, TNF-RII; TNF p75 Receptor; Death Receptor, tPA, transferrin, transforming growth factor beta, , Troponin peptides, Truncated monocyte chemotactic protein 2 (6-76), Truncated monocyte chemotactic protein 2 (6-76), Truncated RANTES protein (3-68), tumour necrosis factor, Urate Oxidase, urokinase, Vasopressin (Neurophysin II), VEGF R-3; flt-4, VEGF Receptor; KDR; flk-1, VEGF-110, VEGF-121, VEGF-138, VEGF-145, VEGF-162, VEGF-165, VEGF-182, VEGF-189, VEGF-206, VEGF-D, VEGF-E; VEGF-X, von Willebrand's factor, , Wild type monocyte chemotactic protein 2, Wild type monocyte chemotactic protein 2, ZTGF-beta 9.

### Chemotherapy drugs

13-cis-Retinoic Acid, 2-CdA, 2-Chlorodeoxyadenosine, 5-Azacitidine, 5-Fluorouracil, 5-FU, 6-Mercaptopurine, 6-MP, 6-TG, 6-Thioguanine, A, Abraxane, Accutane ®, Actinomycin-D, Adriamycin ®, Adrucil ®, Agrylin ®, Ala-Cort ®, Aldesleukin, Alemtuzumab, ALIMTA, Alitretinoin, Alkaban-AQ ®, Alkeran ®, All-transretinoic Acid, Alpha Interferon, Altretamine, Amethopterin, Amifostine, Aminoglutethimide, Anagrelide, Anandron ®, Anastrozole, Arabinosyl-cytosine, Ara-C, Aranesp ®, Aredia ®, Arimidex ®, Aromasin ®, Arranon ®, Arsenic Trioxide, Asparaginase, ATRA, Avastin ®, Azacitidine, BCG, BCNU, Bevacizumab, Bexarotene, BEXXAR ®, Bicalutamide, BiCNU, Blenoxane ®, Bleomycin, Bortezomib, Busulfan, Busulfex ®, C225 , Calcium Leucovorin, Campath ®, Camptosar ®, Camptothecin-11, Capecitabine, Carac ™, Carboplatin, Carmustine, Carmustine Wafer, Casodex ®, CC-5013, CCNU, CDDP, CeeNU, Cerubidine ®, Cetuximab, Chlorambucil, Cisplatin, Citrovorum Factor, Cladribine, Cortisone, Cosmegen ®, CPT-11, Cyclophosphamide, Cytadren ®, Cytarabine, Cytarabine Liposomal, Cytosar-U ®, Cytoxan ®, Dacarbazine, Dacogen, Dactinomycin, Darbepoetin Alfa, Dasatinib, Daunomycin, Daunorubicin, Daunorubicin Hydrochloride, Daunorubicin Liposomal, DaunoXome ®, Decadron, Decitabine, Delta-Cortef ®, Deltasone ®, Denileukin diftitox, DepoCyt ™, Dexamethasone, Dexamethasone acetate , Dexamethasone Sodium Phosphate , Dexasone, Dexrazoxane, DHAD, DIC, Diodex, Docetaxel, Doxil ®, Doxorubicin, Doxorubicin liposomal, Droxia ™, DTIC, DTIC-Dome ®, Duralone ®, Efudex ®, Eligard ™, Ellence ™, Eloxatin ™, Elspar ®, Emcyt ®, Epirubicin, Epoetin alfa, Erbitux ™, Erlotinib, Erwinia L-asparaginase, Estramustine, Ethyol , Etopophos ®, Etoposide, Etoposide Phosphate, Eulexin ®, Evista ®, Exemestane, Fareston ®, Faslodex ®, Femara ®, Filgrastim, Floxuridine, Fludara ®, Fludarabine, Fluoroplex ®, Fluorouracil, Fluorouracil (cream), Fluoxymesterone, Flutamide, Folinic Acid, FUDR ®, Fulvestrant, G-CSF, Gefitinib, Gemcitabine, Gemtuzumab ozogamicin, Gemzar ®, Gleevec ™, Gliadel ® Wafer, GM-CSF, Goserelin, Granulocyte - Colony Stimulating Factor, Granulocyte Macrophage Colony Stimulating Factor, Halotestin ®, Herceptin ®, Hexadrol, Hexalen ®, Hexamethylmelamine, HMM, Hycamtin ®, Hydrea ®, Hydrocort Acetate ®, Hydrocortisone, Hydrocortisone Sodium Phosphate, Hydrocortisone Sodium Succinate, Hydrocortone Phosphate, Hydroxyurea, Ibritumomab, Ibritumomab Tiuxetan , Idamycin ®, Idarubicin, Ifex ®, IFN-alpha , Ifosfamide, IL-11 , IL-2 , Imatinib mesylate, Imidazole Carboxamide , Interferon alfa, Interferon Alfa-2b (PEG Conjugate), Interleukin - 2, Interleukin-11, Intron A® (interferon alfa-2b), Iressa ®, Irinotecan, Isotretinoin, Kidrolase ®, Lanacort ®, Lapatinib, L-asparaginase, LCR, Lenalidomide, Letrozole, Leucovorin, Leukeran, Leukine ™, Leuprolide, Leurocristine, Leustatin ™, Liposomal Ara-C, Liquid Pred ®, Lomustine, L-PAM, L-Sarcolysin, Lupron ®, Lupron Depot ®, M, Matulane ®, Maxidex, Mechlorethamine, Mechlorethamine Hydrochloride, Medralone ®, Medrol ®, Megace ®, Megestrol, Megestrol Acetate, Melphalan, Mercaptopurine, Mesna, Mesnex ™, Methotrexate, Methotrexate Sodium, Methylprednisolone, Meticorten ®, Mitomycin, Mitomycin-C, Mitoxantrone, M-Prednisol ®, MTC, MTX, Mustargen ®, Mustine , Mutamycin ®, Myleran ®, Mylocel ™, Mylotarg ®, Navelbine ®, Nelarabine, Neosar ®, Neulasta ™, Neumega ®, Neupogen ®, Nexavar ®, Nilandron ®, Nilutamide, Nipent ®, Nitrogen Mustard, Novaldex ®, Novantrone ®, Octreotide, Octreotide acetate, Oncospar ®, Oncovin ®, Ontak ®, Onxal ™, Oprevelkin, Orapred ®, Orasone ®, Oxaliplatin, Paclitaxel, Paclitaxel Protein-bound, Pamidronate, Panitumumab, Panretin ®, Paraplatin ®, Pediapred ®, PEG Interferon, Pegaspargase, Pegfilgrastim, PEG-INTRON ™, PEG-L-asparaginase, PEMETREXED, Pentostatin, Phenylalanine Mustard, Platinol ®, Platinol-AQ ®, Prednisolone, Prednisone, Prelone ®, Procarbazine, PROCRIT ®, Proleukin ®, Prolifeprospan 20 with Carmustine Implant, Purinethol ®, R, Raloxifene, Revlimid ®, Rheumatrex ®, Rituxan ®, Rituximab, Roferon-A ® (Interferon Alfa-2a), Rubex ®, Rubidomycin hydrochloride, Sandostatin ®, Sandostatin LAR ®, Sargramostim, Solu-Cortef ®, Solu-Medrol ®, Sorafenib, SPRYCEL ™, STI-571, Streptozocin, SU11248, Sunitinib, Sutent ®, Tamoxifen, Tarceva ®, Targretin ®, Taxol ®, Taxotere ®, Temodar ®, Temozolomide, Teniposide, TESPA, Thalidomide, Thalomid ®, TheraCys ®, Thioguanine, Thioguanine Tabloid ®, Thiophosphoamide, Thioplex ®, Thiotepa, TICE ®, Toposar ®, Topotecan, Toremifene, Tositumomab, Trastuzumab, Tretinoin, Trexall ™, Trisenox ®, TSPA, TYKERB ®, VCR, Vectibix ™, Velban ®, Velcade ®, VePesid ®, Vesanoid ®, Viadur ™, Vidaza ®, Vinblastine, Vinblastine Sulfate, Vincasar Pfs ®, Vincristine, Vinorelbine, Vinorelbine tartrate, VLB, VM-26, Vorinostat, VP-16, Vumon ®, Xeloda ®, Zanosar ®, Zevalin ™, Zinecard ®, Zoladex ®, Zoledronic acid, Zolinza, Zometa ®.

### Radiopharmaceuticals

Carbon-11, Carbon-14, Chromium-51, Cobalt-57, Cobalt-58, Erbium-169, Fluorine-18, Gallium-67, Gold-198, Indium-111, Indium-113m, Iodine-123, Iodine-125, Iodine-131, Iron-59, Krypton-81 m, Nitrogen-13, Oxygen-15, Phosphorous-32, Rhenium-186, Rubidium-82, Samarium-153, Selenium-75, Strontium-89, Technetium-99m, Thallium-201, Tritium, Xenon-127, Xenon-133, Yttrium-90.

### Imaging agents

Gadolinium, magnetite, manganese, technetium, I125, I131, P32, TI201, lopamidol, PET-FDG.

### Purification tags

(Ala-Trp-Trp-Pro) n, avidin/streptavidin/Strep-tag, BCCP, B-tag (VP7 protein region of bluetongue virus), calmodulin binding protein (CBP), cellulose binding domains (CBD's), chitin binding domain, chloramphenicol acetyltransferase, c-myc, dihydrofolate reductase (DHFR), FLAG™ peptide (DYKDDDDK), galactose-binding protein, glutathione-S-transferase (GST), green flourescent protein (GFP), Growth hormone, N-terminus, hemagglutinin influenza virus (HAI), His-patch thioredoxin, His-tag, HSB-tag, KSI, lacZ (β-Galactosidase), maltose binding protein (MBP), NusA, ompT/ompA/peIB/DsbA/DsbC, polyarginine, polyaspartic acid, polycysteine, polyphenyalanine, S-tag, staphylococcal protein A, streptococcal protein G, T4 gp55, T7gene10, T7-tag, thioredoxin, trpE, ubiquitin.

### Diagnostic compounds

The use of diagnostic agents or biological "contrast" agents are well known to the art. A diagnostic agent is any pharmaceutical product used as part of a diagnostic test (i.e. together with the equipment and procedures that are needed to assess the test result). The diagnostic agent may be used in vivo, ex vivo or in vitro. For example, US 4,945,239 describes a procedure developed for detecting the presence of breast cancer cells by using a transillumination imaging device. It is known that normal breast cells and benign breast tumor cells have no expression of transferrin receptors whereas breast carcinomas have a very high expression of transferrin receptors. Human transferrin has been chemically modified in such a way that it developed a strong absorption in the visible region of electromagnetic radiation, where most human proteins, fatty acids and other body fluids do not significantly absorb light. More particularly, fluorescein has been covalently coupled with transferrin using isothiocyanate coupling method. The conjugated protein (FITC-Tf) has a very strong absorption at 496 nm. In a preliminary study, it was demonstrated the FITC-Tf conjugate can be used for detecting cancer cells. EMT-6 mouse mammary tumor cells were grown in an RPMI 1640 culture medium containing 10% fetal bovine serum in a carbon dioxide incubator and maintained in log phase grown by frequent splitting. For the experiments, the cells were trypsinized and washed with phosphate buffered saline (PBS), pH 7.4, and incubated in PBS for 30 minutes at 37.°C. to remove bound transferrin from the cell surface transferrin receptors. The cells were then centrifuged at 500 g for 5 minutes and washed once more with PBS. In each of two 15 ml conical centrifuge tubes, one million cells were suspended in 10 ml of PBS, pH 7.4, and 1.0 ml of 2.0 mg/ml FITC-Tf or transferrin was added to each tube. These tubes were incubated at 4. °C. for 45 minutes. The cells were then washed twice with PBS to remove unbound material. The washed pellets were then suspended in 0.2 ml of PBS, and the cells were transferred to a 96-well clear plastic titer plate, available from the Dynatech company, for imaging purposes.

### Alignment and identity

The HST variant may have at least 40 % identity with SEQ ID NO: 1, particularly at least 45%, 50%, 55%, 60%, 65%, 70%, 75 %, at least 80 %, at least 85 %, at least 90 %, at least 95 %, at least 97 %, at least 98 % or at least 99 % identity.

The Lactoferrin variant may have at least 40 % identity with SEQ ID NO: 11, particularly at least 45%, 50%, 55%, 60%, 65%, 70%, 75 %, at least 80 %, at least 85 %, at least 90 %, at least 95 %, at least 97 %, at least 98 % or at least 99 % identity.

The melanotransferrin variant may have at least 40 % identity with SEQ ID NO: 15, particularly at least 45%, 50%, 55%, 60%, 65%, 70%, 75 %, at least 80 %, at least 85 %, at least 90 %, at least 95 %, at least 97 %, at least 98 % or at least 99 % identity.

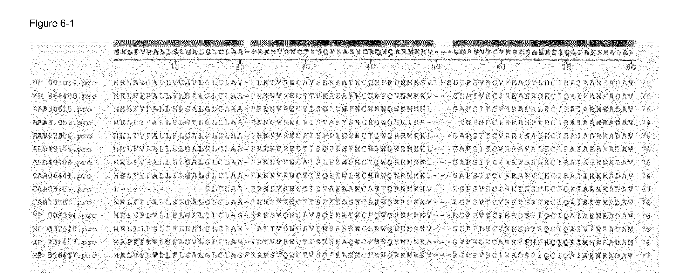

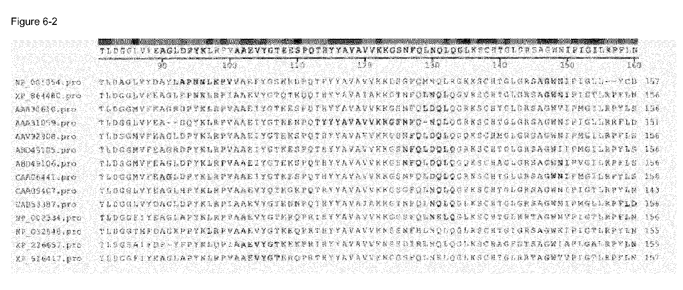

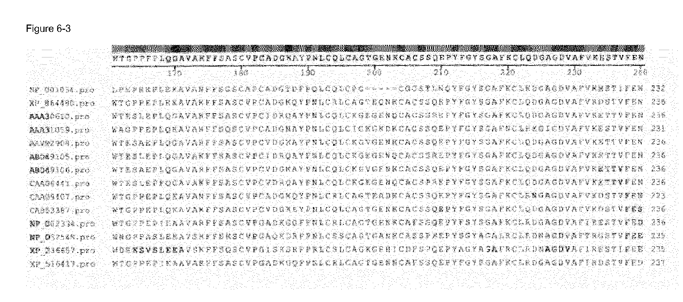

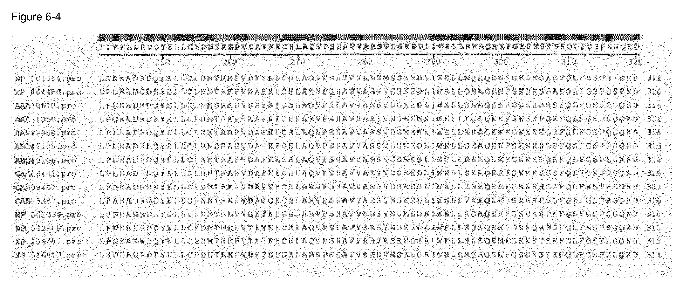

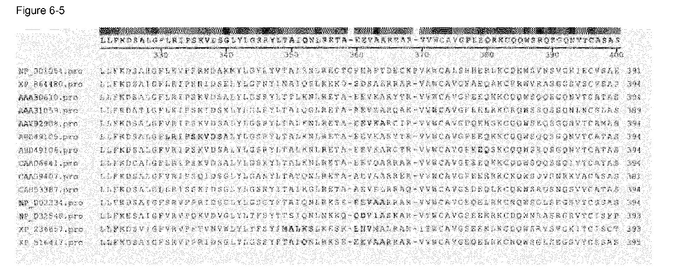

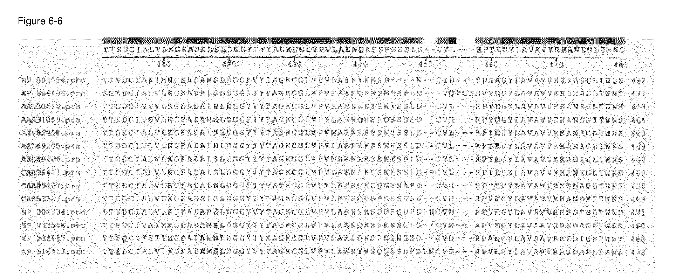

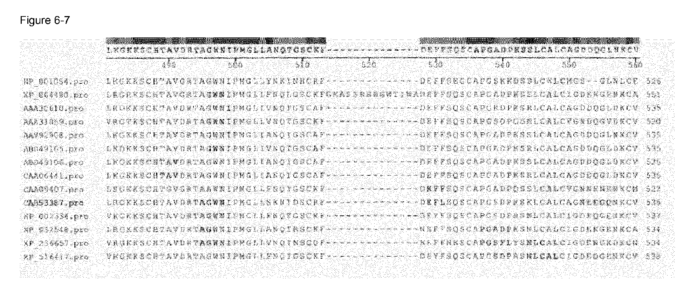

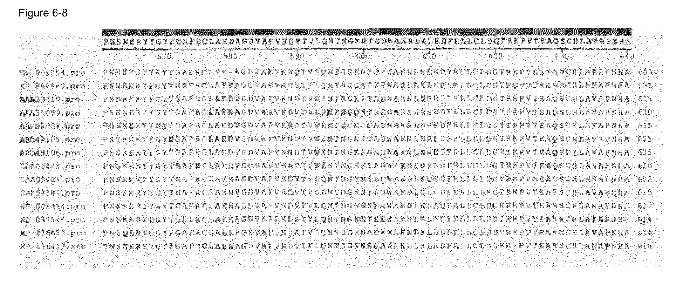

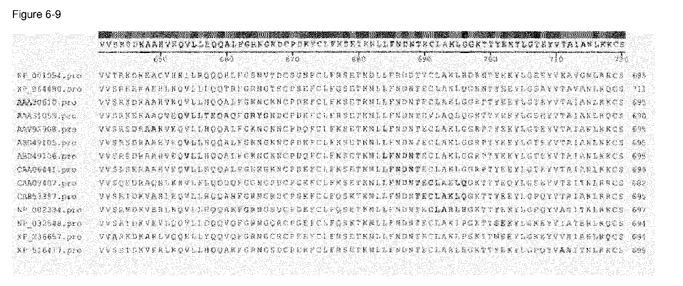

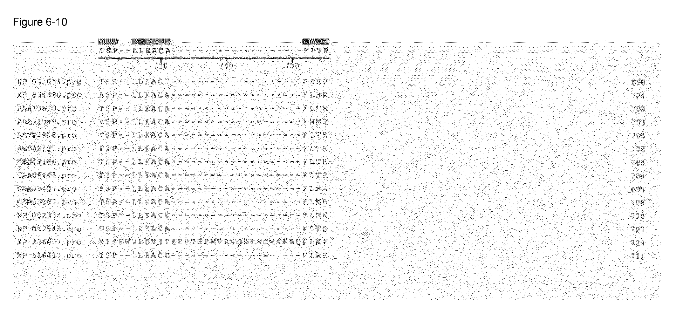

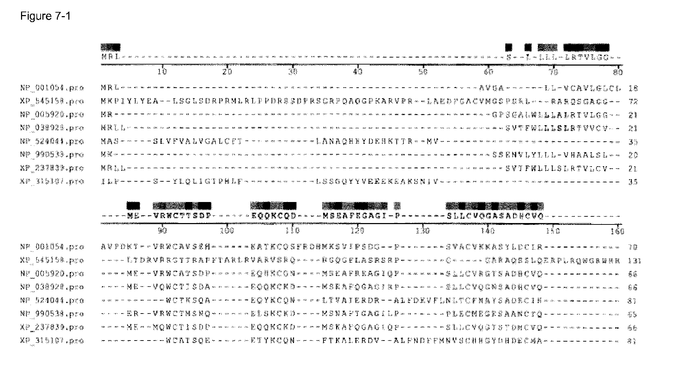

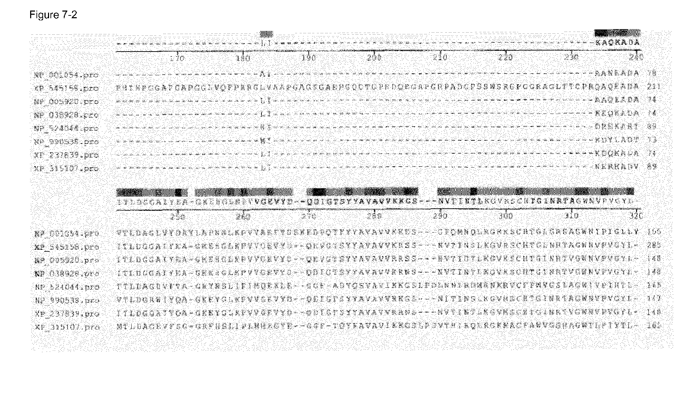

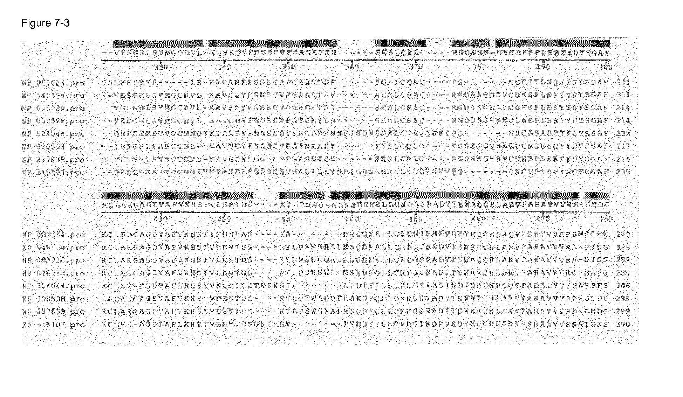

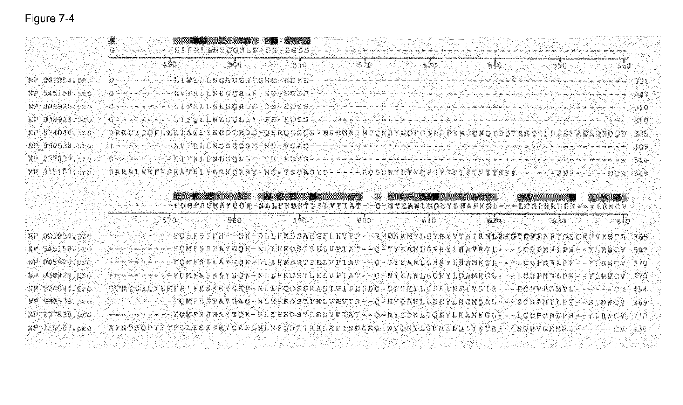

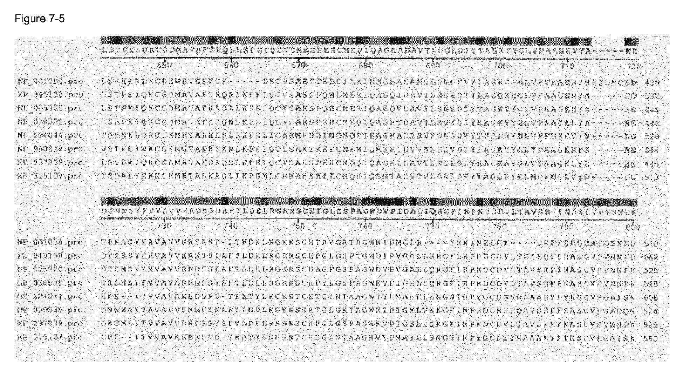

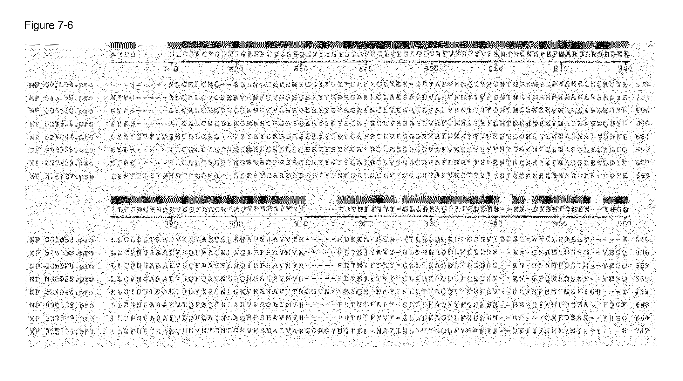

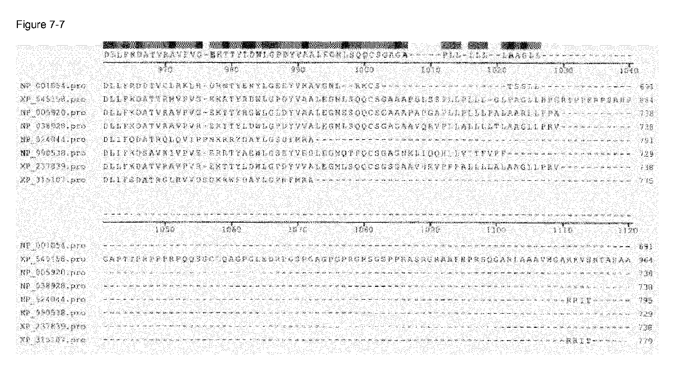

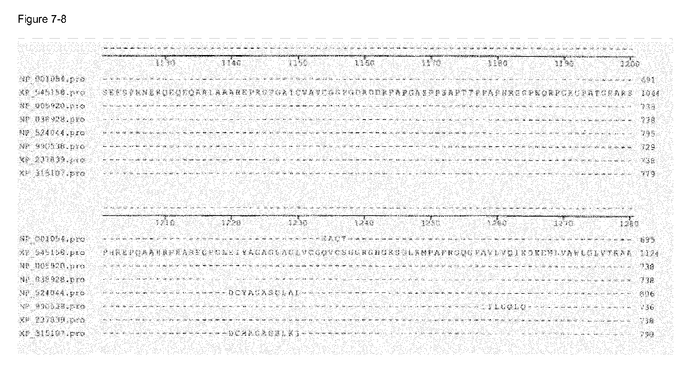

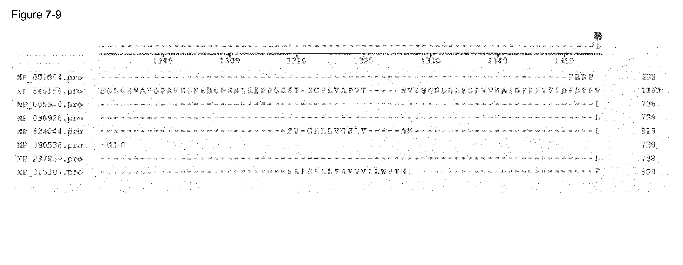

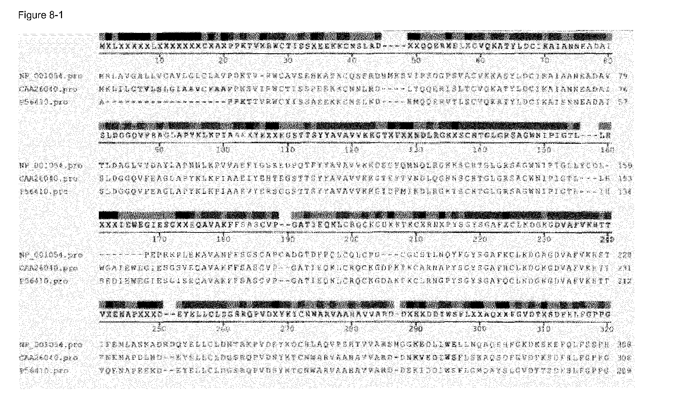

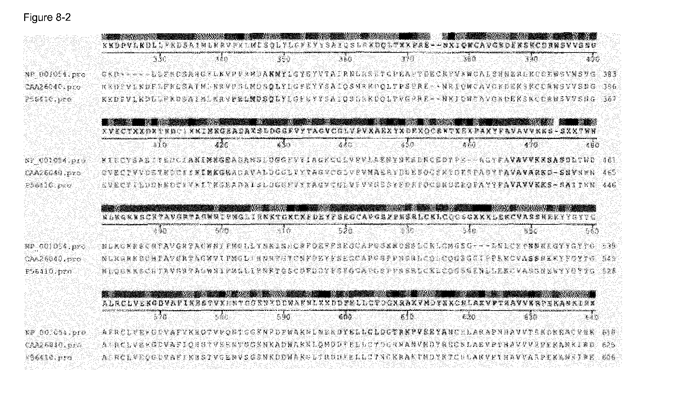

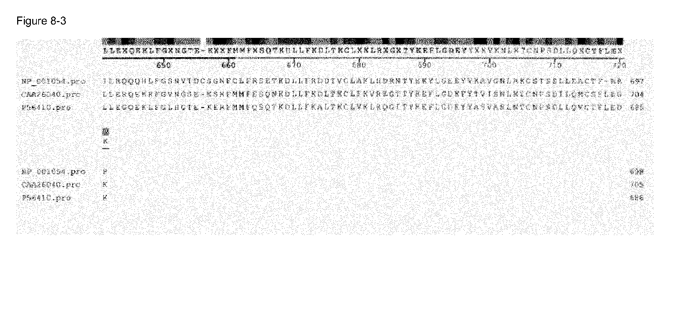

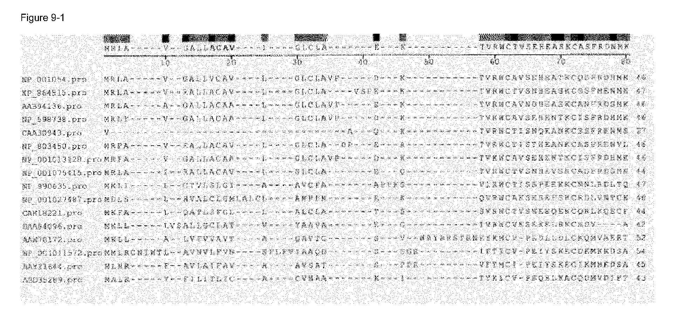

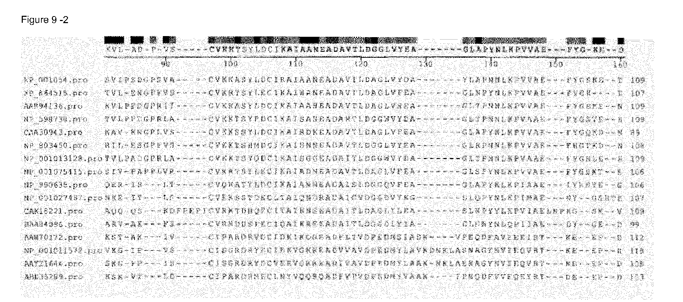

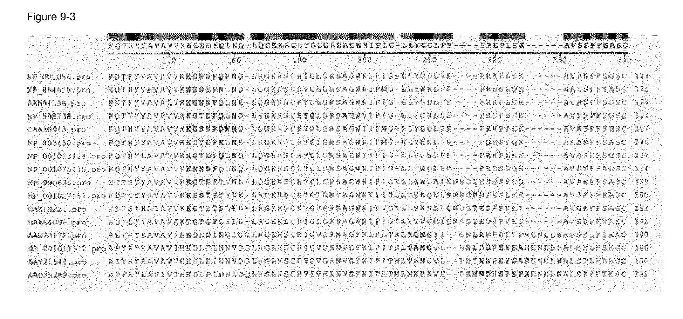

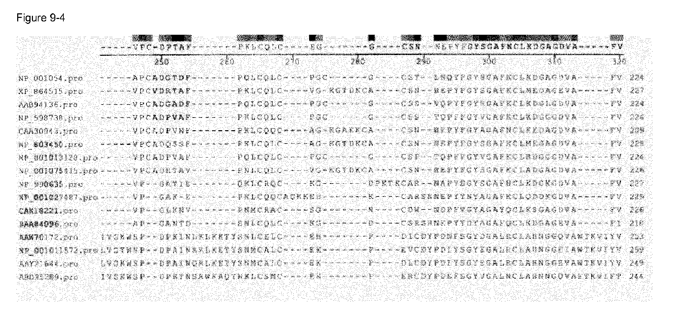

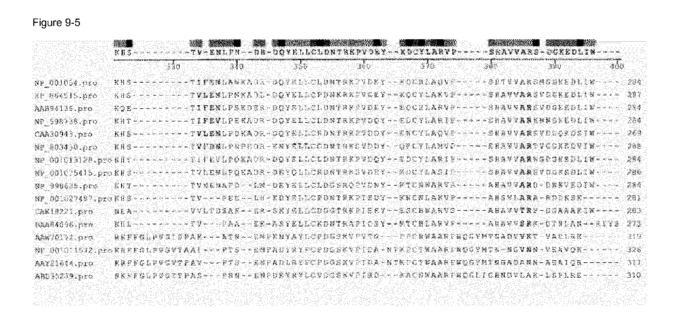

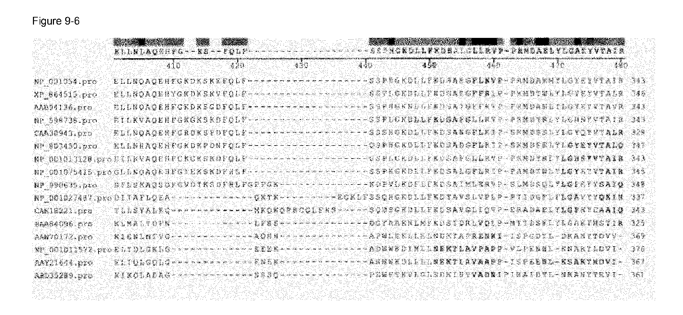

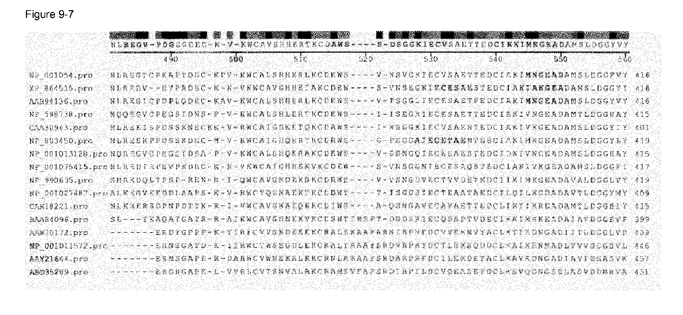

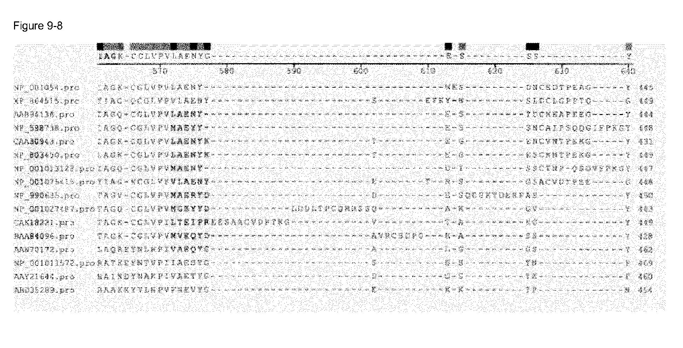

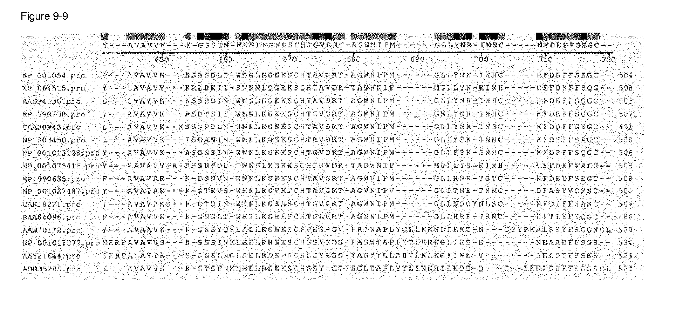

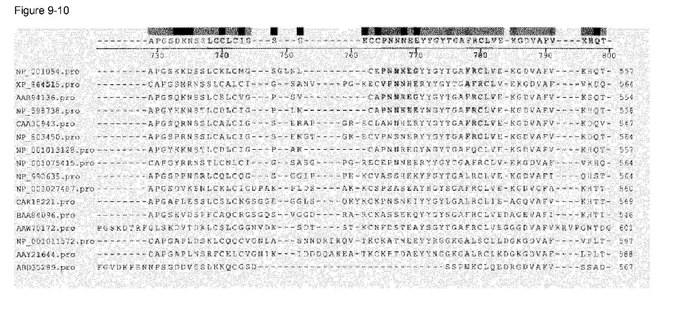

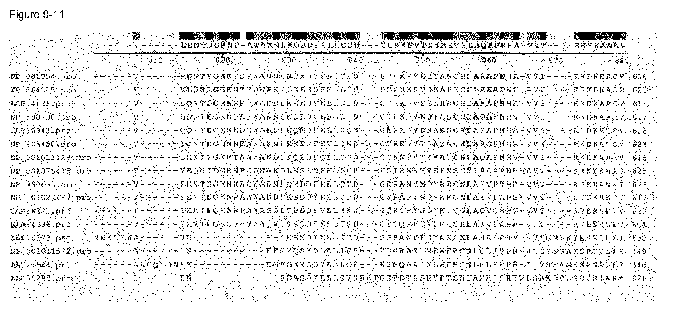

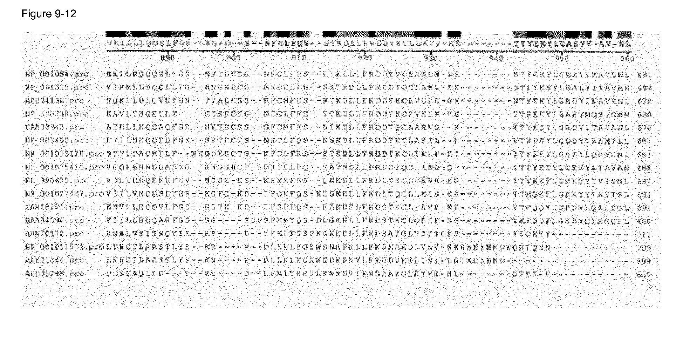

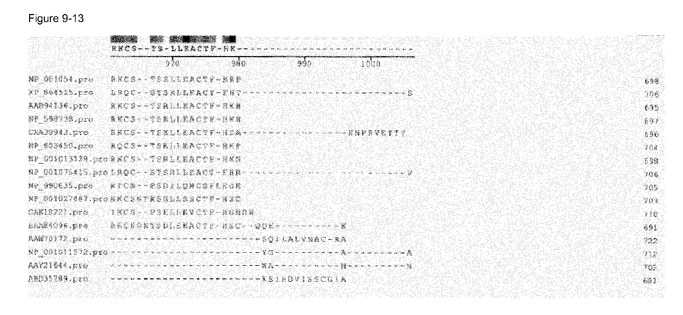

Figures 6-9 show alignments of a number of transferrin family proteins with HST (SEQ ID NO: 1). A structural alignment of human lactoferrin with HST is described by J. Wally et al., Biometals (2007) 20:249-262. These alignments can be used to identify regions and amino acid residues corresponding to those in HST selected as described above. For other lactoferrin family proteins, primary sequence alignment can be performed by a number of procedures known to the art.

An example of such a procedure is the MegAlign program (version 7) developed by DNASTAR Inc., part of the Lasergene suite, based on Hein, J.J. (1990). "Unified approach to alignment and phylogenies." In Methods in Enzymology, Vol. 183: pp. 626-645. Using the Jotun Hein Method and the settings GAP PENALTY = 11, GAP LENGTH PENALTY = 3 for multiple alignments and KTUPLE = 2 for pairwise alignments a series of percentage identity values can be calculated.

The alignment of two amino acid sequences may also be determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between a given amino acid sequence and SEQ ID NO: 1 is calculated as the number of exact matches in an alignment of the two sequences, divided by the length of the shorter of the two sequences. The result is expressed in percent identity. An exact match occurs when the two sequences have identical amino acid residues in the same positions of the overlap. The length of a sequence is the number of amino acid residues in the sequence.

### Mobility (RMSF) of residues in 3D model

The root mean square fluctuations of the C-alpha carbon atoms during the last nanosecond of the simulation were calculated using the Gromacs tool "g_rmsf", version 3.3, based on D. van der Spoel, E. Lindahl, B. Hess, G. Groenhof, A. E. Mark and H. J. C. Berendsen: GROMACS: Fast, Flexible and Free, J. Comp. Chem. 26 pp. 1701-1718 (2005).

### Solvent accessibility of residues in 3D model

The solvent accessible surface area is calculated for each residue using the DSSP software (W.Kabsch and C.Sander, Biopolymers 22 (1983) 2577-2637). Each solvent accessible surface area is divided by a standard value for the particular amino acid found in that position and multiplied by 100, thereby obtaining a percentage of the standard value for each residue.

The standard solvent accessible surface areas for the 20 different amino acids are defined as (using one-letter codes for the amino acids): A=62, C=92, D=69, E=156, F=123, G=50, H=130, I=84, K=174, L=97, M=103, N=85, P=67, Q=127, R=211, S=64, T=80, V=81, W=126, Y=104.

### Ligand binding

The polypeptide may include insertions, deletions and substitutions, either conservative or non-conservative, where such changes do not substantially reduce the useful ligand-binding, immunological or receptor binding properties of transferrin. The polypeptide may have at least 5%, 10%, 15%, 20%, 30%, 40% or 50%, 60%, 70%, at least 80%, 90%, 95%, 100%, 105% or moreof human transferrin's receptor binding activity, mole for mole. The polypeptide may have increased affinity for the receptor or reduced release of iron (Adams et al., 2003 J Biol Chem, 278 (8), 6027-33; Baker et al., 2007 Acta Crystallogr D Biol Crystallogr, 63 (Pt 3), 408-14; He and Mason, 2002 Molecular and Cellular Iron Transport, 5-123; Mason et al., 2005 Biochemistry, 44 (22), 8013-21.).

The polypeptide may display modified (e.g. reduced) glycosylation, such as, but not limited to reduced N-linked glycosylation or reduced O-linked glycosylation. The N-linked glycosylation pattern of a transferrin molecule can be modified by adding/removing amino acid glycosylation consensus sequences such as N-X-S/T, at any or all of the N, X, or S/T position. Transferrin mutants may have altered ability to release iron and/or altered recycling time such that the efficacy of a mutant as a bioactive carrier is improved (Lao et al., 2007 J Control Release, 117 (3), 403-12.). Transferrin mutants may be altered in their natural binding to metal ions and/or other proteins, such as transferrin receptor. An example of a transferrin mutant modified in this manner is exemplified below.

We also include naturally-occurring polymorphic variants of human transferrin or human transferrin analogues. Generally, variants or fragments of human transferrin will have at least 5%, 10%, 15%, 20%, 30%, 40%, 50%, 60%, 70%, (preferably at least 80%, 90%, 95%, 100%, 105% or more) of human transferrin's ligand binding activity (for example iron-binding), mole for mole.

### Iron binding assay

Iron binding capacity refers to the ability of a recombinant protein comprising the sequence of a transferrin mutant to reversibly bind iron. Transferrins devoid of iron are colourless, but when iron is bound they are characteristic salmon-pink colour. Iron binding capacity can therefore be determined spectrophotometrically by 470nm:280nm absorbance ratios for the proteins in their iron-free and fully iron-loaded states. A thiotransferrin is considered to have iron binding capacity if it has at least 5 % of the iron binding capacity of the corresponding polypeptide without Cysteine insertion.

Reagents should be iron-free unless stated otherwise. Iron can be removed from transferrin or the test sample by dialysis against 0.1 M citrate, 0.1 M acetate, 10 mM EDTA pH4.5. The protein should be at approximately 20 mg/mL in 100 mM HEPES, 10 mM Na-HCO₃ pH8.0. Measure the 470nm:280nm absorbance ratio of apo-transferrin (i.e. iron-free control transferrin) (Calbiochem, CN Biosciences, Nottingham, UK) diluted in water so that absorbance at 280nm can be accurately determined spectrophotometrically (0% iron binding). Prepare 20 mM iron-nitrilotriacetate (FeNTA) solution by dissolving 191 mg nitrotriacetic acid in 2 mL 1 M NaOH, then add 2 mL 0.5 M ferric chloride. Dilute to 50 mL with deionised water. Fully load apo-(control) transferrin with iron (100% iron binding) by adding a sufficient excess of freshly prepared 20 mM FeNTA, then dialyse the holo-transferrin preparation completely against 100 mM HEPES, 10m M NaHCO₃ pH 8.0 to remove remaining FeNTA before measuring the absorbance ratio at 470nm:280nm. Repeat the procedure using test sample (i.e. the recombinant protein comprising the sequence of a transferrin mutant in question), which should initially be free from iron, and compare final ratios to the control.

### Procedure for quantitation of Transferrin Total Iron Binding Capacity (TIBC)

Total iron-binding capacity (TIBC) indicates the maximum amount of iron necessary to saturate all available transferrin iron-binding sites. Measurements of TIBC, serum iron, and the ratio of serum iron to TIBC (transferrin saturation) are used routinely for clinical diagnosis and monitoring (Huebers, H. A. et al. (1987), Clin Chem 33, 273-7. The total iron binding capacity of rTf can be determined using a modified method for Determination of Serum Iron and Iron-Binding Capacity described by Caraway, 1963 Clinical Chem 9(2), 188. The rTf was diluted to 5mg.mL⁻¹, prior to addition of 1 mL of Total Iron Binding Buffer (0.5M Tris pH8, 0.5M NaHCO₃). To each tube 0.220g ± 0.005g magnesium carbonate was added (to remove excess iron), and mixed for 45 minutes using a rock-roll mixer. Samples were centrifuged at 5000 rpm for 25 minutes at 17°C. The supernatants (650 µl) containing transferrin bound iron were transferred to receiving reservoirs of ultrafree®-MC 0.45µ microfuge spin filters, and centrifuged at 8000 rpm for 10 minutes. The receiving reservoirs were removed and vortexed to mix. The samples divided into two aliquots, 100 µµl of each sample was transferred to microfuge tubes for iron analysis, and the remaining 150 µl of each sample was transferred into 200 µl crimp top HPLC vials (02-CTV, Chromacol Ltd) so that rTF concentration could be measured by RP-HPLC (described in purification section).

Total iron in protein samples can be measured using ferene-S a colorimetric reagent for iron and by monitoring the change in absorbance at 595 nm.

A 10 mg.L⁻¹ iron working stock standard was prepared by addition of 50 µl iron atomic spectroscopy standard to 4.95 mL water. Iron standards were prepared at 1, 2, 3, 4, 5 and 10 mg.L⁻¹ in a final volume of 100 µl, so that a standard curve could be created. To each of the standards and 50 µl 1.3M ascorbic acid was added, samples were mixed and incubated at room temperature. After 10 minutes, 50 µl 20% (w/v) trichloroacetic acid was added to all samples. 100 µl of the standards were transferred to 10 x 4 x 45 mm plastic cuvettes and 100 µl of ammonium acetate was added, followed by 100 µl 22.2nmol.L⁻¹ ferene-S solution and 700 µl of water added. The standards were incubated at room temperature for 10 minutes, prior to measuring the absorbance at 595 nm (using Shimadzu UV2501 spectrophotometer or equivalent). A standard curve was constructed of absorbance at 595 nm against iron concentration mg.L⁻¹.

Simultaneously, 50 µl 1.3M ascorbic acid was added to the rTf samples (100 µl), samples were mixed and incubated at room temperature. After 10 minutes, 50 µl 20% (w/v) trichloroacetic acid was added and 50 µl chloroform was added to samples containing protein. All samples were mixed by vortexing and were centrifuged at 14,000 rpm for 3 minutes. The supernatants (100 µl) were transferred to 10 x 4 x 45 mm plastic cuvettes and 100 µl of 40% (w/v) ammonium acetate was added, followed by 100 µl 22.2nmol.L⁻¹ ferene-S solution and 700 µl of water added. The standards were incubated at room temperature for 10 minutes, prior to measuring the absorbance at 595 nm (using Shimadzu UV2501 spectrophotometer or equivalent). The iron concentration mg.L⁻¹ was calculated using the standard curve, and the iron content (mg.g protein ⁻¹) was calculated by dividing the iron concentration by the protein concentration (g.L⁻¹).

### Conjugation

The transferrin mutein (thiotransferrin) of the invention can be covalently linked to the bioactive compound by methods known to the art (http://www.piercenet.com/files/1601361 Crosslink.pdf). These include, but are not limited to incorporating or engineering a thiol reactive group into or onto the bioactive compound, for example by incorporating or engineering another free thiol present on the bioactive compound; or by incorporating or engineering a pyridyl disulphide group on the bioactive compound; or by incorporating or engineering an iodoacetyl group on the bioactive compound or or by incorporating or engineering a maleimide group on the bioactive compound. For example, N-ethylmaleimide (NEM, Pierce), 2-amino-2'-aminoethanethiolsulfonate (Pierce), N-beta-maleimidoprpionic acid (BMPA Pierce), methyl methane thiosulfonate (MMTS, Pierce), fluorescein-5-maleimide (Pierce), 5-iodoacetamido-fluorescein (5-IAF, Pierce) or N-[6-7-amino-4-methylcoumarin-3-acetamido) hexyl]-3'-[2'-pyridyldithio] propionamide (AMCA-HPDP, Pierce).

If the bioactive compound contains at least one thiol group, then the bioactive compound can be cross-linked to the transferrin mutein of the invention by methods known to the art such as, but not limited to, oxidation or by the use of cross-linking reagents such as, but not limited to, 1,4-Bis-maleimidibutane (BMB, Pierce); 1,4-Bis-maleimidyl-2,3-dihydroxybutane (BMDB, Pierce); Bis-maleimidohexane (BMH, Pierce), Bis-maleimidoethane (BMOE, Pierce); 1,8-Bis-Maleimidotriethyleneglycol (BM[PEO]3 Pierce); 1,11-Bis-Maleimidotetraethyleneglycol (BM[PEO]4 Pierce); 1,4-Di-[3'-(2'-pyridyldithio)-propionamido]butane (DPDPB, Pierce); dithuio-bis-maleimidoethane (DTME Pierce); 1,6-Hexane-bis-vinylsulfone (HBVS, Pierce) and Tris-[2-maleimimidoethyl]amine (TMEA, Pierce).

If the bioactive compound does not contain a thiol reactive group then it can be modified to incorporate one or more such groups by either chemical modification or genetic engineering by methods know to the art (Chapman, A.P. (2002) Adv. Drug Deliv. Rev., 54 531-545: Humphreys, D.P. et al. Protein Engineering, Design & Selection vol. 20 no. 5 pp. 227-234, 2007). While these two references describe methodologies to cross-link PEG to an engineered free thiol within an antibody or antibody fragment, the techniques can be used to cross-link an bioactive compound to an engineered free thiol within the transferrin mutein of the invention. Alternatively the Drug Affinity Complex (DAC™) technology developed by ConjuChem Inc. (Montreal, Quebec, Canada, H2X 3Y8) can be used, e.g. as described in WO200069902. There are three parts of each DAC™ construct: 1) the drug component (the portion responsible for biologic activity); 2) a linker attached to the drug component, and 3) a reactive chemistry group at the opposite end of the linker, usually a soft electrophile selective for thiols; a maleimide is the most useful embodiment.

If the bioactive compound does not contain a thiol reactive group but does contain one or more amino groups then it can be modified to incorporate one or more thiol reactive groups by chemical modification by methods known to the art such as the use of cross-linking reagents such as, but not limited to, N-5-azido-2-nitrobenzoyloxysuccinimide (AMAS, Pierce), N-[beta-maleimidopropyloxy] succinimide ester (BMPS, Pierce), N-eta-maleimidocaproic acid (EMCA, Pierce), N-[eta-maleimidocaproyloxy]succinimide ester (EMCS, Pierce), N-[etamaleimidocaproyloxy]sulfosuccinimide ester (sulfo-EMCS, Pierce), N-[gamma-maleimidobutyryloxy]succinimide ester (GMBS, Pierce), N-[gamma-maleimidobutyryloxy]sulfosuccinimide ester (sulfo-GMBS, Pierce), N-kappa-maleimidoundecanoic acid (KMUA, Pierce), N-[kappa -maleimidoundecanoic acid]hydrazide (KMUH, Pierce), N-[kappa -maleimidoundecanoyloxy]sulfosuccinimide ester (sulfo-KMUS, Pierce), m-maleimidobenzoyl-N-hydroxysuccinimide (MBS, Pierce), m-maleimidobenzoyl-N-hydroxysulfosuccinimide ester (sulfo-MBS, Pierce), N-succinimidyl S-acetylthioacetate (SA-TA, Pierce), N-succinimidyl S-acetylthiopropionate (SATP, Pierce), succinimidyl 3-[bromoacetamido]propionate (SBAP, Pierce), N-succinimidyl iodoacetate (SIA, Pierce), N-succinimidyl[4-iodoacetyl]aminobenzoate (SIAB, Pierce), sulfosuccinimidyl[4-iodoacetyl]aminobenzoate (sulfo-SIAB, Pierce), succinimidyl [4-[N-maleimidomethyl]cyclohexane-1-carboxylate (SMCC, Pierce), sulfosuccinimidyl [4-[N-maleimidomethyl]cyclohexane-1-carboxylate (sulfo-SMCC, Pierce), succinimidyl-[4-[N-maleimidomethyl]cyclohexane-1-carboxy-[6-amidocaproate (LC-SMCC, Pierce), 4-succinimidyloxycarbonyl-methyl-alpha[2-pyridyldithio]toluene (SMPT, Pierce), sulfosuccinimidyl-6-[alpha-methyl-alpha2-pyridyldithio)toluamido]hexanoate (sulfo-LC-SMPT, Pierce), succinimidyl 4-[p-maleimidophenyl]-butyrate (SMPB, Pierce), sulfosuccinimidyl 4-[p-maleimidophenyl]-butyrate (sulfo-SMPB, Pierce), succinimidyl-6-[(beta-maleimidopropionamido)hexanoate] (SMPH, Pierce), N-succinimidyl 3-[2-pyridyldithio]propionate (SPDP, Pierce), succinimidyl [3-(2-pyridyldithio)propionamido]hexanoate (LC-S P D P , P i e rce), sulfosuccinimidyl [3'-(2-pyridyldithio)propionamido]hexanoate (sulfo-LC-S P D P, Pierce) and N-succinimidyl-[4-vinylsulfonyl]benzoate (SVSB Pierce). It may be advantageous to block certain amine residue as described by Kavimandan et al., (2006) Bioconjugate Chem. 17, 1376-1384.

If the bioactive compound does not contain a thiol reactive group but does contain one or more carbonyl (oxidised carbohydrate) groups then it can be modified to incorporate one or more thiol reactive groups by chemical modification by methods known to the art such as the use of cross-linking reagents such as, but not limited to, N-[eta-maleimidocaproic acid]hydrazide (EMCH, Pierce), 4-[N-maleimidomethyl]cyclohexane-1carboxylhydrazide·HCl·1/2 dioxane (M2C2H, Pierce), 3-maleimidophenyl boronic acid (MPBH, Pierce) and 3-[2-pyridyldithio]propionyl hydrazide (PDPH, Pierce).

If the bioactive compound does not contain a thiol reactive group but does contain one or more hydroxyl groups then it can be modified to incorporate one or more thiol reactive groups by chemical modification by methods known to the art such as the use of cross-linking reagents such as, but not limited to, N-[p-maleimidophenyl]isocyanate (PMPI, Pierce).

### Conjugation competence of transferrin variant

The conjugation competence of polypeptides of the invention may be tested by fluorescent labelling and cellular uptake, as described by McGraw et al., (1987), The Journal of Cell Biology, 105, 207-214 and Presley et al., (1993), The Journal of Cell Biology, 122, 1231-1241.

Preferred embodiments of the invention incude:
1. A polypeptide which:
   has iron binding capacity,
   binds to a receptor, and
   has an amino acid sequence which is at least 60% identical to residues 1-679 or 339-679 of SEQ ID NO: 1, residues 1-691 of SEQ ID NO: 11 or residues 1-738 of SEQ ID NO: 15, and comprises at least one cysteine residue with a free thiol group.
2. The polypeptide of embodiment 1 having at least 95% sequence identity to a serum transferrin, particularly derived from a vertebrate, a mammal or human.
3. The polypeptide of embodiment 1 or 2 which comprises substitution of an amino acid with cysteine, insertion or deletion of cysteine at a position corresponding to any of V1-T5, E13-H25, M26-S36, K42-S44, Y85-T93, K103-Q108, L112-K115, T120-W128, L139-P145, F154-G156, A159-F167, P168-L170; P175-C177, G178-F186, G187-K196, D197-D201, I210-N213, L214-N216, K217-R220, D221-Q222, L226-P234, S255-K259, E260-H273, F274-H300, V305-D310, Y317-E328, G329- P341, C402-N417, C418-D420, K434-T440, W441-N443, L444-G446, Y468-K470, I471-R475, A485-S501, G502-N504, L505-Y515, G516-V526, T537-Q540, N541-D548, P549-A551, K552-N555, D558-Y559, C563-T567, R568-P570, E572-N576, E594-F608, G609-V636, L641-T646, R663-S668 or S669-R677 of SEQ ID NO: 1.
4. The polypeptide of any of embodiments 1, 2 or 3 which comprises substitution, insertion or deletion of an amino acid with cysteine at a position corresponding to any of V1-K4, M26-P35, K42-S44, Y85-T93, K103-Q108, L112-K115, T120-W128, L139-P145, F154-S155, A159-F167, G178-F186, D197-D201, L214-N216, D221-Q222, L226-P234, S255-K259, F274-H300, V305-D310, G329- P341, C402-N417, K434-T440, L444-G446, I471-R475, A485-S501, L505-Y515, N541-D548, K552-N555, D558, C563-T567, G609-V636, L641-T646, R663-S668 of SEQ ID NO: 1.
5. The polypeptide of any of embodiments 1 to 4 which comprises substitution, insertion or deletion of an amino acid with cysteine at a position corresponding to any of V1-K4, K103-Q108, L112-K115, L139-P145, A159-F167, G178-F186, F274-H300, V305-D310, G329-P341, K434-T440, L444-G446, I471-R475, A485-S501, L505-Y515, G609-V636, L641-T646 of SEQ ID NO: 1.
6. The polypeptide of any of embodiments 1 to 5 which comprises substitution, insertion or deletion of an amino acid with cysteine at a position corresponding to any of V1, P2, D3, K4 T5, H14, Q20, S21, D24, K27, S28, V29, P31, S32, D33, A43, E89, D104, G106, G114, L122, G123, P145, S155, D163, T165, D166, P168, P175, G176, G178, C179, S180, T181, L182, Q184, F187, S189, D197, G198, E212, A215, N216, A218, D221, D229, G257, N268, D277, K278, K280, E281, S287, P288, H289, K291, S298, P307, L326, T330, P335, T336, N413, S415, D416, D420, K434, S435, A436, S437, D438, D442, N443, G446, N469, N472, G487, K489, D491, S501, G502, L503, N510, T518, P539, Q540, G543, G544, K545, P547, D548, P549, K552, N553, N555, D558, D565, T567, P570, N576, A595, S610, N611, V612, T613, D614, S616, G617, T626, D634, D643, S666, T667 or S669 of SEQ ID NO: 1.
7. The polypeptide of embodiment 1, wherein at least one cysteine residue has been substituted with a different amino acid residue, particularly Ser, Thr, Val or Ala.
8. The polypeptide of embodiment 7, wherein the polypeptide has at least 95% sequence identity to residues 1-679 or 339-679 of SEQ ID NO: 1, and the at least one cysteine residue is selected among C19, C158, C161, C177, C179, C194, C227, C331, C339, C402, C418, C474, C495, C448, C506, C523, C563, C596, C615, C620, C665.
9. The polypeptide of any of embodiments 1 to 8 which further comprises at least one mutation that reduces N- or O-linked glycosylation.
10. The polypeptide embodiment 9, which compared to SEQ ID NO: 1 comprises a substitution of an amino acid at a position corresponding to S32, N413, S415, N611 or T613 to an amino acid which does not allow glycosylation at the position corresponding to S32, N413 or N611.
11. The polypeptide of any of embodiments 1 to 10, having at least 99% sequence identity to SEQ ID NO: 1 and comprises one or more mutations selected among: V1C, S28C, S32C, D104C, T165C, P175C, A215C, P288C, T336C, S415C, D146C, C171A, S415C+deletion of D416, S415A+insertion of C before D416, S501C, N553C, N611C, T613C, D643C and S28C+S415C.
12. The polypeptide of any embodiments 1 to 8, having at least 95% sequence identity to SEQ ID NO: 11 and comprises the mutation S421C.
13. A polynucleotide which encodes the polypeptide of any embodiments 1 to 12,
14. A plasmid comprising the polynucleotide of embodiment 13.
15. A host cell comprising a polynucleotide of embodiment 13 or a plasmid of embodiment 14.
16. The host cell of embodiment 15, which is a yeast cell.
17. A conjugate which comprises a bioactive compound and a polypeptide according to any of embodiments 1 to 12, wherein the bioactive compound is linked through the free thiol group of the cysteine residue of the polypeptide.
18. A method or producing a polypeptide of any of embodiments 1 to 12, comprising culturing the host cell of embodiment 15 or 16 under conditions that allows expression of the polypeptide and recovering the polypeptide.
19. A method of producing the conjugate of embodiment 17, which comprises inking the polypeptide of any of embodimens 1 to 19 and a bioactive compound linked through the sulphur atom of the cysteine residue in the polypeptide.
20. A composition comprising a conjugate of embodiment 17 and at least one pharmaceutically acceptable carrier or diluent.
21. The use of a conjugate according to any of embodiments 1 to 20 for treatment of disease, illness and diagnosis.
22. A method of preparing a polypeptide, comprising:
   providing a three-dimensional model comprising at least one instance of a transferrin sequence, an iron atom and a receptor,
   selecting an amino acid residue in the transferrin sequence which corresponds to V1, P2 or D3 in SEQ ID NO: 1 or which in each instance of the transferrin sequence fulfils the following conditions:
      i) RMFS above 0.14,
      ii) solvent-surface Accessibility above 100%,
   substituting the selected residue with cysteine or inserting cysteine at the N-side or C-side of the selected residue,
   optionally, making additional alterations to the transferrin sequence where each alteration is an amino acid deletion, substitution, or insertion, and
   preparing a polypeptide having the resulting amino acid sequence.
23. The method of embodiment 22, which further comprises determining the Fe binding capacity and/or the receptor binding capacity and/or the conjugation competence of the polypeptide and selecting a polypeptide which has Fe binding capacity and/or receptor binding capacity and/or conjugation competence.

### EXAMPLES

### MATERIALS AND METHODS

Chemicals used in the examples below are provided from Merch unless otherwise stated.

### Urea gel electrophoresis

Urea gel electrophoresis is performed using a modification of the procedure of Makey and Seal (Monthony et al, 1978, Clin. Chem., 24, 1825-1827; Harris & Aisen, 1989, Physical biochemistry of the transferrins, VCH; Makey & Seal, 1976, Biochim. Biophys. Acta., 453, 250-256; Evans & Williams, 1980, Biochem. J., 189, 541-546) with commercial minigels (6% homogeneous TBE Urea, Invitrogen). Samples containing approximately 10 µg protein are diluted 1:1 in TBE-Urea sample buffer (Invitrogen), separated at 180 V for 550 to 600 Vh and stained with GelCode^{®} Blue reagent (Pierce). Apo-transferrin is prepared by dialysis against 0.1 M citrate, 0.1 M acetate, 10 mM EDTA pH 4.5. Solutions are filtered (0.22µm), concentrated to 10 mg/ml using a Vivaspin polyethersulphone 10,000 NMWCO centrifugal concentrator and diafiltered against 10 volumes water followed by 10 volumes of 0.1 M HEPES, 0.1 M NaHCO₃ pH 8.0. Samples are recovered from the concentrator with a rinse and made up to a final concentration of 5 mg/ml. Reconstituted holo-transferrin is prepared from this solution by addition of 10 µl 1 mM FeNTA (prepared freshly as an equimolar solution of ferric chloride in disodium nitrilotriacetic acid) to a 50 µl aliquot and allowed to stand for 10 minutes to permit CO₂ dissolution for completion of iron binding before electrophoretic analysis. This technique separates four molecular forms with different iron loadings namely (in order of increasing mobility) apo-transferrin, C-lobe and N-lobe bound monoferric transferrins and holo-transferrin. Separation of the four forms of transferrin is believed to be due to partial denaturation in 4-6M urea; where iron binding in any lobe causes a change in conformation resulting in increased resistance to denaturation. Thus the presence of iron in a lobe results in a more compact structure with higher electrophoretic mobility. Since the N-lobe has fewer disulphide bonds than the C-lobe (8 versus 11 respectively) it unfolds further in the absence of iron, making the monoferric form with iron bound to the C-lobe the least mobile.

### Free thiol assay

The number of free thiols on a protein can be determined spectrophotometrically using Ellman's reagent. Ellman's reagent (5'5'-dithio-bis(2-nitronenzoic acid) (DTNB)) is an aromatic disulphide which reacts with thiol groups to form a mixed disulphide of the protein and one mole of 2-nitro-5-thio-benzoate (NTB) (per mole of protein sulphidyl group). Formation of NTB can be monitored by measuring absorbance at 412nm. The molar absorbance coefficient is 13,600 M-1 cm-1

The free thiol assay using Ellman's reagent can be used to determine number of free thiol groups for proteins. To determine the number of free thiol groups the sample protein was diluted to a known concentration (e.g. 10 mg/mL) in 7.4 mM phosphate buffer pH 7.4 in a final volume of 1 mL. The protein solution (600 mL) was treated with 5,5'-dithiobis(2-nitrobenzoic acid) (DTNB) prepared freshly in 37 mM phosphate buffer, pH 8.3. Following 45 minutes at room temperature in the presence of EDTA, the absorbance of the solution was measured at 412 nm against reagent blanks and number of reacting sulfydryls calculated using E 412 =13600 M⁻¹cm⁻¹.

### Microorganism.

The host strain used in the examples below identified as Strain 1 is an *hsp150-*deficient version of DXY1, disclosed in S. M. Kerry-Williams et al. (1998) Yeast 14:161-169. WO 95/33833 teaches the skilled person how to prepare *hsp150*-deficient yeast.

### EXAMPLE 1: CONSTRUCTION OF TRANSFERRIN MUTEIN EXPRESSION PLASMIDS

Expression plasmids for transferrin variants of this invention can be constructed in similarity with the following description for Tf variant S415A, T613A.

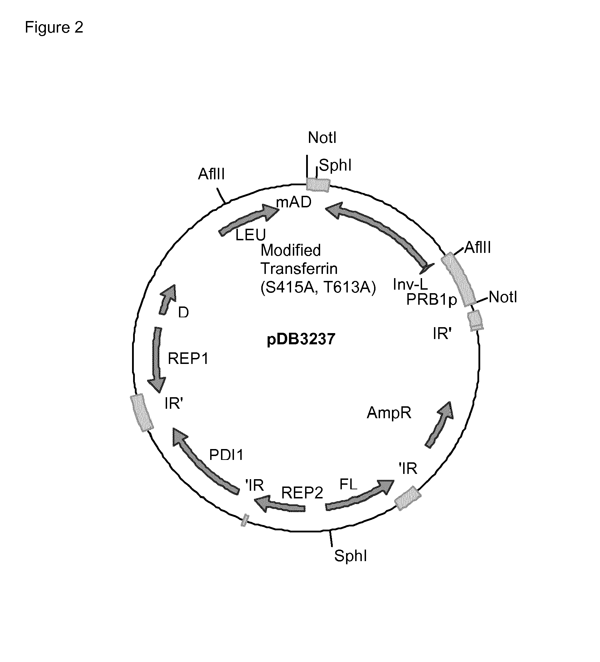

Transferrin muteins are made by modification of a plasmid called pDB3237 (Figure 2) by site directed mutagenesis. Overlapping mutagenic oligonucleotide sequences will be used to modify the codon of the selected residue(s) to any DNA sequence which encodes a cysteine residue (TGT or TGC) using the procedures indicated by a commercially available kit (such as Stratagene's Quikchange™ Kit).

### Construction of Transferrin (S415A, T613A) expression plasmid, pDB3237

Overlapping oligonucleotide primers were used to create a synthetic DNA encoding the invertase leader sequence operationally linked to the human transferrin (S415A, T613A) which is codon optimised for expression in S. cerevisiae.

SEQ ID No. 2 is a DNA sequence based on the mature human transferrin C1 variant protein sequence, modified at serine 415 and threonine 613 to alanine codons to prevent N-linked glycosylation at the asparagine 413 and asparagine 611 sites, respectively. The sequence is flanked by *Sphl* and *Afl*II restriction endonuclease sites to facilitate cloning. SEQ ID NO: 2 comprises the mature human transferrin C1 variant protein encoding sequence modified at serine 415 and threonine 613 to alanine codons to prevent N-linked glycosylation at the asparagine 413 and asparagine 611 sites (nucleotides 124-2160); two translation stop codons (nucleotides 2161-2166); the invertase leader (signal) protein encoding sequence (nucleotides 67-123); the 3' UTR and part of the ADH1 gene terminator up to an *Sphl* cloning sites (nucleotides 2167-2359); the 5' UTR and part of the *PRB1* gene promoter up to an *Afl*II cloning sites (nucleotides 1-66).

The invertase leader (signal) protein encoding sequence (nucleotides 67-123) encodes the signal peptide MLLQAFLFLLAGFAAKISA (-19 TO -1 OF SEQ ID NO: 3). 1 -679 of SEQ ID NO: 3 comprises the mature human transferrin C1 variant protein encoding sequence modified at serine 415 and threonine 613 to alanine codons to prevent N-linked glycosylation at the asparagine 413 and asparagine 611 sites.

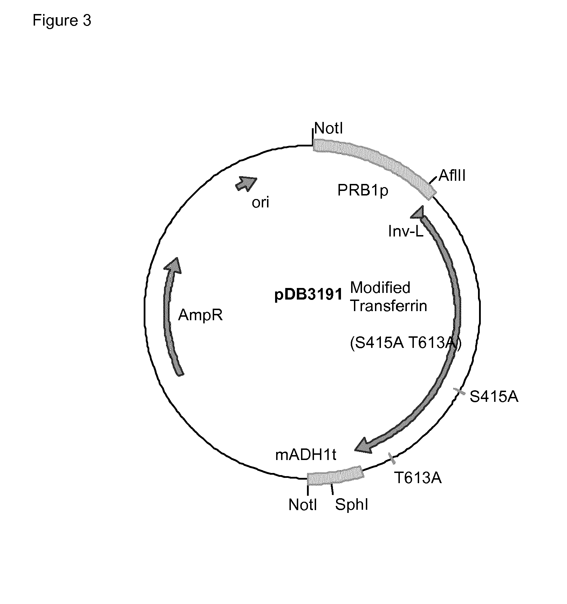

The synthetic DNA sequence encoding the invertase leader sequence operationally linked to the human transferrin (S415A, T613A) (SEQ ID NO: 2) was digested to completion with *Sphl* and *Afl*II to create a 2.357kb fragment. Plasmid pDB2241 (4.383kb), described in WO 00/44772 was digested to completion using restriction endonucleases *Sph*I and *Afl*II to create a 4.113kb fragment, which was subsequently dephosphorylated using calf alkaline intestinal phosphatase. The 2.537 kb invertase leader sequence human transferrin (S415A, T613A) DNA fragment was ligated into the 4.113kb *Sph*I/*Afl*II fragment from pDB2241 to create plasmid pDB3191 (Figure 3). Plasmid pDB3191 is digested to completion with Notl restriction endonuclease to release the 3.259kb invertase leader sequence human transferrin (S415A, T613A) expression cassette.

The construction of plasmid pDB2690 is described in WO/2005061719 A1. Plasmid pDB2690 (13.018kb) is digested to completion with restriction endonuclease Notl and dephosphorylated using calf alkaline intestinal phosphatase and ligated with the 3.259kb Notl transferrin (S415A, T613A) expression cassette to produce 16.306kb pDB3237 which has the transferrin (S415A, T613A) expression cassette in the opposite direction to the *LEU2* gene (Figure 2).

### Construction of a thiotransferrin mutant expression plasmids

Alternatively expression plasmids for thiotransferrin variants of this invention can be made by subcloning synthesized DNA fragments into plasmid pDB3191 (Figure 3) prior to subcloning of Notl transferrin variant expression cassettes into pDB2690.

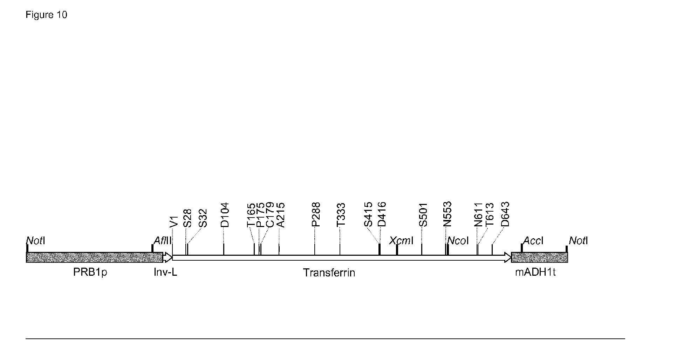

The transferrin DNA sequence of pDB3191 (Figure 3) contains unique *Afl*II, *Xcm*I, *Nco*I and *Acc*I restriction endonuclease sites. The positions of the proposed mutations were mapped on the transferrin expression cassette sequence of pDB3191 (Figure 10). SEQ ID No. 4, 5, 6 and 7 are DNA sequences based on the part of mature human transferrin C1 variant protein sequence, modified at serine 415 to an alanine codon to prevent N-linked glycosylation at the asparagine 413 site. The sequence is flanked by *Afl*II and *Xcm*I restriction endonuclease sites to facilitate cloning. Fourteen thiotransferrin variants were created by modification of the DNA sequence between the *Afl*II and *Xcm*I restriction site (**Table** 2). SEQ ID No: 4 comprises part of the mature human transferrin C1 variant protein encoding sequence modified at serine 415 to alanine to prevent N-linked glycosylation at the asparagine 413 site up to an *Xcm*I cloning site (nucleotides 124-1487); the invertase leader (signal) protein encoding sequence (nucleotides 67-123) and part of the *PRB1* gene promoter up to an *Afl*II cloning site (nucleotides 1-66). Eleven SEQ ID No: 4 variants were synthesized wherein the transferrin protein encoding sequence is modified at a selected codon to a cysteine codon.

For Thiotransferrin variant (V1C) the transferrin protein encoding sequence is modified such that the valine codon at position 1 is substituted to a cysteine codon (TGT).

For Thiotransferrin variant (S28C) the transferrin protein encoding sequence is modified such that the serine codon at position 28 is substituted to a cysteine codon (TGT).

For Thiotransferrin variant (S32C) the transferrin protein encoding sequence is modified such that the serine codon at position 32 is substituted to a cysteine codon (TGT).

For Thiotransferrin variant (D104C) the transferrin protein encoding sequence is modified such that the aspartic acid codon at position 104 is substituted to a cysteine codon (TGT).

For Thiotransferrin variant (T165C) the transferrin protein encoding sequence is modified such that the threonine codon at position 165 is substituted to a cysteine codon (TGT).

For Thiotransferrin variant (P175C) the transferrin protein encoding sequence is modified such that the proline codon at position 175 is substituted to a cysteine codon (TGT).

For Thiotransferrin variant (A215C) the transferrin protein encoding sequence is modified such that the alanine codon at position 215 is substituted to a cysteine codon (TGT).

For Thiotransferrin variant (P288C) the transferrin protein encoding sequence is modified such that the proline codon at position 288 is substituted to a cysteine codon (TGT).

For Thiotransferrin variant (T336C) the transferrin protein encoding sequence is modified such that the threonine codon at position 336 is substituted to a cysteine codon (TGT).

For Thiotransferrin variant (S415C) the transferrin protein encoding sequence is modified such that the serine codon at position 415 is substituted to a cysteine codon (TGT).

For Thiotransferrin variant (D416C) the transferrin protein encoding sequence is modified such that the aspartic acid codon at position 416 is substituted to a cysteine codon (TGT).

SEQ ID No:5 comprises part of the mature human transferrin C1 variant protein encoding sequence modified at serine 415 to alanine to prevent N-linked glycosylation at the asparagine 413 sites, and modified at cysteine 171 to an alanine codon to create an unpaired cysteine at the cysteine 179 site, up to an *Xcm*I cloning site (nucleotides 124-1487); the invertase leader (signal) protein encoding sequence (nucleotides 67-123) and part of the *PRB1* gene promoter up to an *Afl*II cloning site (nucleotides 1-66).

The appropriate SEQ ID No: 4 variant or SEQ ID No: 5 DNA sequence was digested to completion with *Afl*II and *Xcm*I to create a 1.479kb DNA fragment. Plasmid pDB3191 (6.47kb) was digested to completion using restriction endonucleases *Afl*II and *Xcm*I to create a 4.991 kb fragment, which was subsequently dephosphorylated using shrimp alkaline phosphatase. The appropriate 1.479kb Thiotransferrin variant DNA fragment was sublconed into the 4.991kb *Afl*II/*Xcm*I fragment from pDB3191 to create plasmids pDB3714, pDB3715, pDB3752, pDB3716, pDB3717, pDB3754, pDB3740, pDB3741 pDB3742, pDB3755, pDB3744, or pDB3756 (**Table** 2).

SEQ ID No. 6 is a DNA sequence based on the mature human transferrin C1 variant protein sequence, modified by substitution of serine 415 and aspartic acid 416 codons to a cysteine codon (so that the amino acid chain length is reduced). The sequence is flanked by *Af*/II and *Xcm*I restriction endonuclease sites to facilitate cloning. SEQ ID No: 6 comprises part of the mature human transferrin C1 variant protein encoding sequence modified by substitution of two residues, serine 415 and aspartic acid 416, to a cysteine codon (the amino acid chain length is reduced) up to an *Xcm*I cloning site (nucleotides 124-1484); the invertase leader (signal) protein encoding sequence (nucleotides 67-123) and part of the *PRB1* gene promoter up to an *Afl*II cloning sites (nucleotides 1-66).

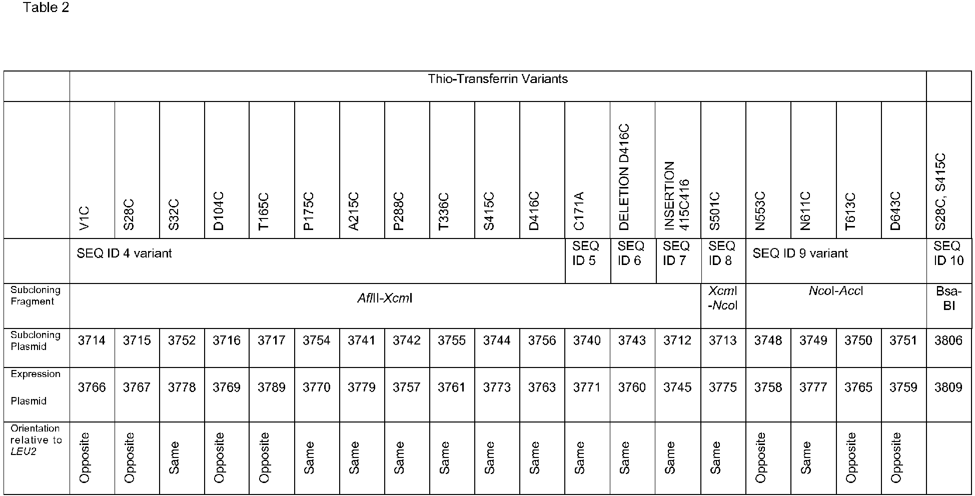

The SEQ ID No: 6 DNA sequence was digested to completion with *Afl*II and *Xcm*I to create a 1.476kb fragment. Plasmid pDB3191 (6.47kb) was digested to completion using restriction endonucleases *Afl*II and *Xcm*I to create a 4.991kb fragment, which was subsequently dephosphorylated using shrimp alkaline phosphatase. The 1.476kb Thiotransferrin variant (Deletion 416C) DNA fragment was sublconed into the 4.991 kb *Afl*II/*Xcm*I fragment from pDB3191 to create 6.467kb plasmid pDB3743 (Table 2**Table** ).

SEQ ID No. 7 is a DNA sequence based on the mature human transferrin C1 variant protein sequence, modified at serine 415 to an alanine codon to prevent N-linked glycosylation at the asparagine 413 site and modified such that a cysteine codon is inserted at the N-terminal side of aspartic acid 416 residue (so that the amino chain length is increased). The sequence is flanked by *Afl*II and *Xcm*I restriction endonuclease sites to facilitate cloning. SEQ ID No: 7 comprises part of the mature human transferrin C1 variant protein encoding sequence modified at serine 415 to an alanine residue to prevent N-linked glycosylation at the asparagine 413 site and at aspartic acid 416 where a cysteine codon is inserted at the N-terminal side of aspartic acid 416 up to an *Xcm*I cloning site (nucleotides 124-1490); the invertase leader (signal) protein encoding sequence (nucleotides 67-123) and part of the *PRB1* gene promoter up to an *Afl*II cloning sites (nucleotides 1-66).

The SEQ ID No: 7 DNA sequence was digested to completion with *Afl*II and *Xcm*lI to create a 1.482kb fragment. Plasmid pDB3191 (6.47kb) was digested to completion using restriction endonucleases *Afl*II and *Xcm*I to create a 4.991kb fragment, which was subsequently dephosphorylated using shrimp alkaline phosphatase. The 1.482kb thiotransferrin variant (Insertion 415C416) DNA fragment was sublconed into the 4.991 kb *Afl*II/*Xcm*I fragment from pDB3191 to create a 6.473kb plasmid,. pDB3712 (Table 2).

SEQ ID No. 8 is a DNA sequence based on part of the mature human transferrin C1 variant protein sequence. The sequence is flanked by *Xcm*I and *Nco*I restriction endonuclease sites to facilitate cloning. SEQ ID No. 8: comprises the part mature human transferrin C1 variant protein encoding sequence modified at serine 501 to a cysteine codon (TGT) (1-301 nucleotides).

The SEQ ID No: 8 DNA sequence was digested to completion with *Xcm*I and *Nco*I to create a 0.288kb fragment. Plasmid pDB3191 (6.47kb) was digested to completion using restriction endonucleases *Xcm*I and *Nco*I to create a 6.182kb fragment, which was subsequently dephosphorylated using shrimp alkaline phosphatase. The 0.288kb Thiotransferrin (S415A, S501C, T613A) variant DNA fragment was sublconed into the 6.182kb *Xcm*I/*Nco*I fragment from pDB3191 to create a 6.47kb plasmid, pDB3713 (Table 2).

SEQ ID No. 9 a DNA sequence is based on part of the mature human transferrin C1 variant protein sequence, modified at threonine 613 to an alanine codon to prevent N-linked glycosylation at Asparagine 611 site. The sequence is flanked by *Nco*I and *Acc*I restriction endonuclease sites to facilitate cloning.

Four Thiotransferrin variants were created by modification of the DNA sequence between the *Nco*I and *Acc*I restriction site.

SEQ ID No: 9 comprises part of the mature human transferrin C1 variant protein encoding sequence from an Ncol cloning site and modified at threonine 613 to an alanine codon to prevent N-linked glycosylation at the Asparagine 611 site (nucleotides 1-393); two translation stop codons (nucleotides 394-399); the 3' UTR and part of the ADH1 gene terminator up to an *Acc*I cloning site (nucleotides 1-462).

Four SEQ ID No: 9 variants were synthesized wherein the transferrin protein encoding sequence is modified at a selected residue the to a cysteine codon (TGT).

For Thiotransferrin variant (N553C) the amino acid sequence is modified such that the asparagine codon at position 553 is substituted to a cysteine codon (TGT).

For Thiotransferrin variant (N611 C) the amino acid sequence is modified such that the asparagine codon at position 611 is substituted to a cysteine codon (TGT).

For Thiotransferrin variant (T613C) the amino acid sequence is modified such that the threonine codon at position 613 is substituted to a cysteine codon (TGT).

For Thiotransferrin variant (D643C) the amino acid sequence is modified such that the aspartic acid codon at position 643 is substituted to a cysteine codon (TGT).

The appropriate SEQ ID No: 9 Thiotransferrin variant DNA sequence was digested to completion with Ncol and *Acc*I to create a 0.457kb DNA fragment. Plasmid pDB3191 (6.47kb) was digested to completion using restriction endonucleases *Nco*I and *Acc*I to create a 6.013kb fragment, which was subsequently dephosphorylated using shrimp alkaline phosphatase. The appropriate 0.457kb Thiotransferrin variant DNA fragment was subcloned into the 6.013kb *Nco*I/*Acc*I fragment from pDB3191 to create 6.47kb plasmid pDB3748, pDB3749, pDB3750, pDB3751 (Table 2).

The appropriate Thiotransferrin variant subcloning plasmid pDB3714, pDB3715, pDB3752, pDB3716, pDB3717, pDB3754, pDB3740, pDB3741 pDB3742, pDB3755, pDB3744, pDB3756, pDB3713, pDB3748, pDB3749, pDB3750 or pDB3751 were digested to completion with *Not*I restriction endonuclease to release the appropriate 3.259kb Thiotransferrin variant expression cassette.

The construction of plasmid pDB2690 has been described in WO/2005061719 A1. Plasmid pDB2690 (13.018kb) was digested to completion with restriction endonuclease *Not*I and dephosphorylated using shrimp alkaline phosphatase and ligated with the 3.259kb *Not*I thiotransferrin variant expression cassette to produce 16.306kb plasmids pDB3778, pDB3770, pDB3779, pDB3757, pDB3761, pDB3773, pDB3763, pDB3771, pDB3775, pDB3777 which has the thiotransferrin variant expression cassette in the same orientation to the *LEU2* gene, or pDB3766, pDB3767, pDB3769, pDB3789, pDB3758, pDB3765, pDB3759 which has the thiotransferrin variant expression cassette in the opposite direction to the *LEU2* gene. These examples indicate that the expression cassette may be cloned in either orientation in the expression vector as part of this invention.

One example is described in which a free thiol is introduced by insertion of a cysteine codon (the amino chain length is increased). Plasmid pDB3712 was digested to completion with restriction endonuclease *Not*I to release the 3.262kb thiotransferrin (Insertion 415C416) variant expression cassette. Plasmid pDB2690 (13.018kb) was digested to completion with restriction endonuclease *Not*I and dephosphorylated using shrimp alkaline phosphatase and ligated with the 3.262kb *Not*I thiotransferrin variant expression cassette to produce 16.309kb plasmid pDB3745 which has the thiotransferrin variant expression cassette in the same orientation to the *LEU2* gene.

One example is described in which a free thiol is introduced by substitution of two codons for a cysteine codon (the amino chain length is reduced). Plasmid pDB3743 was digested to completion with restriction endonuclease Notl to release the 3.256kb thiotransferrin (Deletion 416C) variant expression cassette. Plasmid pDB2690 (13.018kb) was digested to completion with restriction endonuclease *Not*I and dephosphorylated using shrimp alkaline phosphatase and ligated with the 3.256kb *Not*I transferrin variant expression cassette to produce 16.303kb plasmid pDB3760 which has the transferrin variant expression cassette in the same orientation to the *LEU2* gene.

### Construction of a thiotransferrin (S28C, S415C) mutant expression plasmid

One example is described in which two free thiol groups are introduced by substitution of the serine 28 codon to a cysteine codon and the serine 415 codon to a cysteine codon.

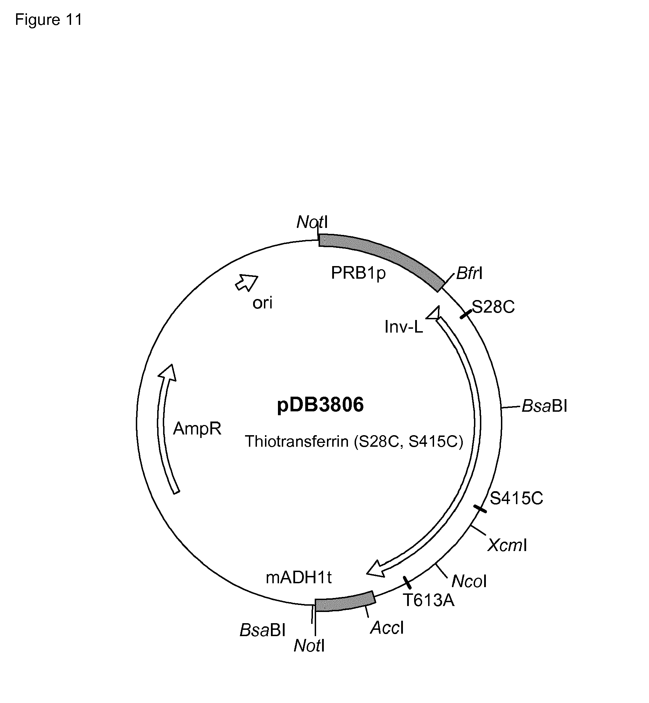

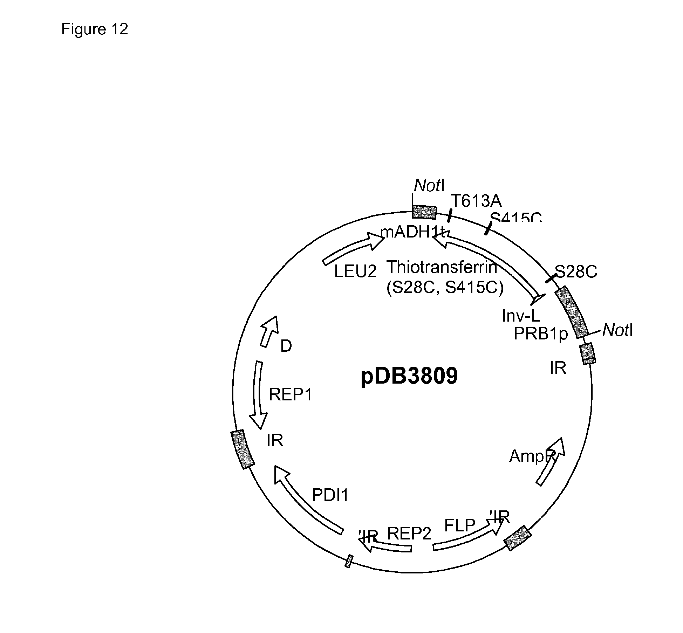

SEQ ID 10 is a DNA sequence based on the mature human transferrin C1 variant protein encoding sequence modified at threonine 613 to an alanine codon to prevent N-linked glycosylation at the asparagine 611 site, and modified at serine 415 to a cysteine codon (TGT) and prevent N-linked glycosylation at the asparagine 413 site, and modified at serine 28 to a cysteine codon (TGT) such that more than one (two) free thiol groups are created (nucleotides 124-2160), two translation stop codons (nucleotides 2161-2166); the invertase leader (signal) protein encoding sequence (nucleotides 67-123); the 3' UTR and part of the ADH1 gene terminator up to an *Sph*I cloning sites (nucleotides 2167-2359); the 5' UTR and part of the PRB1 gene promoter up to an *Afl*II cloning sites (nucleotides 1-66). The expression plasmid was constructed by sub-cloning the S415C, T613A mutation present on a 1.737kb *Bsa*BI DNA fragment of pDB3744 into plasmid pDB3715. Plasmid pDB3744 was digested to completion with restriction endonuclease *Bsa*BI to release 1.737kb part of thiotransferrin (S415C) variant expression cassette. Plasmid pDB3715 was digested to completion with restriction endonuclease *Bsa*BI, the 4.733kb fragment was recovered by gel extraction and dephosphorylated using shrimp alkaline phosphatase. The 4.733kb fragment from plasmid pDB3715 was ligated with the 1.737kb *Bsa*BI thiotransferrin variant fragment from plasmid pDB3744 to produce 6.470kb plasmid pDB3806 (Figure 11). The Thiotransferrin (S28C, S415C, T613A) variant subcloning plasmid pDB3806 was digested to completion with Notl restriction endonuclease to release the 3.259kb Thiotransferrin (S28C, S415C, T613A) variant expression cassette. Plasmid pDB2690 (13.018kb) was digested to completion with restriction endonuclease *Not*I and dephosphorylated using shrimp alkaline phosphatase and ligated with the 3.259kb *Not*I thiotransferrin (S28C, S415C, T613A) variant expression cassette to produce 16.306kb plasmid pDB3809 (Figure 12) which has the thiotransferrin (S28C, S415C, T613A) variant expression cassette in the opposite orientation to the *LEU2* gene.

A *S. cerevisiae* strain (Strain 1) was transformed to leucine prototrophy with transferrin expression plasmid pDB3237 and thiotransferrin expression plasmids pDB3766, pDB3767, pDB3778, pDB3769, pDB3789 pDB3770, pDB3771, pDB3779, pDB3757, pDB3761, pDB3773, pDB3763, pDB3760, pDB3745, pDB3775, pDB3758, pDB3777 pDB3765, pDB3809. Yeast were transformed using a modified lithium acetate method (Sigma yeast transformation kit, YEAST-1, protocol 2; Ito et al, 1983, J. Bacteriol., 153, 16; Elble, 1992, Biotechniques, 13, 18). Transformants were selected on BMMD-agar plates, and subsequently patched out on BMMD-agar plates. The composition of BMMD is described by Sleep et al., 2002, Yeast, 18, 403. Cryopreserved stocks were prepared in 20% (w/v) trehalose from 10mL BMMD shake flask cultures (24 hrs, 30°C, 200rpm).

### Construction of Lactoferrin mutein expression plasmids

The transferrins form a group of proteins with high sequence homology, including but not limited to human serum transferrin (HST), lactoferrin, melanotransferrin and ovotransferrin.

Overlapping oligonucleotide primers were used to create a synthetic DNA encoding the invertase leader sequence operationally linked to the human lactoferrin (T139A, T480A, S625A) which may or may not be codon optimised for expression in S. cerevisiae. SEQ ID No. 11 is based on the mature human lactoferrin variant protein sequence (Accession number NP_002334), modified at threonine 139, threonine 480 and serine 625 to alanine codons to prevent N-linked glycosylation at the asparagine 137, asparagine 478 and asparagine 623 sites, respectively.

SEQ ID No. 12 is the *S cerevisiae* native DNA sequence encoding the invertase leader protein sequence operationally linked to the human native DNA sequence (Accession NM_002343) encoding human lactoferrin (T139A, T480A, S625A) variant protein modified at threonine 139, threonine 480 and serine 625 to alanine codons to prevent N-linked glycosylation at the asparagine 137, asparagine 478 and asparagine 623 sites, respectively (nucleotides 124-2196), two translation stop codons (nucleotides 2197-2202); the native *S cerevisiae* invertase leader (signal) protein encoding sequence (nucleotides 67-123); the 3' UTR and part of the ADH1 gene terminator up to an *Sph*I cloning sites (nucleotides 2203-2395); the 5' UTR and part of the *PRB1* gene promoter up to an *Afl*II cloning sites (nucleotides 1-66). The sequence is flanked by *Sphl* and *Afl*II restriction endonuclease sites to facilitate cloning.

SEQ ID No. 13 is a DNA sequence encoding the invertase leader protein sequence codon optimised for expression in *S cerevisiae* operationally linked to a DNA sequence encoding mature human lactoferrin (T139A, T480A, S625A) variant protein sequence modified at threonine 139, threonine 480 and serine 625 to alanine codons to prevent N-linked glycosylation at the asparagine 137, asparagine 478 and asparagine 623 sites, respectively codon optimised for expression in *S. cerevisiae* (nucleotides 124-2196), two translation stop codons (nucleotides 2197-2202); the invertase leader (signal) protein encoding sequence codon optimised for expression in *S. cerevisiae* (nucleotides 67-123); the 3' UTR and part of the ADH1 gene terminator up to an *Sph*I cloning sites (nucleotides 2203-2395); the 5' UTR and part of the *PRB1* gene promoter up to an *Afl*II cloning sites (nucleotides 1-66). The sequence is flanked by *Sph*I and *Afl*II restriction endonuclease sites to facilitate cloning.

Sequence alignment of human transferrin protein and human lactoferrin protein was used to identify an amino acid residue within the lactoferrin protein sequence suitable for modification to a cysteine residue such that a free thiol group is created. Amino acid residues of the human transferrin protein sequence selected for modification to a cysteine residue described in Table 2 were mapped onto an alignment of human transferrin and human lactoferrin protein sequences. The serine residue at position 421 on lactoferrin protein sequence corresponded to the serine residue at position 415 on the transferrin protein sequence, thus serine 421 was selected for modification of a cysteine residue.

SEQ ID No. 14 is a DNA sequence encoding the invertase leader protein sequence codon optimised for expression in *S cerevisiae* operationally linked to a DNA sequence encoding mature human lactoferrin (T139A, S421C, T480A, S625A) variant protein encoding sequence modified at threonine 139, threonine 480 and serine 625 to alanine codons to prevent N-linked glycosylation at the asparagine 137, asparagine 478 and asparagine 623 sites, respectively and at modified at serine 421 to a cysteine codon (TGT), codon optimised for expression in *S. cerevisiae* (nucleotides 124-2196), two translation stop codons (nucleotides 2197-2202); the invertase leader (signal) protein encoding sequence codon optimised for expression in *S. cerevisiae* (nucleotides 67-123); the 3' UTR and part of the ADH1 gene terminator up to an *Sph*I cloning sites (nucleotides 2203-2395); the 5' UTR and part of the *PRB1* gene promoter up to an *Afl*II cloning sites (nucleotides 1-66). The sequence is flanked by *Sph*I and *Afl*II restriction endonuclease sites to facilitate cloning.

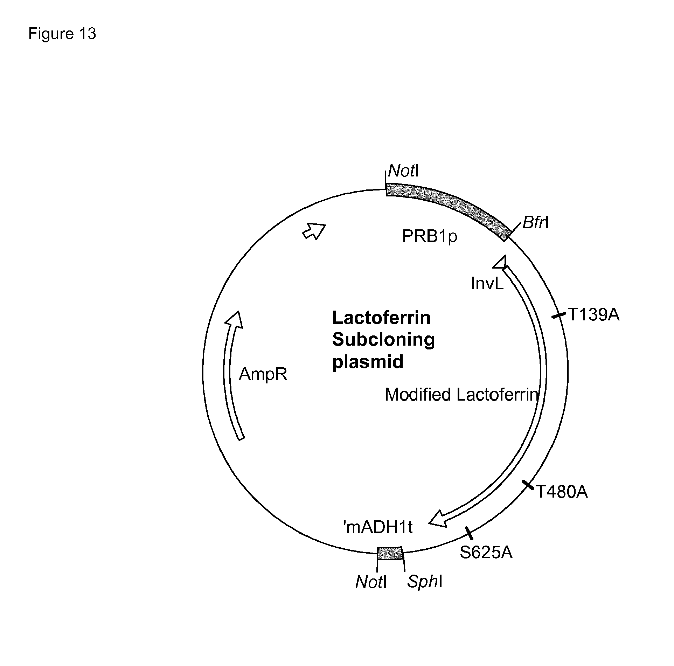

The synthetic DNA sequence (SEQ ID No. 12) encoding the invertase leader sequence operationally linked to the human lactoferrin (T139A, T480A, S625A) was digested to completion with *Sph*I and *Afl*II to create a 2.393kb fragment. Plasmid pDB3191 (6.470kb) (Figure 3), was digested to completion using restriction endonucleases *Sph*I and *Afl*II to create a 4.113kb fragment, which was subsequently dephosphorylated using shrimp alkaline intestinal phosphatase. The 2.393 kb invertase leader sequence-human lactoferrin (T139A, T480A, S625A) DNA fragment was ligated into the 4.113kb *Sph*I/*Afl*II fragment from pDB3191 to create plasmid pDB3815 (Figure 13).

The synthetic DNA sequence codon optimised for expression in S cerevisiae (SEQ ID No. 13) encoding the invertase leader sequence operationally linked to the human lactoferrin (T139A, T480A, S625A) was digested to completion with *Sph*I and *Afl*II to create a 2.393kb fragment. Plasmid pDB3191 (6.470kb) (Figure 3), was digested to completion using restriction endonucleases *Sph*I and *Afl*II to create a 4.113kb fragment, which was subsequently dephosphorylated using shrimp alkaline intestinal phosphatase. The 2.393 kb invertase leader sequence-human lactoferrin (T139A, T480A, S625A) DNA fragment was ligated into the 4.113kb *Sph*I/*Afl*II fragment from pDB3191 to create plasmid pDB3816 (Figure 13)

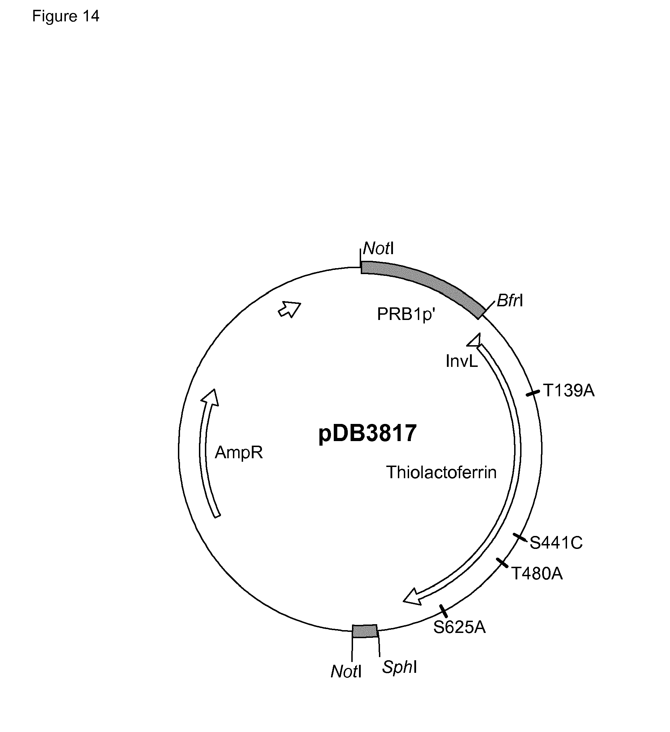

The synthetic DNA sequence codon optimised for expression in S cerevisiae (SEQ ID No. 14) encoding the invertase leader sequence operationally linked to the human lactoferrin (T139A, S421C, T480A, S625A) was digested to completion with *Sph*I and *Afl*II to create a 2.393kb fragment. Plasmid pDB3191 (6.470kb) (Figure 3), was digested to completion using restriction endonucleases *Sph*I and *Afl*II to create a 4.113kb fragment, which was subsequently dephosphorylated using shrimp alkaline intestinal phosphatase. The 2.393 kb invertase leader sequence-human lactoferrin (T139A, T480A, S625A) DNA fragment was ligated into the 4.113kb *Sph*I/*Afl*II fragment from pDB3191 to create plasmid pDB3817 (Figure 14).

Plasmids pDB3815 and pDB3816 were digested to completion with *Not*I restriction endonuclease to release a 3.259kb DNA sequence encoding invertase leader sequence human lactoferrin (T139A, T480A, S625A) expression cassette. Plasmid pDB3817 was digested to completion with *Not*I restriction endonuclease to release the 3.259kb invertase leader sequence human lactoferrin (T139A, T480A, S421C, S625A) expression cassette.

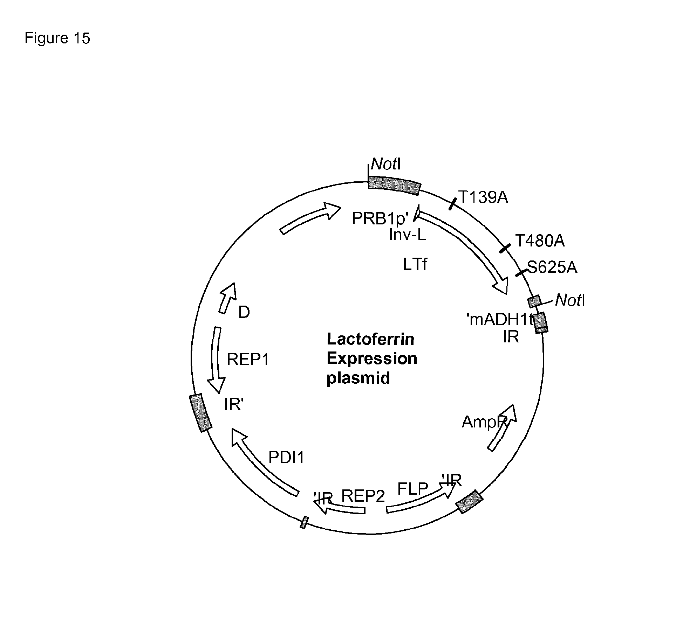

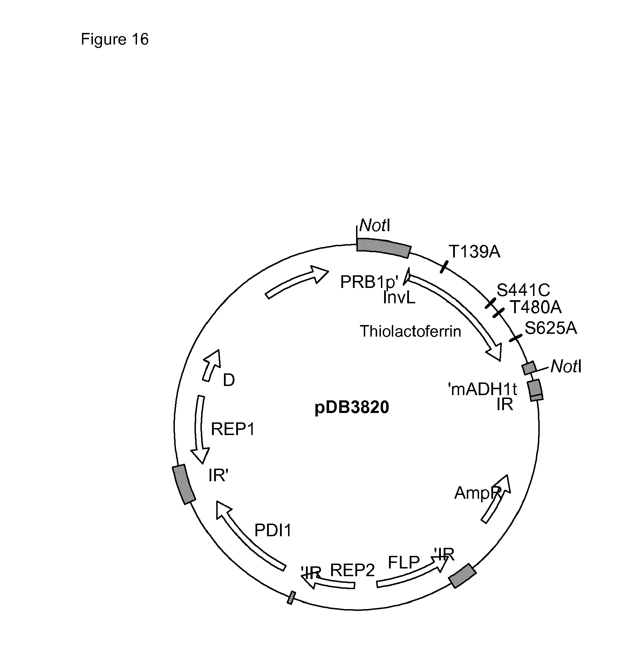

The construction of plasmid pDB2690 has been described in WO/2005061719 A1. Plasmid pDB2690 (13.018kb) was digested to completion with restriction endonuclease *Not*I, dephosphorylated using shrimp alkaline phosphatase and ligated with the appropriate 3.259kb Notl lactoferrin variant expression cassette from pDB3815, pDB3816 and pDB3817 to produce 16.342kb plasmids pDB3818, pDB3819 (Figure 15) and pDB3820 (Figure 16) respectively which has the lactoferrin variant expression cassette in the same orientation to the *LEU2* gene

A S. cerevisiae strain (Strain 1) was transformed to leucine prototrophy with lactoferrin expression plasmids pDB3818, pDB3819 and pDB3820. Yeast were transformed using a modified lithium acetate method (Sigma yeast transformation kit, YEAST-1, protocol 2; Ito et al, 1983, J. Bacteriol., 153, 16; Elble, 1992, Biotechniques, 13, 18). Transformants were selected on BMMD-agar plates, and subsequently patched out on BMMD-agar plates. The composition of BMMD is described by Sleep et al., 2002, Yeast, 18, 403. Cryopreserved stocks were prepared in 20% (w/v) trehalose from 10mL BMMD shake flask cultures (24 hrs, 30°C, 200rpm).

In the examples given codon TGT was used for the cysteine codon, however, codon TGC could also be used in this invention.

### EXAMPLE 2: PURIFICATION OF TRANSFERRIN AND THIOTRANSFERRIN MUTEINS

The transformants from Example 1 were cultivated as follows:

Shake flasks cultures, with 10 ml media in 50 ml conical flasks, were grown at 30°C, 200 rpm with YEPD medium (1% (w/v) yeast extract, 2% (w/v) Bactopeptone, 2% (w/v) glucose) or BMMD (buffered minimal medium, 0.67% (w/v) Bacto Yeast Nitrogen Base without amino acids, 36 mM citric acid/126 mM disodium hydrogen orthophosphate, pH 6.5, 2% (w/v) glucose).

Yeast transformants were cultured for 5 days in 10mL BMMD shake flask. After centrifugation of the cells, the thiotransferrin variant proteins secreted into supernatant were compared to recombinant human transferrin (S415A, T613A) expressed from Strain 1 [pDB3237] by 4-12% gradient SDS non-reducing gel (SDS-PAGE) and 4- 12% gradient SDS reducing gel (SDS-PAGE) (Figure 17). All strains had secreted a proteinaceous band that co-migrated with the recombinant human transferrin (S415A, T613A) band from Strain 1 [pDB3237].

The titres of the recombinant transferrin variants expressed after 5 days in 10mL BMMD shake flask were compared by rocket immunoelectrophoresis to that of Strain 1 [pDB3237] (Figure 18). The titres of secreted transferrin from the thiotransferrin variants appeared to be similar to Strain 1 [pDB3237] from strains Strain 1 [pDB3766], Strain 1 [pDB3767], Strain 1 [pDB3778], Strain 1 [pDB3769], Strain 1 [pDB3789], Strain 1 [pDB3770], Strain 1 [pDB3779], Strain 1 [pDB3757], Strain 1 [pDB3761], Strain 1 [pDB3773], Strain 1 [pDB3775], Strain 1 [pDB3758], Strain 1 [pDB3777] and Strain 1 [pDB3765]. Lower expression was detected from Strain 1 [pDB3771], Strain 1 [pDB3763], Strain 1 [pDB3760] Strain 1 [pDB3745] and Strain 1 [pDB3759] when compared by rocket immunoelectrophoresis to that of Strain 1 [pDB3237].

Thiotransferrin muteins created by insertion of a cysteine residue (the amino acid chain length is increased), substitution of two or more adjacent residues with a cysteine (the amino acid chain length is decreased) or substitution of an amino acid residue with a cysteine (the amino acid chain length is unchanged), deletion of a cysteine residue or combinations of the above as described in this invention are expressed as full length monomeric proteins with the same molecular weight as recombinant transferrin (S415A, T613A) and the same reactivity with a transferrin antibody (measured by rocket immunoelectrophoresis) demonstrating that amino acid modification to a cysteine residue do not adversely affect protein expression.

Fed-batch fermentations were carried out in a 10 L Braun Biostat E fermenter at 30°C; pH was monitored and adjusted by the addition of ammonia or sulphuric acid as appropriate. The ammonia also provided the nitrogen source for the culture. The level of dissolved oxygen were monitored and linked to the stirrer speed to maintain the level at >20% of saturation. Inocula were grown in shake flasks in buffered minimal media. For the batch-phase the culture was inoculated into fermenter media (approximately 50% of the fermenter volume) containing 2% (w/v) sucrose. The feed stage is automatically triggered by a sharp rise in the level of dissolved oxygen. Sucrose was kept at growth-limiting concentrations by controlling the rate of feed to a set nominal growth rate. The feed consisted of fermentation media containing 50% (w/v) sucrose, all essentially as described by Collins, S.H., (1990) (S.H. Collins, Production of secreted proteins in yeast, in: T.J.R. Harris (Ed.) Protein production by biotechnology, Elsevier, London, 1990, pp. 61-77).

Culture supernatant was harvested by centrifugation. A two-step ion exchange chromatography procedure was used to prepare the mutant transferrin (Thiotransferrin). Culture supernatant was diluted with water to give a conductivity suitable for binding to the first column, such as SP-FF. In the case of SP-FF the culture supernatant is diluted to 3.0±0.3 mS/cm after adjustment to pH 5.0 with glacial acetic acid.

The first chromatographic step uses an SP-Sepharose Fast Flow (GE Healthcare) column (bed volume approx 400 ml, bed height 11 cm) equilibrated with 50 mM sodium acetate pH 5.0. Loading was 10-25 mg mutant transferrin/ml matrix. The column was washed with 3 column volumes of equilibration buffer and the protein was eluted with approximately 2 column volumes of 50 mM sodium phosphate, 25 mM sodium chloride pH 7.0. The linear flow rate was 330 cm/h during the loading and wash and 165 cm/h during the elution.

For the second step the eluate was diluted approximately 3-fold, to give a conductivity of < 3.0±0.3 mS/cm after adjustment to pH 9.0-9.3 with sodium hydroxide. This was loaded on to a DEAE-Sepharose Fast Flow (GE Healthcare) column (bed volume approx 800 ml, bed height 11 cm) equilibrated with 15.7 mM potassium tetraborate pH 9.2. Loading was 5-10 mg mutant transferrin/ml matrix. The column was washed with 5 column volumes of equilibration buffer and eluted with 4 - 5 column volumes of 60 mM potassium tetraborate pH 9.35. The linear flow rate was 286 cm/h during loading and wash, 264 cm/h during elution. The eluate was concentrated and diafiltered against 10 mM HEPES buffer using a Pall Centramate Omega® 10,000 NMWCO membrane, to give a final concentration of approximately 20 mg/ml, and stored at -80 °C.

Recombinant thiotransferrin mutant protein concentration was determined by reverse phase high performance liquid chromatography (RP-HPLC) using an Agilent 1100 binary gradient system equipped with UV detection under Shimadzu VP7.3 client server software control. Injections of up to 100µL were made onto a Phenomenex Jupiter C4 300A (50 × 4.6 mm, 5 µm) at 45°C at a flow rate of 1ml/min comprising mobile phase A (0.1 % TFA, 5% Acetonitrile in water), mobile phase B (0.1% TFA, 95% Acetonitrile in water) using a gradient time program of 0 to 3 minutes at 30% B, 3 to 13 minutes from 30 to 55% B (linear gradient), 13 to 14 minutes at 55% B, 14 to 15 minutes 55 to 30% B (linear gradient), 15 to 20 minutes 30% B (isocratic). Peak detection is performed at an absorbance of 214 nm and quantified against a human transferrin (Calbiochem) standard curve from 0.1 to 10µg.

High cell density fed-batch fermentation of Strain 1 [pDB3778] expressing thiotransferrin (S32C, S415A, T613A) variant, Strain 1 [pDB3779] expressing thiotransferrin (A215C, S415A, T613A) variant, and Strain 1 [pDB3758] expressing thiotransferrin (S415A, N553C, T613A) variant gave yields of 1.95, 1.75 ,and 0.61 mg. mL-1 respectively (n=1). High cell density fed-batch fermentation of Strain 1 [pDB3767] expressing thiotransferrin (S28C, S415A, T613A) gave yields ~1.67 mg.mL⁻¹ (n=2) and Strain 1 [pDB3773] expressing thiotransferrin (S415C, T613A) gave yields ~1.06 mg.mL⁻¹ (n=2).

A holoisation procedure was used to prepare iron loaded recombinant thiotransferrin variant proteins. Sodium bicarbonate was added to purified thiotransferrin samples to give a final concentration of 20 mM. The amount of iron (in the form of ammonium iron citrate at 10 mg.mL⁻¹ (16.5-18.5% Fe) to target 2 mol Fe³⁺.mol⁻¹ transferrin was calculated, added to the recombinant transferrin / 20 mM sodium bicarbonate preparation and allowed to mix for a minimum of 60 minutes at ambient temperature (21-25 °C) followed by ultrafiltration into 145 mM NaCl.

### EXAMPLE 3: TRANSFERRIN RECEPTOR BINDING

Transferrin receptor binding was determined as follows.

### ⁵⁵Fe uptake competition in erythroleukemic K562 cells

Human plasma-derived apo-transferrin (Calbiochem 616419, tissue culture grade, pyrogen-free) is supplied lyophilised from 10mM phosphate buffer, pH7.4. The transferrin mutein is expressed, purified and quantified as described above.

For iron-55 uptake from labeled diferric transferrin, K562 erythroleukemic cells, cultured in RPMI cell culture medium under standard conditions (bicarbonate-buffered, 5% (v/v) CO₂, antibiotics, 10% (v/v) fetal calf serum) are washed with serum-free medium containing HEPES-buffer and 1 mg/ml of bovine serum albumin and used at a concentration of 10 million cells/ml in this medium. The samples tested are prepared as equimolar concentrations of apo-transferrin mutein. Apo-transferrin mutein is prepared by dialysis against 0.1 M citrate, 0.1 M acetate, 10 mM EDTA pH 4.5. Solutions are filtered (0.22µm), concentrated to 10 mg/ml using a Vivaspin polyethersulphone 10,000 NMWCO centrifugal concentrator and diafiltered against 10 volumes water followed by 10 volumes of 0.1 M HEPES, 0.1 M NaHCO₃ pH 8.0. Samples are recovered from the concentrator with a rinse and made up to a final concentration of 5 mg/ml. Transferrin mutein is loaded with iron according to a standard procedure (Bates and Schlabach, J Biol Chem, 248, 3228-3232, 1973) using ferric nitrilotriacetate as iron source. Typically 50µl of 1 M NaClO₄ is added to 450µl of the transferrin mutein stock-solution (pH is alkaline to neutral). The ⁵⁵Fe -NTA-loading-buffer is prepared by mixing 8.5µl 50mM NTA (pH 8.25), 18.9µl 0.1M Tris, 98.8µl Millipore-purified water and 7.5µl ⁵⁵FeCl₃ in 0.5 N HCl (NEN products). 500µl of transferrin mutein is carefully mixed (dropwise) with the ⁵⁵Fe -NTA-loading-buffer and then 310µl of 5mM Hepes. NaOH/0.1M NaClO₄ is added. The mixture is incubated at 4°C for 60 min following which it is deslated on a PD-10 column (containing Sephadex G-25) and dialysed. The PD-10 column is equilibrated with 5ml of equilibration buffer (5mM Hepes/NaOH, 0.1 M NaClO₄, 0.1 g BSA (Amresco)) then washed three times with 5ml of of wash buffer (5mM Hepes/NaOH, 0.1M NaClO₄). Iron (⁵⁵Fe)-loaded transferrin mutein is loaded on to the column and eluted with elution buffer (5mM Hepes/NaOH, 0.1 M NaClO₄). The eluted iron (⁵⁵Fe)-loaded transferrin mutein is dialysed at 4°C against 5mM Hepes, 0.15 mM NaCl, pH 7.4.

Increasing concentrations of human plasma transferrin or the transferrin mutein sample (0, 25, 100, 200, 400, 800, 1600 nM), labeled with ⁵⁵Fe, are mixed with 25 µl of medium. The reaction is started by the addition of 300 µl of cell suspension. A second series of parallel experiments is carried out in the presence of a hundredfold excess of unlabeled diferric transferrin to account for unspecific binding. After 25 minutes at 37°C the reaction is stopped by immersion into an ice-bath, three aliquots of 60µl of cell suspension are transferred to new tubes and the cells are centrifuged in the cold and again after addition of an oil layer of diethylphtalate/dibutylphthalate. The supernatant is removed, the cell pellet transferred into a counter vial and lysed with 0.5 M KOH + 1% (v/v) Triton X-100. The lysates are neutralized with 1 M HCl after overnight lysis, mixed with Readysolv scintillation cocktail and counted in the Packard Liquid Scintillation Counter. The results are typically presented as fmol ⁵⁵Fe/million cells.

### Competition of iron uptake into human K562 cells by transferrin mutein and radiolabelled plasma transferrin

Additionally a competition assay is performed using a constant concentration of ⁵⁵Fe-loaded plasma transferrin (100nM) with non-radioactive labelled holotransferrin mutein in concentrations ranging from 0 to 1600nM (0, 25, 100, 200, 400, 800, 1600 nM). Iron (⁵⁵Fe)-loaded plasma transferrin is prepared by described above. K562 cells are added to the incubation mixture to give a cell density of 10⁶cells/ml. RPMI-medium containing 0.1% (w/v) of bovine serum albumin and 10mM Hepes is used for dilution of transferrins and cells. After 25 minutes at 37°C the reaction is stopped by immersion into an ice-bath, three aliquots of 60µl of cell suspension are transferred to new tubes and the cells are centrifuged in the cold and again after addition of an oil layer of diethylphtalate/dibutylphthalate. The supernatant is removed, the cell pellet transferred into a counter vial and lysed with 0.5 M KOH, 1% (v/v) Triton X-100. The lysates are neutralized with 1M HCl after overnight lysis, mixed with Readysolv scintillation cocktail and counted in the Packard Liquid Scintillation Counter. The results are presented as fmol ⁵⁵Fe/million cells.

A thiotransferrin is considered to bind to a transferrin receptor it has at least 5 % of the receptor binding capacity of the corresponding polypeptide without Cys insertion.

### EXAMPLE 4: THREE-DIMENSIONAL MODEL BUILDING

In order to obtain a reasonable model of transferrin, where binding areas for both iron and receptor, as well as surface exposed areas can be identified, the following approach was used:

The initial model building was done in Pymol (Warren L. DeLano "The PyMOL Molecular Graphics System.", DeLano Scientific LLC, San Carlos, CA, USA. http://www.pymol.org). Two structures were used as templates:
1. Chain A of Apo-Human Serum Transferrin, PDB entry 2HAV
2. Human Transferrin Receptor-Transferrin Complex, PDB entry 1SUV

The following steps were used in the model building:
1. Chains A and B of 1SUV were copied directly to the new model and denoted chain R and S, respectively.
2. A copy of chain A of 2HAV was aligned to the D and F chains of 1SUV, using the "align" command in Pymol. Only the C-alpha carbon atoms were used for the alignment. In this position, significant clashes with chain A and B of 1 SUV were observed, so the structure was moved manually to reduce the clashes. The result was copied as chain A of the new model.
3. Another copy of chain A of 2HAV was aligned to chain C of 1 SUV, using the "align" command in Pymol. Only residues up to number 332 of chain A of 2HAV were regarded, and only the C-alpha carbon atoms were used for the alignment. The result was copied as chain B of the new model.

The model was subjected to molecular mechanics simulations using the Gromacs 3.3 software (D. van der Spoel, E. Lindahl, B. Hess, G. Groenhof, A. E. Mark and H. J. C. Berendsen: GROMACS: Fast, Flexible and Free, J. Comp. Chem. 26 pp. 1701-1718 (2005)). A molecular dynamics cascade was employed:
1. 100 steps of steepest descents minimization
2. Embedding in a 16x16x16 nm solvent water box, using periodic boundary conditions.
3. 100 steps of steepest descents minimization.
4. 2 ns of NVT molecular dynamics at 300 K.

A snapshot of the structure was recorded after 400 ps of the molecular dymanics simulation. Three sets of data were obtained for each residue of the model:
1. The trajectory for the 2 ns molecular dymanics simulation was precessed, and the root mean square fluctuations of the C-alpha carbon atoms during the last nanosecond of the simulation were calculated using the Gromacs tool "g_rmsf".
2. The solvent accessible surface area was calculated for each residue in the 400 ps snapshot structure, using the DSSP software (W.Kabsch and C.Sander, Biopolymers 22 (1983) 2577-2637). Each solvent accessible surface area was divided by a standard value for the particular amino acid found in that position and multiplied by 100, thereby obtaining a percentage of the standard value for each residue.
   The standard solvent accessible surface areas for the 20 different amino acids are defined as (using one-letter codes for the amino acids):
   A=62, C=92, D=69, E=156, F=123, G=50, H=130, I=84, K=174, L=97, M=103, N=85, P=67,Q=127, R=211,S=64,T=80,V=81,W=126,Y=104
3. The secondary structure was determined for each residue in the 400 ps snapshot structure, using the DSSP software (W.Kabsch and C.Sander, Biopolymers 22 (1983) 2577-2637). If the secondary structure is defined as H (Helix), B (isolated beta bridge) or E (Extended sheet), the residue is marked '1', otherwise as '0'

The data for chains R and S of the model were discarded. The data for chain A and chain B for each of the three data sets were collected.

### EXAMPLE 5: EXPRESSION OF A THIOTRANSFERRIN MUTANT PROTEIN CONTAINING MORE THAN ONE FREE THIOL GROUP.

In this example two free thiol groups are introduced by substitution of serine-28 to a cysteine residue and serine-415 to a cysteine residue. Secretion of thiotransferrin (S28C, S415C, T613A) variant was compared with secretion of thiotransferrin (S28C, S415A, T613A) variant, thiotransferrin (S415C, T613A) variant and transferrin (S415A, T613A). Construction of the thiotransferrin (S28C, S415C, T613A) variant expression plasmid pDB3809 is described in Example 1.

Strain 1 [pDB3809], Strain 1 [pDB3767], Strain 1 [pDB3773] and Strain 1 [pDB3237] were cultured for 5 days in 10mL BMMD shake flask. After centrifugation of the cells, the thiotransferrin (S28C, S415C, T613A) variant, thiotransferrin (S28C, S415A, T613A) variant, and thiotransferrin (S415C, T613A) variant proteins secreted into supernatant from Strain 1 [pDB3809], Strain 1 [pDB3767], Strain 1 [pDB3773] were compared to recombinant human transferrin (S415A, T613A) secretion from Strain 1 [pDB3237] by 4- 12% gradient SDS non-reducing gel (SDS-PAGE) and 4- 12% gradient SDS reducing gel (SDS-PAGE) (Figure 19). A proteinaceous band corresponding to the thiotransferrin (S28C, S415C, T613A) protein secreted from Strain 1 [pDB3709] was detected which co-migrated with the recombinant human transferrin (S415A, T613A), thiotransferrin (S28C, S415A, T613A), thiotransferrin (S415C, T613A) bands secreted from Strain 1 [pDB3767], Strain 1 [pDB3773] and Strain 1 [pDB3237] respectively, demonstrating that the thiotransferrin (S28C, S415C, T613A) protein secreted from Strain 1 [pDB3709] was secreted as monomeric protein.

The titres of the recombinant thiotransferrin (S28C, S415C, T613A) secreted from Strain 1 [pDB3709] after 5 days in 10mL BMMD shake flask were compared to that of recombinant thiotransferrin (S28C, S415A, T613A) secreted from Strain 1 [pDB3767], recombinant thiotransferrin (S415C, T613A) secreted from Strain 1 [pDB3773] and recombinant transferrin (S415A, T613A) secreted from Strain 1 [pDB3237] by rocket immunoelectrophoresis .(Figure 20) Rocket immunoelectrophoresis and SDS-PAGE analysis of recombinant thiotransferrin (S28C, S415C, T613A) secreted from Strain 1 [pDB3709] demonstrates that modification of more than one selected amino acid residues of transferrin protein to cysteine residues does not do not adversely affect protein expression of full-length monomeric protein.

### EXAMPLE 6: EXPRESSION OF A LACTOFERRIN AND THIOLACTOFERRIN MUTANT PROTEIN

Construction of the lactoferrin (T139A, T480A, S625A) and thiolactoferrin (T139A, S421C, T480A, S625A) expression plasmids pDB3818-20 is described in Example 1.

Strain 1 [pDB3818], Strain 1 [pDB3719], Strain 1 [pDB3720], Strain 1 [pDB3773] and Strain 1 [pDB3237] were cultured for 5 days in 10mL BMMD shake flask. After centrifugation of the cells, the lactoferrin (T139A, T480A, S625A) and thiolactoferrin (T139A, S421C, T480, S625) variant proteins secreted into supernatant from Strain 1 [pDB3818], Strain 1 [pDB3719] and Strain 1 [pDB3720] were compared to recombinant human transferrin (S415A, T613A) secretion from Strain 1 [pDB3237] and thiotransferrin (S415C, T613A) expression from Strain 1 [pDB3773] by 4- 12% gradient SDS non-reducing gel (SDS-PAGE) and 4-12% gradient SDS reducing gel (SDS-PAGE) (Figure 21).

A proteinaceous band corresponding to the lactoferrin (T139A, T480A, S625A) protein secreted from both Strain 1 [pDB3818] and Strain 1 [pDB3819] was detected which migrated at approximately 80 kDa indicating that lactoferrin (T139A, T480A, S625A) can be expressed from a microbial host; expression plasmids may or may not be codon optimised for expression in microbial host. The the serine residue at position 421 on lactoferrin protein sequence is equivalent to the serine residue at position 415 on the transferrin protein sequence (see example 1), thus thiolactoferrin (T139A, S421C, T480A, S625A) expression from Strain 1 [pDB3820] was compared thiotransferrin (S415C, T613A) expression from Strain 1 [pDB3773] (Figure 16). A proteinaceous band corresponding to the lactoferrin (T139A, S421C, T480A, S625A) protein secreted from Strain 1 [pDB3820] was also detected which co-migrated with lactoferrin (T139A, T480A, S625A) demonstrating that modification of a selected amino acid residue to a cysteine residue does not adversely affect protein expression and that other transferrin family proteins may also be used in this invention.

### EXAMPLE 7: IRON BINDING OF THIOTRANSFERRIN MUTANT PROTEINS

The ability of thiotransferrin mutant proteins described in this invention to bind iron was determined using two experimental procedures, urea gel analysis and a spectrophotometric assay for total iron binding capacity and compared to that of recombinant human transferrin (S415A, T613A) standard.

The Iron Binding capability of the recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A) and recombinant thiotransferrin (S415A, N553C, T613A) was compared to that of purified recombinant human transferrin (S415A, T613A) standard. Recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A) and recombinant thiotransferrin (S415A, N553C, T613A) were iron loaded as described in Example 2.

5 µg samples were separated on 6% TBE Urea PAGE (Invitrogen) and stained with Coomassie G250 (Pierce) (Figure 22). This technique separates four molecular forms with different iron loadings namely (in order of increasing mobility) apo-transferrin, C-lobe and N-lobe bound monoferric transferrins and holo-transferrin. Separation of the four forms of transferrin is believed to be due to partial denaturation in 4-6M urea; where iron binding in any lobe causes a change in conformation resulting in increased resistance to denaturation. Thus the presence of iron in a lobe results in a more compact structure with higher electrophoretic mobility. Since the N-lobe has fewer disulphide bonds than the C-lobe (8 versus 11 respectively) it unfolds further in the absence of iron, making the monoferric form with iron bound to the C-lobe the least mobile.

The thiotransferrin variants were able to bind iron, however under the experimental conditions purified recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A) and recombinant thiotransferrin (S415A, N553C, T613A) (lanes 3-7 in Figure 22) did not appear to be fully saturated with iron and showed bands that migrated through the analytical TBE Urea gel more slowly than recombinant transferrin (S415A, T613A) (lane 2 in Figure 22) and heterogeneity was observed. One of the proteinaceous bands co-migrated with the holo-transferrin form of recombinant human transferrin (S415A, T613A) band corresponding to diferric transferrin. Thiotransferrin variants also appeared to contain a fraction of monoferric transferrin. Some care is required in the interpretation of these results. The presence of the band corresponding to monoferric (thio)transferrin variants showed that iron addition did not result in the higher electrophoretic mobility typical of saturated diferric transferrin. However, since the technique depends upon the stabilisation on iron binding, it is not a safe conclusion that the mutation prevents iron binding; destabilisation of the lobe to urea denaturation would give the same result.

Total iron-binding capacity (TIBC) recombinant thiotransferrin described in this invention and recombinant human transferrin(S415A, T613A) (Deltaferrin^{™}) standard was determined using a modified method for Determination of Serum Iron and Iron-Binding Capacity described by Caraway, 1963 Clinical Chem 9 (2), 188, as described in the present application.

The Total iron-binding capacity (TIBC) of recombinant human transferrin (S415A, T613A) was measured as 2.14. The total iron binding capacity of recombinant thiotransferrin (S28C, S415A, T613A) variant was measured as 1.91 recombinant thiotransferrin (A215C, S415A, T613A) variant was measured as 2.23, recombinant thiotransferrin (S415A, N553C, T613A) variant was measured as 2.00, recombinant thiotransferrin (S415C, T613A) variant was measured as 2.18 and recombinant thiotransferrin (S32C, S415A, T613A) variant was measured as 2.42 (**Table 3**) indicating that modification of a selected residue to a cysteine does not alter the gross structure of transferrin mutein or prevent the thiotransferrin mutein being able to bind to iron.

### EXAMPLE 8: TRANSFERRIN RECEPTOR BINDING CAPABILITY OF RECOMBINANT THIOTRANSFERRIN VARIANTS COMPARED TO RECOMBINANT TRANSFERRIN (S415A, T613A)

The receptor binding capability of the recombinant transferrin variants was assessed by Surface Plasmon Resonance (SPR) analysis. The binding activity of a transferrin sample to transferrin receptor can be measured using surface plasmon resonance (SPR) a non- invasive optical technique in which the SPR response is a measure of change in mass concentration at the detector surface as molecules bind or dissociate. A sample is sent onto the surface of the sensor chip via a micro flow system at a constant flow rate. In this analysis, if the transferrin sample is able to bind to the TfR the mass on the surface sensor chip is increased due to binding between TfR and Tf molecules creating a surface plasmon wave, and a shift of the SPR signal proportional to the binding quantity can be detected as a change in the resonance unit (RU). A response of 1 RU is equivalent to change in a surface concentration of about 1 pg .mm⁻².

Biacore sensor chips for interaction analysis between transferrin and the transferrin receptor were prepared by first immobilizing Transferrin receptor antibody prior to addition of the transferrin. Specifically, anti- Transferrin receptor (anti-TfR) antibody was immobilized to the CM5 sensor chip surface (GE Healthcare catalogue number BR-1000-14) using amine coupling chemistry at 25 °C. The carboxymethylated dextran surface on a CM5 sensor chip flow cell were converted to active succinamide esters by the addition of *N*-hydroxysuccinimide:*N*-ethyl-N'-(dimethylaminopropyl) carbodiimide (NHS:EDC).

**Table 3:**

| Sample | Replicate | Protein post clarification | Absorbance | Iron Conc. | | | |
|---|---|---|---|---|---|---|---|
| | | (g/L) | (595nm) | (mg/L) | (mg.g protein) | Mean (mg.g protein) | (mole.mole) |
| Blank | 1 | 0.000 | 0.019 | 0.689 | | | |
| | 2 | 0.000 | 0.020 | 0.726 | | | |
| | 3 | 0.000 | 0.021 | 0.764 | | | |
| Transferrin (S415A, T613A) | 1 | 0.830 | 0.053 | 1.967 | 1.494 | 1.595 | 2.14 |
| | 2 | 0.826 | 0.057 | 2.117 | 1.684 | | |
| | 3 | 0.842 | 0.056 | 2.079 | 1.607 | | |
| Thiotransferrin (S28C, S415A, T613A) | 1 | 0.648 | 0.046 | 1.703 | 1.508 | 1.420 | 1.91 |
| | 2 | 0.665 | 0.044 | 1.628 | 1.357 | | |
| | 3 | 0.647 | 0.044 | 1.628 | 1.394 | | |
| Thiotransferrin (A215C, T613A) | 1 | 0.548 | 0.041 | 1.515 | 1.440 | 1.661 | 2.23 |
| | 2 | 0.497 | 0.042 | 1.553 | 1.664 | | |
| | 3 | 0.500 | 0.045 | 1.666 | 1.879 | | |
| Thiotransferrin (S415A, N553C, T613A) | 1 | 0.366 | 0.034 | 1.252 | 1.438 | 1.485 | 2.00 |
| | 2 | 0.377 | 0.034 | 1.252 | 1.396 | | |
| | 3 | 0.371 | 0.036 | 1.328 | 1.621 | | |
| Thiotransferrin (S415C, T613A) | 1 | 0.689 | 0.050 | 1.854 | 1.637 | 1.624 | 2.18 |
| | 2 | 0.708 | 0.049 | 1.816 | 1.540 | | |
| | 3 | 0.732 | 0.053 | 1.967 | 1.695 | | |
| Thiotransferrin (S32C, S415A, T613A) | 1 | 0.325 | 0.035 | 1.290 | 1.735 | 1.804 | 2.42 |
| | 2 | | | | | | |
| | 3 | 0.321 | 0.036 | 1.328 | 1.874 | | |

The (Transferrin) receptor specific binding can be confirmed by concurrently preparing a sensor chip having an immobilized protein other than transferrin receptor and deducting the change in the resonance unit when the sample specimen is allowed to flow onto this chip to exclude a so-called bulk effect by a solvent or the like. The Anti-TfR antibody (AbD Serotec catalogue number MCA1148) was diluted to 10 µg.mL⁻¹ in 10mM sodium acetate pH 5.0 (GE Healthcare catalogue number BR-1003-50) and injected over flow cell 2 only. Whereas 50 µL of the transferrin receptor (TfR) (AbD Serotec catalogue number 9110-300) (diluted in HBS-EP (10 mM HEPES, 150 mM NaCl, 3 mM EDTA, 0.005 % surfactant P-20, pH 7.4) to 10-20 µg.mL⁻¹) was injected over both flow cells. Excess ester groups on sensor chip surface were deactivated using ethanolamine hydrochloride (1 M pH 8.5).

HBS-EP was used as running buffer and dilution buffer for interaction analysis. Purified iron-loaded recombinant transferrin (S415A, T613A) or recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A), and recombinant thiotransferrin (S415A, N553C, T613A) purified by the first chromatographic step was diluted to 20 µg.mL⁻¹ and 50 µL injected over both flow cells. Replicates were carried out to ensure reproducibility, The prepared Biacore sensor chip surface was regenerated between addition purified recombinant transferrin variants by 6-12 s injections of 10 mM sodium acetate pH 4.5 (GE Healthcare catalogue number BR-1003-50) between sample injections. Up to three injections were made, as required until baseline was restored.

Samples of recombinant thiotransferrin (S28C, S415A, T613A), recombinant thiotransferrin (S32C, S415A, T613A), recombinant thiotransferrin (A215C, S415A, T613A), recombinant thiotransferrin (S415C, T613A) and recombinant thiotransferrin (S415A, N553C, T613A) purified by the first chromatographic step were compared to purified iron-loaded recombinant transferrin (S415A, T613A) for their ability to bind the Transferrin receptor.

A thiotransferrin is considered to bind to a transferrin receptor it has at least 5 % of the receptor binding capacity of the corresponding polypeptide without cysteine insertion. All thiotransferrin muteins were able to bind the transferrin receptor by qualitative SPR analysis.

### EXAMPLE 9: MASS SPECTROMETRY OF RECOMBINANT THIOTRANSFERRIN VARIANTS

For this example samples of purified recombinant thiotransferrin variants were prepared as in example 2 purified by the first chromatographic step, analysed by ESITOF mass spectrometry and compared to that of purified transferrin (S415A, T613A).

Samples were prepared for mass spectrometry as aqueous solutions of test proteins which were desalted/concentrated using reversed phase (RP-) HPLC with recovered protein at concentrations of typically 20-100 nmol/mL. The RP-HPLC desalting was carried on a Brownlee Aquapore BU-300(C4)7mm, 100x2.1 mm column, the method utilised a binary gradient of 0.1 %(v/v) Trifluoracetic acid (TFA) as solvent A and 70%(v/v) acetonitrile, 0.1 %(v/v) TFA as solvent B with collection of eluting components detected by UV absorbance at 280 nm. For Time-of-Flight mass spectrometry samples were introduced into a hybrid quadrupole time-of flight mass spectrometer (QqOaTOF, Applied Biosystems, OSTAR-XL®), equipped with an IonSprayTM source in positive ion mode, using flow injection analysis (FIA). The only instrument parameter that was actively tuned was the Decoupling Potential (DP) this was typically set to 250V Typically 2 minutes of sample scans were averaged. For protein analysis the TOF analyser was calibrated against protonated molecular ions of equine myoglobin (Sigma) and resolution was typically 12,000. Instrument control and data acquisition and processing were performed using AnalystTM QS v1.1 software (Applied Biosystems).

Figure 23 shows mass spectra of thiotransferrin (S28C, S415A, T613A) variant, thiotransferrin (A215C, S415A, T613A) variant, thiotransferrin (S415C, T613A) variant, thiotransferrin (S415A, N553C, T613A) variant and thiotransferrin (S32C, S415A, T613A) variant compared with transferrin (S415A, T613A). Mass spectrometric analysis of transferrin (S415A, T613A) shows two peaks. In this case one peak (marked "A" in Figure 23) is that corresponding to the unmodified transferrin (S415A, T613A) molecule with a nominal mass of 75097 (theoretical mass 75098Da) (Figure 23). There is also a large peak (marked "B" in Figure 23) with the expected 162 Dalton increment for a single hexose addition. This probably represents O-linked glycosylation.

Spectra B shows the mass spectrum of thiotransferrin (S28C, S415A, T613A) variant from high cell density fermentation of Strain 1 [pDB3767] purified by the first chromatographic step. A mass of 75114 is expected when the serine residue at position 28 is modified to a cysteine, and the molecule is folded such that 38 cysteine residues are disulfided bonded and there is one free thiol. In this case the largest single peak (marked "C" in Figure 23) is that corresponding to the unmodified molecule with a nominal mass of 75116 (theoretical mass 75114Da). There is a large peak (marked "D" in Figure 23) with the expected 162 Dalton increment for a single hexose addition. This probably represents O-linked glycosylation. Spectra C shows the mass spectrum of thiotransferrin (A215C, S415A, T613A) variant from high cell density fermentation of Strain 1 [pDB3779] purified by the first chromatographic step. A mass of 75130 is expected when the alanine residue at position 215 is modified to a cysteine, and the molecule is folded such that 38 cysteine residues are disulfided bonded and there is one free thiol. In this case the largest single peak (marked "E" in Figure 23) is that corresponding to the unmodified molecule with a nominal mass of 75130 (theoretical mass 75130 Da). There is a large peak (marked "F" in Figure 23) with the expected 162 Dalton increment for a single hexose addition. This probably represents O-linked glycosylation.

Spectra D shows the mass spectrum of thiotransferrin (S415C, T613A) variant from high cell density fermentation of Strain 1 [pDB3773] purified by the first chromatographic step. A mass of 75130 is expected when the serine residue at position 415 is modified to a cysteine, and the molecule is folded such that 38 cysteine residues are disulfided bonded and there is one free thiol. In this case the largest single peak (marked "G" in Figure 23) is that corresponding to the unmodified molecule with a nominal mass of 75127 (theoretical mass 75130 Da). There is a large peak (marked "H" in Figure 23) with the expected 162 Dalton increment for a single hexose addition. This probably represents O-linked glycosylation.

Spectra E shows the mass spectrum of thiotransferrin (S415A, N553C, T613A) variant from high cell density fermentation of Strain 1 [pDB3758] purified by the first chromatographic step. A mass of 75087 is expected when the asparagine residue at position 553 is modified to a cysteine, and the molecule is folded such that 38 cysteine residues are disulfided bonded and there is one free thiol. In this case the largest single peak (marked "I" in Figure 23) is that corresponding to the unmodified molecule with a nominal mass of 75082 (theoretical mass 75087 Da). There is a large peak (marked "J" in Figure 23) with the expected 162 Dalton increment for a single hexose addition. This probably represents O-linked glycosylation.

Spectra F shows the mass spectrum of thiotransferrin (S32C, S415A, T613A) variant from high cell density fermentation of Strain 1 [pDB3778] purified by the first chromatographic step. Mass spectrometric analysis of thiotransferrin (S32C, S415A, T613A) shows only one main peak. A mass of 75114 is expected when the serine residue at position 32 is modified to a cysteine, and the molecule is folded such that 38 cysteine residues are disulfided bonded and there is one free thiol. In this case the largest single peak (marked "K" in Figure 23) is that corresponding to the unmodified molecule with a nominal mass of 75110 (theoretical mass 75114Da). In contrast to the other transferrin/thiotransferrin mass spectrometric analyses no additional peak corresponding to a 162 Dalton increment for a single hexose addition was detected indicating that mutation of serine-32 to a cysteine prevented O-linked glycosylation at this position.

### EXAMPLE 10: MASS SPECTROMETRY OF RECOMBINANT THIOTRANSFERRIN VARIANTS TREATED WITH ELLMAN'S REAGENT (5'5'-DITHIO-BIS(2-NITRONENZOIC ACID) (DTNB)

The number of free thiols on a protein can be determined spectrophotometrically using Ellman's reagent. Ellman's reagent (5'5'-dithio-bis(2-nitronenzoic acid) (DTNB)) is an aromatic disulphide which reacts with thiol groups to form a mixed disulphide of the protein and one mole of 2-nitro-5-thio-benzoate (TNB) (per mole of protein sulphidyl group). Alternatively the number of free thiols on a protein can be determined using mass spectrometric analysis of protein sample treated with DTNB reagent. 5-thio-2-nitrobenzoic acid (TNB) has a molecular weight of 199Da, thus an increase in mass of 197Da (TNB minus H₂ lost during disulphide bridge formation with the free thiol group on the test protein) indicates presence of one free thiol group on the protein sample.

100µl of the test protein sample (20 mg.mL⁻¹) was added to 100 µl Buffer 2 (4 mg.mL⁻¹ DTNB and 500mM Sodium Phosphate, pH 7.0) and 900µl Buffer 1 (0.1 M TRIS-HCl, 100mM EDTA, pH8.0). The preparation was allowed to mix 25 minutes at ambient temperature (21-25 °C) followed by filtration through a low molecular mass cut-off filter (Vivaspin 2 - 10000 MWCO Sartorius Stedim Germany). The filter was washed with two volumes of 0.1% Trifluoroacetic acid (TFA) and the sample was resuspended in 300µl of 0.1 % TFA. TNB labelled samples were prepared for mass spectrometric analysis by desalting/concentrating using reversed phase (RP-) HPLC. The RP-HPLC desalting is carried on a Brownlee Aquapore BU-300(C4)7mm, 100x2.1mm column, the method utilises a binary gradient of 0.1 % (v/v) Trifluoracetic acid (TFA) as solvent A and 70 % (v/v) acetonitrile , 0.1 % (v/v) TFA as solvent B with collection of eluting components detected by UV absorbance at 280 nm.

For Time-of-Flight mass spectrometry samples were introduced into a hybrid quadrupole time-of flight mass spectrometer (QqOaTOF, Applied Biosystems, OSTAR-XL®), equipped with an IonSprayTM source in positive ion mode, using flow injection analysis (FIA) as described in Example 9.

Figure 24 shows mass spectra of thiotransferrin (S28C, S415A, T613A) variant, thiotransferrin (A215C, S415A, T613A) variant, thiotransferrin (S415C, T613A) variant, thiotransferrin (S415A, N553C, T613A) variant and thiotransferrin (S32C, S415A, T613A) variant treated with DTNB compared with transferrin (S415A, T613A) treated with DTNB. Mass spectrometric analysis of transferrin (S415A, T613A) treated with DTNB shows two peaks. In this case one peak (marked "A" in Figure 24) is that corresponding to the unmodified transferrin (S415A, T613A) molecule with a nominal mass of 75097 (theoretical mass 75098Da) (Figure 24)). There is also a large peak (marked "B" in Figure 24) with the expected 162 Dalton increment for a single hexose addition. This probably represents O-linked glycosylation. A mass shift of 195 Da was not detected in the mass spectra confirming that all 38 cysteine residues in transferrin (S415A, T613A) (DeltaferrinTM standard) are disulfide bonded and that no free thiol groups are present.

Spectrum B shows the mass spectrum of thiotransferrin (S28C, S415A, T613A) variant from high cell density fermentation of Strain 1 [pDB3767] purified by the first chromatographic step which has been treated with DTNB. A mass of 75311 is expected when the serine residue at position 28 is modified to a cysteine, and the molecule is folded such that 38 cysteine residues are disulfided bonded and one mole of NTB is bound to the thiol group. In this case the largest single peak (marked "C" in Figure 24) is that corresponding to one mole of NTB bound to thiotransferrin molecule with a nominal mass of 75311 (theoretical mass 75311 Da). There is a large peak (marked "D" in Figure 24) with the expected 162 Dalton increment for a single hexose addition. This probably represents O-linked glycosylation.

Spectrum C shows the mass spectrum of thiotransferrin (A215C, S415A, T613A) variant from high cell density fermentation of Strain 1 [pDB3779] purified by the first chromatographic step which has been treated with DTNB. A mass of 75327 is expected when the alanine residue at position 215 is modified to a cysteine, and the molecule is folded such that 38 cysteine residues are disulfided bonded and one mole of NTB is bound to the thiol group. In this case the largest single peak (marked "E" in Figure 24)) is that corresponding to one mole of NTB bound to thiotransferrin molecule with a nominal mass of 75325 (theoretical mass 75327 Da). There is a large peak (marked "F" in Figure 24) with the expected 162 Dalton increment for a single hexose addition. This probably represents O-linked glycosylation.

Spectrum D shows the mass spectrum of thiotransferrin (S415C, T613A) variant from high cell density fermentation of Strain 1 [pDB3773] purified by the first chromatographic step which has been treated with DTNB. A mass of 75327 is expected when the serine residue at position 415 is modified to a cysteine, and the molecule is folded such that 38 cysteine residues are disulfided bonded and one mole of NTB is bound to the thiol group. In this case the largest single peak (marked "G" in Figure 24)) is that corresponding to one mole of NTB bound to thiotransferrin molecule with a nominal mass of 75324 (theoretical mass 75327 Da). There is a large peak (marked "H" in Figure 24) with the expected 162 Dalton increment for a single hexose addition. This probably represents 0-!inked glycosylation.

Spectrum E shows the mass spectrum of thiotransferrin (S415A, N553C, T613A) variant from high cell density fermentation of Strain 1 [pDB3758] purified by the first chromatographic step which has been treated with DTNB. A mass of 75284 is expected when the asparagine residue at position 553 is modified to a cysteine, and the molecule is folded such that 38 cysteine residues are disulfided bonded and one mole of NTB is bound to the thiol group. In this case the largest single peak (marked "I" in Figure 24) is that corresponding to one mole of NTB bound to thiotransferrin molecule with a nominal mass of 75281 (theoretical mass 75284 Da). There is a large peak (marked "J" in Figure 24) with the expected 162 Dalton increment for a single hexose addition. This probably represents O-linked glycosylation.

Spectrum F shows the mass spectrum of thiotransferrin (S32C, S415A, T613A) variant from high cell density fermentation of Strain 1 [pDB3778] purified by the first chromatographic step which has been treated with DTNB. Mass spectrometric analysis of thiotransferrin (S32C) shows only one main peak. A mass of 75311 is expected when the serine residue at position 32 is modified to a cysteine, and the molecule is folded such that 38 cysteine residues are disulfided bonded and one mole of NTB is bound to the thiol group. In this case the largest single peak (marked "K" in Figure 24) is that corresponding to one mole of NTB bound to thiotransferrin molecule with a nominal mass of 75307 (theoretical mass 75311 Da). This result indicated that mutation of serine-32 prevented O-linked glycosylation at this position.

The mass spectrometry analysis of DTNB treated thiotransferrin variants confirms each of the thiotransferrin muteins has one free thiol, whereas the transferrin (S415A, T613A) standard cannot be labelled using DTNB under the same experimental conditions.

### EXAMPLE 11: CONJUGATION OF HORSERADISH PEROXIDASE PROTEIN TO THIOTRANSFERRIN VARIANTS

The thiotransferrins of the invention were assayed for their ability to be covalently linked to a bioactive compound by methods known to the art. Maleimide groups react predominantly with sulfhydryls at pH 6.5-7.5 forming a stable thioether bond. Maleimide labeling reagents can be used to label protein molecules containing free thiol groups with bioactive molecules such as proteins, drugs and imaging agents. At pH 7, the maleimide group is ~1,000 times more reactive toward a thiol group than to an amine group (Pierce). Purified recombinant human transferrin (Deltaferrin^{™}) and thiotransferrin variants purified by the first chromatographic step were diluted to 50 µg.mL⁻¹ in phosphate buffered saline (PBS) and mixed with a 4 fold molar excess of EZ-Link® Maleimide Activated Horseradish Peroxidase (Pierce) overnight at 4°C. Proteins were separated by non-reducing 4-12% SDS-PAGE, and stained using GelCode^{®} Blue reagent (Pierce) (Figure 25). A proteinaceous band that co-migrated with the recombinant human transferrin (Deltaferrin^{™}) (labelled A in lane 2 of Figure 25) was detected for each of the Thiotransferrin variant proteins which had not been reacted with EZ-Link® Maleimide Activated Horseradish Peroxidase (Pierce) (labelled A in lane 4, 6, 9, 11, and 13 of Figure 25). A band corresponding to un-reacted EZ-Link® Maleimide Activated Horseradish Peroxidase (Pierce) was detected in the lanes of samples treated with EZ-Link® Maleimide Activated Horseradish Peroxidase (Pierce) (labelled B in Lanes 3, 5, 7, 10, 12 and 14 of Figure 25). A feint band was also detected labelled C in lane 2 of Figure 25 which was thought to correspond to none specific binding of maleimide to transferrin (S415A, T613A) at primary amine groups. A more prominent band corresponding to thiotransferrin variant conjugated to Horseradish Peroxidase was detected in the lanes of samples treated with EZ-Link® Maleimide Activated Horseradish Peroxidase (Pierce) (labelled C in Lanes 3, 5, 7, 10, 12 and 14 of Figure 25). This example demonstrates that the thiotransferrin mutein proteins which contain one or more unpaired cysteine residues such as to introduce free thiol groups described in this invention can be covalently linked to bioactive molecules such as maleimide activated proteins.

### EXAMPLE 12: CONJUGATION OF FLUORESCEIN TO THIOTRANSFERRIN VARIANTS

Thiotransferrin (S28C, S415A, T613A) and thiotransferrin (S415C, T613A) were assayed for their ability to be covalently linked to fluorescein-5-maleimide by methods known to the art. Maleimide groups react predominantly with sulfhydryls at pH 6.5-7.5 forming a stable thioether bond. At pH 7, the maleimide group is ~1,000 times more reactive toward a thiol group than to an amine group (Pierce).

Thiotransferrin variants prepared as described in Example 2 were subjected to size exclusion chromatography prior to conjugation with fluorescein-5-maleimide. There is a possibility that in the thiotransferrin preparations described in Example 2 that thiotransferrin dimers are present. It is therefore advantageous to include a size exclusion chromatography as a polishing step, despite the high degree of purity that may be achieved using the method described herein. The polishing step removes low or trace levels of these contaminants.

A column (26 x 920mm, 488mL) was packed with Superdex 200 Prep Grade media (GE Healthcare). The column was sanitised with 0.5 M NaOH prior to loading of protein samples. Thiotransferrin (S28C, S415A, T613A) or Thiotransferrin (S415C, T613A) prepared by the protocol described in example 2 were loaded onto the column.

Sample loading sizes were 2% of the column volume. Flow rates were 3.5mL.min⁻¹. The running buffer was Dulbecco's phosphate buffered saline (DPBS, Sigma) or any other buffer in which the protein is stable and fractions collected every 1 minute (3.5mL fractions) across the peak. Each fraction was assayed for monomer content using a GP.HPLC method. The GP-HPLC method consisted of a Tosoh TSK3000GW_{xL} column run in 25 mM sodium phosphate, 0.1 M sodium sulphate, 0.05 % sodium azide, pH 7.0 at 1ml.min⁻¹ with a 25µL injection.

The thiotransferrin (S28C, S415A, T613A) and thiotransferrin (S415C, T613A) preparations which had been purified by the size exclusion chromatography step were divided in two. One sample was conjugated with fluorescein using fluorescein-5-maleimide, the other sample was stored as a 'unconjugated' thiotransferrin sample.

A sample of fluorescein conjugated thiotransferrin (S28C, S415A, T613A) was prepared by addition of approximately a 15 fold molar excess of fluorescein-5-maleimide to thiotransferrin (S28C, S415A, T613A) which was allowed to mix for 75 minutes at ambient temperature (21-25 °C).

A sample of fluorescein conjugated thiotransferrin (S415C, T613A) was prepared by addition of approximately a 15 fold molar excess of fluorescein-5-maleimide to thiotransferrin (S415C, T613A) which was allowed to mix for 110 minutes at ambient temperature (21-25 °C).

The fluorescein conjugated thiotransferrin (S28C, S415A, T613A), and unconjugated thiotransferrin (S28C, S415A, T613A), fluorescein conjugated thiotransferrin (S415C, T613A), fluorescein conjugated thiotransferrin (S415C, T613A) preparations were each divided into two samples. One sample was subjected to an iron loading step, whilst the other sample was subjected to a step which removed iron.

To prepare iron free recombinant fluorescein conjugated thiotransferrin proteins or recombinant unconjugated thiotransferrin proteins which had been further purified by the size exclusion chromatography step the sample was incubated in 0.1 M sodium citrate, 0.1 M sodium acetate, 10 mM EDTA pH 4.5 for a minimum of 180 minutes at ambient temperature, followed by ultrafiltration first into water to remove the stripped iron and then into 100 mM HEPES, 10 mM sodium carbonate buffer pH 8.0.

Iron free thiotransferrin mutein samples were prepared: iron free fluorescein conjugated thiotransferrin (S28C, S415A, T613A), iron free unconjugated thiotransferrin (S28C, S415A, T613A), iron free fluorescein conjugated thiotransferrin (S415C, T613A), and iron free unconjugated thiotransferrin (S415C, T613A). These samples were further analysed in Examples 13, 14 and 16.

The iron loading (holoisation) procedure described in Example 3 was used to prepare iron loaded recombinant fluorescein conjugated thiotransferrin proteins or recombinant unconjugated thiotransferrin proteins which had been further purified by the size exclusion chromatography step. Sodium bicarbonate was added to purified fluorescein thiotransferrin samples to give a final concentration of 20 mM. The amount of iron (in the form of ammonium iron citrate at 10 mg.mL⁻¹ (16.5-18.5% Fe) to target 2 mol Fe³⁺.mol⁻¹ transferrin was calculated, added to the recombinant transferrin / 20 mM sodium bicarbonate preparation and allowed to mix for a minimum of 60 minutes at ambient temperature (21-25 °C) followed by ultrafiltration into 145 mM NaCl.

Thus four iron loaded thiotransferrin mutein samples were prepared: iron loaded fluorescein conjugated thiotransferrin (S28C, S415A, T613A), iron loaded unconjugated thiotransferrin (S28C, S415A, T613A), iron loaded fluorescein conjugated thiotransferrin (S415C, T613A), and iron loaded unconjugated thiotransferrin (S415C, T613A). These samples were further analysed in Examples 13, 14 and 15.

### EXAMPLE 13: MASS SPECTROMETRY OF FLUORESCEIN CONJUGATED THIOTRANSFERRIN VARIANTS

Fluorescein conjugated recombinant thiotransferrin (S28C, S415A, T613A) and fluorescein conjugated recombinant thiotransferrin (S415C, T613A) were analysed by ESITOF mass spectrometry and compared to that of recombinant thiotransferrin (S28C, S415A, T613A) and recombinant thiotransferrin (S415C, T613A) Both iron loaded (holo) thiotransferrin and iron free (Apo) thiotransferrin (S28C, S415A, T613A) and thiotransferrin (S415C, T613A) preparations were analysed. Iron free thiotransferrin samples (data not shown) gave equivalent results to iron loaded (holo) samples shown.

Samples of fluorescein conjugated and unconjugated thiotransferrin (S28C, S415A, T613A) and thiotransferrin (S415C, T613A) prepared in Example 12 were prepared for mass spectrometry using the protocol described in Example 9.

Figure 26 shows mass spectra of unconjugated thiotransferrin (S28C, S415A, T613A) variant and fluorescein conjugated thiotransferrin (S28C, S415A, T613A) variant. Spectrum A shows the mass spectrum of thiotransferrin (S28C, S415A, T613A)). Mass spectrometric analysis of thiotransferrin (S28C, S415A, T613A) shows two peaks. A mass of 75114 is expected when the serine residue at position 28 is modified to a cysteine, and the molecule is folded such that 38 cysteine residues are disulfided bonded and there is one free thiol. In this case the largest single peak (marked "A" in Figure 26) is that corresponding to the unmodified molecule with a nominal mass of 75111 (theoretical mass 75114Da). There is a large peak (marked "B" in Figure 26) with the expected 162 Dalton increment for a single hexose addition. This probably represents O-linked glycosylation. Spectrum B shows the mass spectrum of fluorescein conjugated thiotransferrin (S28C, S415A, T613A). Mass spectrometric analysis of fluorescein conjugated thiotransferrin (S28C, S145A, T613A) shows two peaks. A mass of 75541 is expected when the serine residue at position 28 is modified to a cysteine, and the molecule is folded such that 38 cysteine residues are disulfided bonded and there is fluorescein molecule conjugated to the free thiol group. In this case the largest single peak (marked "A" in Figure 26) is that corresponding to the fluorescein conjugated molecule with a nominal mass of 75555 (theoretical mass 75541 Da). There is a large peak (marked "B" in Figure 26) with the expected 162 Dalton increment for a single hexose addition. This probably represents O-linked glycosylation.

Figure 27 shows mass spectra of unconjugated thiotransferrin (S28C, S415A, T613A) variant and fluorescein conjugated thiotransferrin (S28C, S415A, T613A) variant. Spectrum A shows the mass spectrum of thiotransferrin (S415C, T613A) variant. A mass of 75130 is expected when the serine residue at position 415 is modified to a cysteine, and the molecule is folded such that 38 cysteine residues are disulfided bonded and there is fluorescein molecule conjugated to the free thiol group. In this case the largest single peak (marked "A" in Figure 27) is that corresponding to the unmodified molecule with a nominal mass of 75126 (theoretical mass 75130 Da). There is a large peak (marked "B" in Figure 27) with the expected 162 Dalton increment for a single hexose addition. This probably represents O-linked glycosylation. Spectrum B shows the mass spectrum of fluorescein conjugated thiotransferrin (S415C, T613A) variant. A mass of 75557 is expected when the serine residue at position 415 is modified to a cysteine, and the molecule is folded such that 38 cysteine residues are disulfided bonded and there is one free thiol. In this case the largest single peak (marked "A" in Figure 27) is that corresponding to the fluorescein conjugated molecule with a nominal mass of 75574 (theoretical mass 75557 Da). There is a large peak (marked "B" in Figure 27) with the expected 162 Dalton increment for a single hexose addition. This probably represents O-linked glycosylation.

### EXAMPLE 14: MASS SPECTROMETRY OF FLUORESCEIN CONJUGATED THIOTRANSFERRIN VARIANTS TREATED WITH ELLMAN'S REAGENT (5'5'-DITHIO-BIS(2-NITRONENZOIC ACID) (DTNB)

To confirm that the fluorescein-5-maleimide reagent reacted with the free thiol group on thiotransferrin (S28C, S415A, T613A) variant and thiotransferrin (S415C, T613A) variant to produce fluorescein conjugated thiotransferrin variant the fluorescein conjugated thiotransferrin samples were assayed using the free thiol assay described in Example 10 and compared to mass spectra of the 'unconjugated' thiotransferrin samples.

Samples were treated with DTNB and prepared for mass spectrometry as described in Example 10.

Figure 28 shows mass spectra of unconjugated thiotransferrin (S28C, S415A, T613A) variant and fluorescein conjugated thiotransferrin (S28C, S415A, T613A) variant treated with DTNB.

Spectrum A shows the mass spectrum of thiotransferrin (S28C, S415A, T613A) which has been treated with DTNB. Mass spectrometric analysis of thiotransferrin (S28C, S415A, T613A) shows two peaks. A mass of 75311 is expected when the serine residue at position 28 is modified to a cysteine, and the molecule is folded such that 38 cysteine residues are disulfided bonded and one mole of NTB is bound to the thiol group. In this case the largest single peak (marked "A" in Figure 28) is that corresponding to the unmodified molecule with a nominal mass of 75307 (theoretical mass 75311 Da). There is a large peak (marked "B" in Figure 28) with the expected 162 Dalton increment for a single hexose addition. This probably represents O-linked glycosylation.

Spectrum B shows the mass spectrum of fluorescein conjugated thiotransferrin (S28C, S415A, T613A) which has been treated with DTNB. Mass spectrometric analysis of fluorescein conjugated thiotransferrin (S28C, S415A, T613A) shows two peaks. A mass of 75541 is expected when the serine residue at position 28 is modified to a cysteine, and the molecule is folded such that 38 cysteine residues are disulfided bonded and there is fluorescein molecule conjugated to the free thiol group. In this case the largest single peak (marked "C" in Figure 28) is that corresponding to the fluorescein conjugated molecule with a nominal mass of 75561 (theoretical mass 75541 Da). There is a large peak (marked "D" in Figure 28) with the expected 162 Dalton increment for a single hexose addition. This probably represents O-linked glycosylation. This spectrum is equivalent to that seen in the absence of DTNB (Figure 26) indicating that the fluorescein is conjugated through the free thiol group at serine 28, since addition of DTNB does not result in an additional mass shift of 197 Da.

Figure 29 shows mass spectra of unconjugated thiotransferrin (S415C, T613A) variant and fluorescein conjugated thiotransferrin (S415C, T613A) variant treated with DTNB.

Spectrum A shows the mass spectrum of thiotransferrin (S415C, T613A) which has been treated with DTNB. Mass spectrometric analysis of thiotransferrin (S415C, T613A) shows two peaks. A mass of 75327 is expected when the serine residue at position 28 is modified to a cysteine, and the molecule is folded such that 38 cysteine residues are disulfided bonded and one mole of NTB is bound to the thiol group. In this case the largest single peak (marked "A" in Figure 29) is that corresponding to the unmodified molecule with a nominal mass of 75328 (theoretical mass 75327Da). There is a large peak (marked "B" in Figure 29) with the expected 162 Dalton increment for a single hexose addition. This probably represents O-linked glycosylation.

Spectrum B shows the mass spectrum of fluorescein conjugated thiotransferrin (S415C, T613A) which has been treated with DTNB. Mass spectrometric analysis of fluorescein conjugated thiotransferrin (S415C, T613A) shows two peaks. A mass of 75557 is expected when the serine residue at position 415 is modified to a cysteine, and the molecule is folded such that 38 cysteine residues are disulfided bonded and there is fluorescein molecule conjugated to the free thiol group. In this case the largest single peak (marked "C" in Figure 29) is that corresponding to the fluorescein conjugated molecule with a nominal mass of 75573 (theoretical mass 75557 Da). There is a large peak (marked "D" in Figure 29) with the expected 162 Dalton increment for a single hexose addition. This probably represents O-linked glycosylation. This spectrum is equivalent to that seen in the absence of DTNB (Figure 27) indicating that the fluorescein is conjugated through the free thiol group at serine 415, since addition of DTNB does not result in an additional mass shift of 197 Da.

The mass spectrometry analysis of DTNB treated thiotransferrin (S28C, S415A, T613A) and thiotransferrin (S415C, T613A) confirms thiotransferrin (S28C, S415A, T613A) and thiotransferrin (S415C, T613A) has one free thiol, whereas fluorescein conjugated thiotransferrin (S28C, S415A, T613A) and fluorescein conjugated thiotransferrin (S415C, T613A) can not be labelled using DTNB under the same experimental conditions demonstrating that the fluorescein-5 maleimide labelled thiotransferrin (S28C, S415A, T613A) and thiotransferrin (S415C, T613A) through their free thiol groups.

### EXAMPLE 15: TRANSFERRIN RECEPTOR BINDING (MEASURED BY SPR)

Thiotransferrin muteins and fluorescein conjugated thiotransferrin muteins expressed, purified and quantified as described in Example 12 were compared for transferrin receptor binding capacity measured by surface plasmon resonance (SPR) using the same protocol described in Example 8.

Thiotransferrin (S28C, S415A, T613A) sample which has been conjugated with fluorescein was able to bind to the transferrin receptor (measured by SPR). Measurement of transferrin receptor binding by SPR analysis in Example 8 showed that modification of a selected residue such as serine 28 to a cysteine residue does not alter the gross structure of transferrin or prevent binding of the transferrin to its receptor.

Similarly, fluorescein conjugated thiotransferrin (S415C, T613A) samples was able to bind to the transferrin receptor by SPR analysis in a manner equivalent to the unconjugated sample. Measurement of transferrin receptor binding by SPR analysis in Example 8 showed that modification of a selected residue such as serine 415 to a cysteine residue does not alter the gross structure of transferrin or prevent binding of the transferrin to its receptor.

This example demonstrates that when a bioactive molecule is conjugated to the thiotransferrin mutein through the sulphur atom of the Cysteine residue that the thiotransferrin mutein retains its ability to bind to the transferrin receptor.

### EXAMPLE 16: TRANSFERRIN RECEPTOR BINDING

Transferrin receptor binding can be determined by measuring ⁵⁵Fe uptake competition in erythroleukemic K562 cells. The transferrin receptor binding of thiotransferrin (S28C, S415A, T613A), fluorescein conjugated thiotransferrin (S28C, S415A, T613A), thiotransferrin (S415C, T613A), fluorescein conjugated thiotransferrin (S415C, T613A) was compared to that of transferrin (S415A, T613A) and human plasma-derived apo-transferrin.

Iron free samples of thiotransferrin (S28C, S415A, T613A), fluorescein conjugated thiotransferrin (S28C, S415A, T613A), thiotransferrin (S415C, T613A), fluorescein conjugated thiotransferrin (S415C, T613A), were prepared at equimolar concentrations (- 5 mg/mL) as described in Example 11 and assayed for their ability to deliver ⁵⁵Fe to erythroleukemic K562 cells by the protocol described in Example 3.

A thiotransferrin is considered to bind to a transferrin receptor it has at least 5 % of the receptor binding capacity of the corresponding polypeptide without cysteine insertion. To assay for transferrin-mediated iron uptake, a cell model in need of iron must be chosen. Since all proliferating cells need iron as an essential growth factor, any fast-growing cell culture line can be used. The classical model for this type of assay is the human erythroleukemic K562 cell line, which originates from a patient suffering from chronic myeloic leukaemia in blast crisis. Experiments with this cell type were essential for clarification of the mechanism of receptor-mediated endocytosis (Klausner et al., 1983). [Klausner, R. D., Van Renswoude, J., Ashwell, G., Kempf, C., Schechter, A. N., Dean, A. & Bridges, K. R. (1983) Receptor-mediated endocytosis of transferrin in K562 cells. J Biol Chem 258, 4715-4724].

Iron uptake via receptor-mediated endocytosis can easily be assayed for by labelling transferrin with radioactive iron (⁵⁵Fe) and measuring cellular radioactivity after incubation of appropriate cells with labelled transferrin at different concentrations in appropriate time intervals. Iron uptake proceeds linearly with time provided the endocytosis-release system works properly, which can easily be seen from the counting data.

Unspecific components of the uptake process can be assayed for by addition of a large excess of unlabeled diferric transferrin. The residual radioactivity measured under these conditions can be accounted for by unspecific uptake and is discounted from the total. Unspecific binding and resulting iron uptake from recombinant transferrins or thiotransferrins must not be significantly higher than from native transferrin, otherwise, they may deliver iron to cells in an uncontrolled way leading to possible cell damage.

Since the number of receptors is the limiting factor of the uptake process, affinity and maximal capacity measured will represent binding affinity and maximal number of binding sites of the transferrin receptor.

Iron uptake from labelled diferric transferrins was performed as follows. K562 erythroleukemic cells, cultured in RPMI cell culture medium under standard conditions (bicarbonate-buffered, 5% CO₂, antibiotics, 10% foetal calf serum) were washed with serum-free medium containing HEPES-buffer and 1mg/ml of bovine serum albumin and used at a concentration of 10 million cells/ml in this medium.

Increasing concentrations of plasma transferrin or the respective recombinant transferrin sample (12.5, 25, 100, 200, 300, 500, 600, 800, 1200, 1600, 2000 nM), labelled with ⁵⁵Fe, were mixed with 100µl of medium. The reaction was started by the addition of 50µl of cell suspension.

A second series of parallel identical experiments was carried out in the presence of a hundredfold excess of unlabeled diferric transferrin to account for unspecific binding.

After 25min at 37°C the reaction was stopped by immersion into an ice-bath, three aliquots of 30µL of cell suspension were transferred to new tubes and the cells were centrifuged in the cold and again after addition of an oil layer of diethylphtalate/dibutylphthalate. The supernatant was removed, the cell pellet transferred into a counter vial and lysed with 0.5 M KOH+1% Triton X-100. The lysates were neutralized with 1M HCl after overnight lysis, mixed with Readysolv scintillation cocktail and counted in the Packard Liquid Scintillation Counter.

Transferrin was loaded with iron according to a standard procedure using ferric nitrilotriacetate as iron source (Bates and Schlabach, 1973). [Bates, G. W. and M. R. Schlabach (1973). "The reaction of ferric salts with transferrin." J Biol Chem 248(9): 3228-32.]

The results are presented as fmol ⁵⁵Fe/million cells. All data are mean of three experiments ± S.E.M. Results are provided in Table 4. It can be seen from these results that all transferrin samples were able to deliver iron to the K562 cells by receptor-mediated endocytosis (specific iron uptake) at greater than 5% of the level for the transferrin (S415A, T613A) control. The Kd values indicate that the transferrin (S415A, T613A) binds the receptor at least as well as the plasma-derived transferrin control, whereas the thiotransferrin (S28C, S415A, T613A) and thiotransferrin (S415C, T613A) appear to have slightly lower receptor binding (i.e. higher Kd values). Surprisingly, conjugation of fluorocein to the thiotransferrin variants has not caused a decrease in binding to the transferrin receptor in this experiment.

**Table 4**

| **Sample** | **Total Iron Uptake** | | **Specific iron ptake** | | |
|---|---|---|---|---|---|
| | **Bmax (fmol⁵⁵Fe/10⁶cells x 25min)** | **R²** | **Bmax (fmol⁵⁵Fe/10⁶cells x 25min)** | **Kd (nM transferrin)** | **R²** |
| Plasma-derived transferrin | 4252±185 | 0.9376 | 3277±164 | 166±33 | 0.8997 |
| Transferrin (S415A, T613A) | 4046±128 | 0.9613 | 2829±117 | 130±23 | 0.9028 |
| Thiotransferrin (S28C, S415A, T613A), | 4211±133 | 0.9713 | 3023±105 | 209±27 | 0.9501 |
| Fluorescein conjugated thiotransferrin (S28C, S415A, T613A) | 3294±170 | 0.9093 | 2430±125 | 154±32 | 0.8820 |
| Thiotransferrin (S415C, T613A) | 3691±256 | 0.8938 | 2359±150 | 203±47 | 0.8389 |
| Fluorescein conjugated thiotransferrin (S415C, T613A), | 4165±216 | 0.9288 | 3246±217 | 189±47 | 0.8559 |

## Claims

1. A transferrin polypeptide which:
has iron binding capacity,
binds to a receptor, and
has an amino acid sequence which is at least 80% identical to residues 1-679 or 339-679 of SEQ ID NO: 1, residues 1-691 of SEQ ID NO: 11 or residues 1-738 of SEQ ID NO: 15, and comprises a cysteine residue substituted at or inserted adjacent one or more positions corresponding to S28, S32, A215, S415, S501 and/or N611 of SEQ ID NO: 1 and wherein the cysteine residue at the one or more positions corresponding to S28, S32, A215, S415, S501 and/or N611 of SEQ ID NO: 1 is unpaired.

2. The transferrin polypeptide of claim 1 having at least 95% sequence identity SEQ ID NO: 1.

3. The transferrin polypeptide of claim 1 or 2 which comprises substitution of an amino acid with cysteine, insertion or deletion of cysteine at a position corresponding to any of V1-T5, E13-H25, M26-S36, K42-S44, Y85-T93, K103-Q108, L112-K115, T120-W128, L139-P145, F154-G156, A159-F167, P168-L170; P175-C177, G178-F186, G187-K196, D197-D201, I210-N213, L214-N216, K217-R220, D221-Q222, L226-P234, S255-K259, E260-H273, F274-H300, V305-D310, Y317-E328, G329- P341, C402-N417, C418-D420, K434-T440, W441-N443, L444-G446, Y468-K470, I471-R475, A485-S501, G502-N504, L505-Y515, G516-V526, T537-Q540, N541-D548, P549-A551, K552-N555, D558-Y559, C563-T567, R568-P570, E572-N576, E594-F608, G609-V636, L641-T646, R663-S668 or S669-R677 of SEQ ID NO: 1.

4. The transferrin polypeptide of any preceding claim which comprises substitution, insertion or deletion of an amino acid with cysteine at a position corresponding to any of V1-K4, M26-P35, K42-S44, Y85-T93, K103-Q108, L112-K115, T120-W128, L139-P145, F154-S155, A159-F167, G178-F186, D197-D201, L214-N216, D221-Q222, L226-P234, S255-K259, F274-H300, V305-D310, G329- P341, C402-N417, K434-T440, L444-G446, 1471-R475, A485-S501, L505-Y515, N541-D548, K552-N555, D558, C563-T567, G609-V636, L641-T646, R663-S668 of SEQ ID NO: 1.

5. The transferrin polypeptide of any preceding claim which comprises substitution, insertion or deletion of an amino acid with cysteine at a position corresponding to any of V1-K4, K103-Q108, L112-K115, L139-P145, A159-F167, G178-F186, F274-H300, V305-D310, G329-P341, K434-T440, L444-G446, I471-R475, A485-S501, L505-Y515, G609-V636, L641-T646 of SEQ ID NO: 1.

6. The transferrin polypeptide of any preceding claim which comprises substitution, insertion or deletion of an amino acid with cysteine at a position corresponding to any of V1, P2, D3, K4 T5, H14, Q20, S21, D24, K27, S28, V29, P31, S32, D33, A43, E89, D104, G106, G114, L122, G123, P145, S155, D163, T165, D166, P168, P175, G176, G178, C179, S180, T181, L182, Q184, F187, S189, D197, G198, E212, A215, N216, A218, D221, D229, G257, N268, D277, K278, K280, E281, S287, P288, H289, K291, S298, P307, L326, T330, P335, T336, N413, S415, D416, D420, K434, S435, A436, S437, D438, D442, N443, G446, N469, N472, G487, K489, D491, S501, G502, L503, N510, T518, P539, Q540, G543, G544, K545, P547, D548, P549, K552, N553, N555, D558, D565, T567, P570, N576, A595, S610, N611, V612, T613, D614, S616, G617, T626, D634, D643, S666, T667 or S669 of SEQ ID NO: 1.

7. The transferrin polypeptide of any preceeding claim which further comprises at least one mutation that reduces N- or O-linked glycosylation.

8. The transferrin polypeptide claim of 7 which, compared to SEQ ID NO: 1, comprises a substitution of an amino acid at a position corresponding to S32, N413, S415, N611 or T613 to an amino acid which does not allow glycosylation at the position corresponding to S32, N413 or N611.

9. The transferrin polypeptide of any preceeding claim, having at least 99% sequence identity to SEQ ID NO: 1 and comprises one or more mutations selected among: V1 C, S28C, S32C, D104C, T165C, P175C, A215C, P288C, T336C, S415C, D146C, C171A, S415C+deletion of D416, S415A+insertion of C before D416, S501 C, N553C, N61 1 C, T613C, D643C and S28C+S415C.

10. The transferrin polypeptide of any of claims 1-8, having at least 95% sequence identity to SEQ ID NO: 11 and comprises the mutation S421 C.

11. A polynucleotide which encodes the transferrin polypeptide of any of claims 1-10,

12. A plasmid comprising the polynucleotide of claim 11.

13. A host cell comprising a polynucleotide of claim 11 or a plasmid of claim 12.

14. The host cell of claim 13, which is a yeast cell.

15. A conjugate which comprises a bioactive compound and a transferrin polypeptide according to any of claims 1-10, wherein the bioactive compound is linked through the free thiol group of the cysteine residue of the transferrin polypeptide.

16. A method of producing a transferrin polypeptide of any of claims 1-10, comprising culturing the host cell of claim 13 or 15 under conditions that allows expression of the transferrin polypeptide and recovering the transferrin polypeptide.

17. A method of producing the conjugate of claim 15 which comprises linking the polypeptide of any preceding claim and a bioactive compound linked through the sulphur atom of the cysteine residue in the transferrin polypeptide.

18. A composition comprising a conjugate of claim 15 and at least one pharmaceutically acceptable carrier or diluent.

19. The use of a conjugate according to claim 15 or produced according to any of claims 16 or 17 for treatment of disease, illness and diagnosis.

20. A method of preparing a transferrin polypeptide, comprising:
providing a three-dimensional model comprising at least one instance of a transferrin sequence, an iron atom and a receptor,
selecting an amino acid residue in the transferrin sequence which corresponds to V1, P2 or D3 in SEQ ID NO: 1 or which in each instance of the transferrin sequence fulfils the following conditions:
i) RMFS above 0.14,
ii) solvent-surface Accessibility above 100%,
substituting the selected residue with cysteine or inserting cysteine at the N-side or C-side of the selected residue,
optionally, making additional alterations to the transferrin sequence where each alteration is an amino acid deletion, substitution, or insertion, and
preparing a transferrin polypeptide having the resulting amino acid sequence.

21. The method of claim 20, which further comprises determining the Fe binding capacity and/or the receptor binding capacity and/or the conjugation competence of the transferrin polypeptide; and selecting a transferrin polypeptide which has Fe binding capacity and/or receptor binding capacity and/or conjugation competence.


SEQUENCE LISTING

<110>  Novozymes Biopharma DK A/S

<120>  Transferrin variants and conjugates

<130>  11195-EP-ETD

<160>  15

<170>  PatentIn version 3.5

<210>  1
<211>  679
<212>  PRT
<213>  Homo Sapiens

<400>  1

Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val
            420                 425                 430

Lys Lys Ser Ala Ser Asp Leu Thr Trp Asp Asn Leu Lys Gly Lys Lys
        435                 440                 445

Ser Cys His Thr Ala Val Gly Arg Thr Ala Gly Trp Asn Ile Pro Met
    450                 455                 460

Gly Leu Leu Tyr Asn Lys Ile Asn His Cys Arg Phe Asp Glu Phe Phe
465                 470                 475                 480

Ser Glu Gly Cys Ala Pro Gly Ser Lys Lys Asp Ser Ser Leu Cys Lys
                485                 490                 495

Leu Cys Met Gly Ser Gly Leu Asn Leu Cys Glu Pro Asn Asn Lys Glu
            500                 505                 510

Gly Tyr Tyr Gly Tyr Thr Gly Ala Phe Arg Cys Leu Val Glu Lys Gly
        515                 520                 525

Asp Val Ala Phe Val Lys His Gln Thr Val Pro Gln Asn Thr Gly Gly
    530                 535                 540

Lys Asn Pro Asp Pro Trp Ala Lys Asn Leu Asn Glu Lys Asp Tyr Glu
545                 550                 555                 560

Leu Leu Cys Leu Asp Gly Thr Arg Lys Pro Val Glu Glu Tyr Ala Asn
                565                 570                 575

Cys His Leu Ala Arg Ala Pro Asn His Ala Val Val Thr Arg Lys Asp
            580                 585                 590

Lys Glu Ala Cys Val His Lys Ile Leu Arg Gln Gln Gln His Leu Phe
        595                 600                 605

Gly Ser Asn Val Thr Asp Cys Ser Gly Asn Phe Cys Leu Phe Arg Ser
    610                 615                 620

Glu Thr Lys Asp Leu Leu Phe Arg Asp Asp Thr Val Cys Leu Ala Lys
625                 630                 635                 640

Leu His Asp Arg Asn Thr Tyr Glu Lys Tyr Leu Gly Glu Glu Tyr Val
                645                 650                 655

Lys Ala Val Gly Asn Leu Arg Lys Cys Ser Thr Ser Ser Leu Leu Glu
            660                 665                 670

Ala Cys Thr Phe Arg Arg Pro
        675

<210>  2
<211>  2359
<212>  DNA
<213>  Artificial

<220>
<223>  Synthetic

<220>
<221>  CDS
<222>  (67)..(2160)

<220>
<221>  sig_peptide
<222>  (67)..(123)

<220>
<221>  mat_peptide
<222>  (124)..(2160)

<400>  2
cttaagagtc caattagctt catcgccaat aaaaaaacaa gcttaaccta attctaacaa      60

gcaaag atg ttg ttg caa gct ttt ttg ttt ttg ttg gct ggt ttt gct      108
       Met Leu Leu Gln Ala Phe Leu Phe Leu Leu Ala Gly Phe Ala
                       -15                 -10

gct aaa att tct gct gtt cca gat aaa aca gtt aga tgg tgt gct gtt      156
Ala Lys Ile Ser Ala Val Pro Asp Lys Thr Val Arg Trp Cys Ala Val
-5              -1  1               5                   10

tct gaa cat gaa gct act aaa tgt caa tct ttt aga gat cat atg aaa      204
Ser Glu His Glu Ala Thr Lys Cys Gln Ser Phe Arg Asp His Met Lys
            15                  20                  25

tct gtt att cca tct gat ggt cca tct gtt gct tgt gtt aaa aaa gct      252
Ser Val Ile Pro Ser Asp Gly Pro Ser Val Ala Cys Val Lys Lys Ala
        30                  35                  40

tct tat ttg gat tgt att aga gct att gct gct aat gaa gct gat gct      300
Ser Tyr Leu Asp Cys Ile Arg Ala Ile Ala Ala Asn Glu Ala Asp Ala
    45                  50                  55

gtt act ttg gat gct ggt tta gtt tat gat gct tat ttg gct cca aac      348
Val Thr Leu Asp Ala Gly Leu Val Tyr Asp Ala Tyr Leu Ala Pro Asn
60                  65                  70                  75

aat ttg aaa cca gtt gtt gct gaa ttt tat ggt tct aag gaa gat cca      396
Asn Leu Lys Pro Val Val Ala Glu Phe Tyr Gly Ser Lys Glu Asp Pro
                80                  85                  90

caa act ttt tat tat gct gta gcc gtt gta aaa aag gat tca ggt ttt      444
Gln Thr Phe Tyr Tyr Ala Val Ala Val Val Lys Lys Asp Ser Gly Phe
            95                  100                 105

caa atg aat caa ttg aga ggt aaa aaa tct tgt cat act ggt tta ggt      492
Gln Met Asn Gln Leu Arg Gly Lys Lys Ser Cys His Thr Gly Leu Gly
        110                 115                 120

aga tct gct gga tgg aat att cca att ggt ttg ttg tat tgt gat ttg      540
Arg Ser Ala Gly Trp Asn Ile Pro Ile Gly Leu Leu Tyr Cys Asp Leu
    125                 130                 135

cca gaa cca aga aaa cca ttg gaa aaa gct gtt gct aat ttt ttt tct      588
Pro Glu Pro Arg Lys Pro Leu Glu Lys Ala Val Ala Asn Phe Phe Ser
140                 145                 150                 155

ggt tct tgt gct cca tgt gct gat ggt aca gat ttt cca caa ttg tgt      636

Gly Ser Cys Ala Pro Cys Ala Asp Gly Thr Asp Phe Pro Gln Leu Cys
                160                 165                 170

caa tta tgt cca ggt tgt ggt tgt tct act ttg aat caa tat ttt ggt      684
Gln Leu Cys Pro Gly Cys Gly Cys Ser Thr Leu Asn Gln Tyr Phe Gly
            175                 180                 185

tat tct ggt gct ttt aaa tgt ttg aaa gat ggt gct ggt gat gtt gct      732
Tyr Ser Gly Ala Phe Lys Cys Leu Lys Asp Gly Ala Gly Asp Val Ala
        190                 195                 200

ttt gtt aaa cat tct act att ttt gaa aat ttg gca aac aaa gct gat      780
Phe Val Lys His Ser Thr Ile Phe Glu Asn Leu Ala Asn Lys Ala Asp
    205                 210                 215

aga gat caa tat gaa ttg ttg tgt ttg gat aat act aga aaa cca gtt      828
Arg Asp Gln Tyr Glu Leu Leu Cys Leu Asp Asn Thr Arg Lys Pro Val
220                 225                 230                 235

gat gaa tat aaa gat tgt cat ttg gct caa gtt cca tct cat act gtt      876
Asp Glu Tyr Lys Asp Cys His Leu Ala Gln Val Pro Ser His Thr Val
                240                 245                 250

gtt gct aga tct atg ggt ggt aaa gaa gat ttg att tgg gaa ttg ttg      924
Val Ala Arg Ser Met Gly Gly Lys Glu Asp Leu Ile Trp Glu Leu Leu
            255                 260                 265

aat caa gct caa gaa cat ttt ggt aaa gat aaa tct aaa gaa ttt caa      972
Asn Gln Ala Gln Glu His Phe Gly Lys Asp Lys Ser Lys Glu Phe Gln
        270                 275                 280

ttg ttt tct tct cca cat ggt aaa gat ttg ttg ttt aaa gat tct gct     1020
Leu Phe Ser Ser Pro His Gly Lys Asp Leu Leu Phe Lys Asp Ser Ala
    285                 290                 295

cat ggt ttt ttg aaa gtt cca cca aga atg gat gct aaa atg tat ttg     1068
His Gly Phe Leu Lys Val Pro Pro Arg Met Asp Ala Lys Met Tyr Leu
300                 305                 310                 315

ggt tat gaa tac gtt act gct att aga aat ttg aga gaa ggt act tgt     1116
Gly Tyr Glu Tyr Val Thr Ala Ile Arg Asn Leu Arg Glu Gly Thr Cys
                320                 325                 330

cca gaa gct cca act gat gaa tgt aaa cca gtt aaa tgg tgt gct ttg     1164
Pro Glu Ala Pro Thr Asp Glu Cys Lys Pro Val Lys Trp Cys Ala Leu
            335                 340                 345

tct cat cat gaa aga tta aaa tgt gat gaa tgg tct gtt aat tct gtt     1212
Ser His His Glu Arg Leu Lys Cys Asp Glu Trp Ser Val Asn Ser Val
        350                 355                 360

ggt aaa att gaa tgt gtt tct gct gaa act aca gaa gat tgt att gct     1260
Gly Lys Ile Glu Cys Val Ser Ala Glu Thr Thr Glu Asp Cys Ile Ala
    365                 370                 375

aaa att atg aat ggt gaa gct gat gct atg tct tta gat ggt ggt ttt     1308
Lys Ile Met Asn Gly Glu Ala Asp Ala Met Ser Leu Asp Gly Gly Phe
380                 385                 390                 395

gta tat att gct ggt aaa tgt ggt tta gtt cca gtt ttg gct gaa aat     1356
Val Tyr Ile Ala Gly Lys Cys Gly Leu Val Pro Val Leu Ala Glu Asn
                400                 405                 410

tat aac aaa gct gat aat tgt gaa gat act cca gaa gct ggt tat ttt     1404
Tyr Asn Lys Ala Asp Asn Cys Glu Asp Thr Pro Glu Ala Gly Tyr Phe

415                    420                    425

gct gtt gct gtt gtt aaa aaa tct gct tct gat ttg act tgg gat aat        1452
Ala Val Ala Val Val Lys Lys Ser Ala Ser Asp Leu Thr Trp Asp Asn
        430                    435                    440

cta aaa gga aaa aag agt tgc cat aca gct gtt gga aga aca gcc gga        1500
Leu Lys Gly Lys Lys Ser Cys His Thr Ala Val Gly Arg Thr Ala Gly
    445                    450                    455

tgg aac att cca atg gga ttg cta tac aac aaa att aat cat tgt aga        1548
Trp Asn Ile Pro Met Gly Leu Leu Tyr Asn Lys Ile Asn His Cys Arg
460                    465                    470                    475

ttt gat gaa ttt ttt tct gaa ggt tgt gct cca ggt tct aaa aaa gat        1596
Phe Asp Glu Phe Phe Ser Glu Gly Cys Ala Pro Gly Ser Lys Lys Asp
                480                    485                    490

tct tct ttg tgt aaa ttg tgt atg ggt tct gga ttg aat ttg tgt gaa        1644
Ser Ser Leu Cys Lys Leu Cys Met Gly Ser Gly Leu Asn Leu Cys Glu
            495                    500                    505

cca aac aac aag gaa ggt tat tat ggt tat act ggt gct ttt aga tgt        1692
Pro Asn Asn Lys Glu Gly Tyr Tyr Gly Tyr Thr Gly Ala Phe Arg Cys
        510                    515                    520

tta gtt gaa aaa ggt gat gtt gct ttt gtt aaa cat caa aca gtt cca        1740
Leu Val Glu Lys Gly Asp Val Ala Phe Val Lys His Gln Thr Val Pro
    525                    530                    535

caa aat act ggt ggt aaa aat cca gat cca tgg gct aaa aat ttg aat        1788
Gln Asn Thr Gly Gly Lys Asn Pro Asp Pro Trp Ala Lys Asn Leu Asn
540                    545                    550                    555

gaa aaa gat tac gaa tta cta tgt tta gat ggt aca aga aag cca gtt        1836
Glu Lys Asp Tyr Glu Leu Leu Cys Leu Asp Gly Thr Arg Lys Pro Val
                560                    565                    570

gag gaa tac gct aat tgt cat tta gct aga gca cca aat cat gct gtt        1884
Glu Glu Tyr Ala Asn Cys His Leu Ala Arg Ala Pro Asn His Ala Val
            575                    580                    585

gtt act aga aaa gat aaa gaa gct tgt gtt cat aaa att ttg aga caa        1932
Val Thr Arg Lys Asp Lys Glu Ala Cys Val His Lys Ile Leu Arg Gln
        590                    595                    600

caa caa cat ttg ttt ggt tct aat gtt gct gat tgt tct ggt aat ttt        1980
Gln Gln His Leu Phe Gly Ser Asn Val Ala Asp Cys Ser Gly Asn Phe
    605                    610                    615

tgt ttg ttt aga tct gaa act aaa gat tta ttg ttt aga gat gat act        2028
Cys Leu Phe Arg Ser Glu Thr Lys Asp Leu Leu Phe Arg Asp Asp Thr
620                    625                    630                    635

gtt tgt ttg gct aaa ttg cat gat aga aat act tat gaa aaa tat ttg        2076
Val Cys Leu Ala Lys Leu His Asp Arg Asn Thr Tyr Glu Lys Tyr Leu
                640                    645                    650

ggt gaa gaa tac gtt aaa gct gtt ggt aat ttg aga aaa tgt tct act        2124
Gly Glu Glu Tyr Val Lys Ala Val Gly Asn Leu Arg Lys Cys Ser Thr
            655                    660                    665

tct tct ttg ttg gaa gct tgt act ttt aga agg cca taataagctt        2170
Ser Ser Leu Leu Glu Ala Cys Thr Phe Arg Arg Pro
        670                    675

aattcttatg atttatgatt tttattatta aataagttat aaaaaaaata agtgtataca 2230

aattttaaag tgactcttag gttttaaaac gaaaattctt attcttgagt aactctttcc 2290

tgtaggtcag gttgctttct caggtatagc atgaggtcgc tcttattgac cacacctcta 2350

ccggcatgc 2359

<210> 3
<211> 698
<212> PRT
<213> Artificial

<220>
<223> Synthetic Construct

<400> 3

Met Leu Leu Gln Ala Phe Leu Phe Leu Leu Ala Gly Phe Ala Ala Lys
-15 -10 -5

Ile Ser Ala Val Pro Asp Lys Thr Val Arg Trp Cys Ala Val Ser Glu
-1 1 5 10

His Glu Ala Thr Lys Cys Gln Ser Phe Arg Asp His Met Lys Ser Val
15 20 25

Ile Pro Ser Asp Gly Pro Ser Val Ala Cys Val Lys Lys Ala Ser Tyr
30 35 40 45

Leu Asp Cys Ile Arg Ala Ile Ala Ala Asn Glu Ala Asp Ala Val Thr
50 55 60

Leu Asp Ala Gly Leu Val Tyr Asp Ala Tyr Leu Ala Pro Asn Asn Leu
65 70 75

Lys Pro Val Val Ala Glu Phe Tyr Gly Ser Lys Glu Asp Pro Gln Thr
80 85 90

Phe Tyr Tyr Ala Val Ala Val Val Lys Lys Asp Ser Gly Phe Gln Met
95 100 105

Asn Gln Leu Arg Gly Lys Lys Ser Cys His Thr Gly Leu Gly Arg Ser
110 115 120 125

Ala Gly Trp Asn Ile Pro Ile Gly Leu Leu Tyr Cys Asp Leu Pro Glu
130 135 140

Pro Arg Lys Pro Leu Glu Lys Ala Val Ala Asn Phe Phe Ser Gly Ser
145 150 155

Cys Ala Pro Cys Ala Asp Gly Thr Asp Phe Pro Gln Leu Cys Gln Leu

160                 165                 170

Cys Pro Gly Cys Gly Cys Ser Thr Leu Asn Gln Tyr Phe Gly Tyr Ser
    175                 180                 185

Gly Ala Phe Lys Cys Leu Lys Asp Gly Ala Gly Asp Val Ala Phe Val
190                 195                 200                 205

Lys His Ser Thr Ile Phe Glu Asn Leu Ala Asn Lys Ala Asp Arg Asp
                210                 215                 220

Gln Tyr Glu Leu Leu Cys Leu Asp Asn Thr Arg Lys Pro Val Asp Glu
            225                 230                 235

Tyr Lys Asp Cys His Leu Ala Gln Val Pro Ser His Thr Val Val Ala
        240                 245                 250

Arg Ser Met Gly Gly Lys Glu Asp Leu Ile Trp Glu Leu Leu Asn Gln
    255                 260                 265

Ala Gln Glu His Phe Gly Lys Asp Lys Ser Lys Glu Phe Gln Leu Phe
270                 275                 280                 285

Ser Ser Pro His Gly Lys Asp Leu Leu Phe Lys Asp Ser Ala His Gly
                290                 295                 300

Phe Leu Lys Val Pro Pro Arg Met Asp Ala Lys Met Tyr Leu Gly Tyr
            305                 310                 315

Glu Tyr Val Thr Ala Ile Arg Asn Leu Arg Glu Gly Thr Cys Pro Glu
        320                 325                 330

Ala Pro Thr Asp Glu Cys Lys Pro Val Lys Trp Cys Ala Leu Ser His
    335                 340                 345

His Glu Arg Leu Lys Cys Asp Glu Trp Ser Val Asn Ser Val Gly Lys
350                 355                 360                 365

Ile Glu Cys Val Ser Ala Glu Thr Thr Glu Asp Cys Ile Ala Lys Ile
                370                 375                 380

Met Asn Gly Glu Ala Asp Ala Met Ser Leu Asp Gly Gly Phe Val Tyr
            385                 390                 395

Ile Ala Gly Lys Cys Gly Leu Val Pro Val Leu Ala Glu Asn Tyr Asn
        400                 405                 410

Lys Ala Asp Asn Cys Glu Asp Thr Pro Glu Ala Gly Tyr Phe Ala Val
    415                 420                 425

Ala Val Val Lys Lys Ser Ala Ser Asp Leu Thr Trp Asp Asn Leu Lys
430                 435                 440                 445

Gly Lys Lys Ser Cys His Thr Ala Val Gly Arg Thr Ala Gly Trp Asn
                450                 455                 460

Ile Pro Met Gly Leu Leu Tyr Asn Lys Ile Asn His Cys Arg Phe Asp
            465                 470                 475

Glu Phe Phe Ser Glu Gly Cys Ala Pro Gly Ser Lys Lys Asp Ser Ser
        480                 485                 490

Leu Cys Lys Leu Cys Met Gly Ser Gly Leu Asn Leu Cys Glu Pro Asn
    495                 500                 505

Asn Lys Glu Gly Tyr Tyr Gly Tyr Thr Gly Ala Phe Arg Cys Leu Val
510                 515                 520                 525

Glu Lys Gly Asp Val Ala Phe Val Lys His Gln Thr Val Pro Gln Asn
                530                 535                 540

Thr Gly Gly Lys Asn Pro Asp Pro Trp Ala Lys Asn Leu Asn Glu Lys
            545                 550                 555

Asp Tyr Glu Leu Leu Cys Leu Asp Gly Thr Arg Lys Pro Val Glu Glu
        560                 565                 570

Tyr Ala Asn Cys His Leu Ala Arg Ala Pro Asn His Ala Val Val Thr
    575                 580                 585

Arg Lys Asp Lys Glu Ala Cys Val His Lys Ile Leu Arg Gln Gln Gln
590                 595                 600                 605

His Leu Phe Gly Ser Asn Val Ala Asp Cys Ser Gly Asn Phe Cys Leu
                610                 615                 620

Phe Arg Ser Glu Thr Lys Asp Leu Leu Phe Arg Asp Asp Thr Val Cys
            625                 630                 635

Leu Ala Lys Leu His Asp Arg Asn Thr Tyr Glu Lys Tyr Leu Gly Glu
        640                 645                 650

Glu Tyr Val Lys Ala Val Gly Asn Leu Arg Lys Cys Ser Thr Ser Ser
    655                 660                 665

Leu Leu Glu Ala Cys Thr Phe Arg Arg Pro
670                 675

<210>  4
<211>  1487
<212>  DNA
<213>  Artificial

<220>
<223>  Synthetic

<400>  4
cttaagagtc caattagctt catcgccaat aaaaaaacaa gcttaaccta attctaacaa      60

gcaaagatgt tgttgcaagc ttttttgttt ttgttggctg gttttgctgc taaaatttct      120

gctgttccag ataaaacagt tagatggtgt gctgtttctg aacatgaagc tactaaatgt      180

caatctttta gagatcatat gaaatctgtt attccatctg atggtccatc tgttgcttgt      240

gttaaaaaag cttcttattt ggattgtatt agagctattg ctgctaatga agctgatgct      300

gttactttgg atgctggttt agtttatgat gcttatttgg ctccaaacaa tttgaaacca      360

gttgttgctg aattttatgg ttctaaggaa gatccacaaa ctttttatta tgctgtagcc      420

gttgtaaaaa aggattcagg ttttcaaatg aatcaattga gaggtaaaaa atcttgtcat      480

actggtttag gtagatctgc tggatggaat attccaattg gtttgttgta ttgtgatttg      540

ccagaaccaa gaaaaccatt ggaaaaagct gttgctaatt ttttttctgg ttcttgtgct      600

ccatgtgctg atggtacaga ttttccacaa ttgtgtcaat tatgtccagg ttgtggttgt      660

tctactttga atcaatattt tggttattct ggtgctttta aatgtttgaa agatggtgct      720

ggtgatgttg cttttgttaa acattctact atttttgaaa atttggcaaa caaagctgat      780

agagatcaat atgaattgtt gtgtttggat aatactagaa aaccagttga tgaatataaa      840

gattgtcatt tggctcaagt tccatctcat actgttgttg ctagatctat gggtggtaaa      900

gaagatttga tttgggaatt gttgaatcaa gctcaagaac attttggtaa agataaatct      960

aaagaatttc aattgttttc ttctccacat ggtaaagatt tgttgtttaa agattctgct      1020

catggttttt tgaaagttcc accaagaatg gatgctaaaa tgtatttggg ttatgaatac      1080

gttactgcta ttagaaattt gagagaaggt acttgtccag aagctccaac tgatgaatgt      1140

aaaccagtta aatggtgtgc tttgtctcat catgaaagat taaaatgtga tgaatggtct      1200

gttaattctg ttggtaaaat tgaatgtgtt tctgctgaaa ctacagaaga ttgtattgct      1260

aaaattatga atggtgaagc tgatgctatg tctttagatg gtggttttgt atatattgct      1320

ggtaaatgtg gtttagttcc agttttggct gaaaattata acaaagctga taattgtgaa      1380

gatactccag aagctggtta ttttgctgtt gctgttgtta aaaaatctgc ttctgatttg      1440

acttgggata atctaaaagg aaaaaagagt tgccatacag ctgttgg      1487

<210>  5
<211>  1487
<212>  DNA
<213>  Artificial

<220>
<223>  Synthetic

<400>  5
cttaagagtc caattagctt catcgccaat aaaaaaacaa gcttaaccta attctaacaa      60

gcaaagatgt tgttgcaagc ttttttgttt ttgttggctg gttttgctgc taaaatttct     120

gctgttccag ataaaacagt tagatggtgt gctgtttctg aacatgaagc tactaaatgt     180

caatctttta gagatcatat gaaatctgtt attccatctg atggtccatc tgttgcttgt     240

gttaaaaaag cttcttattt ggattgtatt agagctattg ctgctaatga agctgatgct     300

gttactttgg atgctggttt agtttatgat gcttatttgg ctccaaacaa tttgaaacca     360

gttgttgctg aattttatgg ttctaaggaa gatccacaaa ctttttatta tgctgtagcc     420

gttgtaaaaa aggattcagg ttttcaaatg aatcaattga gaggtaaaaa atcttgtcat     480

actggtttag gtagatctgc tggatggaat attccaattg gtttgttgta ttgtgatttg     540

ccagaaccaa gaaaaccatt ggaaaaagct gttgctaatt ttttttctgg ttcttgtgct     600

ccatgtgctg atggtacaga ttttccacaa ttggctcaat tatgtccagg ttgtggttgt     660

tctactttga atcaatattt tggttattct ggtgctttta aatgtttgaa agatggtgct     720

ggtgatgttg cttttgttaa acattctact atttttgaaa atttggcaaa caaagctgat     780

agagatcaat atgaattgtt gtgtttggat aatactagaa aaccagttga tgaatataaa     840

gattgtcatt tggctcaagt tccatctcat actgttgttg ctagatctat gggtggtaaa     900

gaagatttga tttgggaatt gttgaatcaa gctcaagaac attttggtaa agataaatct     960

aaagaatttc aattgttttc ttctccacat ggtaaagatt tgttgtttaa agattctgct    1020

catggttttt tgaaagttcc accaagaatg gatgctaaaa tgtatttggg ttatgaatac    1080

gttactgcta ttagaaattt gagagaaggt acttgtccag aagctccaac tgatgaatgt    1140

aaaccagtta aatggtgtgc tttgtctcat catgaaagat taaaatgtga tgaatggtct    1200

gttaattctg ttggtaaaat tgaatgtgtt tctgctgaaa ctacagaaga ttgtattgct    1260

aaaattatga atggtgaagc tgatgctatg tctttagatg gtggttttgt atatattgct    1320

ggtaaatgtg gtttagttcc agttttggct gaaaattata acaaagctga taattgtgaa    1380

gatactccag aagctggtta ttttgctgtt gctgttgtta aaaaatctgc ttctgatttg    1440

acttgggata atctaaaagg aaaaaagagt tgccatacag ctgttgg                  1487

<210>  6
<211>  1484
<212>  DNA
<213>  Artificial

<220>
<223>  Synthetic

<400>  6
   cttaagagtc caattagctt catcgccaat aaaaaaacaa gcttaaccta attctaacaa 60
   gcaaagatgt tgttgcaagc tttttgtttt ttgttggctg gttttgctgc taaaatttct 120
   gctgttccag ataaaacagt tagatggtgt gctgtttctg aacatgaagc tactaaatgt 180
   caatctttta gagatcatat gaaatctgtt attccatctg atggtccatc tgttgcttgt 240
   gttaaaaaag cttcttattt ggattgtatt agagctattg ctgctaatga agctgatgct 300
   gttactttgg atgctggttt agtttatgat gcttatttgg ctccaaacaa tttgaaacca 360
   gttgttgctg aattttatgg ttctaaggaa gatccacaaa ctttttatta tgctgtagcc 420
   gttgtaaaaa aggattcagg ttttcaaatg aatcaattga gaggtaaaaa atcttgtcat 480
   actggtttag gtagatctgc tggatggaat attccaattg gtttgttgta ttgtgatttg 540
   ccagaaccaa gaaaaccatt ggaaaaagct gttgctaatt ttttttctgg ttcttgtgct 600
   ccatgtgctg atggtacaga ttttccacaa ttgtgtcaat tatgtccagg ttgtggttgt 660
   tctactttga atcaatattt tggttattct ggtgctttta aatgtttgaa agatggtgct 720
   ggtgatgttg cttttgttaa acattctact atttttgaaa atttggcaaa caaagctgat 780
   agagatcaat atgaattgtt gtgtttggat aatactagaa aaccagttga tgaatataaa 840
   gattgtcatt tggctcaagt tccatctcat actgttgttg ctagatctat gggtggtaaa 900
   gaagatttga tttgggaatt gttgaatcaa gctcaagaac attttggtaa agataaatct 960
   aaagaatttc aattgttttc ttctccacat ggtaaagatt tgttgtttaa agattctgct 1020
   catggttttt tgaaagttcc accaagaatg gatgctaaaa tgtatttggg ttatgaatac 1080
   gttactgcta ttagaaattt gagagaaggt acttgtccag aagctccaac tgatgaatgt 1140
   aaaccagtta aatggtgtgc tttgtctcat catgaaagat taaaatgtga tgaatggtct 1200
   gttaattctg ttggtaaaat tgaatgtgtt tctgctgaaa ctacagaaga ttgtattgct 1260
   aaaattatga atggtgaagc tgatgctatg tctttagatg gtggttttgt atatattgct 1320
   ggtaaatgtg gtttagttcc agttttggct gaaaattata acaaatgtaa ttgtgaagat 1380
   actccagaag ctggttattt tgctgttgct gttgttaaaa aatctgcttc tgatttgact 1440
   tgggataatc taaaaggaaa aaagagttgc catacagctg ttgg 1484

<210> 7
<211> 1490
<212> DNA
<213> Artificial

<220>
<223> Synthetic

<400> 7
cttaagagtc caattagctt catcgccaat aaaaaaacaa gcttaaccta attctaacaa 60

gcaaagatgt tgttgcaagc tttttgtttt ttgttggctg gttttgctgc taaaatttct 120

gctgttccag ataaaacagt tagatggtgt gctgtttctg aacatgaagc tactaaatgt 180

caatctttta gagatcatat gaaatctgtt attccatctg atggtccatc tgttgcttgt 240

gttaaaaaag cttcttattt ggattgtatt agagctattg ctgctaatga agctgatgct 300

gttactttgg atgctggttt agtttatgat gcttatttgg ctccaaacaa tttgaaacca 360

gttgttgctg aattttatgg ttctaaggaa gatccacaaa ctttttatta tgctgtagcc 420

gttgtaaaaa aggattcagg ttttcaaatg aatcaattga gaggtaaaaa atcttgtcat 480

actggtttag gtagatctgc tggatggaat attccaattg gtttgttgta ttgtgatttg 540

ccagaaccaa gaaaaccatt ggaaaaagct gttgctaatt ttttttctgg ttcttgtgct 600

ccatgtgctg atggtacaga ttttccacaa ttgtgtcaat tatgtccagg ttgtggttgt 660

tctactttga atcaatattt tggttattct ggtgctttta aatgtttgaa agatggtgct 720

ggtgatgttg cttttgttaa acattctact atttttgaaa atttggcaaa caaagctgat 780

agagatcaat atgaattgtt gtgtttggat aatactagaa aaccagttga tgaatataaa 840

gattgtcatt tggctcaagt tccatctcat actgttgttg ctagatctat gggtggtaaa 900

gaagatttga tttgggaatt gttgaatcaa gctcaagaac attttggtaa agataaatct 960

aaagaatttc aattgttttc ttctccacat ggtaaagatt tgttgtttaa agattctgct 1020

catggttttt tgaaagttcc accaagaatg gatgctaaaa tgtatttggg ttatgaatac 1080

gttactgcta ttagaaattt gagagaaggt acttgtccag aagctccaac tgatgaatgt 1140

aaaccagtta aatggtgtgc tttgtctcat catgaaagat taaaatgtga tgaatggtct 1200

gttaattctg ttggtaaaat tgaatgtgtt tctgctgaaa ctacagaaga ttgtattgct 1260

aaaattatga atggtgaagc tgatgctatg tctttagatg gtggttttgt atatattgct 1320

ggtaaatgtg gtttagttcc agttttggct gaaaattata acaaagcttg tgataattgt 1380

gaagatactc cagaagctgg ttattttgct gttgctgttg ttaaaaaatc tgcttctgat 1440

ttgacttggg ataatctaaa aggaaaaaag agttgccata cagctgttgg 1490

<210> 8
<211> 301
<212> DNA
<213> Artificial

<220>
<223> Synthetic

<400> 8
ccatacagct gttggaagaa cagccggatg gaacattcca atgggattgc tatacaacaa 60

aattaatcat tgtagatttg atgaattttt ttctgaaggt tgtgctccag gttctaaaaa 120

agattcttct ttgtgtaaat tgtgtatggg ttgtggattg aatttgtgtg aaccaaacaa 180

caaggaaggt tattatggtt atactggtgc ttttagatgt ttagttgaaa aaggtgatgt 240

tgcttttgtt aaacatcaaa cagttccaca aaatactggt ggtaaaaatc cagatccatg 300

g                                                                     301

<210>  9
<211>  462
<212>  DNA
<213>  Artificial

<220>
<223>  Synthetic

<400>  9
ccatgggcta aaaatttgaa tgaaaaagat tacgaattac tatgtttaga tggtacaaga      60

aagccagttg aggaatacgc taattgtcat ttagctagag caccaaatca tgctgttgtt     120

actagaaaag ataaagaagc ttgtgttcat aaaattttga gacaacaaca acatttgttt     180

ggttctaatg ttgctgattg ttctggtaat ttttgtttgt ttagatctga aactaaagat      240

ttattgttta gagatgatac tgtttgtttg gctaaattgc atgatagaaa tacttatgaa     300

aaatatttgg gtgaagaata cgttaaagct gttggtaatt tgagaaaatg ttctacttct     360

tctttgttgg aagcttgtac ttttagaagg ccataataag cttaattctt atgatttatg     420

atttttatta ttaaataagt tataaaaaaa ataagtgtat ac                        462

<210>  10
<211>  2359
<212>  DNA
<213>  Artificial

<220>
<223>  Synthetic

<400>  10
cttaagagtc caattagctt catcgccaat aaaaaaacaa gcttaaccta attctaacaa      60

gcaaagatgt tgttgcaagc tttttgtttt ttgttggctg gttttgctgc taaaatttct     120

gctgttccag ataaaacagt tagatggtgt gctgtttctg aacatgaagc tactaaatgt     180

caatctttta gagatcatat gaaatgtgtt attccatctg atggtccatc tgttgcttgt     240

gttaaaaaag cttcttattt ggattgtatt agagctattg ctgctaatga agctgatgct     300

gttactttgg atgctggttt agtttatgat gcttatttgg ctccaaacaa tttgaaacca     360

gttgttgctg aattttatgg ttctaaggaa gatccacaaa ctttttatta tgctgtagcc     420

gttgtaaaaa aggattcagg ttttcaaatg aatcaattga gaggtaaaaa atcttgtcat     480

actggtttag gtagatctgc tggatggaat attccaattg gtttgttgta ttgtgatttg     540

ccagaaccaa gaaaaccatt ggaaaaagct gttgctaatt ttttttctgg ttcttgtgct     600

ccatgtgctg atggtacaga ttttccacaa ttgtgtcaat tatgtccagg ttgtggttgt     660

tctactttga atcaatattt tggttattct ggtgctttta aatgtttgaa agatggtgct     720

ggtgatgttg cttttgttaa acattctact atttttgaaa atttggcaaa caaagctgat      780

agagatcaat atgaattgtt gtgtttggat aatactagaa aaccagttga tgaatataaa     840

gattgtcatt tggctcaagt tccatctcat actgttgttg ctagatctat gggtggtaaa 900

gaagatttga tttgggaatt gttgaatcaa gctcaagaac attttggtaa agataaatct 960

aaagaatttc aattgttttc ttctccacat ggtaaagatt tgttgtttaa agattctgct 1020

catggttttt tgaaagttcc accaagaatg gatgctaaaa tgtatttggg ttatgaatac 1080

gttactgcta ttagaaattt gagagaaggt acttgtccag aagctccaac tgatgaatgt 1140

aaaccagtta aatggtgtgc tttgtctcat catgaaagat taaaatgtga tgaatggtct 1200

gttaattctg ttggtaaaat tgaatgtgtt tctgctgaaa ctacagaaga ttgtattgct 1260

aaaattatga atggtgaagc tgatgctatg tctttagatg gtggttttgt atatattgct 1320

ggtaaatgtg gtttagttcc agttttggct gaaaattata acaaatgtga taattgtgaa 1380

gatactccag aagctggtta ttttgctgtt gctgttgtta aaaaatctgc ttctgatttg 1440

acttgggata atctaaaagg aaaaaagagt tgccatacag ctgttggaag aacagccgga 1500

tggaacattc caatgggatt gctatacaac aaaattaatc attgtagatt tgatgaattt 1560

ttttctgaag gttgtgctcc aggttctaaa aaagattctt ctttgtgtaa attgtgtatg 1620

ggttctggat tgaatttgtg tgaaccaaac aacaaggaag gttattatgg ttatactggt 1680

gcttttagat gtttagttga aaaaggtgat gttgcttttg ttaaacatca aacagttcca 1740

caaaatactg gtggtaaaaa tccagatcca tgggctaaaa atttgaatga aaaagattac 1800

gaattactat gtttagatgg tacaagaaag ccagttgagg aatacgctaa ttgtcattta 1860

gctagagcac caaatcatgc tgttgttact agaaaagata aagaagcttg tgttcataaa 1920

attttgagac aacaacaaca tttgtttggt tctaatgttg ctgattgttc tggtaatttt 1980

tgtttgttta gatctgaaac taaagattta ttgtttagag atgatactgt ttgtttggct 2040

aaattgcatg atagaaatac ttatgaaaaa tatttgggtg aagaatacgt taaagctgtt 2100

ggtaatttga gaaaatgttc tacttcttct ttgttggaag cttgtacttt tagaaggcca 2160

taataagctt aattcttatg atttatgatt tttattatta aataagttat aaaaaaaata 2220

agtgtataca aattttaaag tgactcttag gttttaaaac gaaaattctt attcttgagt 2280

aactctttcc tgtaggtcag gttgctttct caggtatagc atgaggtcgc tcttattgac 2340

cacacctcta ccggcatgc 2359

<210> 11
<211> 691
<212> PRT
<213> Homo sapiens

<400> 11

Gly Arg Arg Arg Ser Val Gln Trp Cys Ala Val Ser Gln Pro Glu Ala
1               5                   10                  15

Thr Lys Cys Phe Gln Trp Gln Arg Asn Met Arg Lys Val Arg Gly Pro
            20                  25                  30

Pro Val Ser Cys Ile Lys Arg Asp Ser Pro Ile Gln Cys Ile Gln Ala
        35                  40                  45

Ile Ala Glu Asn Arg Ala Asp Ala Val Thr Leu Asp Gly Gly Phe Ile
    50                  55                  60

Tyr Glu Ala Gly Leu Ala Pro Tyr Lys Leu Arg Pro Val Ala Ala Glu
65                  70                  75                  80

Val Tyr Gly Thr Glu Arg Gln Pro Arg Thr His Tyr Tyr Ala Val Ala
                85                  90                  95

Val Val Lys Lys Gly Gly Ser Phe Gln Leu Asn Glu Leu Gln Gly Leu
            100                 105                 110

Lys Ser Cys His Thr Gly Leu Arg Arg Thr Ala Gly Trp Asn Val Pro
        115                 120                 125

Ile Gly Thr Leu Arg Pro Phe Leu Asn Trp Ala Gly Pro Pro Glu Pro
    130                 135                 140

Ile Glu Ala Ala Val Ala Arg Phe Phe Ser Ala Ser Cys Val Pro Gly
145                 150                 155                 160

Ala Asp Lys Gly Gln Phe Pro Asn Leu Cys Arg Leu Cys Ala Gly Thr
                165                 170                 175

Gly Glu Asn Lys Cys Ala Phe Ser Ser Gln Glu Pro Tyr Phe Ser Tyr
            180                 185                 190

Ser Gly Ala Phe Lys Cys Leu Arg Asp Gly Ala Gly Asp Val Ala Phe
        195                 200                 205

Ile Arg Glu Ser Thr Val Phe Glu Asp Leu Ser Asp Glu Ala Glu Arg
    210                 215                 220

Asp Glu Tyr Glu Leu Leu Cys Pro Asp Asn Thr Arg Lys Pro Val Asp
225                 230                 235                 240

Lys Phe Lys Asp Cys His Leu Ala Arg Val Pro Ser His Ala Val Val
                245                 250                 255

Ala Arg Ser Val Asn Gly Lys Glu Asp Ala Ile Trp Asn Leu Leu Arg
            260                 265                 270

Gln Ala Gln Glu Lys Phe Gly Lys Asp Lys Ser Pro Lys Phe Gln Leu

275                 280                 285

Phe Gly Ser Pro Ser Gly Gln Lys Asp Leu Leu Phe Lys Asp Ser Ala
    290                 295                 300

Ile Gly Phe Ser Arg Val Pro Pro Arg Ile Asp Ser Gly Leu Tyr Leu
305                 310                 315                 320

Gly Ser Gly Tyr Phe Thr Ala Ile Gln Asn Leu Arg Lys Ser Glu Glu
                325                 330                 335

Glu Val Ala Ala Arg Arg Ala Arg Val Val Trp Cys Ala Val Gly Glu
            340                 345                 350

Gln Glu Leu Arg Lys Cys Asn Gln Trp Ser Gly Leu Ser Glu Gly Ser
        355                 360                 365

Val Thr Cys Ser Ser Ala Ser Thr Thr Glu Asp Cys Ile Ala Leu Val
    370                 375                 380

Leu Lys Gly Glu Ala Asp Ala Met Ser Leu Asp Gly Gly Tyr Val Tyr
385                 390                 395                 400

Thr Ala Gly Lys Cys Gly Leu Val Pro Val Leu Ala Glu Asn Tyr Lys
                405                 410                 415

Ser Gln Gln Ser Ser Asp Pro Asp Pro Asn Cys Val Asp Arg Pro Val
            420                 425                 430

Glu Gly Tyr Leu Ala Val Ala Val Val Arg Arg Ser Asp Thr Ser Leu
        435                 440                 445

Thr Trp Asn Ser Val Lys Gly Lys Lys Ser Cys His Thr Ala Val Asp
    450                 455                 460

Arg Thr Ala Gly Trp Asn Ile Pro Met Gly Leu Leu Phe Asn Gln Ala
465                 470                 475                 480

Gly Ser Cys Lys Phe Asp Glu Tyr Phe Ser Gln Ser Cys Ala Pro Gly
                485                 490                 495

Ser Asp Pro Arg Ser Asn Leu Cys Ala Leu Cys Ile Gly Asp Glu Gln
            500                 505                 510

Gly Glu Asn Lys Cys Val Pro Asn Ser Asn Glu Arg Tyr Tyr Gly Tyr
        515                 520                 525

Thr Gly Ala Phe Arg Cys Leu Ala Glu Asn Ala Gly Asp Val Ala Phe
    530                 535                 540

Val Lys Asp Val Thr Val Leu Gln Asn Thr Asp Gly Asn Asn Asn Glu
545                 550                 555                 560

Ala Trp Ala Lys Asp Leu Lys Leu Ala Asp Phe Ala Leu Leu Cys Leu
                565                 570                 575

Asp Gly Lys Arg Lys Pro Val Thr Glu Ala Arg Ser Cys His Leu Ala
            580                 585                 590

Met Ala Pro Asn His Ala Val Val Ser Arg Met Asp Lys Val Glu Arg
        595                 600                 605

Leu Lys Gln Val Leu Leu His Gln Gln Ala Lys Phe Gly Arg Asn Gly
    610                 615                 620

Ala Asp Cys Pro Asp Lys Phe Cys Leu Phe Gln Ser Glu Thr Lys Asn
625                 630                 635                 640

Leu Leu Phe Asn Asp Asn Thr Glu Cys Leu Ala Arg Leu His Gly Lys
                645                 650                 655

Thr Thr Tyr Glu Lys Tyr Leu Gly Pro Gln Tyr Val Ala Gly Ile Thr
            660                 665                 670

Asn Leu Lys Lys Cys Ser Thr Ser Pro Leu Leu Glu Ala Cys Glu Phe
        675                 680                 685

Leu Arg Lys
    690

<210>  12
<211>  2395
<212>  DNA
<213>  Artificial

<220>
<223>  Synthetic

<400>  12
cttaagagtc caattagctt catcgccaat aaaaaaacaa gcttaaccta attctaacaa      60

gcaaagatgc ttttgcaagc cttccttttc cttttggctg gttttgcagc caagatctct     120

gctggccgta ggaggagtgt tcagtggtgc gccgtatccc aacccgaggc cacaaaatgc     180

ttccaatggc aaaggaatat gagaaaagtg cgtggccctc ctgtcagctg cataaagaga     240

gactccccca tccagtgtat ccaggccatt gcggaaaaca gggccgatgc tgtgaccctt     300

gatggtggtt tcatatacga ggcaggcctg gccccctaca aactgcgacc tgtagcggcg     360

gaagtctacg ggaccgaaag acagccacga actcactatt atgccgtggc tgtggtgaag     420

aagggcggca gctttcagct gaacgaactg caaggtctga agtcctgcca cacaggcctt 480

cgcaggaccg ctggatggaa tgtccctata gggacacttc gtccattctt gaattgggct 540

ggtccacctg agcccattga ggcagctgtg gccaggttct tctcagccag ctgtgttccc 600

ggtgcagata aaggacagtt ccccaacctg tgtcgcctgt gtgcggggac aggggaaaac 660

aaatgtgcct tctcctccca ggaaccgtac ttcagctact ctggtgcctt caagtgtctg 720

agagacgggg ctggagacgt ggcttttatc agagagagca cagtgtttga ggacctgtca 780

gacgaggctg aaagggacga gtatgagtta ctctgcccag acaacactcg gaagccagtg 840

gacaagttca aagactgcca tctggcccgg gtcccttctc atgccgttgt ggcacgaagt 900

gtgaatggca aggaggatgc catctggaat cttctccgcc aggcacagga aaagtttgga 960

aaggacaagt caccgaaatt ccagctcttt ggctccccta gtgggcagaa agatctgctg 1020

ttcaaggact ctgccattgg gttttcgagg gtgcccccga ggatagattc tgggctgtac 1080

cttggctccg gctacttcac tgccatccag aacttgagga aaagtgagga ggaagtggct 1140

gcccggcgtg cgcgggtcgt gtggtgtgcg gtgggcgagc aggagctgcg caagtgtaac 1200

cagtggagtg gcttgagcga aggcagcgtg acctgctcct cggcctccac cacagaggac 1260

tgcatcgccc tggtgctgaa aggagaagct gatgccatga gtttggatgg aggatatgtg 1320

tacactgcag gcaaatgtgg tttggtgcct gtcctggcag agaactacaa atcccaacaa 1380

agcagtgacc ctgatcctaa ctgtgtggat agacctgtgg aaggatatct tgctgtggcg 1440

gtggttagga gatcagacac tagccttacc tggaactctg tgaaaggcaa gaagtcctgc 1500

cacaccgccg tggacaggac tgcaggctgg aatatcccca tgggcctgct cttcaaccag 1560

gctggctcct gcaaatttga tgaatatttc agtcaaagct gtgcccctgg gtctgacccg 1620

agatctaatc tctgtgctct gtgtattggc gacgagcagg gtgagaataa gtgcgtgccc 1680

aacagcaacg agagatacta cggctacact ggggctttcc ggtgcctggc tgagaatgct 1740

ggagacgttg catttgtgaa agatgtcact gtcttgcaga acactgatgg aaataacaat 1800

gaggcatggg ctaaggattt gaagctggca gactttgcgc tgctgtgcct cgatggcaaa 1860

cggaagcctg tgactgaggc tagaagctgc catcttgcca tggccccgaa tcatgccgtg 1920

gtgtctcgga tggataaggt ggaacgcctg aaacaggtgt tgctccacca acaggctaaa 1980

tttgggagaa atggagctga ctgcccggac aagttttgct tattccagtc tgaaaccaaa 2040

aaccttctgt tcaatgacaa cactgagtgt ctggccagac tccatggcaa aacaacatat 2100

gaaaaatatt tgggaccaca gtatgtcgca ggcattacta atctgaaaaa gtgctcaacc 2160

tcccccctcc tggaagcctg tgaattcctc aggaagtaat aagcttaatt cttatgattt 2220

atgattttta ttattaaata agttataaaa aaaataagtg tatacaaatt ttaaagtgac 2280

tcttaggttt taaaacgaaa attcttattc ttgagtaact ctttcctgta ggtcaggttg 2340

ctttctcagg tatagcatga ggtcgctctt attgaccaca cctctaccgg catgc 2395

<210> 13
<211> 2394
<212> DNA
<213> Artificial

<220>
<223> Synthetic

<400> 13
ttaagagtcc aattagcttc atcgccaata aaaaaacaag cttaacctaa ttctaacaag 60

caaagatgtt gttgcaagct tttttgtttt tgttggctgg ttttgctgct aaaatttctg 120

ctggtagaag aagatctgtt caatggtgtg ctgtttctca acctgaagct actaaatgtt 180

ttcaatggca aagaaacatg agaaaagtta gaggtccacc agtttcttgt attaaaagag 240

attctccaat tcaatgtatt caagctattg ctgaaaatag agctgatgct gttactttgg 300

atggtggttt catttatgaa gctggtttgg ctccatacaa attaagacca gttgctgctg 360

aagtttatgg tactgaaaga caaccaagaa ctcattatta tgctgttgct gttgttaaaa 420

aaggtggttc tttccaattg aatgaattgc aaggtttgaa atcttgtcat actggtttga 480

gaagaactgc tggttggaat gttccaattg gtactttaag accatttttg aattgggctg 540

gtccaccaga accaattgaa gctgctgttg ctagattttt ttctgcttct tgtgttccag 600

gtgctgataa aggtcaattt ccaaacttgt gtagattgtg tgctggtact ggtgaaaaca 660

aatgtgcttt ctcttctcaa gaaccatatt tttcttactc tggtgctttt aaatgtttga 720

gagatggtgc tggtgatgtt gcttttatta gagaatctac tgttttcgaa gatttgtctg 780

atgaagctga aagagatgaa tacgaattgt tgtgtccaga taatactaga aaaccagttg 840

ataagttcaa agattgtcat ttggctagag ttccatctca tgctgttgtt gctagatctg 900

ttaatggtaa agaagatgct atttggaatt tgttgagaca agctcaagaa aaatttggta 960

aggataagtc tccaaagttt caattgtttg gttctccatc tggtcaaaaa gatttgttgt 1020

tcaaggattc tgctattggt ttttctagag ttccaccaag aattgattct ggtttgtatt 1080

tgggttctgg ttattttact gctattcaaa acttgagaaa gtctgaagaa gaagttgctg 1140

ctagaagagc tagagttgtt tggtgtgcag ttggtgaaca agaattgaga aagtgtaatc 1200

aatggtctgg tttgtctgaa ggttctgtta cttgttcttc tgcttctact actgaagatt 1260

gtattgcttt ggttttgaaa ggtgaagctg atgctatgtc attagatggt ggttacgttt 1320

acactgctgg taaatgtggt ttggttccag ttttggctga aaattacaag tctcaacaat 1380

cttctgatcc agatccaaat tgtgttgata gaccagttga aggttatttg gctgttgcag 1440

ttgttagaag atctgatact tctttgactt ggaactctgt taaaggtaaa aagtcttgtc 1500

atacagctgt tgatagaaca gcaggttgga atattcctat gggtttgttg tttaatcaag 1560

ctggttcttg taaatttgat gaatacttct ctcaatcttg tgctccaggt tcagatccaa 1620

gatctaattt gtgtgctttg tgtattggtg atgaacaagg tgaaaacaag tgtgttccaa 1680

attctaatga aagatactat ggttatacag gtgcatttag atgtttagct gaaaatgcag 1740

gtgatgttgc atttgttaag gatgttacag ttttgcaaaa tactgatggt aacaacaatg 1800

aagcttgggc taaagatttg aaattggctg attttgcttt gttgtgtttg gatggtaaaa 1860

gaaaacctgt tactgaagct agatcttgtc atttagctat ggctccaaat catgcagttg 1920

tttctagaat ggataaggtt gaaagattga agcaagtttt gttgcatcaa caagctaaat 1980

ttggtagaaa tggtgctgat tgtccagata agttttgttt gttccaatct gaaactaaga 2040

acttgttgtt caacgataat actgaatgtt tggctagatt gcatggtaaa actacttacg 2100

aaaaatactt gggtccacaa tacgttgctg gtattactaa tttgaagaag tgttctactt 2160

ctccattgtt ggaagcttgt gaatttttga gaaagtaata agcttaattc ttatgattta 2220

tgatttttat tattaaataa gttataaaaa aaataagtgt atacaaattt taaagtgact 2280

cttaggtttt aaaacgaaaa ttcttattct tgagtaactc tttcctgtag gtcaggttgc 2340

tttctcaggt atagcatgag gtcgctctta ttgaccacac ctctaccggc atgc 2394

<210> 14
<211> 2395
<212> DNA
<213> Artificial

<220>
<223> Synthetic

<400> 14
cttaagagtc caattagctt catcgccaat aaaaaaacaa gcttaaccta attctaacaa 60

gcaaagatgt tgttgcaagc tttttgtttt ttgttggctg gttttgctgc taaaatttct 120

gctggtagaa gaagatctgt tcaatggtgt gctgtttctc aacctgaagc tactaaatgt 180

tttcaatggc aaagaaacat gagaaaagtt agaggtccac cagtttcttg tattaaaaga 240

gattctccaa ttcaatgtat tcaagctatt gctgaaaata gagctgatgc tgttactttg 300

gatggtggtt tcatttatga agctggtttg gctccataca aattaagacc agttgctgct 360

gaagtttatg gtactgaaag acaaccaaga actcattatt atgctgttgc tgttgttaaa 420

aaaggtggtt ctttccaatt gaatgaattg caaggtttga aatcttgtca tactggtttg 480

agaagaactg ctggttggaa tgttccaatt ggtactttaa gaccattttt gaattgggct 540

ggtccaccag aaccaattga agctgctgtt gctagatttt tttctgcttc ttgtgttcca 600

ggtgctgata aaggtcaatt tccaaacttg tgtagattgt gtgctggtac tggtgaaaac 660

aaatgtgctt tctcttctca agaaccatat ttttcttact ctggtgcttt taaatgtttg 720

agagatggtg ctggtgatgt tgcttttatt agagaatcta ctgttttcga agatttgtct 780

gatgaagctg aaagagatga atacgaattg ttgtgtccag ataatactag aaaaccagtt 840

gataagttca aagattgtca tttggctaga gttccatctc atgctgttgt tgctagatct 900

gttaatggta aagaagatgc tatttggaat ttgttgagac aagctcaaga aaaatttggt 960

aaggataagt ctccaaagtt tcaattgttt ggttctccat ctggtcaaaa agatttgttg 1020

ttcaaggatt ctgctattgg tttttctaga gttccaccaa gaattgattc tggtttgtat 1080

ttgggttctg gttattttac tgctattcaa aacttgagaa agtctgaaga agaagttgct 1140

gctagaagag ctagagttgt ttggtgtgca gttggtgaac aagaattgag aaagtgtaat 1200

caatggtctg gtttgtctga aggttctgtt acttgttctt ctgcttctac tactgaagat 1260

tgtattgctt tggttttgaa aggtgaagct gatgctatgt cattagatgg tggttacgtt 1320

tacactgctg gtaaatgtgg tttggttcca gttttggctg aaaattacaa gtctcaacaa 1380

tcttgtgatc cagatccaaa ttgtgttgat agaccagttg aaggttattt ggctgttgca 1440

gttgttagaa gatctgatac ttctttgact tggaactctg ttaaaggtaa aaagtcttgt 1500

catacagctg ttgatagaac agcaggttgg aatattccta tgggtttgtt gtttaatcaa 1560

gctggttctt gtaaatttga tgaatacttc tctcaatctt gtgctccagg ttcagatcca 1620

agatctaatt tgtgtgcttt gtgtattggt gatgaacaag gtgaaaacaa gtgtgttcca 1680

aattctaatg aaagatacta tggttataca ggtgcattta gatgtttagc tgaaaatgca 1740

ggtgatgttg catttgttaa ggatgttaca gttttgcaaa atactgatgg taacaacaat 1800

gaagcttggg ctaaagattt gaaattggct gattttgctt tgttgtgttt ggatggtaaa 1860

agaaaacctg ttactgaagc tagatcttgt catttagcta tggctccaaa tcatgcagtt 1920

gtttctagaa tggataaggt tgaaagattg aagcaagttt tgttgcatca acaagctaaa 1980

tttggtagaa atggtgctga ttgtccagat aagttttgtt tgttccaatc tgaaactaag 2040

aacttgttgt tcaacgataa tactgaatgt ttggctagat tgcatggtaa aactacttac 2100

gaaaaatact tgggtccaca atacgttgct ggtattacta atttgaagaa gtgttctact 2160

tctccattgt tggaagcttg tgaatttttg agaaagtaat aagcttaatt cttatgattt 2220

atgattttta ttattaaata agttataaaa aaaataagtg tatacaaatt ttaaagtgac 2280

tcttaggttt taaaacgaaa attcttattc ttgagtaact ctttcctgta ggtcaggttg 2340

ctttctcagg tatagcatga ggtcgctctt attgaccaca cctctaccgg catgc 2395

<210> 15
<211> 738
<212> PRT
<213> Homo sapiens

<400> 15

Met Arg Gly Pro Ser Gly Ala Leu Trp Leu Leu Leu Ala Leu Arg Thr
1               5                   10                  15

Val Leu Gly Gly Met Glu Val Arg Trp Cys Ala Thr Ser Asp Pro Glu
            20                  25                  30

Gln His Lys Cys Gly Asn Met Ser Glu Ala Phe Arg Glu Ala Gly Ile
        35                  40                  45

Gln Pro Ser Leu Leu Cys Val Arg Gly Thr Ser Ala Asp His Cys Val
    50                  55                  60

Gln Leu Ile Ala Ala Gln Glu Ala Asp Ala Ile Thr Leu Asp Gly Gly
65                  70                  75                  80

Ala Ile Tyr Glu Ala Gly Lys Glu His Gly Leu Lys Pro Val Val Gly
                85                  90                  95

Glu Val Tyr Asp Gln Glu Val Gly Thr Ser Tyr Tyr Ala Val Ala Val
            100                 105                 110

Val Arg Arg Ser Ser His Val Thr Ile Asp Thr Leu Lys Gly Val Lys
        115                 120                 125

Ser Cys His Thr Gly Ile Asn Arg Thr Val Gly Trp Asn Val Pro Val
    130                 135                 140

Gly Tyr Leu Val Glu Ser Gly Arg Leu Ser Val Met Gly Cys Asp Val
145                 150                 155                 160

Leu Lys Ala Val Ser Asp Tyr Phe Gly Gly Ser Cys Val Pro Gly Ala
                165                 170                 175

Gly Glu Thr Ser Tyr Ser Glu Ser Leu Cys Arg Leu Cys Arg Gly Asp
            180                 185                 190

Ser Ser Gly Glu Gly Val Cys Asp Lys Ser Pro Leu Glu Arg Tyr Tyr
        195                 200                 205

Asp Tyr Ser Gly Ala Phe Arg Cys Leu Ala Glu Gly Ala Gly Asp Val
    210                 215                 220

Ala Phe Val Lys His Ser Thr Val Leu Glu Asn Thr Asp Gly Lys Thr
225                 230                 235                 240

Leu Pro Ser Trp Gly Gln Ala Leu Leu Ser Gln Asp Phe Glu Leu Leu
                245                 250                 255

Cys Arg Asp Gly Ser Arg Ala Asp Val Thr Glu Trp Arg Gln Cys His
            260                 265                 270

Leu Ala Arg Val Pro Ala His Ala Val Val Val Arg Ala Asp Thr Asp
        275                 280                 285

Gly Gly Leu Ile Phe Arg Leu Leu Asn Glu Gly Gln Arg Leu Phe Ser
    290                 295                 300

His Glu Gly Ser Ser Phe Gln Met Phe Ser Ser Glu Ala Tyr Gly Gln
305                 310                 315                 320

Lys Asp Leu Leu Phe Lys Asp Ser Thr Ser Glu Leu Val Pro Ile Ala
                325                 330                 335

Thr Gln Thr Tyr Glu Ala Trp Leu Gly His Glu Tyr Leu His Ala Met
            340                 345                 350

Lys Gly Leu Leu Cys Asp Pro Asn Arg Leu Pro Pro Tyr Leu Arg Trp
        355                 360                 365

Cys Val Leu Ser Thr Pro Glu Ile Gln Lys Cys Gly Asp Met Ala Val
    370                 375                 380

Ala Phe Arg Arg Gln Arg Leu Lys Pro Glu Ile Gln Cys Val Ser Ala
385                 390                 395                 400

Lys Ser Pro Gln His Cys Met Glu Arg Ile Gln Ala Glu Gln Val Asp
                405                 410                 415

Ala Val Thr Leu Ser Gly Glu Asp Ile Tyr Thr Ala Gly Lys Thr Tyr
            420                 425                 430

Gly Leu Val Pro Ala Ala Gly Glu His Tyr Ala Pro Glu Asp Ser Ser
        435                 440                 445

Asn Ser Tyr Tyr Val Val Ala Val Val Arg Arg Asp Ser Ser His Ala
    450                 455                 460

Phe Thr Leu Asp Glu Leu Arg Gly Lys Arg Ser Cys His Ala Gly Phe
465                 470                 475                 480

Gly Ser Pro Ala Gly Trp Asp Val Pro Val Gly Ala Leu Ile Gln Arg
                485                 490                 495

Gly Phe Ile Arg Pro Lys Asp Cys Asp Val Leu Thr Ala Val Ser Glu
            500                 505                 510

Phe Phe Asn Ala Ser Cys Val Pro Val Asn Asn Pro Lys Asn Tyr Pro
        515                 520                 525

Ser Ser Leu Cys Ala Leu Cys Val Gly Asp Glu Gln Gly Arg Asn Lys
    530                 535                 540

Cys Val Gly Asn Ser Gln Glu Arg Tyr Tyr Gly Tyr Arg Gly Ala Phe
545                 550                 555                 560

Arg Cys Leu Val Glu Asn Ala Gly Asp Val Ala Phe Val Arg His Thr
                565                 570                 575

Thr Val Phe Asp Asn Thr Asn Gly His Asn Ser Glu Pro Trp Ala Ala
            580                 585                 590

Glu Leu Arg Ser Glu Asp Tyr Glu Leu Leu Cys Pro Asn Gly Ala Arg
        595                 600                 605

Ala Glu Val Ser Gln Phe Ala Ala Cys Asn Leu Ala Gln Ile Pro Pro
    610                 615                 620

His Ala Val Met Val Arg Pro Asp Thr Asn Ile Phe Thr Val Tyr Gly
625                 630                 635                 640

Leu Leu Asp Lys Ala Gln Asp Leu Phe Gly Asp Asp His Asn Lys Asn
                645                 650                 655

Gly Phe Lys Met Phe Asp Ser Ser Asn Tyr His Gly Gln Asp Leu Leu
            660                 665                 670

Phe Lys Asp Ala Thr Val Arg Ala Val Pro Val Gly Glu Lys Thr Thr
        675                 680                 685

Tyr Arg Gly Trp Leu Gly Leu Asp Tyr Val Ala Ala Leu Glu Gly Met
    690                 695                 700

Ser Ser Gln Gln Cys Ser Gly Ala Ala Ala Pro Ala Pro Gly Ala Pro
705                 710                 715                 720

Leu Leu Pro Leu Leu Leu Pro Ala Leu Ala Ala Arg Leu Leu Pro Pro
                725                 730                 735

Ala Leu